# EUROPEAN PATENT APPLICATION

(11) **EP 1 767 628 A1**
(43) Date of publication of application: **28.03.2007**
(21) Application number: 05765118.4
(22) Date of filing: 24.06.2005
(51) Int. Cl.: C12N 15/09, C12P 21/02, C12N 9/48, C12R 1/19

(54) **PROCESS FOR PRODUCING DIPEPTIDES OR DIPEPTIDE DERIVATIVES**

(30) Priority: 25.06.2004 JP 2004189008
(71) Applicant: KYOWA HAKKO KOGYO CO., LTD., Chiyoda-ku, Tokyo 100-8185 (JP)
(72) Inventor: HASHIMOTO, Shin-ichi c/o Technical Research Lab, YAMAGUCHI 747-8522 (JP); IKEDA, Hajime C/O Technical Research Lab., Yamaguchi 747-8522 (JP); TABATA, Kazuhiko c/o Technical Research Lab., Yamaguchi 747-8522 (JP)
(74) Representative: Vossius & Partner
(86) International application number: PCT/JP2005/011638
(87) International publication number: WO 2006/001381

(57) **Abstract**

The present invention provides a process for producing a dipeptide or a dipeptide derivative by using a protein having the activity to form the dipeptide or dipeptide derivative from one or more kinds of amino acids or amino acid derivatives, or a culture of cells having the ability to produce the protein or a treated matter of the culture as a enzyme source, which comprise; allowing the enzyme sorce, one or more kinds of amino acids or amino acid derivatives and ATP to be present in an aqueous medium;
allowing the dipeptide or dipeptide derivative to form and accumulate in the medium; and
recovering the dipeptide or dipeptide derivative from the medium.

## Description

### Technical Field

The present invention relates to a process for producing a dipeptide or a dipeptide derivative, by using a protein having the activity to form the dipeptide or the dipeptide derivative, a culture of cells comprising a DNA encoding the protein or a treated matter of the culture, from one or more kinds of amino acids or amino acid derivatives.

### Background of the Invention

Chemical synthesis methods (liquid phase method and solid phase method), enzymatic synthesis methods and biological synthesis methods utilizing recombinant DNA techniques are known as the methods for large-scale peptide synthesis. Currently, the enzymatic synthesis methods and biological synthesis methods are employed for the synthesis of long-chain peptides longer than 50 residues, and the chemical synthesis methods and enzymatic synthesis methods are mainly employed for the synthesis of dipeptides.

In the synthesis of dipeptides by the chemical synthesis methods, operations such as introduction and removal of protective groups for functional groups are necessary, and racemates are also formed. The chemical synthesis methods are thus considered to be disadvantageous in respect of cost and efficiency. They are unfavorable also from the viewpoint of environmental hygiene because of the use of large amounts of organic solvents and the like.

As to the synthesis of dipeptides by the enzymatic methods, the following methods are known: a method utilizing reverse reaction of protease (non-patent publication No. 1); methods utilizing thermostable aminoacyl t-RNA synthetase (Patent publication No.1 to 4); and methods utilizing non-ribosomal peptide synthetase (hereinafter referred to as NRPS) (non-patent publication No.2, 3 and patent publication 5, 6).

However, the method utilizing reverse reaction of protease requires introduction and removal of protective groups for functional groups of amino acids used as substrates, which causes difficulties in raising the efficiency of peptide-forming reaction and in preventing peptidolytic reaction. The methods utilizing thermostable aminoacyl t-RNA synthetase have the defects that the expression of the enzyme and the prevention of side reactions forming by-products other than the desired products are difficult. The methods utilizing NRPS are inefficient in that the expression of the enzyme by recombinant DNA techniques is difficult because of its large enzyme molecule size and that the supply of coenzyme 4'-phosphopantetheine is necessary.

On the other hand, there exist a group of peptide synthetases that have enzyme molecular weight lower than that of NRPS and do not require coenzyme 4'-phosphopantetheine; for example, γ-glutamylcysteine synthetase, glutathione synthetase, D-alanyl-D-alanine (D-Ala-D-Ala) ligase, and poly-γ-glutamate synthetase. Most of these enzymes utilize D-amino acids as substrates or catalyze peptide bond formation at the γ-carboxyl group. Because of such properties, they can not be used for the synthesis of dipeptides by peptide bond formation at the α-carboxyl group of L-amino acid.

The only known example of an enzyme capable of dipeptide synthesis by the activity to form a peptide bond at the α-carboxyl group of L-amino acid is bacilysin (dipeptide antibiotic derived from a microorganism belonging to the genus Bacillus) synthetase. Bacilysin synthetase is known to have the activity to synthesize bacilysin [L-alanyl-L-anticapsin (L-Ala-L-anticapsin)] and L-alanyl-L-alanine (L-Ala-L-Ala), but there is no information about its activity to synthesize other peptides (non-patent publication No.4 and 5).

As for the bacilysin biosynthetase genes in Bacillus subtilis 168 whose whole genome information has been clarified (non-patent publication No.6), it is known that the productivity of bacilysin is increased by amplification of bacilysin operons containing ORFs ywfA-F (patent publication No.7). However, it is not known whether an ORF encoding a protein having the activity to ligate two or more amino acids by peptide bond is contained in these ORFs, and if contained, which ORF encodes the protein.

Certain microorganisms are known to form a compound having the cyclic dipeptide (diketopiperazine) structure wherein two amino acids are cyclically bound (non-patent publication No. 7, 8 and 9). With regard to the biosynthesis of diketopiperazine, it is reported that the cyclo-(L-4-nitrotryptophyl-L-phenylalanine) structure is synthesized by NRPS in the Thaxtomin biosynthesis process of Streptomyces acidiscabies (non-patent publication No. 10) and that cyclo(phenylalanyl-proline) is formed from phenylalanine and proline by the action of a part of the modules of NRPS of bacteria belonging to the genus Bacillus (non-patent publication No. 11).

It is also reported that a protein bearing no similarity to NRPS (albC gene product) is responsible for the synthesis of the cyclo(L-phenylalanyl-L-leucine) structure in Streptomyces noursei ATCC 11455 known as a strain producing the antibiotic albonoursin and that albonoursin was detected when cyclo dipeptide oxidase was made to act on the culture broth of Escherichia coli and Streptomyces lividans into which the albC gene was introduced (non-patent publication No. 12). However, there is no report that the albC gene product forms a linear dipeptide.

As described above, there is no information that enzymes included in the bacylicin synthetase group have the activity to form various dipeptide derivatives from amino acids or amino acids derivatives.
Patent publication No. 1:
   Japanese Published Unexamined Patent Application No. 146539/83
Patent publication No. 2:
   Japanese Published Unexamined Patent Application No. 209991/83
Patent publication No. 3:
   Japanese Published Unexamined Patent Application No. 209992/83
Patent publication No. 4:
   Japanese Published Unexamined Patent Application No. 106298/84
Patent publication No. 5:
   U.S. Patent No. 5,795,738 Patent publication No. 6:
   U.S. Patent No. 5,652,116 Patent publication No. 7:
   WO00/03009 pamphlet
Non-patent publication No. 1:
   J. Biol. Chem., 119, 707-720(1937) Non-patent publication No. 2:
   Chem. Biol., 7, 373-384(2000) Non-patent publication No. 3:
   FEBS Lett., 498, 42-45(2001)
Non-patent publication No. 4:
   J. Ind. Microbiol., 2, 201-208(1987) Non-patent publication No. 5:
   Enzyme. Microbial. Technol., 29, 400-406(2001) Non-patent publication No. 6:
   Nature, 390, 249-256 (1997)
Non-patent publication No. 7:
   J. Nat. Prod., 59, 293-296(1996)
Non-patent publication No. 8:
   Tetrahedron, 28, 2999(1972)
Non-patent publication No. 9:
   J. Appl. Microbiol., 86, 29-53(1999)
Non-patent publication No. 10:
   Mol. Microbiol., 38, 794-804(2000)
Non-patent publication No. 11:
   J. Biol. Chem., 273, 22773-22781(1998)
Non-patent publication No. 12:
   Chemistry & Biol., 9, 1355-1364(2002)

### Disclosure of the Invention

### Problems to be Solved by the Invention

An object of the present invention is to provide a process for producing dipeptide derivatives from amino acids or amino acid derivatives.

### Means for Solving the Problems

The present invention relates to the following (1) to (26).
(1) A process for producing a dipeptide or a dipeptide derivative(PI) (hereinafter referred to as dipeptide or dipeptide derivative PI), which comprises:
   allowing the protein shown in any of the following [1] to [7], one or more kinds of amino acids or amino acid derivatives and ATP to be present in an aqueous medium;
   allowing the dipeptide or dipeptide derivative PI to form and accumulate in the aqueous medium; and
   recovering the dipeptide or dipeptide derivative PI from the aqueous medium;
      [1] a protein having the amino acid sequence shown in any of SEQ ID NOS: 1 to 8;
      [2] a protein consisting of an amino acid sequence wherein one or more amino acid residues are deleted, substituted or added in the amino acid sequence shown in any of SEQ ID NOS: 1 to 8 and having the activity to form the dipeptide or dipeptide derivative PI from one or more kinds of amino acids or amino acid derivatives;
      [3] a protein consisting of an amino acid sequence which has 65% or more homology to the amino acid sequence shown in any of SEQ ID NOS: 1 to 8 and having the activity to form the dipeptide or dipeptide derivative PI from one or more kinds of amino acids or amino acid derivatives;
      [4] a protein consisting of an amino acid sequence which has 80% or more homology to the amino acid sequence shown in SEQ ID NO: 17 and having the activity to form the dipeptide or dipeptide derivative PI from one or more kinds of amino acids or amino acid derivatives;
      [5] a protein having the amino acid sequence shown in SEQ ID NO: 37 or 38;
      [6] a protein consisting of an amino acid sequence wherein one or more amino acid residues are deleted, substituted or added in the amino acid sequence shown in SEQ ID NO: 37 or 38 and having the activity to form the dipeptide or dipeptide derivative PI from one or more kinds of amino acids or amino acid derivatives;
      [7] a protein consisting of an amino acid sequence which has 65% or more homology to the amino acid sequence shown in SEQ ID NO: 37 or 38 and having the activity to form the dipeptide or dipeptide derivative PI from one or more kinds of amino acids or amino acid derivatives;
   provided that a compound wherein the amino acids selected from the following Amino acid group A are the same or different, and are bound by peptide bond is excluded from the dipeptide or dipeptide derivative PI:
      Amino acid group A; L-alanine, L-glutamine, L-glutamic acid, L-valine, L-leucine, L-isoleucine, L-proline, L-phenylalanine, L-tryptophan, L-methionine,
      L-serine, L-threonine, L-cysteine, L-asparagine, L-tyrosine, L-lysine, L-arginine, L-histidine, L-aspartic acid, L-α-aminobutanoic acid, L-azaserine,
      L-theanine, L-4-hydroxyproline, L-3-hydroxyproline, L-ornithine, L-citrulline, L-6-diazo-5-oxo-norleucine, glycine and β-alanine.
(2) A process for producing a dipeptide or a dipeptide derivative (hereinafter referred to as dipeptide or dipeptide derivative PII), which comprises:
   allowing the protein shown in any of following [1] to [7], one or more kinds of amino acids or amino acid derivatives and ATP to be present in an aqueous medium;
   allowing dipeptide or dipeptide derivative PI to form and accumulate in the aqueous medium;
   subjecting the dipeptide or dipeptide derivative PI, as such or after recovery, to modification to form the dipeptide or dipeptide derivative PII; and
   recovering the dipeptide or dipeptide derivative PII;
      [1] a protein having the amino acid sequence shown in any of SEQ ID NOS: 1 to 8;
      [2] a protein consisting of an amino acid sequence wherein one or more amino acid residues are deleted, substituted or added in the amino acid sequence shown in any of SEQ ID NOS: 1 to 8 and having the activity to form the dipeptide or dipeptide derivative PI from one or more kinds of amino acids or amino acid derivatives;
      [3] a protein consisting of an amino acid sequence which has 65% or more homology to the amino acid sequence shown in any of SEQ ID NOS: 1 to 8 and having the activity to form the dipeptide or dipeptide derivative PI from one or more kinds of amino acids or amino acid derivatives;
      [4] a protein consisting of an amino acid sequence which has 80% or more homology to the amino acid sequence shown in SEQ ID NO: 17 and having the activity to form the dipeptide or dipeptide derivative PI from one or more kinds of amino acids or amino acid derivatives;
      [5] a protein having the amino acid sequence shown in SEQ ID NO: 37 or 38;
      [6] a protein consisting of an amino acid sequence wherein one or more amino acid residues are deleted, substituted or added in the amino acid sequence shown in SEQ ID NO: 37 or 38 and having the activity to form the dipeptide or dipeptide derivative PI from one or more kinds of amino acids or amino acid derivatives;
      [7] a protein consisting of an amino acid sequence which has 65% or more homology to the amino acid sequence shown in SEQ ID NO: 37 or 38 and having the activity to form the dipeptide or dipeptide derivative PI from one or more kinds of amino acids or amino acid derivatives;
   provided that a compound wherein the amino acids selected from the Amino acid group A described in the above are the same or different, and are bound by peptide bond is excluded from the dipeptide or dipeptide derivative PII.
(3) A process for producing a dipeptide or a dipeptide derivative PI, which comprises:
   allowing an enzyme source and one or more kinds of amino acids or amino acid derivatives to be present in an aqueous medium, said enzyme source being a culture or a treated matter of the culture of cells having the DNA selected from the following [1] to [5]:
      [1] DNA having the nucleotide sequence shown in any of SEQ ID NOS: 9 to 16 and 36;
      [2] DNA which hybridizes with DNA having a nucleotide sequence complementary to the nucleotide sequence shown in any of SEQ ID NOS: 9 to 16 and 36 under stringent conditions and which encodes a protein having the activity to form the dipeptide or dipeptide derivative PI from one or more kinds of amino acids or amino acid derivatives;
      [3] DNA which hybridizes with DNA having a nucleotide sequence complementary to the nucleotide sequence shown in SEQ ID NO: 18 under stringent conditions and which encodes a protein having the activity to form the dipeptide or dipeptide derivative PI from one or more kinds of amino acids or amino acid derivatives;
      [4] DNA having the nucleotide sequence shown in SEQ ID NO: 39 or 40;
      [5] DNA which hybridizes with DNA having a nucleotide sequence complementary to the nucleotide sequence shown in SEQ ID NO: 39 or 40 under stringent conditions and which encodes a protein having the activity to form the dipeptide or dipeptide derivative PI from one or more kinds of amino acids or amino acid derivatives;
   provided that a compound wherein the amino acids selected from the Amino acid group A described in the above are the same or different, and are bound by peptide bond is excluded from the dipeptide or dipeptide derivative PI.
(4) A process for producing a dipeptide or a dipeptide derivative PII, which comprises:
   allowing an enzyme source and one or more kinds of amino acids or amino acid derivatives to be present in an aqueous medium, said enzyme source being a culture or a treated matter of the culture of cells having the DNA selected from the following [1] to [5];
   allowing dipeptide or dipeptide derivative PI to form and accumulate in the aqueous medium;
   subjecting the dipeptide or dipeptide derivative PI, as such or after recovery, to modification to form the dipeptide or dipeptide derivative PII; and
   recovering the dipeptide or dipeptide derivative PII;
      [1] DNA having the nucleotide sequence shown in any of SEQ ID NOS: 9 to 16 and 36;
      [2] DNA which hybridizes with DNA having a nucleotide sequence complementary to the nucleotide sequence shown in any of SEQ ID NOS: 9 to 16 and 36 under stringent conditions and which encodes a protein having the activity to form the dipeptide or dipeptide derivative PI from one or more kinds of amino acids or amino acid derivatives;
      [3] DNA which hybridizes with DNA having a nucleotide sequence complementary to the nucleotide sequence shown in SEQ ID NO: 18 under stringent conditions and which encodes a protein having the activity to form the dipeptide or dipeptide derivative PI from one or more kinds of amino acids or amino acid derivatives;
      [4] DNA having the nucleotide sequence shown in SEQ ID NO: 39 or 40;
      [5] DNA which hybridizes with DNA having a nucleotide sequence complementary to the nucleotide sequence shown in SEQ ID NO: 39 or 40 under stringent conditions and which encodes a protein having the activity to form the dipeptide or dipeptide derivative PI from one or more kinds of amino acids or amino acid derivatives.
(5) The process according to any of the above (1) to (4), wherein the amino acids or amino acid derivatives are amino acids or amino acid derivatives represented by formula (I): 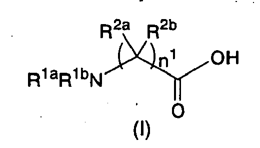 (wherein n¹ represents an integer of 1 to 3;
   R^{1a} and R^{1b}, which may be the same or different, each represent a hydrogen atom, substituted or unsubstituted lower alkyl, substituted or unsubstituted lower alkenyl, substituted or unsubstituted lower alkynyl, substituted or unsubstituted aralkyl, substituted or unsubstituted lower alkanoyl, substituted or unsubstituted lower alkoxycarbonyl, substituted or unsubstituted aryl, or substituted or unsubstituted aroyl, or either R^{1a} or
   R^{1b} may form a substituted or unsubstituted heterocyclic group together with the adjacent nitrogen atom, the carbon atom adjacent to the nitrogen atom and either R^{2a} or R^{2b} on the carbon atom; and
   R^{2a} and R^{2b}, which may be the same or different, each represent a hydrogen atom, substituted or unsubstituted lower alkyl, substituted or unsubstituted aralkyl, substituted or unsubstituted aryl, or substituted or unsubstituted heterocyclic alkyl, or either R^{2a} or R^{2b} on the carbon atom adjacent to R^{1a}R^{1b}N may form a substituted or unsubstituted heterocyclic group together with the adjacent carbon atom, the nitrogen atom adjacent to the carbon atom and either R^{1a} or R^{1b}, and when n¹ is 2 or 3, two or three R^{2a}s and two or three R^{2b}s may be the same or different, respectively), or formula (II): 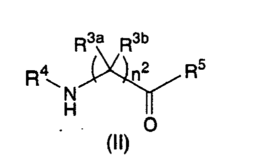 [wherein n² has the same significance as the above n¹;
   R^{3a} and R^{3b}, which may be the same or different, each represent a hydrogen atom, substituted or unsubstituted lower alkyl, substituted or unsubstituted aralkyl, substituted or unsubstituted aryl, or substituted or unsubstituted heterocyclic alkyl, or either R^{3a} or R^{3b} on the carbon atom adjacent to R⁴HN may form a substituted or unsubstituted heterocyclic group together with the adjacent carbon atom, the nitrogen atom adjacent to the carbon atom and R⁴, and when n² is 2 or 3, two or three R^{3a}s and two or three R^{3b}s may be the same or different, respectively;
   R⁴ represents a hydrogen atom, substituted or unsubstituted lower alkyl, substituted or unsubstituted lower alkenyl, substituted or unsubstituted lower alkynyl, substituted or unsubstituted aralkyl, substituted or unsubstituted lower alkanoyl, substituted or unsubstituted lower alkoxycarbonyl, substituted or unsubstituted aryl, or substituted or unsubstituted aroyl, or R⁴ may form a substituted or unsubstituted heterocyclic group together with the adjacent nitrogen atom, the carbon atom adjacent to the nitrogen atom and either R^{3a} or
   R^{3b} on the carbon atom; and
   R⁵ represents amino, hydroxy, substituted or unsubstituted lower alkoxy, mono(substituted or unsubstituted lower alkyl)amino, di(substituted or unsubstituted lower alkyl)amino, or an alicyclic heterocyclic group],
   provided that when all the amino acids or amino acid derivatives are amino acids or amino acid derivatives represented by formula (I), at least one of R^{1a} and R^{1b} is a hydrogen atom, and when all the amino acids or amino acid derivatives are amino acids or amino acid derivatives represented by formula (II), R⁵ is hydroxy.
(6) The process according to any of the above (1) to (4), wherein the amino acids or amino acid derivatives are amino acids or amino acid derivatives represented by formula (III): 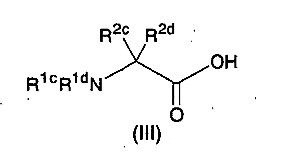 (wherein R^{1c} and R^{1d}, which may be the same or different, each represent a hydrogen atom, substituted or unsubstituted lower alkyl, substituted or unsubstituted lower alkenyl, substituted or unsubstituted lower alkynyl, substituted or unsubstituted aralkyl, substituted or unsubstituted lower alkanoyl, substituted or unsubstituted lower alkoxycarbonyl, substituted or unsubstituted aryl, or substituted or unsubstituted aroyl; and
   R^{2c} and R^{2d}, which may be the same or different, each represent a hydrogen atom or substituted or unsubstituted lower alkyl), or formula (IV): 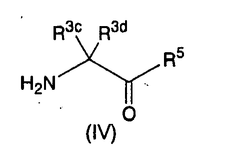 (wherein R^{3c} and R^{3d}, which may be the same or different, each represent a hydrogen atom, substituted or unsubstituted lower alkyl, substituted or unsubstituted aralkyl, or substituted or unsubstituted aryl; and
   R⁵ has the same significance as defined above), provided that when all the amino acids or amino acid derivatives are amino acids or amino acid derivatives represented by formula (III), at least one of R^{1c} and
   R^{1d} is a hydrogen atom, and when all the amino acids or amino acid derivatives are amino acids or amino acid derivatives represented by formula (IV), R⁵ is hydroxy.
(7) The process according to any of the above (1) to (4), wherein the amino acids or amino acid derivatives are amino acids or amino acid derivatives represented by formula (V): 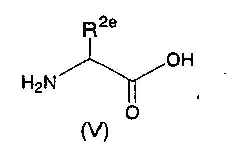 (wherein R^{2e} represents substituted or unsubstituted methyl),
   or formula (VI): 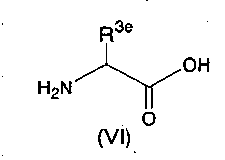 (wherein R^{3e} represents substituted or unsubstituted lower alkyl, substituted or unsubstituted aralkyl, or substituted or unsubstituted aryl).
(8) The process according to any of the above (1) to (4), wherein the amino acids or amino acid derivatives are amino acids selected from the group consisting of L-amino acids, glycine and β-alanine, or derivatives thereof.
(9) The process according to the above (8), wherein the L-amino acid is an L-amino acid selected from the group consisting of L-alanine, L-glutamine, L-glutamic acid, L-valine, L-leucine, L-isoleucine, L-proline, L-phenylalanine, L-tryptophan, L-methionine, L-serine, L-threonine, L-cysteine, L-asparagine, L-tyrosine, L-lysine, L-arginine, L-histidine, L-aspartic acid, L-α-aminobutyric acid, L-azaserine, L-theanine, L-4-hydroxyproline, L-3-hydroxyproline, L-ornithine, L-citrulline and L-6-diazo-5-oxo-norleucine.
(10) The process according to any of the above (1) to (5), wherein the dipeptide or dipeptide derivative PI is a dipeptide or a dipeptide derivative represented by formula (VIIa): 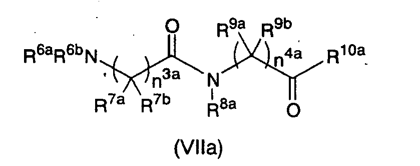 [wherein n^{3a} and n^{4a} each have the same significance as the above n¹;
   R^{6a} and R^{6b}, which may be the same or different, each represent a hydrogen atom, substituted or unsubstituted lower alkyl, substituted or unsubstituted lower alkenyl, substituted or unsubstituted lower alkynyl, substituted or unsubstituted aralkyl, substituted or unsubstituted lower alkanoyl, substituted or unsubstituted lower alkoxycarbonyl, substituted or unsubstituted aryl, or substituted or unsubstituted aroyl, or either R^{6a} or
   R^{6b} may form a substituted or unsubstituted heterocyclic group together with the adjacent nitrogen atom, the carbon atom adjacent to the nitrogen atom and either R^{7a} or R^{7b} on the carbon atom;
   R^{7a} and R^{7b}, which may be the same or different, each represent a hydrogen atom, substituted or unsubstituted lower alkyl, substituted or
   unsubstituted aralkyl, substituted or unsubstituted aryl, or substituted or unsubstituted heterocyclic alkyl, or either R^{7a} or R^{7b} on the carbon atom adjacent to R^{6a}R^{6b}N may form a substituted or unsubstituted heterocyclic group together with the adjacent carbon atom, the nitrogen atom adjacent to the carbon atom and either R^{6a} or R^{6b}, and when n^{3a} is 2 or 3, two or three R^{7a}s and two or three R^{7b}s may be the same or different, respectively;
   R^{8a} represents a hydrogen atom, substituted or unsubstituted lower alkyl, substituted or unsubstituted lower alkenyl, substituted or unsubstituted lower alkynyl, substituted or unsubstituted aralkyl, substituted or unsubstituted lower alkanoyl, substituted or unsubstituted lower alkoxycarbonyl, substituted or unsubstituted aryl, or substituted or unsubstituted aroyl, or R^{8a} may form a substituted or unsubstituted heterocyclic group together with the adjacent nitrogen atom, the carbon atom adjacent to the nitrogen atom and bound to R^{9a} and R^{9b}, and either R^{9a} or R^{9b} on the carbon atom;
   R^{9a} and R^{9b}, which may be the same or different, each represent a hydrogen atom, substituted or unsubstituted lower alkyl, substituted or unsubstituted aralkyl, substituted or unsubstituted aryl, or substituted or unsubstituted heterocyclic alkyl, or either R^{9a} or R^{9b} on the carbon atom adjacent to -R^{8a}N- may form a substituted or unsubstituted heterocyclic group together with the adjacent carbon atom, the nitrogen atom adjacent to the carbon atom and R^{8a}, and when n^{4a} is 2 or 3, two or three R^{9a}s and two or three R^{9b}s may be the same or different, respectively; and
   R^{10a} represents amino, hydroxy, substituted or unsubstituted lower alkoxy, mono(substituted or unsubstituted lower alkyl)amino, di(substituted or unsubstituted lower alkyl)amino, or an alicyclic heterocyclic group].
(11) The process according to any of the above (1) to (5), wherein the dipeptide or dipeptide derivative PII is a dipeptide or a dipeptide derivative represented by formula (VIIb): 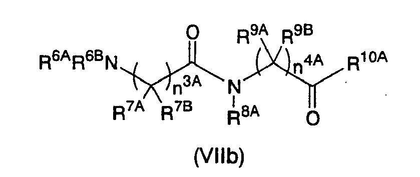 (wherein n^{3A} and n^{4A} each have the same significance, as the above n¹;
   R^{6A} and R^{6B}, which may be the same or different, each represent a hydrogen atom, substituted or unsubstituted lower alkyl, substituted or unsubstituted lower alkenyl, substituted or unsubstituted lower alkynyl, substituted or unsubstituted aralkyl, substituted or unsubstituted lower alkanoyl, substituted or unsubstituted lower alkoxycarbonyl, substituted or unsubstituted aryl, or substituted or unsubstituted aroyl, or either R^{6A} or
   R^{6B} may form a substituted or unsubstituted heterocyclic group together with the adjacent nitrogen atom, the carbon atom adjacent to the nitrogen atom and either R^{7A} or R^{7B} on the carbon atom;
   R^{7A} and R^{7B}, which may be the same or different, each represent a hydrogen atom, substituted or unsubstituted lower alkyl, substituted or unsubstituted aralkyl, substituted or unsubstituted aryl, or substituted or unsubstituted heterocyclic alkyl, or either R^{7A} or R^{7B} on the carbon atom adjacent to R^{6A}R^{6B}N may form a substituted or unsubstituted heterocyclic group together with the adjacent carbon atom, the nitrogen atom adjacent to the carbon atom and either R^{6A} or R^{6B}, and when n^{3A} is 2 or 3, two or three R^{7A}s and two or three R^{7B}s may be the same or different, respectively;
   R^{8A} represents a hydrogen atom, substituted or unsubstituted lower alkyl, substituted or unsubstituted lower alkenyl, substituted or unsubstituted lower alkynyl, substituted or unsubstituted aralkyl, substituted or unsubstituted lower alkanoyl, substituted or unsubstituted lower alkoxycarbonyl, substituted or unsubstituted aryl, or substituted or unsubstituted aroyl, or R^{8A} may form a substituted or unsubstituted heterocyclic group together with the adjacent nitrogen atom, the carbon atom adjacent to the nitrogen atom and bound to R^{9A} and R^{9B}, and either R^{9A} or R^{9B} on the carbon atom;
   R^{9A} and R^{9B}, which may be the same or different, each represent a hydrogen atom, substituted or unsubstituted lower alkyl, substituted or unsubstituted aralkyl, substituted or unsubstituted aryl, or substituted or unsubstituted heterocyclic alkyl, or either R^{9A} or R^{9B} on the carbon atom adjacent to -R^{8A}N- may form a substituted or unsubstituted heterocyclic group together with the adjacent carbon atom, the nitrogen atom adjacent to the carbon atom and R^{8A}, and when n^{4A} is 2. or 3, two or three R^{9A}s and two or three R^{9B}s may be the same or different, respectively; and
   R^{10A} has the same significance as the above R^{10a}).
(12) The process according to any of the above (1) to (6), wherein the dipeptide or dipeptide derivative PI is a dipeptide or a dipeptide derivative represented by formula (VIIIa): 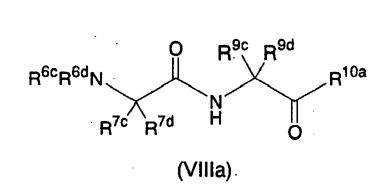 (wherein R^{6c} and R^{6d}, which may be the same or different, each represent a hydrogen atom, substituted or unsubstituted lower alkyl, substituted or unsubstituted lower alkenyl, substituted or unsubstituted lower alkynyl, substituted or unsubstituted aralkyl, substituted or unsubstituted lower alkanoyl, substituted or unsubstituted lower alkoxycarbonyl, substituted or unsubstituted aryl, or substituted or unsubstituted aroyl;
   R^{7c} and R^{7d}, which may be the same or different, each represent a hydrogen atom or substituted or unsubstituted lower alkyl;
   R^{9c} and R^{9d}, which may be the same or different, each represent a hydrogen atom, substituted or unsubstituted lower alkyl, substituted or unsubstituted aralkyl, or substituted or unsubstituted aryl; and
   R^{10a} has the same significance as defined above).
(13) The process according to any of the above (1) to (6), wherein the dipeptide or dipeptide derivative PII is a dipeptide or a dipeptide derivative represented by formula (VIIIb): 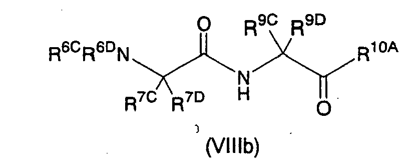 (wherein R^{6C}, R^{6D}, R^{7C}, R^{7D}, R^{9C} and R9^{D} have the same significances as the above R^{6C}, R^{6d}, R^{7c}, R^{7d}, R^{9c} and
   R^{9d}, respectively; and
   R^{10A} has the same significance as defined above).
(14) The process according to any of the above (1) to (7),
   wherein the dipeptide or dipeptide derivative PI is a dipeptide or a dipeptide derivative represented by formula (IXa): 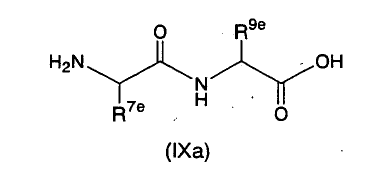 (wherein R^{7e} represents substituted or unsubstituted methyl; and
   R^{9e} represents substituted or unsubstituted lower alkyl, substituted or unsubstituted aralkyl, or substituted or unsubstituted aryl).
(15) The process according to any of the above (1) to (7), wherein the dipeptide or dipeptide derivative PII is a dipeptide or a dipeptide derivative represented by formula (IXb): 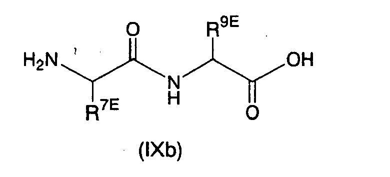 (wherein R^{7E} and R^{9E} have the same significances as the above R^{7e} and R^{9e}, respectively).
(16) The process according to any of the above (1) to (8), wherein the dipeptide or dipeptide derivative PI or dipeptide or dipeptide derivative PII is a dipeptide or a dipeptide derivative in which the same or different amino acids or amino acid derivatives selected from the group consisting of L-amino acids, glycine, β-alanine and their derivatives are bound by peptide bond.
(17) The process according to the above (16), wherein the L-amino acid is an L-amino acid selected from the group consisting of L-alanine, L-glutamine, L-glutamic acid, L-valine, L-leucine, L-isoleucine, L-proline, L-phenylalanine, L-tryptophan, L-methionine, L-serine, L-threonine, L-cysteine, L-asparagine, L-tyrosine, L-lysine, L-arginine, L-histidine, L-aspartic acid, L-α-aminobutyric acid, L-azaserine, L-theanine, L-4-hydroxyproline, L-3-hydroxyproline, L-ornithine, L-citrulline and L-6-diazo-5-oxo-norleucine.
(18)The process according to any of the above (3) to (17), wherein the cells are cells of a microorganism.
(19) The process according to the above (18), wherein the microorganism is a procaryote.
(20) The process according to the above (19), wherein the procaryote is a microorganism in which the activities of one or more kinds of peptidases and one or more kinds of proteins having peptide-permeating/transporting activity (hereinafter referred to also as peptide-permeating/transporting proteins) are reduced or lost.
(21) The process according to the above (20), wherein the procaryote is a microorganism in which the activities of three or more kinds of peptidases are reduced or lost.
(22) The process according to the above (20) or (21), wherein the peptidase is a protein having the amino acid sequence shown in any of SEQ ID NOS: 43 to 46, or a protein having an amino acid sequence which has 80% or more homology to the amino acid sequence shown in any of SEQ ID NOS: 43 to 46 and having peptidase activity.
(23) The process according to the above (20) or (22), wherein the peptide-permeating/transporting protein is a protein having the amino acid sequence shown in any of SEQ ID NOS: 47 to 51, or a protein having an amino acid sequence which has 80% or more homology to the amino acid sequence shown in any of SEQ ID NOS: 47 to 51 and having peptide-permeating/transporting activity.
(24) The process according to any of the above (19) to (23), wherein the procaryote is a microorganism belonging to the genus Escherichia, Bacillus or Corynebacterium.
(25) The process according to the above (24), wherein the microorganism belonging to the genus Escherichia, Bacillus or Corynebacterium is Escherichia coli, Corynebacterium glutamicum, Corynebacterium ammoniagenes, Corynebacterium lactofermentum, Corynebacterium flavum, Corynebacterium efficiens, Bacillus subtilis or Bacillus megaterium.
(26) The process according to any of the above (3) to (25), wherein the treated matter of the culture is a treated matter which is selected from the group consisting of concentrated culture, dried culture, cells obtained by centrifuging the culture, dried culture, cells obtained by centrifuging the culture, dried cells, freeze-dried cells, surfactant-treated cells, ultrasonicated cells, mechanically-disrupted cells, solvent-treated cells, enzymatic-treated cells, protein fractionation of cells, immobilized cells, and an enzyme preparation obtained by extracting the cells, and which has the activity to form dipeptide or dipeptide derivative from one or more kinds of amino acids or amino acid derivatives., and which has the activity to form dipeptide or dipeptide derivative from one or more kinds of amino acids or amino acid derivatives.

### Effect of the Invention

In accordance with the present invention, dipeptide derivatives can be efficiently produced from one or more kinds of amino acids or amino acid derivatives.

### Brief Description of the Drawings

Fig. 1 shows the steps for constructing plasmid pPE43.
Fig. 2 shows the steps for constructing plasmid pQE60ywfE.
Fig. 3 shows the steps for constructing pAL-nou and pAL-alb, which are plasmid vectors for the expression of' proteins having the activity to synthesize a linear dipeptide.
Fig. 4 shows the steps for constructing ywfE gene expression-enhanced vector pPE56.

### Explanation of Symbols

ywfE: ywfE gene derived from Bacillus subtilis 168
Ptrp: Tryptophan promoter gene
PT5: T5 promoter
Amp^{r}: Ampicillin resistance gene
lacI^{q}: Lactose repressor gene
albC: albC gene or albC-analogous gene

### Best Modes for Carrying Out the Invention

1. Proteins Used in the Present Invention
   Examples of the proteins used in the present invention include:
   [1] a protein having the amino acid sequence shown in any of SEQ ID NOS: 1 to 8;
   [2] a protein consisting of an amino acid sequence wherein one or more amino acid residues are deleted, substituted or added in the amino acid sequence shown in any of SEQ ID NOS: 1 to 8 and having the activity to form a dipeptide or a dipeptide derivative from one or more kinds of amino acids or amino acid derivatives;
   [3] a protein consisting of an amino acid sequence which has 65% or more homology to the amino acid sequence shown in any of SEQ ID NOS: 1 to 8 and having the activity to form a dipeptide or a dipeptide derivative from one or more kinds of amino acids or amino acid derivatives;
   [4] a protein consisting of an amino acid sequence which has 80% or more homology to the amino acid sequence shown in SEQ ID, NO: 17 and having the activity to form a dipeptide or a dipeptide derivative from one or more kinds of amino acids or amino acid derivatives;
   [5] a protein having the amino acid sequence shown in SEQ ID NO: 37 or 38;
   [6] a protein consisting of an amino acid sequence wherein one or more amino acid residues are deleted, substituted or added in the amino acid sequence shown in SEQ ID NO: 37 or 38 and having the activity to form a dipeptide or a dipeptide derivative from one or more kinds of amino acids or amino acid derivatives;
   [7] a protein consisting of an amino acid sequence which has 65% or more homology to the amino acid sequence shown in SEQ ID NO: 37 or 38 and having the activity to form a dipeptide or a dipeptide derivative from one or more kinds of amino acids or amino acid derivatives.

The above protein consisting of an amino acid sequence wherein one or more amino acid residues are deleted, substituted or added and having the activity to form dipeptide represented by formula (I) can be obtained by introducing a site-directed mutation into DNA encoding a protein consisting of the amino acid sequence shown in any of SEQ ID NOS: 1 to 8, 37 and 38, by site-directed mutagenesis described in Molecular Cloning, A Laboratory Manual, Third Edition, Cold Spring Harbor Laboratory Press (2001) (hereinafter referred to as Molecular Cloning, Third Edition); Current Protocols in Molecular Biology, John Wiley & Sons (1987-1997) (hereinafter referred to as Current Protocols in Molecular Biology); Nucleic Acids Research, 10, 6487 (1982); Proc. Natl. Acad. Sci. USA, 79, 6409 (1982); Gene, 34, 315 (1985); Nucleic Acids Research, 13, 4431 (1985); Proc. Natl. Acad. Sci. USA, 82, 488 (1985) , etc.

The number of amino acid residues which are deleted, substituted or added is not specifically limited, but is within the range where deletion, substitution or addition is possible by known methods such as the above site-directed mutagenesis. The suitable number is 1 to dozens, preferably 1 to 20, more preferably 1 to 10, further preferably 1 to 5.

The expression "one or more amino acid residues are deleted, substituted or added in the amino acid sequence shown in any of SEQ ID NOS: 1 to 8, 37 and 38" means that the amino acid sequence may contain deletion, substitution or addition of a single or plural amino acid residues at an arbitrary position therein.

Amino acid residues that may be substituted are, for example, those which are not conserved in all of the amino acid sequences shown in SEQ ID NOS: 1 to 8, 37 and 38 when the sequences are compared using known alignment software. An example of known alignment software is alignment analysis software contained in gene analysis software Genetyx (Software Development Co., Ltd.). As analysis parameters for the analysis software, default values can be used.

Deletion or addition of amino acid residues may be contained, for example, in the N-terminal region or the C-terminal region of the amino acid sequence shown in any of SEQ ID NOS: 1 to 8, 37 and 38.

Deletion, substitution and addition may be simultaneously contained in one sequence, and amino acids to be substituted or added may be either natural or not. Examples of the natural amino acids are L-alanine, L-asparagine, L-aspartic acid, L-arginine, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine and L-cysteine.

The following are examples of the amino acids capable of mutual substitution. The amino acids in the same group can be mutually substituted.
- Group A:: leucine, isoleucine, norleucine, valine, norvaline, alanine, 2-aminobutanoic acid, methionine, O-methylserine, t-butylglycine, t-butylalanine, cyclohexylalanine
- Group B:: aspartic acid, glutamic acid, isoaspartic acid, isoglutamic acid, 2-aminoadipic acid, 2-aminosuberic acid
- Group C:: asparagine, glutamine
- Group D:: lysine, arginine, ornithine, 2,4-diaminobutanoic acid, 2,3-diaminopropionic acid
- Group E:: proline, 3-hydroxyproline, 4-hydroxyproline
- Group F:: serine, threonine, homoserine
- Group G:: phenylalanine, tyrosine

In order that the protein using in the present invention may have the activity to form dipeptides or dipeptide derivatives, it is desirable that the homology of its amino acid sequence to the amino acid sequence shown in any of SEQ ID NOS: 1 to 8, 37 and 38, preferably SEQ ID NO: 1 or 37, is 65% or more, preferably 75% or more, more preferably 85% or more, further preferably 90% or more, particularly preferably 95% or more, and most preferably 98% or more.

The homology among amino acid sequences and nucleotide sequences can be determined by using algorithm BLAST by Karlin and Altschul [Proc. Natl. Acad. Sci. USA, 90, 5873 (1993)] and FASTA [Methods Enzymol., 183, 63 (1990)]. On the basis of the algorithm BLAST, programs such as BLASTN and BLASTX have been developed [J. Mol. Biol., 215, 403 (1990)]. When a nucleotide sequence is analyzed by BLASTN on the basis of BLAST, the parameters, for instance, are as follows: score=100 and wordlength=12. When an amino acid sequence is analyzed by BLASTX on the basis of BLAST, the parameters, for instance, are as follows: score=50 and wordlength=3. When BLAST and Gapped BLAST programs are used, default parameters of each program are used. The specific techniques for these analyses are known (http://www.ncbi.nlm.nih.gov.).

A protein consisting of an amino acid sequence which has 65% or more, preferably 75% or more, more preferably 85% or more, further preferably 90% or more, particularly preferably 95% or more, most preferably 98% or more homology to the amino acid sequence shown in any of SEQ ID NOS: 1 to 8, 37 and 38, preferably SEQ ID NO: 1 or 37, and having the activity to form dipeptides or dipeptide derivatives is also included in the proteins used in the present invention. The homology among amino acid sequences can be determined by using BLAST or FASTA as described above.

The amino acid sequence shown in SEQ ID NO: 17 is a region conserved among the proteins having the amino acid sequences shown in SEQ ID NOS: 1 to 7 and is also a region corresponding to the consensus sequnece of proteins having Ala-Ala ligase activity derived from various microorganisms.

A protein consisting of an amino acid sequence which has 80% or more, preferably 90% or more, more preferably 95% or more homology to the amino acid sequence shown in SEQ ID NO: 17 and having the activity to form dipeptides of dipeptide derivatives is also included in the proteins used in the present invention.

In order that the protein having an amino acid sequence which has 80% or more, preferably 90% or more, further preferably 95% or more homology to the amino acid sequence shown in SEQ ID NO: 17 may have the activity to form dipeptides or dipeptide derivatives, it is desirable that the homology of its amino acid sequence to the amino acid sequence shown in any of SEQ ID NOS: 1 to 8 is at least 80% or more, usually 90% or more, and particularly 95% or more.

The homology among amino acid sequences can be determined by using BLAST or FASTA as described above.

It is possible to confirm that the protein used in the present invention is a protein having the activity to form dipeptides or dipeptide derivatives, for example, in the following manner. That is, a transformant expressing the protein used in the present invention is prepared by recombinant DNA techniques, the protein used in the present invention is produced using the transformant, and then the protein, one or more kinds of L-amino acids and amino acid derivatives and ATP are allowed to be present in an aqueous medium, followed by HPLC analysis or the like to know whether a dipeptide or a dipeptide derivative is formed and accumulated in the aqueous medium.

### 2. DNAs Used in the Present Invention

Examples of the DNAs used in the present invention include DNAs:
[1] DNA having the nucleotide sequence shown in any of SEQ ID NOS: 9 to 16 and 36;
[2] DNA which hybridizes with DNA having a nucleotide sequence complementary to the nucleotide sequence shown in any of SEQ ID NOS: 9 to 16 and 36 under stringent conditions and which encodes a protein having the activity to form a dipeptide or a dipeptide derivative from one or more kinds of amino acids or amino acid derivatives;
[3] DNA which hybridizes with DNA having a nucleotide sequence complementary to the nucleotide sequence in SEQ ID NO: 18 and encoding a protein having the activity to form a dipeptide or a dipeptide derivative from one or more kinds of amino acids or amino acid derivatives;
[4] DNA having the nucleotide sequence shown in SEQ ID NO: 39 or 40; and
[5] DNA which hybridizes with DNA having a nucleotide sequence complementary to the nucleotide sequence shown in SEQ ID NO: 39 or 40 under stringent conditions and which encodes a protein having the activity to form a dipeptide or a dipeptide derivative from one or more kinds of amino acids or amino acid derivatives.

The above DNA capable of hybridization under stringent conditions refers to DNA which is obtained by colony hybridization, plaque hybridization, Southern blot hybridization, or the like using a part or the whole of the DNA having a nucleotide sequence complementary to the nucleotide sequence shown in any of SEQ ID NOS: 9 to 16, 18, 36, 39 and 40 as a probe. A specific example of such DNA is DNA which can be identified by performing hybridization at 65°C in the presence of 0.7 to 1.0 mol/l, preferably 0.9 mol/l sodium chloride using a filter with colony- or plaque-derived DNA immobilized thereon, and then washing the filter at 65°C with a 0.1 to 2-fold conc., preferably 0.1-fold conc. SSC solution (1-fold conc. SSC solution: 150 mmol/l sodium chloride and 15 mmol/l sodium citrate). Hybridization can be carried out according to the methods described in Molecular Cloning, Third Edition; Current Protocols in Molecular Biology; DNA Cloning 1: Core Techniques, A Practical Approach, Second Edition, Oxford University (1995), etc. Specifically, the hybridizable DNA includes DNA having at least 75% or more homology, preferably 85% or more homology, further preferably 90% or more homology, particularly preferably 95% or more homology to the nucleotide sequence shown in any of SEQ ID NOS: 9 to 16, 18, 36, 39 and 40 as calculated by use of BLAST or FASTA described above based on the above parameters.

It is possible to confirm that the DNA which hybridizes with DNA having the nucleotide sequence shown in any of SEQ ID NOS: 9 to 16, 18, 36, 39 and 40 under stringent conditions is DNA encoding a protein having the activity to form dipeptides or dipeptide derivatives, for example, by producing a protein encoded by the DNA by recombinant DNA techniques and measuring the activity of the protein as described above.

### 4. Preparation of DNA Used in the Present Invention

The DNA used in the present invention can be obtained by Southern hybridization of a chromosomal DNA library from a microorganism belonging to the genus Bacillus or Streptomyces using a probe designed based on the nucleotide sequence shown in SEQ ID NOS: 9 to 16, 36, 39 or 40, or by PCR [PCR Protocols, Academic Press (1990)] using primer DNAs designed based on the nucleotide sequence shown in SEQ ID NOS: 9 to 16, 36, 39 or 40, as a template, the chromosomal DNA of a microorganism belonging to the genus Bacillus.

The DNA used in the present invention can also be obtained by conducting a search through various gene sequence databases for a sequence having 75% or more homology, preferably 85% or more homology, more preferably 90% or more homology, further preferably 95% or more homology, particularly preferably 98% or more homology to the nucleotide sequence of DNA encoding the amino acid sequence shown in any of SEQ ID NOS: 1 to 8, 17, 37 and 38, and obtaining the desired DNA, based on the nucleotide sequence obtained by the search, from a chromosomal DNA or cDNA library of an organism having the nucleotide sequence according to the above-described method.

The obtained DNA, as such or after cleavage with appropriate restriction enzymes, is inserted into a vector by a conventional method, and the obtained recombinant DNA is introduced into a host cell. Then, the nucleotide sequence of the DNA can be determined by a conventional sequencing method such as the dideoxy method [Proc. Natl. Acad. Sci., USA, 74, 5463 (1977)] or by using a nucleotide sequencer such as 373A DNA Sequencer (Perkin-Elmer Corp.).

In cases where the obtained DNA is found to be a partial DNA by the analysis of nucleotide sequence, the full length DNA can be obtained by Southern hybridization of a chromosomal DNA library using the partial DNA as a probe. ,

It is also possible to prepare the desired DNA by chemical synthesis using a DNA synthesizer (e.g., Model 8905, PerSeptive Biosystems) based on the determined nucleotide sequence of the DNA.

Examples of the DNAs that can be obtained by the above-described method are DNAs having the nucleotide sequences shown in SEQ ID NOS: 9 to 16, 36, 39 and 40.

Examples of the vectors for inserting the DNA of the present invention and the DNA used in the production process of the present invention include pBluescriptII KS(+) (Stratagene), pDIRECT [Nucleic Acids Res., 18, 6069 (1990)], pCR-Script Amp SK(+) (Stratagene), pT7 Blue (Novagen, Inc.), pCR II (Invitrogen Corp.) and pCR-TRAP (Genhunter Corp.).

As the host cell, microorganisms belonging to the genus Escherichia, etc. can be used. Examples of the microorganisms belonging to the genus Escherichia include Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Escherichia coli MP347, Escherichia coli NM522 and Escherichia coli ME8415.

Introduction of the recombinant DNA can be carried out by any of the methods for introducing DNA into the above host cells, for example, the method using calcium ion [Proc. Natl. Acad. Sci. USA, 69, 2110 (1972)], the protoplast method (Japanese Published Unexamined Patent Application No. 248394/88) and electroporation [Nucleic Acids Res., 16, 6127 (1988)].

Examples of the microorganisms carrying the DNAs used in the production process of the present invention obtained by the above method are Escherichia coli NM522/pPE43, which is a microorganism carrying a recombinant DNA comprising DNA having the nucleotide sequence shown in SEQ ID NO: 1.

### 4. Preparation of Cells Used in the Present Invention

(1) The cells used in the present invention include:
   i) bacteria belonging to the genus Bacillus which carry the DNA of the above 3 on the chromosomal DNA, preferably, bacteria belonging to the genus Bacillus which have bacilysin-synthesizing activity, more preferably, bacteria belonging to a species selected from the group consisting of Bacillus subtilis, Bacillus amyloliquefaciens, Bacillus coagulans, Bacillus licheniformis, Bacillus megaterium and Bacillus pumilus, further preferably, bacteria selected from the group consisting of the strains Bacillus subtilis ATCC 15245, Bacillus subtilis ATCC 6633, Bacillus subtilis IAM 1213, Bacillus subtilis IAM 1107, Bacillus subtilis IAM 1214, Bacillus subtilis ATCC 9466, Bacillus subtilis IAM 1033, Bacillus subtilis ATCC 21555, Bacillus amyloliquefaciens IFO 3022 and Bacillus pumilus NRRL B-12025;
   ii) bacteria belonging to the genus Streptomyces which carry the DNA of the above 3 on the chromosomal DNA, preferably, bacteria belonging to the genus Streptomyces which have albonoursin-synthesizing activity, more preferably, bacteria belonging to a species selected from Streptomyces albulus and Streptomyces noursei, further preferably, Streptomyces albulus IFO 14147, Streptomyces. noursei ATCC 11455 or IFO 15452; and iii)transformants that can be prepared by introducing DNA obtained by the method of the above 3 into host cells using the methods described in Molecular Cloning, Third Edition, Current Protocols in Molecular Biology, etc., for example, according to the method described below.

   On the basis of the DNA used in the present invention, a DNA fragment of an appropriate length comprising a region encoding the protein used in the present invention is prepared according to need. Cells with enhanced productivity of the protein can be obtained by replacing a nucleotide in the nucleotide sequence of the region encoding the protein so as to make a codon most suitable for the expression in a host cell.
   The DNA fragment is inserted downstream of a promoter in an appropriate expression vector to prepare a recombinant DNA.
   A transformant producing the protein of the present invention can be obtained by introducing the recombinant DNA into a host cell suited for the expression vector.
   As the host cell, any cells that are capable of expressing the desired gene can be used. Suitable cells include cells of microorganisms such as procaryotes and yeast, animal cells, insect cells, plant cells, etc., preferably microorganisms, more preferably procaryotes, further preferably bacteria.
   The expression vectors that can be employed are those capable of autonomous replication or integration into the chromosome in the above host cells and comprising a promoter at a position appropriate for the transcription of the DNA of the present invention or the DNA used in the production process of the present invention.
   When a procaryote such as a bacterium is used as the host cell, it is preferred that the recombinant DNA comprising the DNA used in the present invention is a recombinant DNA which is capable of autonomous replication in the procaryote and which comprises a promoter, a ribosome binding sequence, the DNA of the present invention or the DNA used in the production process of the present invention, and a transcription termination sequence. The recombinant DNA may further comprise a gene regulating the promoter.
   Examples of suitable expression vectors are pBTrp2, pBTac1 and pBTac2 (products of Boehringer Mannheim GmbH), pHelix1 (Roche Diagnostics Corp.), pKK233-2 (Amersham Pharmacia Biotech), pSE280 (Invitrogen Corp.), pGEMEX-1 (Promega Corp.), pQE-8 (Qiagen, Inc.), pET-3 (Novagen, Inc.), pKYP10 (Japanese Published Unexamined Patent Application No. 110600/83), pKYP200 [Agric. Biol. Chem., 48, 669 (1984)], pLSA1 [Agric. Biol. Chem., 53, 277 (1989)], pGEL1 [Proc. Natl. Acad. Sci. USA, 82, 4306 (1985)], pBluescript II SK(+), pBluescript II KS(-) (Stratagene), pTrS30 [prepared from Escherichia coli JM109/pTrS30 (FERM BP-5407)], pTrS32 [prepared from Escherichia coli JM109/pTrS32 (FERM BP-5408)], pPAC31 (WO98/12343), pUC19 [Gene, 33, 103 (1985)], pSTV28 (Takara Shuzo Co., Ltd.), pUC118 (Takara Shuzo Co., Ltd.), pPA1 (Japanese Published Unexamined Patent Application No. 233798/88), pWH1520 (MoBiTec), pCS299P (WO00/63388), pVLT31 [Gene, 123, 17 (1993)] and pIJ702 (Genetic Manipulation of Streptomyces: a Laboratory Manual: John Innes Foundation).
   As the promoter, any promoters capable of functioning in host cells such as Escherichia coli can be used. For example, promoters derived from Escherichia coli or phage, such as trp promoter (Pₜᵣₚ), lac promoter (P_{lac}), P_{L} promoter, P_{R} promoter and P_{SE} promoter, SPO1 promoter, SPO2 promoter and penP promoter can be used. Artificially designed and modified promoters such as a promoter in which two Pₜᵣₚs are combined in tandem, tac promoter, lacT7 promoter and letI promoter, etc. can also be used.
   Also useful are xylA promoter for the expression in microorganisms belonging to the genus Bacillus [Appl. Microbiol. Biotechnol., 35, 594-599 (1991)], P54-6 promoter for the expression in microorganisms belonging to the genus Corynebacterium [Appl. Microbiol. Biotechnol., 53, 674-679 (2000)], tac promoter for the expression in microorganisms belonging to the genus Pseudomonas [Gene, 123, 17-24 (1993)] and xylA promoter for the expression in microorganisms belonging to the genus Streptomyces (Genetic Manipulation of Streptomyces: a Laboratory Manual: John Innes Foundation).
   It is preferred to use a plasmid in which the distance between the Shine-Dalgarno sequence (ribosome binding sequence) and the initiation codon is adjusted to an appropriate length (e.g., 6 to 18 nucleotides).
   In the recombinant DNA wherein the DNA used in the present invention is ligated to an expression vector, the transcription termination sequence is not essential, but it is preferred to place the transcription termination sequence immediately downstream of the structural gene.
   Examples of such recombinant DNAs are pPE43.
   Examples of suitable procaryotes include microorganisms belonging to the genera Escherichia, Serratia, Bacillus, Brevibacterium, Corynebacterium, Microbacterium, Pseudomonas, Agrobacterium, Alicyclobacillus*,* Anabaena, Anacystis, Arthrobacter, Azotobacter, Chromatium, Erwinia, Methylobacterium, Phormidium, Rhodobacter, Rhodopseudomonas, Rhodospirillum, Scenedesmus, Streptomyces, Synechoccus and Zymomonas. Specific examples are Escherichia coli XL1-Blue, Escherichia coli XL2-Blue, Escherichia coli DH1, Escherichia coli DH5α, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Escherichia coli MP347, Escherichia coli NM522, Bacillus subtilis ATCC 33712, Bacillus megaterium, Bacillus sp. FERM BP-6030, Bacillus amyloliquefaciens, Bacillus coagulans, Bacillus licheniformis, Bacillus pumilus, Brevibacterium ammoniagenes, Brevibacterium immariophilum ATCC 14068, Brevibacterium saccharolyticum ATCC 14066, Brevibacterium flavum ATCC 14067, Brevibacterium lactofermentum ATCC 13869, Corynebacterium glutamicum ATCC 13032, Corynebacterium glutamicum ATCC 14297, Corynebacterium acetoacidophilum ATCC 13870, Microbacterium ammoniaphilum ATCC 15354, Serratia ficaria, Serratia fonticola, Serratia liquefaciens, Serratia marcescens, Pseudomonas sp. D-0110, Agrobacterium radiobacter, Agrobacterium rhizogenes, Agrobacterium rubi, Anabaena cylindrica, Anabaena doliolum, Anabaena flos-aquae, Arthrobacter aurescens, Arthrobacter citreus, Arthrobacter globformis, Arthrobacter hydrocarboglutamicus, Arthrobacter mysorens, Arthrobacter nicotianae, Arthrobacter paraffineus, Arthrobacter protophormiae, Arthrobacter roseoparaffinus, Arthrobacter sulfureus, Arthrobacter ureafaciens, Chromatium buderi, Chromatium tepidum, Chromatium vinosum, Chromatium warmingii, Chromatium fluviatile, Erwinia uredovora, Erwinia carotovora, Erwinia ananas, Erwinia herbicola, Erwinia punctata, Erwinia terreus, Methylobacterium rhodesianum, Methylobacterium extorquens, Phormidium sp. ATCC 29409, Rhodobacter capsulatus, Rhodobacter sphaeroides, Rhodopseudomonas blastica, Rhodopseudomonas marina, Rhodopseudomonas palustris, Rhodospirillum rubrum, Rhodospirillum salexigens, Rhodospirillum salinarum, Streptomyces ambofaciens, Streptomyces aureofaciens, Streptomyces aureus, Streptomyces fungicidicus, Streptomyces griseochromogenes, Streptomyces griseus, Streptomyces lividans, Streptomyces olivogriseus, Streptomyces rameus, Streptomyces tanashiensis, Streptomyces vinaceus and Zymomonas mobilis. Preferred procaryotes include bacteria belonging to the genera Escherichia, Serratia, Bacillus, Brevibacterium, Corynebacterium, Pseudomonas and Streptomyces, for example, the above-mentioned species belonging to the genera Escherichia, Serratia, Bacillus, Brevibacterium, Corynebacterium, Pseudomonas and Streptomyces. More preferred bacteria include Escherichia coli, Corynebacterium glutamicum, Corynebacterium ammoniagenes, Corynebacterium lactofermentum, Corynebacterium flavum, Corynebacterium efficiens, Bacillus subtilis, Bacillus megaterium, Serratia marcescens, Pseudomonas putida, Pseudomonas aeruginosa, Streptomyces coelicolor and Streptomyces lividans, among which Escherichia coli is particularly preferred.
   Introduction of the recombinant DNA can be carried out by any of the methods for introducing DNA into the above host cells, for example, the method using calcium ion [Proc. Natl. Acad. Sci. USA, 69, 2110 (1972)], the protoplast method (Japanese Published Unexamined Patent Application No. 248394/88) and electroporation [Nucleic Acids Res., 16, 6127 (1988)].
   When a yeast strain is used as the host cell, YEp13 (ATCC 37115), YEp24 (ATCC 37051), YCp50 (ATCC 37419), pHS19, pHS15, etc. can be used as the expression vector.
   As the promoter, any promoters capable of functioning in yeast strains can be used. Suitable promoters include PHO5 promoter, PGK promoter, GAP promoter, ADH promoter, gal 1 promoter, gal 10 promoter, heat shock polypeptide promoter, MF.α1 promoter and CUP 1 promoter.
   Examples of suitable host cells are yeast strains belonging to the genera Saccharomyces, Schizosaccharomyces, Kluyveromyces, Trichosporon, Schwanniomyces, Pichia and Candida*,* specifically, Saccharomyces cerevisiae, Schizosaccharomyces pombe, Kluyveromyces lactis, Trichosporon pullulans, Schwanniomyces alluvius, Pichia pastoris and Candida utilis.
   Introduction of the recombinant DNA can be carried out by any of the methods for introducing DNA into yeast, for example, electroporation [Methods Enzymol., 194, 182 (1990)], the spheroplast method [Proc. Natl. Acad. Sci. USA, 81, 4889 (1984)] and the lithium acetate method [J. Bacteriol., 153, 163 (1983)].
   When an animal cell is used as the host cell, pcDNAI, pcDM8 (commercially available from Funakoshi Co., Ltd.), pAGE107 (Japanese Published Unexamined Patent Application No. 22979/91), pAS3-3 (Japanese Published Unexamined Patent Application No. 227075/90), pCDM8 [Nature, 329, 840. (1987)], pcDNAI/Amp (Invitrogen Corp.), pREP4 (Invitrogen Corp.), pAGE103 [J. Biochem., 101, 1307 (1987)], pAGE210, pAMo, pAMoA, etc. can be used as the expression vector.
   As the promoter, any promoters capable of functioning in animal cells can be used. Suitable promoters include the promoter of IE (immediate early) gene of cytomegalovirus (CMV), SV40 early promoter, metallothionein promoter, the promoter of a retrovirus, heat shock promoter, SR α promoter, etc. The enhancer of IE gene of human CMV may be used in combination with the promoter.
   Examples of suitable host cells are mouse myeloma cells, rat myeloma cells, mouse hybridomas, human-derived Namalwa cells and Namalwa KJM-1 cells, human embryonic kidney cells, human leukemia cells, African green monkey kidney cells, Chinese hamster-derived CHO cells, and HBT5637 (Japanese Published Unexamined Patent Application No. 299/88).
   The mouse myeloma cells include SP2/0 and NSO; the rat myeloma cells include YB2/0; the human embryonic kidney cells include HEK293 (ATCC CRL-1573); the human leukemia cells include BALL-1; and the African green monkey kidney cells include COS-1 and COS-7.
   Introduction of the recombinant DNA can be carried out by any of the methods for introducing DNA into animal cells, for example, electroporation [Cytotechnology, 3, 133 (1990)], the calcium phosphate method (Japanese Published Unexamined Patent Application No. 227075/90), lipofection [Proc. Natl. Acad. Sci. USA, 84, 7413 (1987)], and the method described in Virology, 52, 456 (1973).
   When an insect cell is used as the host cell, the protein can be produced by using the methods described in Baculovirus Expression Vectors, A Laboratory Manual, W. H. Freeman and Company, New York (1992); Current Protocols in Molecular Biology; Molecular Biology, A Laboratory Manual; Bio/Technology, 6, 47 (1988), etc.
   That is, the recombinant gene transfer vector and a baculovirus are cotransfected into insect cells to obtain a recombinant virus in the culture supernatant of the insect cells, and then insect cells are infected with the recombinant virus, whereby the protein can be produced.
   The gene transfer vectors useful in this method include pVL1392, pVL1393 and pBlueBacIII (products of Invitrogen Corp.).
   An example of the baculovirus is Autographa californica nuclear polyhedrosis virus, which is a virus infecting insects belonging to the family Barathra.
   Examples of the insect cells are ovarian cells of Spodoptera frugiperda, ovarian cells of Trichoplusia ni, and cultured cells derived from silkworm ovary.
   The ovarian cells of Spodoptera frugiperda include Sf9 and Sf21 (Baculovirus Expression Vectors, A Laboratory Manual); the ovarian cells of Trichoplusia ni include High 5 and BTI-TN-5B1-4 (Invitrogen Corp.); and the cultured cells derived from silkworm ovary include Bombyx mori N4.
   Cotransfection of the above recombinant gene transfer vector and the above baculovirus into insect cells for the preparation of the recombinant virus can be carried out by the calcium phosphate method (Japanese Published Unexamined Patent Application No. 227075/90), lipofection [Proc. Natl. Acad. Sci. USA, 84, 7413 (1987)], etc.
   When a plant cell is used as the host cell, Ti plasmid, tobacco mosaic virus vector, etc. can be used as the expression vector.
   As the promoter, any promoters capable of functioning in plant cells can be used. Suitable promoters include 35S promoter of cauliflower mosaic virus (CaMV), rice actin 1 promoter, etc.
   Examples of suitable host cells are cells of plants such as tobacco, potato, tomato, carrot, soybean, rape, alfalfa, rice, wheat and barley.
   Introduction of the recombinant vector can be carried out by any of the methods for introducing DNA into plant cells, for example, the method using Agrobacterium (Japanese Published Unexamined Patent Application Nos. 140885/84 and 70080/85, WO94/00977), electroporation (Japanese Published Unexamined Patent Application No. 251887/85) and the method using particle gun (gene gun) (Japanese Patent Nos. 2606856 and 2517813).
   When the DNA is expressed in yeast, an animal cell, an insect cell or a plant cell, a cell producing a glycosylated protein can be obtained.
(2) The microorganisms preferrably used in the production process of the present invention include microorganisms prepared by the method of the above (1) in which the activities of one or more kinds of peptidases and one or more kinds of proteins having peptide-permeating/transporting activity (hereinafter referred to as peptide-permeating/transporting proteins) are reduced or lost, and those in which the activities of three or more kinds of peptidases are reduced or lost.

Such microorganisms can be obtained, for example, by any of the following methods: (a) methods of imparting, by the method of the above (1), the ability to produce the protein having the dipeptide-forming activity or the ability to produce the protein having polyphosphate kinase activity to microorganisms in which the functions of one or more kinds of peptidases and one or more kinds of proteins having peptide-permeating/transporting activity are reduced or lost, or microorganisms in which the functions of three or more kinds of peptidases are reduced or lost; and (b) methods of reducing or causing loss of the functions of a) one or more kinds of peptidases and one or more kinds of peptide-permeating/transporting proteins or b) three or more kinds of peptidases of microorganisms having the ability to produce the protein having the dipeptide-forming activity or the ability to produce the protein having polyphosphate kinase activity which can be prepared by the method of the above (1).

The microorganisms in which the activities of one or more kinds of peptidases and one or more kinds of peptide-permeating/transporting proteins are reduced or lost include microorganisms in which the activities of one or more arbitrary kinds of peptidases and one or more arbitrary kinds of peptide-permeating/transporting proteins are reduced or lost provided that the microorganisms can normally grow, specifically, microorganisms in which the activities of preferably one to nine kinds, more preferably one to seven kinds, further preferably one to four kinds of peptidases and preferably one to five kinds, more preferably one to three kinds, further preferably one or two kinds, particularly preferably one kind of peptide-permeating/transporting protein are reduced or lost.

Examples of such microorganisms are microorganisms in which the activities of one or more kinds of peptidases and one or more kinds of peptide-permeating/transporting proteins are reduced or lost because the nucleotide sequences of one or more kinds of genes encoding peptidases (hereinafter referred to as peptidase genes) and one or more kinds of genes encoding peptide-permeating/transporting proteins (hereinafter referred to as peptide- permeating/transporting protein genes) among the peptidase genes and peptide-permeating/transporting protein genes existing on the genomic DNA of the microorganisms are entirely or partially deleted or said nucleotide sequences contain nucleotide substitutions or additions.

The expression "the activity of peptidase is reduced" means that the peptidolytic activity is reduced, or reduced to normally 80% or less, preferably 50% or less, more preferably 30% or less, further preferably 20% or less, particularly preferably 10% or less, most preferably 5% or less compared with peptidase encoded by the gene having none of the above deletions, substitutions and additions of nucleotides.

The peptidolytic activity of a microorganism can be measured by allowing a peptide as a substrate and microorganism cells to be present in an aqueous medium, thereby performing peptidolytic reaction, and then determining the amount of the remaining peptide by a known method, e.g., HPLC analysis.

The above peptidases may be any proteins having peptidolytic activity. Preferred are proteins having high dipeptide-hydrolyzing activity. More preferred are dipeptidases.

Examples of peptidases include: those existing in Escherichia coli such as PepA having the amino acid sequence shown in SEQ ID NO: 43, PepB having the amino acid sequence shown in SEQ ID NO: 44, PepD having the amino acid sequence shown in SEQ ID NO: 45, PepN having the amino acid sequence shown in SEQ ID NO: 46, PepP [GenBank accession No. (hereinafter abbreviated as Genbank) AAC75946], PepQ (GenBank AAC76850), PepE (GenBank AAC76991), PepT (GenBank AAC74211), Dcp (GenBank AAC74611) and IadA (GenBank AAC77284); those existing in Bacillus subtilis such as AmpS (GenBank AF012285), PepT (GenBank X99339), YbaC (GenBank Z99104), YcdD (GenBank Z99105), YjbG (GenBank Z99110), YkvY (GenBank Z99111), YqjE (GenBank Z99116) and YwaD (GenBank Z99123); and those existing in Corynebacterium glutamicum such as proteins having the amino acid sequences represented by BAB97732, BAB97858, BAB98080, BAB98880, BAB98892, BAB99013, BAB99598 and BAB99819 (registration Nos. of DNA Data Bank of Japan). Examples of dipeptidases include PepA, PepB, PepD and PepN having the amino acid sequences shown in SEQ ID NOS: 43 to 46, PepQ, PepE and IadA. Proteins having amino acid sequences which have 80% or more, preferably 90% or more, more preferably 95% or more homology to the amino acid sequence shown in any of SEQ ID NOS: 43 to 46 and having peptidase activity are also included in the proteins having high dipeptide-hydrolyzing activity. The homology among amino acid sequences and nucleotide sequences can be determined by using BLAST, FASTA or the like described above.

The expression "the activity of a peptide-permeating/transporting protein is reduced" means that the peptide-uptaking activity is reduced, or reduced to normally 80% or less, preferably 50% or less, more preferably 30% or less, further preferably 20% or less, particularly preferably 10% or less, most preferably 5% or less compared with a peptide-permeating/transporting protein encoded by the gene having none of the above deletions, substitutions and additions of nucleotides.

The peptide-uptaking activity of a microorganism can be measured by allowing a peptide as a substrate and microorganism cells to be present in an aqueous medium, thereby performing peptide-uptaking reaction, and then determining the amount of the remaining peptide by a known method, e.g., HPLC analysis.

The above peptide-permeating/transporting proteins may be any proteins involved in peptide uptake of microorganisms, for example, proteins encoded by genes forming an operon on chromosomal DNA which form a complex on cell membrane to express dipeptide-uptaking activity and those which have peptide-uptaing activity as individual proteins. Preferred are proteins having high peptide-uptaking activity.

Examples of the peptide-permeating/transporting proteins include: those existing in Escherichia coli such as DppA having the amino acid sequence shown in SEQ ID NO: 47, DppB having the amino acid sequence shown in SEQ ID NO: 48, DppC having the amino acid sequence shown in SEQ ID NO: 49, DppD having the amino acid sequence shown in SEQ ID NO: 50, DppF having the amino acid sequence shown in SEQ ID NO: 51, OppA (GenBank AAC76569), OppB (GenBank AAC76568), OppC (GenBank'AAC76567), OppD (GenBank AAC76566), OppF (GenBank AAC76565), YddO (GenBank AAC74556), YddP (GenBank AAC74557), YddQ (GenBank AAC74558), YddR (GenBank AAC74559), YddS (GenBank AAC74560), YbiK (GenBank AAC73915), MppA (GenBank AAC74411), SapA (GenBank AAC74376), SapB (GenBank AAC74375), SapC (GenBank AAC74374), SapD (GenBank AAC74373) and SapF (GenBank AAC74372); those existing in Bacillus subtilis such as DppA (GenBank CAA40002), DppB (GenBank CAA40003), DppC (GenBank CAA40004), DppD (GenBank CAA40005), DppE (GenBank CAA40006), OppA (GenBank CAA39787), OppB (GenBank CAA39788), OppC (GenBank CAA39789), OppD (GenBank CAA39790), OppF (GenBank CAA39791), AppA (GenBank CAA62358), AppB (GenBank CAA62359), AppC (GenBank CAA62360), AppD (GenBank CAA62356), AppF (GenBank CAA62357), YclF (GenBank CAB12175) and YkfD (GenBank CAB13157); and those existing in Corynebacterium glutamicum such as proteins having the amino acid sequences represented by BAB99048, BAB99383, BAB99384, BAB99385, BAB99713, BAB99714, BAB99715, BAB99830, BAB99831 and BAB99832 (registration Nos. of DNA Data Bank of Japan). Examples of the proteins having high peptide-permeating/transporting activity include DppA, DppB, DppC, DppD and DppF having the amino acid sequences shown in SEQ ID NOS: 47 to 51, and proteins having amino acid sequences which have 80% or more, preferably 90% or more, more preferably 95% or more homology to the amino acid sequence shown in any of SEQ ID NOS: 47 to 51.

The homology among amino acid sequences can be determined by using programs such as BLAST and FASTA described above.

The microorganisms in which the activities of three or more kinds of peptidases are reduced or lost include microorganisms in which the activities of three or more arbitrary kinds of peptidases are reduced or lost provided that the microorganisms can normally grow, specifically, microorganisms in which the activities of preferably three to nine kinds, more preferably three to six kinds, further preferably three or four kinds of peptidases are reduced or lost.

Examples of peptidases include the above-described peptidases and dipeptidases existing in Escherichia coli, Bacillus subtilis and Corynebacterium glutamicum. Proteins consisting of amino acid sequences which have 80% or more, preferably 90% or more, more preferably 95% or more homology to the amino acid sequence shown in any of SEQ ID NOS: 43 to 46 and having peptidase activity are also included in the proteins having high dipeptide-hydrolyzing activity.

The homology among amino acid sequences can be determined by using programs such as BLAST and FASTA described above.

The microorganisms in which the activities of peptidases and peptide-permeating/transporting proteins are reduced or lost may be obtained by any method capable of preparing such microorganisms. For example, they can be obtained by introducing a deletion, substitution or addition of a nucleotide into peptidase genes and peptide-permeating/transporting protein genes on chromosomal DNAs of microorganisms as described below.

The methods for introducing a deletion, substitution or addition of a nucleotide into a gene on the chromosomal DNA of a microorganism include methods utilizing homologous recombination. An example of the methods utilizing general homologous recombination is a method using a plasmid for homologous recombination prepared by ligating a mutant gene having an introduced nucleotide deletion, substitution or addition to a plasmid DNA incapable of autonomous replication in a host cell into which the nucleotide deletion or the like is to be introduced and carrying a drug resistance gene.

The plasmid for homologous recombination is introduced into a host cell by an ordinary method, followed by selection of a transformant in which the plasmid for homologous recombination has been integrated into the chromosomal DNA by homologous recombination using the drug resistance as a marker. The obtained transformant is cultured using a medium which does not contain the drug for several hours to one day, and then spread on an agar medium containing the drug and on an agar medium without the drug. By selecting a strain which does not grow on the former medium but can grow on the latter medium, the strain in which second homologous recombination occurred on the chromosomal DNA can be obtained. Introduction of a nucleotide deletion, substitution or addition into a desired gene on the chromosomal DNA can be confirmed by determining the nucleotide sequence of a region of the chromosomal DNA containing the gene into which the deletion or the like has been introduced.

By use of the above method, a nucleotide deletion, substitution or addition can be introduced into desired genes on chromosomal DNAs of microorganisms such as those belonging to the genera Escherichia, Bacillus and Corynebacterium.

Further, a nucleotide deletion, substitution or addition can be efficiently introduced into plural genes by utilizing homologous recombination according to a method using a linear DNA.

Specifically, a linear DNA containing a gene into which a nucleotide deletion, substitution or addition is to be introduced is incorporated into a cell to cause homologous recombination between chromosomal DNA and the introduced linear DNA. This method is applicable to any microorganisms capable of efficiently incorporating a linear DNA. Preferred microorganisms are those belonging to the genera Escherichia and Bacillus. Escherichia coli is more preferred, and Escherichia coli expressing a group of recombinant proteins derived from λ phage (Red recombination system) is further preferred.

An example of Escherichia coli expressing λ Red recombination system is Escherichia coli JM101 carrying pKD46, which is a plasmid DNA comprising a λ Red recombination system gene (available from Escherichia coli Genetic Stock Center, Yale University, U.S.A.).

Examples of the DNAs useful for homologous recombination are as follows:
(a) linear DNA in which DNAs present on the outside of a region of chromosomal DNA to be subjected to introduction of a nucleotide deletion, substitution or addition are present at both termini of a drug resistance gene;
(b) linear DNA in which DNAs present on the outside of a region of chromosomal DNA to be subjected to introduction of a nucleotide deletion, substitution or addition are directly ligated to each other;
(c) linear DNA in which DNAs present on the outside of a region of chromosomal DNA to be subjected to introduction of a nucleotide deletion, substitution or addition are present at both termini of a drug resistance gene and a gene that can be used for negative selection; (d) linear DNA of the above (a) in which a'nucleotide sequence recognized by yeast-derived Flp recombinase [Proc. Natl. Acad. Sci. USA., 82, 5875 (1985)] is additionally present between the drug resistance gene and the DNAs present on the outside of a region of chromosomal DNA.

As the drug resistance gene, any drug resistance genes that impart resistance to a drug to which the host microorganism shows sensitivity can be used. When Escherichia coli is used as the host microorganism, examples of the drug resistance genes are kanamycin resistance gene, chloramphenicol resistance gene, gentamicin resistance gene, spectinomycin resistance gene, tetracycline resistance gene and ampicillin resistance gene.

The "gene that can be used for negative selection" refers to a gene that is-fatal to a host microorganism under certain culture conditions when the gene is expressed in the host microorganism. Examples of the genes are sacB gene derived from a microorganism belonging to the genus Bacillus [Appl. Environ. Microbiol., 59, 1361-1366 (1993)] and rpsL gene derived from a microorganism belonging to the genus Escherichia [Genomics, 72, 99-104 (2001)].

The DNAs having homology to the DNAs present on the outside of a region of chromosomal DNA to be subjected to introduction of a substitution or deletion in the above linear DNAs are located in the same direction as that on the chromosomal DNA, and their length is preferably about 10 bp to 100 bp, more preferably about 20 bp to 50 bp, and further preferably about 30 bp to 40 bp.

The nucleotide sequence recognized by yeast-derived Flp recombinase is not specifically limited so long as it is a nucleotide sequence recognized by the said protein and catalyzing homologous recombination. Preferred examples are DNA having the nucleotide sequence shown in SEQ ID NO: 52, and DNA having a nucleotide sequence wherein one to several nucleotides are deleted, substituted or added in the said DNA and having a nucleotide sequence recognized by yeast-derived Flp recombinase and catalyzing homologous recombination.

The expression "having homology" refers to having such a degree of homology that allows occurrence of homologous recombination between the subject region of chromosomal DNA and the above linear DNA, specifically, having 80% or more homology, preferably 90% or more homology, more preferably 95% or more homology, further preferably 100% homology.

The homology among nucleotide sequences can be determined by using programs such as BLAST and FASTA described above.

The above linear DNA fragments can be prepared by PCR. The desired linear DNA, can also be obtained by constructing DNA containing the above linear DNA on plasmid and then carrying out treatment with restriction enzymes.

Examples of the methods for introducing a nucleotide deletion, substitution or addition into the chromosomal DNA of a microorganism include the following Methods 1 to 4.

### Method 1:

A method which comprises introducing the linear DNA of the above (a) or (d) into a host microorganism and selecting a transformant carrying the linear DNA inserted on its chromosomal DNA by homologous recombination using the drug resistance as a marker.

### Method 2:

A method which comprises introducing the linear DNA of the above (b) into the transformant obtained according to the above Method 1 and eliminating the drug resistance gene inserted on its chromosomal DNA by Method 1 to substitute or delete a region of the chromosomal DNA of the microorganism.

### Method 3:

A method which comprises:
[1] introducing the linear DNA of the above (c) into a host microorganism and selecting a transformant carrying the linear DNA inserted on its chromosomal DNA by homologous recombination using the drug resistance as a marker;
[2] synthesizing DNA by ligating DNAs having homology to the DNAs present on the outside of a region of chromosomal DNA to be subjected to introduction of a substitution or deletion in the same direction as that on the chromosomal DNA, and introducing the synthesized DNA into the transformant obtained in the above [1]; and
[3] culturing the transformant subjected to the operation of the above [2] under conditions such that the gene that can be used for negative selection is expressed, and selecting a strain capable of growing by the culturing as a strain in which the drug resistance gene and the gene that can be used for negative selection are eliminated from the chromosomal DNA.

### Method 4:

A method which comprises:
[1] introducing the linear DNA of the above (d) into a host microorganism and selecting a transformant carrying the linear DNA inserted on its chromosomal DNA by homologous recombination using the drug resistance as a marker; and
[2] introducing a Flp recombinase gene expression plasmid into the transformant obtained in the above [1], and after expression of the gene, obtaining a strain sensitive to the drug used in the above [1].

In the above methods, introduction of the linear DNA into a host microorganism can be carried out by any of the methods for introducing DNA into the microorganism, for example, the method using calcium ion [Proc. Natl. Acad. Sci. USA, 69, 2110 (1972)], the protoplast method (Japanese Published Unexamined Patent Application No. 248394/88) and electroporation [Nucleic Acids Res., 16, 6127 (1988)].

By using a linear DNA in which an arbitrary gene to be inserted to chromosomal DNA is incorporated in the center part of the linear DNA used in Method 2 or Method 3 [2], it is possible to eliminate the drug resistance gene and at the same time to insert an arbitrary gene to the chromosomal DNA.

The above Methods 2 to 4 are methods that leave no foreign genes such as a drug resistance gene and a gene usable for negative selection on the chromosomal DNA of the transformant to be finally obtained. Therefore, it is possible to readily produce a microorganism having nucleotide deletions, substitutions or additions in two or more different regions of the chromosomal DNA by repeating the operations of the Methods using the same drug resistance gene and the same gene usable for negative selection.

### 5. Preparation of the Protein Used in the Present Invention

The protein can be produced by culturing the transformant obtained by the method of the above 4 in a medium, allowing the protein used in the present invention to form and accumulate in the culture, and recovering the protein from the culture.

The host of the above transformant for producing the protein used in the present invention may be any microorganism such as procaryote or yeast, animal cell, insect cell, plant cell or the like, but is preferably a microorganism, more preferably a procaryote, further preferably a bacterium, particularly preferably a bacterium belonging to the genus Escherichia, and most preferably Escherichia coli.

Culturing of the above transformant in a medium can be carried out by conventional methods for culturing the host cell.

For the culturing of the transformant microorganism obtained by using a procaryote such as Escherichia coli or a eucaryote such as yeast as the host cell, any of natural media and synthetic media can be used insofar as it is a medium suitable for efficient culturing of the transformant which contains carbon sources, nitrogen sources, inorganic salts, etc. which can be assimilated by the host used.

As the carbon sources, any carbon sources that can be assimilated by the host can be used. Examples of suitable carbon sources include carbohydrates such as glucose, fructose, sucrose, molasses containing them, starch and starch hydrolyzate; organic acids such as acetic acid and propionic acid; and alcohols such as ethanol and propanol.

As the nitrogen sources, ammonia, ammonium salts of organic or inorganic acids such as ammonium chloride, ammonium sulfate, ammonium acetate and ammonium phosphate, and other nitrogen-containing compounds can be used as well as peptone, meat extract, yeast extract, corn steep liquor, casein hydrolyzate, soybean cake, soybean cake hydrolyzate, and various fermented microbial cells and digested products thereof.

Examples of the inorganic salts include potassium dihydrogenphosphate, dipotassium hydrogenphosphate, magnesium phosphate, magnesium sulfate, sodium chloride, ferrous sulfate, manganese sulfate, copper sulfate and calcium carbonate.

Culturing is usually carried out under aerobic conditions, for example, by shaking culture or submerged spinner culture under aeration. The culturing temperature is preferably 15 to 40°C, and the culturing period is usually 5 hours to 7 days. The pH is maintained at 3.0 to 9.0 during the culturing. The pH adjustment is carried out by using an organic or inorganic acid, an alkali solution, urea, calcium carbonate, ammonia, etc.

If necessary, antibiotics such as ampicillin and tetracycline may be added to the medium during the culturing.

When a microorganism transformed with an expression vector comprising an inducible promoter is cultured, an inducer may be added to the medium, if necessary. For example, in the case of a microorganism transformed with an expression vector comprising lac promoter, isopropyl-β-D-thiogalactopyranoside or the like may be added to the medium; and in the case of a microorganism transformed with an expression vector comprising trp promoter, indoleacrylic acid or the like may be added.

For the culturing of the transformant obtained by using an animal cell as the host cell, generally employed media such as RPMI1640 medium [J. Am. Med. Assoc., 199, 519 (1967)], Eagle's MEM [Science, 122, 501 (1952)], DMEM [Virology, 8, 396 (1959)] and 199 medium [Proc. Soc. Biol. Med., 73, 1 (1950)], media prepared by adding fetal calf serum or the like to these media, etc. can be used as the medium.

Culturing is usually carried out at pH 6 to 8 at 25 to 40°C for 1 to 7 days in the presence of 5% CO₂.

If necessary, antibiotics such as kanamycin, penicillin and streptomycin may be added to the medium during the culturing.

For the culturing of the transformant obtained by using an insect cell as the host cell, generally employed media such as TNM-FH medium (PharMingen, Inc.), Sf-900 II SFM medium (Life Technologies, Inc.), ExCell 400 and ExCell 405 (JRH Biosciences, Inc.) and Grace's Insect Medium [Nature, 195, 788 (1962)] can be used as the medium.

Culturing is usually carried out at pH 6 to 7 at 25 to 30°C for 1 to 5 days.

If necessary, antibiotics such as gentamicin may be added to the medium during the culturing.

The transformant obtained by using a plant cell as the host cell may be cultured in the form of cells as such or after differentiation into plant cells or plant organs. For the culturing of such transformant, generally employed media such as Murashige-Skoog (MS) medium and White medium, media prepared by adding phytohormones such as auxin and cytokinin to these media, etc. can be used as the medium.

Culturing is usually carried out at pH 5 to 9 at 20 to 40° C for 3 to 60 days.

If necessary, antibiotics such as kanamycin and hygromycin may be added to the medium during the culturing.

As described above, the protein used in the present invention can be produced by culturing the transformant derived from microorganisms, insect cells, animal cells or plant cells, and carrying the recombinant DNA wherein the DNA used in the present invention is ligated to an expression vector, allowing the protein used in the present invention to form and accumulate in the culture, and recovering the protein from the culture.

The protein used in the present invention may be produced by intracellular production by host cells, extracellular secretion by host cells or production on outer membranes by host cells. A desirable production method can be adopted by changing the kind of the host cells used or the structure of the protein to be produced.

When the protein used in the present invention is produced in host cells or on outer membranes of host cells, it is possible to force the protein to be secreted outside the host cells by applying the method of Paulson, et al. [J. Biol. Chem., 264, 17619 (1989)], the method of Lowe, et al. [Proc. Natl. Acad., Sci. USA, 86, 8227 (1989); Genes Develop., 4, 1288 (1990)], or the methods described in Japanese Published Unexamined Patent Application No. 336963/93, WO94/23021, etc.

That is, extracellular secretion of the protein of the present invention by host cells can be caused by expressing it in the form of a protein in which a signal peptide is added upstream of a protein containing the active site of the protein of the present invention by the use of recombinant DNA techniques.

It is also possible to increase the protein production by utilizing a gene amplification system using a dihydrofolate reductase gene or the like according to the method described in Japanese Published Unexamined Patent Application No. 227075/90.

Further, the protein used in the present invention can be produced using an animal having an introduced gene (non-human transgenic animal) or a plant having an introduced gene (transgenic plant) constructed by redifferentiation of animal or plant cells carrying the introduced gene.

When the transformant producing the protein used in the present invention is an animal or plant, the protein can be produced by raising or culturing the animal or plant in a usual manner, allowing the protein to form and accumulate therein, and recovering the protein from the animal or plant.

Production of the protein used in the present invention using an animal can be carried out, for example, by producing the protein in an animal constructed by introducing the gene according to known methods [Am. J. Clin. Nutr., 63, 639S (1996); Am. J. Clin. Nutr., 63, 627S (1996); Bio/Technology, 9, 830 (1991)].

In the case of an animal, the protein used in the present invention can be produced, for example, by raising a non-human transgenic animal carrying the introduced DNA used in the present invention, allowing the protein to form and accumulate in the animal, and recovering the protein from the animal. The places where the protein is formed and accumulated include milk (Japanese Published Unexamined Patent Application No. 309192/88), egg, etc. of the animal. As the promoter in this process, any promoters capable of functioning in an animal can be used. Preferred promoters include mammary gland cell-specific promoters such as a casein promoter, β casein promoter, β lactoglobulin promoter and whey acidic protein promoter.

Production of the protein used in the present invention using a plant can be carried out, for example, by culturing a transgenic plant carrying the introduced DNA encoding the protein used in the present invention according to known methods [Soshiki Baiyo (Tissue Culture), 20, (1994); Soshiki Baiyo, 21, (1995); Trends Biotechnol., 15, 45 (1997)], allowing the protein to form and accumulate in the plant, and recovering the protein from the plant.

The protein used in the present invention produced by using the transformant producing the protein can be isolated and purified by conventional methods for isolating and purifying enzymes.

For example, when the protein used in the present invention is produced in a soluble form, in cells, the cells are recovered by centrifugation after the completion of culturing and suspended in an aqueous buffer, followed by disruption using a sonicator, French press, Manton Gaulin homogenizer, Dynomill or the like to obtain a cell-free extract.

A purified protein preparation can be obtained by centrifuging the cell-free extract to obtain the supernatant and then subjecting the supernatant to ordinary means for isolating and purifying enzymes, e.g., extraction with a solvent, salting-out with ammonium sulfate, etc., desalting, precipitation with an organic solvent, anion exchange chromatography using resins such as diethylaminoethyl (DEAE)-Sepharose and DIAION HPA-75 (Mitsubishi Chemical Corporation), cation exchange chromatography using resins such as S-Sepharose FF (Pharmacia), hydrophobic chromatography using resins such as butyl Sepharose and phenyl Sepharose, gel filtration using a molecular sieve, affinity chromatography, chromatofocusing, and electrophoresis such as isoelectric focusing, alone or in combination.

When the protein is produced as an inclusion body in cells, the cells are similarly recovered and disrupted, followed by centrifugation to obtain a precipitate fraction. After the protein is recovered from the precipitate fraction by an ordinary method, the inclusion body of the protein is solubilized with a protein-denaturing agent.

The solubilized protein solution is diluted with or dialyzed against a solution containing no protein-denaturing agent or a solution containing the protein-denaturing agent at such a low concentration that denaturation of protein is not caused, whereby the protein is renatured to have normal higher-order structure. Then, a purified protein preparation can be obtained by the same isolation and purification steps as described above.

When the protein used in the present invention or its derivative such as a glycosylated form is extracellularly secreted, the protein or its derivative such as a glycosylated form can be recovered in the culture supernatant.

That is, the culture is treated by the same means as above, e.g., centrifugation, to obtain a soluble fraction. A purified protein preparation can be obtained from the soluble fraction by using the same isolation and purification methods as described above.

Examples of the proteins obtained in the above manner are proteins respectively consisting of the amino acid sequences shown in SEQ ID NOS: 1 to 8, 37 and 38.

It is also possible to produce the protein used in the present invention as a fusion protein with another protein and to purify it by affinity chromatography using a substance having affinity for the fused protein.

Examples of the proteins to be fused include β-galactosidase, protein A, immunoglobulin G-binding region of protein A, chloramphenicol acetyltransferase, poly(Arg), poly(Glu), protein G, maltose-binding protein, glutathione S-transferase, polyhistidine chain (His-tag), S peptide, DNA-binding protein domain, Tac antigen, thioredoxin, green fluorescent protein, FLAG peptide and arbitrary antibody epitopes [Akio Yamakawa, Experimental Medicine, 13, 469-474 (1995)].

Examples of the substances having affinity for the above proteins to be fused include antibodies recognizing β-galactosidase, protein A, immunoglobulin G-binding region of protein A, chloramphenicol acetyltransferase, poly(Arg), poly(Glu), protein G, maltose-binding protein, glutathione S-transferase, polyhistidine chain (His-tag), S peptide, DNA-binding protein domain, Tac antigen, thioredoxin, green fluorescent protein, FLAG peptide and arbitrary antibody epitopes, such as immunoglobulin G.

Specifically, when the protein used in the present invention is produced as a fusion protein with protein A, the fusion protein can be purified according to the method of Lowe, et al. [Proc. Natl. Acad. Sci. USA, 86, 8227 (1989); Genes Develop., 4, 1288 (1990)] and the methods described in Japanese Published Unexamined Patent Application No. 336963/93 and WO94/23021. When the protein used in the present invention is produced as a fusion protein with a Flag peptide, the fusion protein can be purified according to the methods described in Proc. Natl. Acad. Sci. USA, 86, 8227 (1989); Genes Develop., 4, 1288 (1990), etc. The protein can also be purified by affinity chromatography using an antibody against said protein.

The protein used in the present invention can also be produced by chemical synthetic methods such as the Fmoc method (the fluorenylmethyloxycarbonyl method) and the tBoc method (the t-butyloxycarbonyl method) based on the amino acid information on the protein obtained above. Further, the protein can be chemically synthesized by using peptide synthesizers from Advanced ChemTech, Perkin-Elmer, Pharmacia, Protein Technology Instrument, Synthecell-Vega, PerSeptive, Shimadzu Corporation, etc.

### 6. Process for Producing Dipeptide Derivatives of the Present Invention

(1) The production process by the enzymatic method
   The production processes of dipeptide derivatives by the enzymatic method include:
   i) a process for producing dipeptide or dipeptide derivative PI, which comprises:
      allowing the protein above 1, one or more kinds of amino acids or amino acid derivatives and ATP to be present in an aqueous mdedium;
      allowing dipeptide or dipeptide dervative PI to form and
      accumulate in the aqueous medium; and
      recovering dipeptide or dipeptide derivative PI from the medium; and
   ii) a process for producing dipeptide or'dipeptide derivative PII, which comprises:
      allowing the protein above 1, one or more kinds of amino acids or amino acid derivatives and ATP to be present in an aqueous medium;
      allowing dipeptide or dipeptide derivative PI to form and
      accumulate in the aqueous medium;
      subjecting the dipeptide or dipeptide derivative PI, as such or after recovery, to modification to form the dipeptide or dipeptide derivative PII; and
      recovering the dipeptide or dipeptide derivative PII.

   Modification of dipeptide or dipeptide derivative PI to form dipeptide or dipeptide'derivative PII can be carried out by known organic synthesis techniques.
   In the process above, as one or more kinds of amino acids or amino acid derivatives, any amino acid and amino acid derivative that can be used as the substrate of the protein of above 1 can be used as a substrate.
   The above amino acids or amino acid derivatives include amino acids or amino acid derivatives represented by formula (I): 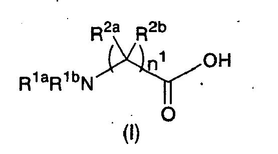 (wherein n¹, R^{1a}, R^{1b}, R^{2a} and R^{2b} have the same significances as defined above) or formula (II): 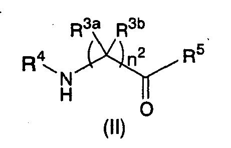 (wherein n², R^{3a}, R^{3b}, R⁴ and R⁵ have the same significances as defined above), provided that when all the amino acids or amino acid derivatives used are amino acids or amino acid derivatives represented by formula (I), at least one of R^{1a} and R^{1b} is a hydrogen atom, and when all the amino acids or amino acid derivatives used are amino acids or amino acid derivatives represented by formula (II), R⁵ is hydroxy. Preferred amino acids or amino acid derivatives are those represented by formula (III): 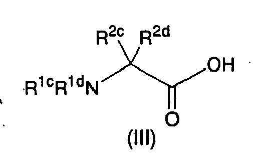 (wherein R^{1c}, R^{1d}, R^{2c} and R^{2d} have the same significances as defined above) or formula (IV): 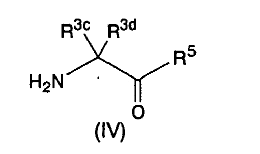 (wherein R^{3c}, R^{3d} and R⁵ have the same significances as defined above), provided that when all the amino acids or amino acid derivatives used are amino acids or amino acid derivatives represented by formula (III), at least one of R^{1c} and R^{1d} is a hydrogen atom, and when all the amino acids or amino acid derivatives used are amino acids or amino acid derivatives represented by formula (IV), R⁵ is hydroxy. More preferred amino acids or amino acid derivatives are those represented by formula (V): 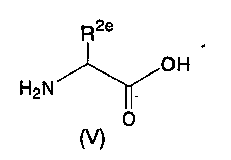 (wherein R^{2e} has the same significance as defined above) or formula (VI): 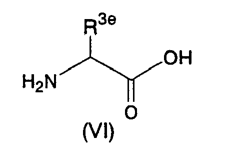 (wherein R^{3e} has the same significance as defined above).
   Examples of dipeptide or dipeptide derivative PI produced by the above process include dipeptides or dipeptide derivatives represented by formula (VIIa): 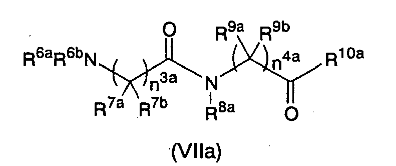 (wherein n^{3a}, n^{4a}, R^{6a}, R^{6b}, R^{7a}, R^{7b}, R^{8a}, R^{9a}, R^{9b} and R^{10a} have the same significances as defined above), preferably, dipeptides or dipeptide derivatives represented by formula (VIIIa): 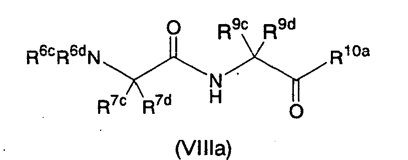 (wherein R^{6c}, R^{6d}, R^{7c}, R^{7d}, R^{9c}, R^{9d} and R^{10a} have the same significances as defined above), more preferably, dipeptides or dipeptide derivatives represented by formula (IXa): 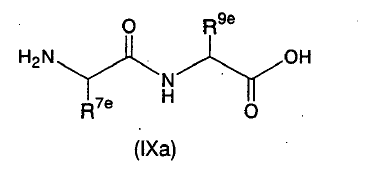 (wherein R^{7e} and R^{9e} have the same significances as defined above).
   Examples of dipeptide or dipeptide derivative PII produced by the above process include dipeptides or dipeptide derivatives represented by formula (VIIb): 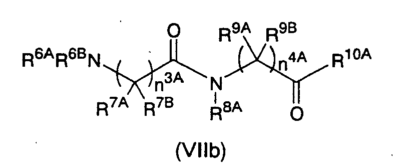 (wherein n^{3A}, n^{4A}, R^{6A}, R^{6B}, R^{7A}, R^{7B}, R^{8A}, R^{9A}, R^{9B} and R^{10A} have the same significances as defined above), preferably, dipeptides or dipeptide derivatives represented by formula (VIIIb): 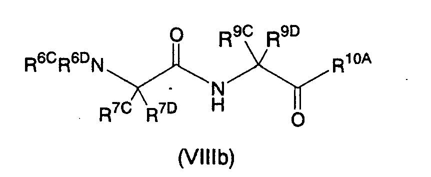 (wherein R^{6C}, R^{6D}, R^{7C}, R^{7D}, R^{9C}, R^{9D} and R^{10A} have the same significances as defined above), more preferably, dipeptides or dipeptide derivatives represented by formula (IXb): 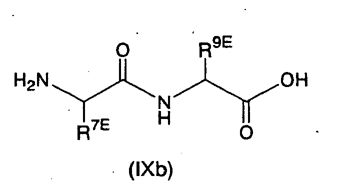 (wherein R^{7E} and R^{9E} have the same significances as defined above). The above dipeptides or dipeptide derivatives do not include compounds in which the same or different amino acids selected from the group consisting of L-alanine, L-glutamine, L-glutamic acid, L-valine, L-leucine, L-isoleucine, L-proline, L-phenylalanine, L-tryptophan, L-methionine, L-serine, L-threonine, L-cysteine, L-asparagine, L-tyrosihe, L-lysine, L-arginine, L-histidine, L-aspartic acid, L-α-aminobutyric acid, L-azaserine, L-theanine, L-4-hydroxyproline, L-3-hydroxyproline, L-ornithine, L-citrulline, L-6-diazo-5-oxo-norleucine, glycine and β-alanine are bound by peptide bond.
   In the definitions of the groups in formulae (I) to (VI), (VIIa), (VIIb), (VIIIa), (VIIIb), (IXa) and (IXb), the lower alkyl moiety of the lower alkyl, the lower alkoxy, the lower alkanoyl, the lower alkoxycarbonyl, the mono(lower alkyl)amino and the di(lower alkyl)amino includes alkyl groups with 1 to 10 carbon atoms having a linear structure, a branched-chain structure, a cyclic structure or a combination thereof. Examples of the linear or branced-chain alkyl groups are methyl, ethyl, n-propyl, isopropyl, n-butyl, isobutyl, sec-butyl, tert-butyl, n-pentyl, neopentyl, n-hexyl, n-heptyl, n-octyl, n-nonyl and n-decyl. Examples of the cyclic alkyl groups are cyclopropyl, cyclobutyl, cyclopentyl, cyclohexyl, cycloheptyl, cyclooctyl, cyclodecyl, noradamantyl, adamantyl, bicyclo[2.2.1]heptyl, bicyclo[2.2.2]octyl, bicyclo[3.3.0]octyl and bicyclo[3.3.1]nonyl. Examples of the alkyl groups having a combination of a linear or branched-chain structure and a cyclic structure are cyclopropylmethyl, cyclopentylmethyl and cyclooctylethyl. Two lower alkyl moieties of the di(lower alkyl)amino may be the same or different.
   The lower alkenyl includes linear or branched-chain alkenyl groups having 2 to 10 carbon atoms, such as vinyl, allyl, 1-propenyl, 1-butenyl, 3-butenyl, 2-pentenyl, 4-pentenyl, 2-hexenyl, 5-hexenyl, 2-decenyl and 9-decenyl.
   The lower alkynyl includes linear or branched-chain alkynyl groups having 2 to 10 carbon atoms, such as ethynyl, 2-propynyl, 3-butynyl, 4-pentynyl, 5-hexynyl and 9-decynyl.
   The aryl moiety of the aryl, the aralkyl and the aroyl includes monocyclic aryl groups and condensed-ring aryl groups in which two or more rings are condensed, specifically, aryl groups having 6 to 14 ring-constituting carbon atoms, such as phenyl, naphthyl, indenyl and anthranyl.
   The alicyclic heterocyclic group includes monocyclic ones and condensed-ring ones in which two or more rings are condensed. Though the kind and number of heteroatoms contained in the alicyclic heterocyclic group are not specifically limited, the alicyclic heterocyclic group may contain, for example, one or more heteroatoms selected from the group consisting of nitrogen atom, sulfur atom and oxygen atom. Specific examples of the alicyclic heterocyclic groups are pyrrolidinyl, 2,5-dioxopyrrolidinyl, thiazolidinyl, oxazolidinyl, piperidyl,
   1,2-dihydropyridyl, piperazinyl, homopiperazinyl, morpholinyl, thiomorpholinyl, pyrazolinyl, oxazolinyl, dioxolanyl, tetrahydropyranyl, tetrahydrothiopyranyl, tetrahydrofuryl, tetrahydroquinolyl, tetrahydroisoquinolyl, tetrahydroquinoxalinyl, octahydroquinolyl, dihydroindolyl and 1,3-dioxoisoindolinyl.
   The heterocyclic group moiety of the heterocyclic alkyl includes, for example, aromatic heterocyclic groups and alicyclic heterocyclic groups. The aromatic heterocyclic groups include monocyclic ones and condensed-ring ones in which two or more rings are condensed. Though the kind and number of heteroatoms contained in the aromatic heterocyclic group are not specifically limited, the aromatic heterocyclic group may contain, for example, one or more heteroatoms selected from the group consisting of nitrogen atom, sulfur atom and oxygen atom. Specific examples of the aromatic heterocyclic groups are those having 5 to 14 ring-constituting atoms, such as furyl, thienyl, pyrrolyl, imidazolyl, pyrazolyl, triazolyl,' tetrazolyl, oxazolyl, oxadiazolyl, thiazolyl, pyridyl, pyrazinyl, pyrimidinyl, pyridazinyl, triazinyl, indolyl, indazolyl, benzimidazolyl, benzoxazolyl, benzothiazolyl, quinolyl, isoquinolyl, phthalazinyl, naphthyridinyl, quinoxalinyl, quinazolinyl, cinnolinyl, purinyl and coumarinyl. The alicyclic heterocyclic group has the same significance as defined above.
   The alkylene moiety of the aralkyl and the heterocyclic alkyl has the same significance as the linear or branched-chain alkyl among the above-described lower alkyl groups except one hydrogen atom is removed therefrom.
   The heterocyclic group formed together with the adjacent nitrogen atom and the carbon atom adjacent to the nitrogen atom and the heterocyclic group formed together with the adjacent carbon atom and the nitrogen atom adjacent to the carbon atom include 5- or 6-membered monocyclic alicyclic heterocyclic groups containing at least one nitrogen atom (the monocyclic alicyclic heterocyclic groups may also contain another nitrogen atom, oxygen atom or sulfur atom), and bicyclic or tricyclic condensed-ring heterocyclic groups containing at least one nitrogen atom in which 3- to 8-membered rings are condensed (the condensed-ring heterocyclic groups may also contain another nitrogen atom, oxygen atom or sulfur atom), specifically, pyrrolidinyl, piperidyl, piperazinyl, morpholinyl, thiomorpholinyl, homopiperidinyl, homopiperazinyl, tetrahydropyridyl, tetrahydroquinolyl and tetrahydroisoquinolyl.
   The substituted lower alkyl, the substituted lower alkenyl, the substituted lower alkynyl, the substituted lower alkoxy, the substituted lower alkanoyl, the substituted lower alkoxycarbonyl, the substituted aralkyl, the substituted aryl, the substituted aroyl, the substituted heterocyclic alkyl, the mono(substituted lower alkyl)amino, the lower alkyl(substituted lower alkyl)amino, the di(substituted lower alkyl)amino, the substituted heterocyclic group formed together with the adjacent nitrogen atom and the carbon atom adjacent to the nitrogen atom, and the substituted heterocyclic group formed together with the adjacent carbon atom and the nitrogen atom adjacent to the carbon atom each have one to a substitutable number, preferably 1 to 3 substituents which are the same or different. Examples of the substituents include halogen, amino, nitro, hydroxy, mercapto, guanidino, ureido, cyano, formyl, carboxyl, aminocarbonyl, diazoacetyl, lower alkyl, lower alkoxy, lower alkanoyl, lower alkoxycarbonyl, mono or di(lower alkyl)aminocarbonyl, lower alkylthio, aryl, aralkyl, aroyl and heterocyclic carbonyl. The lower alkyl moiety of the lower alkyl, the lower alkoxy, the lower alkanoyl and the lower alkoxycarbonyl, the aryl moiety of the aryl, the aralkyl and the aroyl, and the alkylene moiety of the aralkyl have the same significances as defined above, respectively. The lower alkyl moiety of the mono or di(lower alkyl)aminocarbonyl and the lower alkylthio has the same significance as the above lower alkyl, and the heterocyclic group moiety of the heterocyclic carbonyl has the same significance as the heterocyclic group moiety of the above heterocyclic alkyl. The halogen includes fluorine, chlorine, bromine and iodine atoms. Two lower alkyl moieties of the di(lower alkyl)aminocarbonyl may be the same or different.
   Preferred amino acids or amid acid derivatives above include those selected from the group consisting of L-amino acids, glycine (Gly), β-alanine (βAla) and their derivatives. Examples of L-amino acids are L-alanine (L-Ala), L-glutamine (L-Gln), L-glutamic acid (L-Glu), L-valine (L-Val), L-leucine (L-Leu), L-isoleucine (L-Ile), L-proline (L-Pro), L-phenylalanine (L-Phe), L-tryptophan (L-Trp), L-methionine (L-Met), L-serine (L-Ser), L-threonine (L-Thr), L-cysteine (L-Cys), L-asparagine (L-Asn), L-tyrosine (L-Tyr), L-lysine (L-Lys), L-arginine (L-Arg), L-histidine (L-His), L-aspartic acid (L-Asp), L-α-aminobutyric acid (L-α-AB), L-azaserine, L-theanine, L-4-hydroxyproline (L-4-HYP), L-3-hydroxyproline (L-3-HYP), L-ornithine (L-Orn), L-citrulline (L-Cit) and L-6-diazo-5-oxo-norleucine.
   Combinations of amino acids or amino acid derivatives used in the above production processes include the following: a combination of one kind of amino acid selected from the group consisting of L-Ala, Gly, L-Met, L-Ser, L-Thr and β-Ala, or its derivative, and one kind of amino acid selected from the group consisting of L-Ala, L-Gln, L-Glu, Gly, L-Val, L-Leu, L-Ile, L-Pro, L-Phe, L-Trp, L-Met, L-Ser, L-Thr, L-Cys, L-Asn, L-Tyr, L-Lys, L-Arg, L-His, L-Asp, L-α-AB, β-Ala, L-azaserine, L-theanine, L-4-HYP, L-3-HYP, L-Orn, L-Cit and L-6-diazo-5-oxo-norleucine, or its derivative; a combination of L-Gln or its derivative and L-Phe or its derivative; and a combination of L-α-AB or its derivative, and one kind of amino acid selected from the group consisting of L-Gln, L-Arg and L-α-AB, or its derivative. Further preferred combinations are: a combination of L-Ala or its derivative, and one kind of amino acid selected from the group consisting of L-Gln, Gly, L-Val, L-Leu, L-Ile, L-Phe, L-Trp, L-Met, L-Ser, L-Thr, L-Cys, L-Asn, L-Tyr, L-Lys, L-Arg, L-His, L-α-AB, L-azaserine, L-Cit and L-theanine, or its derivative; a combination of Gly or its derivative, and one kind of amino acid selected from the group consisting of L-Gln, Gly, L-Phe, L-Trp, L-Met, L-Ser, L-Thr, L-Cys, L-Tyr, L-Lys, L-Arg, L-α-AB and L-Cit, or its derivative; a combination of L-Met or its derivative, and one kind of amino acid selected from the group consisting of L-Phe, L-Met, L-Ser, L-Thr, L-Cys, L-Tyr, L-Lys and L-His, or its derivative; a combination of L-Ser or its derivative, and one kind of amino acid selected from the group consisting of L-Gln, L-Phe, L-Ser, L-Thr, L-Tyr, L-His and L-α-AB, or its derivative; a combination of L-Thr or its derivative, and one kind of amino acid selected from the group consisting of L-Gln, L-Phe, L-Leu, L-Thr and L-α-AB, or its derivative; a combination of L-Gln or its derivative and L-Phe or its derivative; a combination of β-Ala or its derivative, and one kind of amino acid selected from the group consisting of L-Phe, L-Met, L-His and L-Cit, or its derivative; and a combination of L-α-AB or its derivative, and one kind of amino acid selected from the group consisting of L-Gln, L-Arg and L-α-AB, or its derivative.
   The amount of the protein used in the present invention in the aqueous medium is 0.01 to 100 mg, preferably 0.1 to 10 mg per mg of amino acid or amino acid derivative used as a substrate.
   In the above production processes, the amino acids or amino acid derivatives used as substrates are added to the aqueous medium at the start or in the course of reaction to give a concentration of 0.1 to 500 g/l, preferably 0.2 to 200 g/l.
   In the above production processes, ATP used as energy sourece is added to the aqueous medium at the concentration of 0.5 mmol/l to 10 mol/l.
   The aqueous medium used in the above production processes may comprise any components and may have any composition so far as the dipeptide- or dipeptide derivative-forming reaction is not inhibited. Suitable aqueous media include water and buffers such as phosphate buffer, carbonate buffer, acetate buffer, borate buffer, citrate buffer and Tris buffer. The aqueous medium may comprise alcohols such as methanol and ethanol, esters such as ethyl acetate, ketones such as acetone, and amides such as acetamide.
   The dipeptide-forming reaction is carried out in the aqueous medium at pH 5 to 11, preferably pH 6 to 10, at 20 to 50°C, preferably 25 to 45°C, for 2 to 150 hours, preferably 6 to 120 hours.
(2)The production process using the culture of the cells or the treated matters of the culture as an enzyme source
   The production processes of dipeptides using the culture of the cells or the treated matters of the culture as an enzyme source include:
   i) the process for the production of a dipeptide or a dipeptide derivative PI which comprise:
      using the culture of the cells or the treated matters of the culture obtained by the method of above 4 as an enzyme source;
      allowing the enzyme source and one or more kinds of amino acids of amino acid derivatives to be present in an aqueous medium;
      allowing the dipeptide or dipeptide derivative PI to form and accumulate in the aqueous medium; and
      recovering the dipeptide or dipeptide derivative PI from the medium; and
   ii) the process for the production of dipeptides or dipeptide derivatives which comprises:
      using the culture of cells or the treated matters of the culture as an enzyme source;
      allowing the enzyme source and one or more kinds of amino acids or amino acid derivatives to be present in an aqueous medium;
      allowing the dipeptide or dipeptide derivative PI to form and accumulate in the medium;
      subjecting the dipeptide or dipeptide derivative PI, as such or after recovery, to modification to form dipeptide or dipeptide derivative PII; and
      recovering the dipeptide or dipeptide derivative PII.

   Modification of dipeptide or dipeptide derivative PI to form dipeptide or dipeptide derivative PII can be carried out by known organic synthesis techniques.
   In the production process above, species of amino acids and amino acid derivatives used as substrates, the concentration and the time of addition of the substrate and dipeptide which can produced are the same as the enzymatic method above 6(1).
   The treated matters of the culture include treated matters which are selected from the group consisting of concentrated culture, dried culture, cells obtained by centrifuging the culture, dried cells, freeze-dried cells, surfactant-treated cells, ultrasonicated cells, mechanically-disrupted cells, solvent-treated cells, enzymatic-treated cells, protein fractionation of cells, immobilized cells, and an enzyme preparation obtained by extracting the cells, and which have the activity to form dipeptides or dipeptide derivatives from one or more kinds of amino acids or amino acid derivatives.
   The culture of cells used as an enzyme source also used as an aqueous medium in addition to the aqueous medium used in the enzymatic method above 6(1) in the production process using the culture of the cells or the treated matters of the culture as an enzyme source.
   In the above production processes, as sources of ATP, ATP or substances from which cells can produce ATP by the metabolism, for example, sugars such as glucose, alcohols such as ethanol, and organic acids such as acetic acid, can be used instead of ATP according to need.
   Further, a surfactant or an organic solvent may be added to the aqueous medium according to need. Suitable surfactants include nonionic surfactants such as polyoxyethylene octadecylamine (e.g., Nymeen S-215, NOF Corporation), cationic surfactants such as cetyltrimethylammonium bromide and alkyldimethylbenzylammonium chloride (e.g., Cation F2-40E, NOF Corporation), anionic surfactants such as lauroyl sarcosinate, and tertiary amines such as alkyldimethylamine (e.g., Tertiary Amine FB, NOF Corporation). Suitable organic solvents include xylene, toluene, aliphatic alcohols, acetone and ethyl acetate. Any surfactants and organic solvents may be used alone or in combination provided that the cells used in the production processes of the present invention have the activity to form dipeptide or dipeptide derivative PI from amino acids or amino acid derivatives. The kind and concentration of the surfactant and organic solvent can be arbitrarily selected so long as the cells used in the present invention have the above activity. For example, the surfactant is usually used at a concentration of 0.1 to 50 g/l, and the organic solvent is usually used at a concentration of 0.1 to 50 ml/l.
   When the culture of cells or a treated matter of the culture is used as the enzyme source, the amount of the enzyme source to be added varies according to its specific activity, etc., but is, for example, 5 to 1000 mg (wet cell weight), preferably 10 to 400 mg per mg of amino acid or amino acid derivative used as a substrate.
   The dipeptide- or dipeptide derivative-forming reaction is carried out in the aqueous medium at pH 5 to 11, preferably pH 6 to 10, at 20 to 65°C, preferably 25 to 55° C, more preferably 30 to 45°C, usually for 1 minute to 150 hours, preferably 3 minutes to 120 hours, more preferably 30 minutes to 100 hours.
   In the above production processes of 6(1) or (2), recovery of the dipeptide or dipeptide derivative formed and accumulated in the aqueous medium can be carried out by ordinary methods using active carbon, ion-exchange resins, etc. or by means such as extraction with an organic solvent, crystallization, thin layer chromatography and high performance liquid chromatography.
(3)The production process for dipeptide derivative by modifying dipeptide or dipeptide derivative
   Various dipeptide derivatives can be obtained by subjecting the dipeptide or dipeptide derivative obtained in the above 6(1) or (2) to known organic synthesis method [see Comprehensive Organic Transformations, R. C. Larock, 1989, for example] and the like.
   The product of the production process above can be isolated or purified by the method of the usual organic synthesis like filtration, extraction, washing, drying, condensation, cystalization, various chromatography or the appropriate combination thereof.
   The methods for preparing the DNA, protein and cells used in the present invention are illustrated in the following experimental examples, but the preparation methods are not limited to the following experimental examples.

### Experimental Example 1

### Search for a Protein Having the Dipeptide-Synthesizing Activity Utilizing a Database

By using, as a query, the amino acid sequence of D-Ala-D-Ala ligase gene derived from Bacillus subtilis 168 [Nature, 390, 249-256 (1997)], a search for a gene encoding a protein having homology which is present in the genomic DNA sequences of Bacillus subtilis 168 was carried out using the homology search function of Subtilist (http://genolist.pasteur.fr/SubtiList/) which is a database of the genomic DNA of Bacillus subtilis 168.

From the sequences obtained as a result of the search, genes encoding the amino acid sequences shown in SEQ ID NOS: 33, 34 and 35 which are D-Ala-D-Ala ligase motifs [Biochemistry, 30, 1673 (1991)] and encoding proteins whose function had already been clarified were excluded. Of the remaining sequences, the sequence showing the highest homology (29.1%) to the D-Ala-D-Ala ligase motif was selected as a gene of unknown function ywfE.

The nucleotide sequence of ywfE is shown in SEQ ID NO: 9, and the amino acid sequence of the protein encoded by the nucleotide sequence is shown in SEQ ID NO: 1.

### Experimental Example 2

### Construction of a Strain Expressing ywfE Gene

On the basis of the information on the nucleotide sequence obtained in Experimental Example 1, a ywfE gene fragment of Bacillus subtilis was obtained in the following manner.

That is, Bacillus subtilis 168 (ATCC 23857) was inoculated into LB medium [10 g/l Bacto-tryptone (Difco), 5 g/l yeast extract (Difco) and 5 g/l sodium chloride] and subjected to static culture overnight at 30°C. After the culturing, the chromosomal DNA of the microorganism was isolated and purified according to the method using saturated phenol described in Current Protocols in Molecular Biology.

By using a DNA synthesizer (Model 8905, PerSeptive Biosystems, Inc.), DNAs having the nucleotide sequences shown in SEQ ID NOS: 19 to 22 (hereinafter referred to as primer A, primer B, primer C and primer D, respectively) were synthesized. Primer A has a sequence wherein a nucleotide sequence containing the XhoI recognition sequence is added to the 5' end of a region of the Bacillus subtilis chromosomal DNA containing the initiation codon of ywfE. Primer B has a sequence wherein a nucleotide sequence containing the BamHI recognition sequence is added to the 5' end of a nucleotide sequence complementary to a sequence containing the termination codon of ywfE. Primer C has a sequence wherein a nucleotide sequence containing the EcoRI recognition sequence is added to the 5' end of the nucleotide sequence of trp promoter region of expression vector pTrS30 containing trp promoter [prepared from Escherichia coli JM109/pTrS30 (FERM BP-5407)]. Primer D has a sequence wherein a nucleotide sequence containing the XhoI recognition sequence is added to the 5' end of a sequence complementary to the sequence of trp promoter region of expression vector pTrS30 containing trp promoter.

A ywfE gene fragment was amplified by PCR using the above primer A and primer B, and as a template, the chromosomal DNA of Bacillus subtilis. A trp promoter region fragment was amplified by PCR using primer C and primer D, and as a template, pTrS30. PCR was carried out by 30 cycles, one cycle consisting of reaction at 94°C for one minute, reaction at 55°C for 2 minutes and reaction at 72° C for 3 minutes, using 40 µl of a reaction mixture comprising 0.1 µg of the chromosomal DNA or 10 ng of pTrS30 as a template, 0.5 µmol/l each of the primers, 2.5 units of Pfu DNA polymerase (Stratagene), 4 µl of buffer for Pfu DNA polymerase (10 x) (Stratagene) and 200 µmol/l each of dNTPs (dATP, dGTP, dCTP and dTTP).

One-tenth of each of the resulting reaction mixtures was subjected to agarose gel electrophoresis to confirm that a ca. 1.4 kb DNA fragment corresponding to the ywfE gene fragment and a ca. 0.3 kb DNA fragment corresponding to the trp promoter region fragment were respectively amplified in the PCR using primer A and primer B and the PCR using primer C and primer D. Then, the remaining reaction mixture was mixed with an equal amount of phenol/chloroform (1 vol/l vol) saturated with TE [10 mmol/l Tris-HCl (pH 8.0), 1 mmol/l EDTA]. The resulting solution was centrifuged, and the obtained upper layer was mixed with a two-fold volume of cold ethanol and allowed to stand at -80°C for 30 minutes. The resulting solution was centrifuged to precipitate DNA, and the obtained DNA was dissolved in 20 µl of TE.

The thus obtained solutions (5 µl each) were respectively subjected to reaction to cleave the DNA amplified using primer A and primer B with restriction enzymes XhoI and BamHI and to reaction to cleave the DNA amplified using primer C and primer D with restriction enzymes EcoRI and XhoI. DNA fragments were separated by agarose gel electrophoresis, and a 1.4 kb fragment containing ywfE and a 0.3 kb fragment containing trp promoter region were respectively recovered using GENECLEAN II Kit (BIO 101).

pTrS30 [a trp promoter-containing expression vector prepared from Escherichia coli JM109/pTrS30 (FERM BP-5407), 0.2 µg] was cleaved with restriction enzymes EcoRI and BamHI. DNA fragments were separated by agarose gel electrophoresis and a 4.5 kb DNA fragment was recovered in the same manner as above.

The 1.4 kb fragment containing ywfE, the 0.3 kb fragment containing trp promoter region and the 4.5 kb DNA fragment obtained above were subjected to ligation reaction using a ligation kit (Takara Bio Inc.) at 16°C for 16 hours.

Escherichia coli NM522 (Stratagene) was transformed using the reaction mixture according to the method using calcium ion [Proc. Natl. Acad. Sci. USA, 69, 2110 (1972)], spread on LB agar medium containing 50 µg/ml ampicillin, and cultured overnight at 30°C..

A plasmid was extracted from a colony of the transformant that grew on the medium according to a known method and the structure of the plasmid was analyzed using restriction enzymes, whereby it was confirmed that expression vector pPE43 containing ywfE ligated downstream of the trp promoter was obtained (Fig. 1).

### Experimental Example 3

### Production of a Dipeptide

Escherichia coli NM522 carrying pPE43 (Escherichia. coli NM522/pPE43) obtained in Experimental Example 2 was inoculated into 8 ml of LB medium containing 50 µg/ml ampicillin in a test tube, and cultured at 28°C for 17 hours. The resulting culture was centrifuged to obtain wet cells.

A reaction mixture (0.1 ml) comprising 60 mg/ml (final concentration) wet cells, 120 mmol/l potassium phosphate buffer (pH 7.4), 60 mmol/l magnesium chloride, 60 mmol/l ATP, 30 mmol/l L-Ala, 30 mmol/l L-Gln and 0.4% Nymeen S-215 was prepared, and reaction was carried out at 37°C for 3 minutes.

After the completion of reaction, the reaction product was derivatized by the dinitrophenol method and then analyzed by HPLC. The HPLC analysis was carried out using, as a separation column, Lichrosorb-RP-18 column (Kanto Kagaku) and, as an eluent, 1% (v/v) phosphoric acid and 25% (v/v) acetonitrile at a flow rate of 0.7 ml/min. As a result, it was confirmed that 120 mg/l L-alanyl-L-glutamine (L-Ala-L-Gln) was formed and accumulated in the reaction mixture.

Formation of L-Ala-L-Gln was not observed when the reaction was carried out using cells of Escherichia coli NM522/pTrS31, which is a control strain carrying only a vector.

### Experimental Example 4

### Purification of C-Terminal His-Tagged Recombinant Dipeptide Synthetase

By using the above DNA synthesizer, DNAs having the nucleotide sequences shown in SEQ ID NOS: 23 and 24 (hereinafter referred to as primer E and primer F, respectively) were synthesized. Primer E has a nucleotide sequence containing a region wherein the initiation codon of ywfE (atg) is substituted by the NcoI recognition sequence (ccatgg). Primer F has a nucleotide sequence containing a region wherein the termination codon of ywfE is substituted by the BamHI recognition sequence (ggatcc).

PCR was carried out using the chromosomal DNA of Bacillus subtilis 168 (ATCC 23857) as a template and the above primer E and primer F as a set of primers. That is, PCR was carried out by 30 cycles, one cycle consisting of reaction at 94°C for one minute, reaction at 55°C for 2 minutes and reaction at 72°C for 3 minutes, using 40 µl of a reaction mixture comprising 0.1 µg of the chromosomal DNA, 0.5 µmol/l each of the primers, 2.5 units of Pfu DNA polymerase, 4 µl of buffer for Pfu DNA polymerase (10 x) and 200 µmol/l each of dNTPs.

One-tenth of the resulting reaction mixture was subjected to agarose gel electrophoresis to confirm that a ca. 1.4 kb fragment corresponding to the ywfE fragment was amplified. Then, the remaining reaction mixture was mixed with an equal amount of phenol/chloroform saturated with TE. The resulting solution was centrifuged, and the obtained upper layer was mixed with a two-fold volume of cold ethanol and allowed to stand at -80°C for 30 minutes. The resulting solution was centrifuged, and the obtained DNA precipitate was dissolved in 20 µl of TE.

The thus obtained solution (5 µl) was subjected to reaction to cleave the amplified DNA with restriction enzymes NcoI and BamHI. DNA fragments were separated by agarose gel electrophoresis, and a 1.4 kb DNA fragment containing ywfE was recovered using GENECLEAN II Kit.

C-Terminal His-tagged recombinant expression vector pQE60 (Qiagen, Inc.) (0.2 g) was cleaved with restriction enzymes NcoI and BamHI. DNA fragments were separated by agarose gel electrophoresis, and a 3.4 kb DNA fragment was recovered in the same manner as above.

The 1.4 kb DNA fragment containing ywfE and the 3.4 kb DNA fragment obtained above were subjected to ligation reaction using a ligation kit at 16°C for 16 hours.

Escherichia coli NM522 was transformed using the ligation reaction mixture according to the method using calcium ion, spread on LB agar medium containing 50 µg/ml ampicillin, and cultured overnight at 30°C.

A plasmid was extracted from a colony of the transformant that grew on the medium according to a known method and the structure of the plasmid was analyzed using restriction enzymes, whereby it was confirmed that pQE60ywfE, which is a C-terminal His-tagged ywfE expression vector, was obtained (Fig. 2).

Escherichia coli NM522 carrying pQE60ywfE (Escherichia coli NM522/pQE60ywfE) was inoculated into 8 ml of LB medium containing 50 µg/ml ampicillin in a test tube, and cultured at 28°C for 17 hours. The resulting culture was inoculated into 50 ml of LB medium containing 50 *µ*g/ml ampicillin in a 250-ml Erlenmeyer flask, and cultured at 30° C for 3 hours. Then, isopropyl- β -D-thiogalactopyranoside (IPTG) was added to give a final concentration of 1 mmol/l, followed by further culturing at 30°C for 4 hours. The resulting culture was centrifuged to obtain wet cells, and a His-tagged recombinant enzyme was purified from the wet cells using HisTrap (His-tagged protein purification kit, Amersham Pharmacia Biotech) according to the instructions attached thereto.

### Experimental Example 5

### Production of Dipeptides Using the His-Tagged Recombinant Enzyme (1)

(i) A reaction mixture (0.1 ml) comprising 0.04 mg of the purified His-tagged recombinant enzyme obtained in Experimental Example 4, 100 mmol/l Tris-HCl (pH 8.0), 60 mmol/l magnesium chloride, 60 mmol/l ATP, 30 mmol/l L-Ala and 30 mmol/l L-Gln was prepared, and reaction was carried out at 37°C for 16 hours.
   After the completion of reaction, the reaction product was analyzed in the same manner as in Experimental Example 3 above, whereby it was confirmed that 3.7 g/l L-Ala-L-Gln and 0.3 g/l L-alanyl-L-alanine (L-Ala-L-Ala) were formed and accumulated in the reaction mixture.
(ii) Reactions were carried out under the same conditions as in the above (i) using reaction mixtures having the same composition as that of the reaction mixture of the above (i) except that 0.01 mg of the enzyme was used and L-Phe, L-Met, L-Leu and L-Val, respectively, were used in place of L-Gln.
   After the completion of reactions, the reaction products were analyzed in the same manner as in Experimental Example 3 above, whereby it was confirmed that the following dipeptides were formed and accumulated in the respective reaction mixtures: 7.0 g/l L-alanyl-L-phenylalanine (L-Ala-L-Phe) alone; 7.0 g/l L-alanyl-L-methionine (L-Ala-L-Met) and 0.03 g/l L-Ala-L-Ala; 5.0 g/l L-alanyl-L-leucine (L-Ala-L-Leu) and 0.2 g/l L-Ala-L-Ala; and 1.6 g/l L-alanyl-L-valine (L-Ala-L-Val) and 0.3 g/l L-Ala-L-Ala.
(iii) Reactions were carried out under the same conditions as in the above (i) using reaction mixtures having the same composition as that of the reaction mixture of the above (i) except that 0.01 mg of the enzyme was used, Gly was used in place of L-Ala, and L-Phe and L-Met, respectively, were used in place of L-Gln.
   After the completion of reactions, the reaction products were analyzed in the same manner as in Experimental Example 3 above, whereby it was confirmed that 5.2 g/l glycyl-L-phenylalanine (Gly-L-Phe) and 1.1 g/l glycyl-L-methionine (Gly-L-Met) were formed and accumulated in the respective reaction mixtures.
   When ATP was excluded from the compositions of the above reaction mixtures, no dipeptide was formed.
   The above results revealed that the ywfE gene product has the activity to produce, in the presence of ATP, the following dipeptides: L-Ala-L-Gln plus L-Ala-L-Ala, L-Ala-L-Phe, L-Ala-L-Met plus L-Ala-L-Ala, L-Ala-L-Leu plus L-Ala-L-Ala, or L-Ala-L-Val plus L-Ala-L-Ala from L-Ala plus L-Gln, L-Phe, L-Met, L-Leu or L-Val; and Gly-L-Phe or Gly-L-Met from Gly plus L-Phe or L-Met.

### Experimental Example 6

### Production of Dipeptides Using the His-Tagged Recombinant Enzyme (2)

A reaction mixture (0.1 ml) comprising 0.04 mg of the purified His-tagged recombinant enzyme obtained in Experimental Example 4, 100 mmol/l Tris-HCl (pH 8.0), 60 mmol/l magnesium chloride and 60 mmol/l ATP was prepared. To this mixture were respectively added combinations of various L-amino acids, Gly and β-Ala selected from the amino acids shown in the first row of Table 1 and in the leftmost column of Table 1 to give a concentration of 30 mmol/l each, and the resulting mixtures were subjected to reaction at 37°C for 16 hours. After the completion of reactions, the reaction products were analyzed by HPLC, whereby it was confirmed that the dipeptides shown in Table 1 were formed.

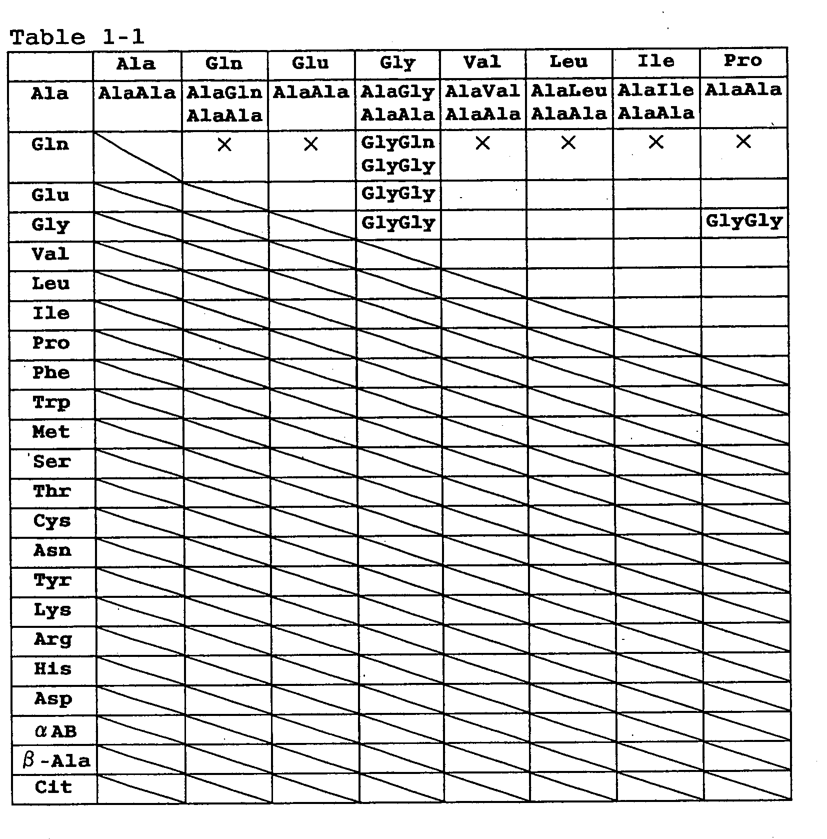

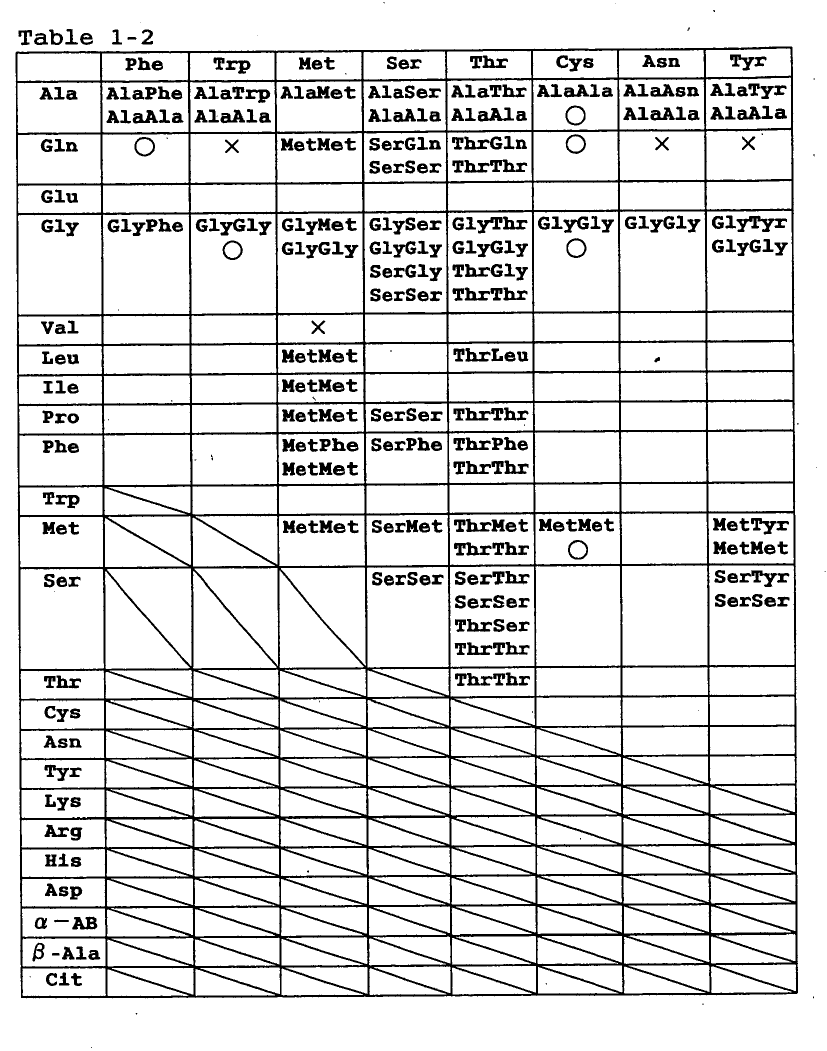

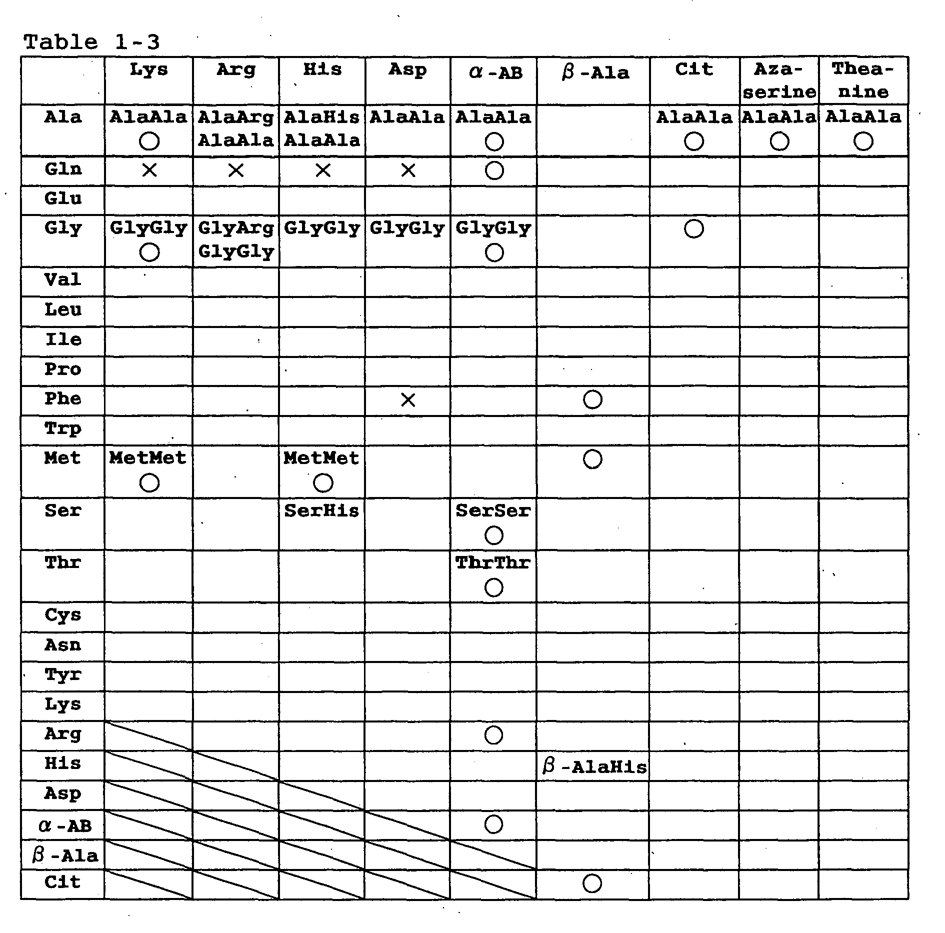

The dipeptides formed by the reaction using, as substrates, two (or one) kinds of L-amino acids, Gly and β-Ala shown in the first row and the leftmost column of Table 1 are shown in the respective cells of the table. In the table, ○ means that a dipeptide was formed though its sequence was unidentified; × means that formation of a dipeptide was not confirmed; and a blank means that reaction was not carried out.

### Experimental Example 7

### Production of a Dipeptide Using the Strain Expressing the His-Tagged Recombinant Enzyme

Escherichia coli NM522/pQE60ywfE obtained in Experimental Example 4 was inoculated into 8 ml of LB medium containing 50 µg/ml ampicillin in a test tube, and cultured at 28°C for 17 hours. The resulting culture was inoculated into 50 ml of LB medium containing 50 *µ*g/ml ampicillin in a 250-ml Erlenmeyer flask, and cultured at 30°C for 3 hours. Then, IPTG was added to give a final concentration of 1 mmol/l, followed by further culturing at 30°C for 4 hours. The resulting culture was centrifuged to obtain wet cells.

A reaction mixture (20 ml, pH 7.2) comprising 200 g/l wet cells, 50 g/l glucose, 5 g/l phytic acid '(diluted to neutrality with 33% conc. sodium hydroxide solution), 15 g/l potassium dihydrogenphosphate, 5 g/l magnesium sulfate heptahydrate, 4 g/l Nymeen S-215, 10 ml/l xylene, 200 mmol/l L-Ala and 200 mmol/l L-Gln was put in a 50-ml beaker, and reaction was carried out at 32°C at 900 rpm for 2 hours. During the reaction, the pH of the reaction mixture was maintained at 7.2 by using 2 mol/l potassium hydroxide.

The reaction product was analyzed by the same method as in Experimental Example 3, whereby it was confirmed that 25 mg/l L-Ala-L-Gln was accumulated.

### Experimental Example 8

### Cloning of Genes Corresponding to the ywfE Gene from Various Microorganisms of the Genus Bacillus and Analysis Thereof

On the basis of the nucleotide sequence shown in SEQ ID NO: 9, genes corresponding to the ywfE gene which exist in Bacillus subtilis ATCC 15245, ATCC 6633, IAM 1213, IAM 1107, IAM 1214, ATCC 9466, IAM 1033 and ATCC 21555, Bacillus amyloliquefaciens IFO 3022 and Bacillus pumilus NRRL B-12025 were obtained in the following manner.

That is, Bacillus subtilis ATCC 15245, ATCC 6633, IAM 1213, IAM 1107, IAM 1214, ATCC 9466, IAM 1033 and ATCC 21555, Bacillus amyloliquefaciens IFO 3022 and Bacillus pumilus NRRL B-12025 were respectively inoculated into LB medium and subjected to static culture overnight at 30°C. After the culturing, the chromosomal DNAs of the respective microorganisms were isolated and purified according to the method using saturated phenol described in Current Protocols in Molecular Biology.

By using a DNA synthesizer (Model 8905, PerSeptive Biosystems, Inc.), DNAs having the nucleotide sequences shown in SEQ ID NOS: 25 and 26 (hereinafter referred to as primer G and primer H, respectively) were synthesized. Primer G has a sequence containing a region upstream of the initiation codon of ywfE on the chromosomal DNA of Bacillus subtilis 168, and primer H has a sequence complementary to a sequence containing a region downstream of the termination codon of ywfE .

PCR was carried out using each of the chromosomal DNAs of Bacillus subtilis ATCC 15245, ATCC 6633, IAM 1213, IAM 1107, IAM 1214, ATCC 9466, IAM 1033 and ATCC 21555 and Bacillus amyloliquefaciens IFO 3022 as a template and the above primer G and primer H as a set of primers. That is, PCR was carried out by 30 cycles, one cycle consisting of reaction at 94°C for one minute, reaction at 55°C for 2 minutes and reaction at 72°C for 3 minutes, using 40 *µ*l of a reaction mixture comprising 0.1 *µ*g of the chromosomal DNA, 0.5 *µ* mol/l each of the primers, 2.5 units of Pfu DNA polymerase, 4 *µ*l of buffer for Pfu DNA polymerase (10 x) and 200 *µ*mol/l each of dNTPs.

One-tenth of each of the resulting reaction mixtures was subjected to agarose gel electrophoresis to confirm that a ca. 1.6 kb fragment corresponding to the ywfE fragment was amplified. Then, the remaining reaction mixture was mixed with an equal amount of phenol/chloroform saturated with TE. The resulting solution was centrifuged, and the obtained upper layer was mixed with a two-fold volume of cold ethanol and allowed to stand at -80°C for 30 minutes. The resulting solution was centrifuged, and the obtained DNA precipitate was dissolved in 20 µl of TE.

Each of the thus obtained 1.4 kb DNA fragments derived from the chromosomal DNAs of the respective strains and pCR-blunt (Invitrogen Corp.) were subjected to ligation reaction using a ligation kit at 16°C for 16 hours.

Escherichia coli NM522 was transformed using each ligation reaction mixture according to the method using calcium ion, spread on LB agar medium containing 50 µg/ml ampicillin, and cultured overnight at 30°C.

A plasmid was extracted from a colony of each transformant that grew on the medium according to a known method and the structure of each plasmid was analyzed using restriction enzymes. As a result, it was confirmed that the following plasmids containing a gene corresponding to the ywfE gene were obtained: pYWFE1 (derived from ATCC 15245, DNA having the nucleotide sequence shown in SEQ ID NO: 36), pYWFE2 (derived from ATCC 6633, DNA having the nucleotide sequence shown in SEQ ID NO: 10), pYWFE3 (derived from IAM 1213, DNA having the nucleotide sequence shown in SEQ ID NO: 11), pYWFE4 (derived from IAM 1107, DNA having the nucleotide sequence shown in SEQ ID NO: 12), pYWFE5 (derived from IAM 1214, DNA having the nucleotide sequence shown in SEQ ID NO: 13), pYWFE6 (derived from ATCC 9466, DNA having the nucleotide sequence shown in SEQ ID NO: 9), pYWFE7 (derived from IAM 1033, DNA having the nucleotide sequence shown in SEQ ID NO: 36), pYWFE8 (derived from ATCC 21555, DNA having the nucleotide sequence shown in SEQ ID NO: 14) and pYWFE9 (derived from IFO 3022, DNA having the nucleotide sequence shown in SEQ ID NO: 15).

On the other hand, a gene corresponding to ywfE derived from Bacillus pumilus NRRL B-12025 (DNA having the nucleotide sequence shown in SEQ ID NO: 16) was obtained in the following manner.

PCR was carried out using the chromosomal DNA of the NRRL B-12025 strain prepared above as a template and DNAs respectively consisting of the nucleotide sequences shown in SEQ ID NOS: 27 and 28 as a set of primers. That is, PCR was carried out by 30 cycles, one cycle consisting of reaction at 98°C for 5 seconds, reaction at 55° C for 30 seconds and reaction at 72°C for one minute, using 50 µl of a reaction mixture comprising 0.1 µg of the chromosomal DNA, 0.5 µmol/l each of the primers, 2.5 units of Z-taq polymerase (Takara Bio Inc.), 5 µl of buffer for Z-taq polymerase (10 x) (Takara Bio Inc.) and 200 µmol/l each of dNTPs.

One-tenth of the resulting reaction mixture was subjected to agarose gel electrophoresis to confirm that a ca. 0.8 kb fragment was amplified. Then, the remaining reaction mixture was mixed with an equal amount of phenol/chloroform saturated with TE. The resulting mixture was centrifuged, and the obtained upper layer was mixed with a two-fold volume of cold ethanol and allowed to stand at -80°C for 30 minutes. The resulting solution was centrifuged, and the obtained DNA precipitate was dissolved in 20 µl of TE.

The thus obtained 0.8 kb fragment derived from the chromosomal DNA and pGEM T-easy (Promega Corp.) were subjected to ligation reaction using a ligation kit at 16° C for 16 hours.

Escherichia coli DH5 α was transformed using the reaction mixture according to the method using calcium ion, spread on LB agar medium containing 50 µg/ml ampicillin, and cultured overnight at 30°C.

A plasmid was extracted from the transformant obtained above and the nucleotide sequence of the ca. 0.8 kb DNA insert was determined, whereby a sequence from nucleotides 358 to 1160 in the nucleotide sequence shown in SEQ ID NO: 16 was confirmed.

The above plasmid was cleaved with EcoRI and then subjected to agarose gel electrophoresis to separate a DNA fragment. The DNA fragment was purified using GENECLEAN II Kit, and about 0.5 *µ*g of the purified DNA fragment was DIG-labeled using DIG-High Prime DNA Labeling & Detection Starter Kit I (Roche Diagnostics Corp.) according to the instructions attached thereto.

Southern analysis of the chromosomal DNA of the NRRL B-12025 strain was carried out using the DIG-labeled DNA obtained above.

The chromosomal DNA of the NRRL B-12025 strain was completely digested with BamHI, EcoRI, HindIII, KpnI, PstI, SacI, SalI and SphI, respectively, and subjected to agarose gel electrophoresis to separate DNA fragments, followed by transfer to nylon membrane plus charge (Roche Diagnostics Corp.) according to an ordinary method.

After the DNA fragments were fixed on the nylon membrane by UV irradiation, Southern hybridization was carried out using the above probe DNA and the nylon membrane.

The hybridization was carried out by contacting the nylon membrane with the probe DNA at 65°C for 16 hours, washing the nylon membrane twice with a solution consisting of 0.1% SDS and 2 x SSC at room temperature for 5 minutes, and further washing the membrane twice with a solution consisting of 0.1% SDS and 0.5 x SSC at 65°C for 15 minutes. The other operations and conditions and detection of the hybridized DNA were carried out according to the instructions attached to the above-mentioned DIG-High Prime DNA Labeling & Detection Starter Kit I.

As a result, color development was observed at around 3.5 kbp of the fragments completely digested with HindIII and PstI.

Subsequently, the chromosomal DNA of the NRRL B-12025 strain was completely digested with HindIII and PstI, respectively, and subjected to agarose gel electrophoresis to separate DNA fragments. From the respective restriction enzyme-digested DNAs, 3-4 kbp fragments were purified using GENECLEAN II Kit, followed by autocyclization using a ligation kit.

On the basis of the nucleotide sequence of the 0.8 kb DNA fragment determined above, the nucleotide sequences shown in SEQ ID NOS: 29 and 30 were designed and synthesized, and they were used in PCR using the cyclized DNA obtained above as a template. PCR was carried out by 30 cycles, one cycle consisting of reaction at 98°C for 5 seconds, reaction at 55°C for 30 seconds and reaction at 72°C for 3 minutes and 30 seconds, using 50 *µ*l of a reaction mixture comprising 10 ng of the cyclized DNA, 0.5 µmol/l each of the primers, 2.5 units of pyrobest polymerase (Takara Bio Inc.), 5 µl of buffer for pyrobest polymerase (10 x) (Takara Bio Inc.) and 200 µmol/l each of dNTPs.

One-tenth of the resulting reaction mixture was subjected to agarose gel electrophoresis to confirm that a ca. 3.0 kb fragment was amplified. Then, the remaining reaction mixture was mixed with an equal amount of phenol/chloroform saturated with TE. The resulting mixture was centrifuged, and the obtained upper layer was mixed with a two-fold volume of cold ethanol and allowed to stand at -80°C for 30 minutes. The resulting solution was centrifuged, and the obtained DNA precipitate was dissolved in 20 µl of TE.

The thus obtained DNA fragment and Zero Blunt PCR Cloning Kit (Invitrogen Corp.) were subjected to ligation reaction using a ligation kit.

Escherichia coli NM522 was transformed using the reaction mixture according to the method using calcium ion, spread on LB agar medium containing 50 µg/ml ampicillin, and cultured overnight at 30°C.

A plasmid was extracted from a colony of the transformant that grew on the medium according to a known method and the structure of the plasmid was analyzed using restriction enzymes. As a result, it was confirmed that plasmid pYWFE10 (derived from NRRL B-12025, DNA having the nucleotide sequence shown in SEQ ID NO: 16) containing a gene corresponding to the ywfE gene was obtained.

The nucleotide sequences of the genes corresponding to the ywfE gene which are respectively contained in the plasmids pYWFE1 to pYWFE10 obtained above were determined using 373A DNA Sequencer.

The amino acid sequences of the proteins encoded by the genes respectively contained in pYWFE1, pYWFE6 and pYWFE7 were identical with the amino acid sequence of the protein encoded by the ywfE gene, whereas those of the proteins encoded by the genes respectively contained in pYWFE2, pYWFE3, pYWFE4, pYWFE5, pYWFE8, pYWFE9 and pYWFE10 were different from the amino acid sequence of the protein encoded by the ywfE gene.

The amino acid sequences of the proteins encoded by the genes corresponding to the ywfE gene which are contained in pYWFE2, pYWFE3, pYWFE4, pYWFE5, pYWFE8, pYWFE9 and pYWFE10, and pYWFE1 and pYWFE7 are shown in SEQ ID NOS: 2 to 8 and 1, respectively, and the nucleotide sequences of these genes are shown in SEQ ID NOS: 10 to 16 and 36, respectively.

### Experimental Example 9

### Purification of C-Terminal His-Tagged Recombinant Dipeptide Synthetase

PCR was carried out using each of the chromosomal DNAs of Bacillus subtilis ATCC 15245, ATCC 6633, IAM 1213, IAM 1107, IAM 1214, ATCC 9466, IAM 1033 and ATCC 21555 and Bacillus amyloliquefaciens IFO 3022 as a template and primer A and primer B described in Experimental Example 2 as a set of primers. That is, PCR was carried out by 30 cycles, one cycle consisting of reaction at 94°C for one minute, reaction at 55°C for 2 minutes and reaction at 72° C for 3 minutes, using 40 µl of a reaction mixture comprising 0.1 µg of the chromosomal DNA, 0.5 µmol/l each of the primers, 2.5 units of Pfu DNA polymerase, 4 µl of buffer for Pfu DNA polymerase (10 x) and 200 µmol/l each of dNTPs.

When the chromosomal DNA of Bacillus pumilus NRRL B-12025 was used as a template, PCR was carried out using DNAs respectively having the nucleotide sequences shown in SEQ ID NOS: 31 and 32 as a set of primers under the same conditions as above.

One-tenth of each of the resulting reaction mixtures was subjected to agarose gel electrophoresis to confirm that a ca. 1.4 kb DNA fragment corresponding to the ywfE fragment was amplified. Then, the remaining reaction mixture was mixed with an equal amount of phenol/chloroform saturated with TE. The resulting mixture was centrifuged, and the obtained upper layer was mixed with a two-fold volume of cold ethanol and allowed to stand at -80°C for 30 minutes. The resulting solution was centrifuged, and the obtained DNA precipitate was dissolved in 20 µl of TE.

Each of the thus obtained solutions (5 µl) was subjected to reaction to cleave the amplified DNA with restriction enzymes NcoI and BamHI. DNA fragments were separated by agarose gel electrophoresis, and a 1.4 kb DNA fragment containing a gene corresponding to the ywfE gene was recovered using GENECLEAN II Kit.

Subsequently, 0.2 µg of the C-terminal His-tagged recombinant expression vector pQE60 was cleaved with restriction enzymes NcoI and BamHI. DNA fragments were separated by agarose gel electrophoresis, and a 3.4 kb DNA fragment was recovered in the same manner as above.

Each of the 1.4 kb DNA fragments containing a gene corresponding to the ywfE gene of Bacillus subtilis 168 and the 3.4 kb DNA fragment obtained above were subjected to ligation reaction using a ligation kit at 16°C for 16 hours. Escherichia coli NM522 was transformed using each ligation reaction mixture according to the method using calcium ion, spread on LB agar medium containing 50 µg/ml ampicillin, and cultured overnight at 30° C.

A plasmid was extracted from a colony of each transformant that grew on the medium according to a known method and the structure of each plasmid was analyzed using restriction enzymes. As a result, it was confirmed that the following C-terminal His-tagged gene expression vectors were obtained: pQE60ywfE1 (a vector containing the gene derived from ATCC 15245), pQE60ywfE2 (a vector containing the gene derived from ATCC 6633), pQE60ywfE3 (a vector containing the gene derived from IAM 1213), pQE60ywfE4 (a vector containing the gene derived from IAM 1107), pQE60ywfE5 (a vector containing the gene derived from IAM 1214), pQE60ywfE6 (a vector containing the gene derived from ATCC 9466), pQE60ywfE7 (a vector containing the gene derived from IAM 1033), pQE60ywfE8 (a vector containing the gene derived from ATCC 21555), pQE60ywfE9 (a vector containing the gene derived from IFO 3022) and pQE60ywfE10 (a vector containing the gene derived from NRRL B-12025).

Escherichia coli NM522/pQE60ywfE1 to NM522/pQE60ywfE10 strains obtained above were respectively inoculated into 8 ml of LB medium containing 50 µg/ml ampicillin in a test tube, and cultured at 28°C for 17 hours. Each of the resulting cultures was inoculated into 50 ml of LB medium containing 50 *µ*g/ml ampicillin in a 250-ml Erlenmeyer flask, and cultured at 30°C for 3 hours. Then, IPTG was added to give a final concentration of 1 mmol/l, followed by further culturing at 30°C for 4 hours. The resulting culture was centrifuged to obtain wet cells, and His-tagged recombinant enzymes were purified from the respective wet cells using HisTrap according to the instructions attached thereto.

### Experimental Example 10

### Production of Dipeptides Using Purified Enzymes

Reaction mixtures (0.1 ml each) comprising 0.04 mg of the respective recombinant enzymes obtained in Experimental Example 9, 100 mmol/l Tris-HCl (pH 8.0), 60 mmol/l magnesium chloride, 60 nunol/l ATP, 30 mmol/l L-Ala and 30 mmol/l L-Gln were prepared, and reactions were carried out at 37°C for 16 hours.

After the completion of reactions, the reaction mixtures were analyzed by the method described in Experimental Example 3, whereby it was confirmed that 3.0 to 3.5 g/l L-Ala-L-Gln and 0.25 to 0.3 g/l L-Ala-L-Ala were formed and accumulated.

When ATP was excluded from the compositions of the above reaction mixtures, L-Ala-L-Gln or L-Ala-L-Ala was not formed at all.

The above results revealed that all of the products of the genes obtained in Experimental Example 8 have the activity to produce L-Ala-L-Gln and L-Ala-L-Ala from L-Ala and L-Gln in the presence of ATP.

### Experimental Example 11

### Acquisition of the albC Gene and Its Analogous Gene

The albC gene and its analogous gene were obtained from Streptomyces noursei and Streptomyces albulus based on the nucleotide sequence of the albC gene of Streptomyces noursei [Chemistry & Biol., 9, 1355 (2002)] in the following manner.

Streptomyces noursei IFO15452 and Streptomyces albulus IFO14147 were inoculated into KM73 medium [2 g/l yeast extract (Difco) and 10 g/l soluble starch (Wako Pure Chemical Industries, Ltd.)] containing 1% glycine and KP medium [15 g/l glucose, 10 g/l glycerol, 10 g/l polypeptone (Nihon Pharmaceutical Co., Ltd.), 10 g/l meat extract (Kyokuto Pharmaceutical Industrial Co., Ltd.) and 4 g/l calcium carbonate)], respectively, and subjected to shaking culture overnight at 28°C. Streptomyces noursei IFO15452 and Streptomyces albulus IFO14147 were distributed by National Institute of Technology and Evaluation (NITE) Biological Resource Center (BRC) (2-5-8, Kazusakamatari, Kisarazu-shi, Chiba 292-0818 Japan).

After the culturing, the chromosomal DNAs of the respective microorganisms were isolated and purified according to the method described in Genetic Manipulation of Streptomyces: a Laboratory Manual: John Innes Foundation.

On the basis of the nucleotide sequence of the albC gene, DNAs having the nucleotide sequences shown in SEQ ID NOS: 41 and 42 (hereinafter referred to as primer J and primer K, respectively) were synthesized by using a DNA synthesizer (Model 8905, PerSeptive Biosystems, Inc.). Primer J has a sequence wherein a sequence containing the NcoI recognition sequence is added to the 5' end of a region containing the initiation codon of the albC gene on the chromosomal DNA of Streptomyces noursei. Primer K has a sequence wherein a sequence containing the BglII recognition sequence is added to the 5' end of a sequence complementary to a sequence containing the termination codon of the albC gene.

PCR was carried out using each of the chromosomal DNAs of Streptomyces noursei and Streptomyces albulus as a template and the above primer J and primer K as a set of primers. That is, PCR was carried out by 30 cycles, one cycle consisting of reaction at 94°C for one minute, reaction at 55°C for 30 seconds and reaction at 72°C for one minute, using 50 *µ*l of a reaction mixture comprising 0.1 *µ*g of the chromosomal DNA as a template, 0.5 *µ*mol/l each of the primers, 2.5 units of Ex Taq DNA polymerase (Takara Bio Inc.), 5 *µ*l of buffer for Ex Taq DNA polymerase (10 x) (Takara Bio Inc.), 200 µmol/l each of dNTPs and 5 µl of dimethyl sulfoxide.

One-tenth of each of the resulting reaction mixtures was subjected to agarose gel electrophoresis to confirm that a ca. 0.7 kb DNA fragment was amplified. Then, the remaining reaction mixture was mixed with an equal amount of phenol/chloroform saturated with TE. The resulting solution was centrifuged, and the obtained upper layer was mixed with a two-fold volume of cold ethanol and allowed to stand at -80°C for 30 minutes. The resulting solution was centrifuged to precipitate DNA, and the obtained DNA was dissolved in 20 µl of TE.

Each of the thus obtained solutions (5 µl) was subjected to reaction to cleave the amplified DNA with restriction enzymes NcoI and BglII. DNA fragments were separated by agarose gel electrophoresis, and a 700 bp DNA fragment was recovered using GENECLEAN II Kit.

Subsequently, 0.2 µg of the expression vector pQE60 containing phage T5 promoter was cleaved with restriction enzymes NcoI and BglII. DNA fragments were separated by agarose gel electrophoresis, and a 3.4 kb DNA fragment was recovered in the same manner as above.

Each of the actinomycetes-derived 0.7 kb DNA fragments and the pQE60-derived 3.4 kb DNA fragment obtained above were subjected to ligation reaction using a ligation kit at 16°C for 16 hours.

Escherichia coli NM522 was transformed using each ligation reaction mixture according to the method using calcium ion, spread on LB agar medium containing 50 µg/ml ampicillin, and cultured overnight at 30°C.

A plasmid was extracted from a colony of each transformant that grew on the medium according to a known method, and the structure of each plasmid was analyzed using restriction enzymes. As a result, it was confirmed that expression vector pAL-nou containing the DNA derived from Streptomyces noursei in a downstream position of the phage T5 promoter and expression vector pAL-alb containing the DNA derived from Streptomyces albulus were obtained (Fig. 3).

The nucleotide sequence of each actinomycete-derived DNA inserted into the resective plasmid was determined by using a nucleotide sequencer (373A DNA Sequencer), whereby it was confirmed that pAL-alb contained DNA encoding a protein having the amino acid sequence shown in SEQ ID NO: 37, i.e. DNA having the nucleotide sequence shown in SEQ ID NO: 39, and pAL-nou contained DNA encoding a protein having the amino acid sequence shown in SEQ ID NO: 38, i.e. DNA having the nucleotide sequence shown in SEQ ID NO: 40.

### Experimental Example 12

### Production of Straight-chain Dipeptides by the Use of Cells as an Enzyme Source

Escherichia coli NM522 carrying pAL-nou or pAL-alb obtained in Experimental Example 11 (Escherichia coli NM522/pAL-nou or NM522/pAL-alb) and Escherichia coli NM522 without a plasmid were respectively inoculated into 10 ml of LB medium containing 50 µg/ml ampicillin in a test tube (no addition of ampicillin in the case of a strain carrying no plasmid, hereinafter the same shall apply), and cultured at 30°C for 17 hours. Each of the resulting cultures (0.5 ml) was inoculated into 50 ml of LB medium in a 250-ml Erlenmeyer flask and subjected to shaking culture at 30°C for one hour. Then, IPTG was added to give a final concentration of 1 mmol/l, followed by further culturing for 4 hours. The resulting culture was centrifuged to obtain wet cells.

A reaction mixture (3.0 ml) comprising 100 mg/ml (final concentration) wet cells, 60 mmol/l potassium phosphate buffer (pH 7.2), 10 mmol/l magnesium chloride, 10 mmol/l ATP, 1 g/l L-Leu and 1 g/l L-Phe was prepared, and reaction was carried out at 30°C. One hour after the start of the reaction, the reaction mixture was sampled and acetonitrile was added thereto to a concentration of 20% (v/v). Then, the obtained reaction product was analyzed by HPLC. The HPLC analysis was carried out by using ODS-HA column (YMC Co., Ltd.) as a separation column and 30% (v/v) acetonitrile as an eluent at a flow rate of 0.6 ml/min, and by measuring ultraviolet absorption at 215 nm.

As a result, it was confirmed that 36.7 mg/l cyclo(L-leucyl-L-phenylalanine) [cyclo(L-Leu-L-Phe)] was accumulated in the reaction mixture of Escherichia coli NM522/pAL-nou. However, no cyclo(L-Leu-L-Phe) was detected in the reaction mixture of Escherichia coli NM522. The same reaction mixtures were analyzed by HPLC under the following conditions to measure linear dipeptides (hereinafter reffered to as dipeptides) L-leucyl-L-phenylalanine (L-Leu-L-Phe) and L-phenylalanyl-L-leucine (L-Phe-L-Leu).

Both the dipeptides were derivatized by the F-moc method and then analyzed by HPLC. The HPLC analysis was carried out by using ODS-HG5 (Nomura Kagaku Co., Ltd.) as a separation column and solution A (6 ml/l acetic acid and 20% (v/v) acetonitrile, pH adjusted to 4.8 with triethylamine) and solution B (6 ml/l acetic acid and 70% (v/v) acetonitrile, pH adjusted to 4.8 with triethylamine) as eluents at a flow rate of 0.6 ml/min, and by detecting the dipeptides at an excitation wavelength of 254 nm and a fluorescence wavelength of 630 nm. The ratio of solution A to solution B was 8:2 during the first 5 minutes of elution and thereafter changed with a linear gradient so that the ratio became 1:1 at 20 minutes after the start of elution.

As a result, it was confirmed that 21.9 mg/l L-Leu-L-Phe and 12.0 mg/l L-Phe-L-Leu were accumulated in the reaction mixture of Escherichia coli NM522/pAL-nou and no linear dipeptide was detected in the reaction mixture of Escherichia coli NM522 used as a control strain.

The above result revealed that the cyclodipeptide-synthesizing enzyme obtained in Experimental Example 11 has the ability to synthesize dipeptides.

### Experimental Example 13

### Production of Straight-chain Dipeptides Using the Purified Enzyme (1)

Escherichia coli NM522/pAL-nou was cultured in the same manner as in Experimental Example 12. After the completion of the culturing, centrifugation was carried out to obtain wet cells. The obtained wet cells were washed with a 60 mmol/l potassium phosphate buffer (pH 7.2) and suspended in a 20 mmol/l potassium phosphate buffer containing 10 mmol/l imidazole. The resulting suspension was subjected to ultrasonication at 4°C to obtain a disrupted cell suspension. The obtained suspension (10 ml: containing 0.863 mg of protein) was passed through a His-tag purification column (Amersham Biosciences K.K.) and then 15 ml of a 20 mmol/l potassium phosphate buffer containing 10 mmol/l imidazole was passed through the column for washing to purify a His-tagged albC protein in the column. Then, 2 ml of a reaction mixture having the same composition as that in Example 2 [composition: 60 mmol/l potassium phosphate buffer (pH 7.2), 10 mmol/l magnesium chloride, 10 mmol/l ATP, 1 g/l L-Leu, 1 g/l L-Phe] was put into the column containing the His-tagged albC protein, followed by incubation at 30°C, during which the substrates were held in the column. After 24 hours, the reaction mixture in the column was eluted with 3 ml of a reaction mixture having the same composition, and the cyclodipeptide and linear dipeptides in the reaction mixture were determined in the same manner as in Experimental Example 12.

As a result, it was confirmed that 6.8 mg/l cyclo(L-Leu-L-Phe), 28.7 mg/l L-Leu-L-Phe and 18.5 mg/l L-Phe-L-Leu were formed. No cyclodipeptide or dipeptide was detected in the reaction mixture without ATP incubated in the same manner.

### Experimental Example 14

### Production of Dipeptides Using the Purified Enzyme (2)

Enzymatic reaction was carried out in the same manner as in Experimental Example 13 except that the amino acids as substrates were replaced by another amino acid and the obtained product was analyzed. As the reaction mixture, a mixture having the same composition as that of Experimental Example 13 except that the amino acids as the substrates were replaced by 1 g/l L-Ala, L-Leu or L-Phe was used.

As a result, it was revealed that 9.41 mg/l L-Ala-L-Ala, 7.85 mg/l L-Leu-L-Leu and 5.20 mg/l L-Phe-L-Phe were respectively formed in 24 hours after the start of the reaction.

### Experimental Example 15

### Construction of Escherichia coli for Enhanced Expression of the ywfE Gene

By using a DNA synthesizer (Model 8905, PerSeptive Biosystems, Inc.), DNAs having the sequences shown in SEQ ID NOS: 82 to 85 (hereinafter referred to as primer L, primer M, primer N and primer O, respectively) were synthesized. The sequence of SEQ ID NO: 82 is a sequence wherein a sequence containing the XhoI recognition sequence is added to the 5' end of a region containing the Shine-Dalgarno sequence (ribosome binding sequence) of the ywfE gene on the plasmid pQE60ywfE. The sequence of SEQ ID NO: 83 is a sequence wherein a sequence containing the BamHI recognition sequence is added to the 5' end of a sequence complementary to a sequence containing the termination codon of the ywfE gene. The sequence of SEQ ID NO: 84 is a sequence wherein a sequence containing the EcoRI recognition sequence is added to the 5' end of the sequence of trp promoter region of expression vector pTrS30 containing trp promoter. The sequence of SEQ ID NO: 85 is a sequence wherein a sequence containing the XhoI recognition sequence is added to the 5' end of a sequence complementary to the sequence of trp promoter region of expression vector pTrS30 containing trp promoter.

A ywfE gene fragment and a trp promoter region fragment were amplified by PCR using the above primers L and M and primers N and O as a set of primers, respectively, and the plasmid pQE60ywfE as a template. PCR was carried out by 30 cycles, one cycle consisting of reaction at 94°C for one minute, reaction at 55°C for 2 minutes and reaction at 72°C for 3 minutes, using 40 µl of a reaction mixture comprising 10 ng of pQE60ywfE, 0.5 µmol/l each of the primers, 2.5 units of Pfu DNA polymerase, 4 µl of buffer for Pfu DNA polymerase (10 x) and 200 µmol/l each of dNTPs.

One-tenth of each of the resulting reaction mixtures was subjected to agarose gel electrophoresis to confirm that a ca. 1.4 kb fragment corresponding to the ywfE gene fragment and a ca. 0.3 kb fragment corresponding to the trp promoter region fragment were respectively amplified in the PCR using primer L and primer M and the PCR using primer N and primer O. Then, the remaining reaction mixture was mixed with an equal amount of phenol/chloroform saturated with TE. The resulting solution was centrifuged, and the obtained upper layer was mixed with a two-fold volume of cold ethanol and allowed to stand at -80°C for 30 minutes. The resulting solution was centrifuged, and the obtained DNA was dissolved in 20 µl of TE.

The thus obtained DNA solutions (5 µl each) were respectively subjected to reaction to cleave the DNA amplified using primer L and primer M with restriction enzymes XhoI and BamHI and to reaction to cleave the DNA amplified using primer N and primer O with restriction enzymes EcoRI and XhoI. DNA fragments were separated by agarose gel electrophoresis, and a 1.4 kb fragment containing the ywfE gene and a 0.3 kb fragment containing trp promoter region were respectively recovered using GENECLEAN II Kit.
pTrS30 (a trp promoter-containing expression vector, 0.2 *µ*g) was cleaved with restriction enzymes EcoRI and BamHI. DNA fragments were separated by agarose gel electrophoresis and a 4.5 kb DNA fragment was recovered in the same manner as above.

The 1.4 kb fragment containing the ywfE gene, the 0.3 kb fragment containing trp promoter region and the 4.5 kb DNA fragment obtained above were subjected to ligation reaction using a ligation kit at 16°C for 16 hours.

Escherichia coli NM522 was transformed using the reaction mixture according to the method using calcium ion, spread on LB agar medium containing 50 *µ*g/ml ampicillin, and cultured overnight at 30°C.

A plasmid was extracted from a colony of the transformant that grew on the medium according to a known method, whereby expression vector pPE56 containing the ywfE gene in a downstream position of the trp promoter was obtained. The structure of the vector was confirmed by digestion with restriction enzymes (Fig. 4).

### Experimental Example 16

### Preparation of Strains Having Deletions of the pepD, pepN, pepB and pepA Genes and the dpp Operon

Strains in which specific genes on Escherichia coli chromosomal DNA are deleted were prepared according to the method utilizing the homologous recombination system of lambda phage [Proc. Natl. Acad. Sci. USA, 97, 6641-6645 (2000)].

Plasmids pKD46, pKD3 and pCP20 used below were prepared by extraction, according to a known method, from Escherichia coli strains carrying them which were obtained from Escherichia coli Genetic Stock Center, Yale University, U.S.A.
(1) Cloning of DNA Fragments for Gene Deletion
   For the purpose of deleting the following genes existing on the chromosomal DNA of Escherichia coli K12, DNAs having nucleotide sequences homologous to 36-bp nucleotide sequences that lie upstream and downstream of the respective genes to be deleted on the chromosomal DNA of Escherichia coli K12 and the nucleotide sequence shown in SEQ ID NO: 52 which is recognized by yeast-derived Flp recombinase were synthesized using a DNA synthesizer (Model 8905, PerSeptive Biosystems, Inc.). The genes to be deleted are the pepD gene having the nucleotide sequence shown in SEQ ID NO: 53, the pepN gene having the nucleotide sequence shown in SEQ ID NO: 54, the pepB gene having the nucleotide sequence shown in SEQ ID NO: 55, the pepA gene having the nucleotide sequence shown in SEQ ID NO: 56, the dppA gene having the nucleotide sequence shown in SEQ ID NO: 57, the dppB gene having the nucleotide sequence shown in SEQ ID NO: 58, the dppC gene having the nucleotide sequence shown in SEQ ID NO: 59, the dppD gene having the nucleotide sequence shown in SEQ ID NO: 60 and the dppF gene having the nucleotide sequence shown in SEQ ID NO: 61. In the case of the dppA, dppB, dppC, dppD and dppF genes, which form an operon, DNAs having nucleotide sequences homologous to the nucleotide sequences that lie upstream and downstream of the operon were synthesized.
   That is, DNAs consisting of the following nucleotide sequences were synthesized as respective sets of primers for amplification of DNA fragments for gene deletion: SEQ ID NOS: 62 and 63 for pepD gene deletion; SEQ ID NOS: 64 and 65 for pepN gene deletion; SEQ ID NOS: 66 and 67 for pepA gene deletion; SEQ ID NOS: 68 and 69 for pepB gene deletion; and SEQ ID NOS: 70 and 71 for dpp operon deletion.
   Subsequently, PCR was carried out using each set of the above synthetic DNAs as a set of primers and pKD3 DNA as a template. That is, PCR was carried out by 30 cycles, one cycle consisting of reaction at 94°C for one minute, reaction at 55°C for 2 minutes and reaction at 72°C for 3 minutes, using 40 µl of a reaction mixture comprising 10 ng of the plasmid DNA, 0.5 *µ*mol/l each of the primers, 2.5 units of Pfu DNA polymerase, 4 *µ*l of buffer for Pfu DNA polymerase (10 x) and 200 *µ*mol/l each of deoxyNTPs.
   One-tenth of each of the resulting reaction mixtures was subjected to agarose gel electrophoresis to confirm that the desired fragment was amplified. Then, the remaining reaction mixture was mixed with an equal amount of phenol/chloroform saturated with TE.
   The resulting mixture was centrifuged, and the obtained upper layer was mixed with a two-fold volume of cold ethanol and allowed to stand at -80°C for 30 minutes, followed by centrifugation. By this procedure, chloramphenicol resistance gene-containing DNA fragments for deletion of the pepD, pepN, pepB and pepA genes and the dpp operon were obtained.
(2) Preparation of Escherichia coli JM101 Having pepD Gene Deletion
   Escherichia coli JM101 was'transformed with pKD46, spread on LB agar medium containing 100 mg/l ampicillin, and cultured at 30°C to select Escherichia coli JM101 carrying pKD46 (hereinafter referred to as Escherichia coli JM101/pKD46).
   The plasmid pKD46 carries λ Red recombinase gene the expression of which can be induced by L-arabinose. Accordingly, when Escherichia coli carrying pKD46 grown in the presence of L-arabinose is transformed using a linear DNA, homologous recombination occurs with high frequency. Further, as pKD46 has a thermosensitive replication origin, curing of the plasmid can be readily caused by culturing the strain at 42°C.
   The chloramphenicol resistance gene-containing DNA fragment for pepD gene deletion obtained above was introduced into Escherichia coli JM101/pKD46 obtained by culturing in the presence of 10 mmol/l L-arabinose and 50 µg/ml ampicillin by electroporation. The resulting cells were spread on LB agar medium (10 g/l Bacto-tryptone, 5 g/l Bacto-yeast extract, 5 g/l sodium chloride and 15 g/l agar) containing 25 mg/l chloramphenicol and cultured at 30°C to select a transformant in which the chloramphenicol resistance gene-containing DNA fragment for pepD gene deletion was integrated into the chromosomal DNA of Escherichia coli JM101 by homologous recombination.
   The selected chloramphenicol-resistant strain was inoculated onto LB agar medium containing 25 mg/l chloramphenicol and cultured at 42°C for 14 hours, followed by single colony isolation. Replicas of the obtained colonies were made on LB agar medium containing 25 mg/l chloramphenicol and LB agar medium containing 100 mg/l ampicillin, followed by culturing at 37°C. By selecting a colony showing chloramphenicol resistance and ampicillin sensitivity, a pKD46-cured strain was obtained.
   The pKD46-cured strain thus obtained was transformed using pCP20, followed by selection on LB agar medium containing 100 mg/l ampicillin to obtain a pKD46-cured strain carrying pCP20.
   The plasmid pCP20 carries yeast-derived Flp recombinase gene the expression of which can be induced at a temperature of 42°C.
   The chloramphenicol resistance gene-containing DNA fragments for deletion of the pepD, pepN, pepB and pepA genes and the dpp operon prepared above contain nucleotide sequences recognized by Flp recombinase at both termini of the chloramphenicol resistance gene. Therefore, the resistance gene can be readily deleted by homologous recombination catalyzed by Flp recombinase.
   Further, as pCP20 has a thermosensitive replication origin, expression of Flp recombinase and curing of pCP20 can be simultaneously induced by culturing the pCP20-carrying strain at 42°C.
   The pCP20-carrying pKD46-cured strain obtained above was inoculated onto drug-free LB agar medium and cultured at 42°C for 14 hours, followed by single colony isolation. Replicas of the obtained colonies were made on drug-free LB agar medium, LB agar medium containing 25 mg/l chloramphenicol and LB agar medium containing 100 mg/l ampicillin, followed by culturing at 30°C. Then, colonies showing chloramphenicol sensitivity and ampicillin sensitivity were selected.
   Chromosomal DNAs were prepared from the respective strains selected above according to an ordinary method [Seibutsukogaku Jikkensho (Experiments in Biotechnology), edited by The Society for Biotechnology, Japan, p. 97-98, Baifukan (1992)]. PCR was carried out using, as a set of primers, DNAs having the nucleotide sequences shown in SEQ ID NOS: 72 and 73 which were designed based on an inner nucleotide sequence of the pepD gene to be deleted, and using each of the chromosomal DNAs as a template. That is, PCR was carried out by 30 cycles, one cycle consisting of reaction at 94°C for one minute, reaction at 55°C for 2 minutes and reaction at 72°C for 3 minutes, using 40 µl of a reaction mixture comprising 0.1 µg of the chromosomal DNA, 0.5 µmol/l each of the primers, 2.5 units of Pfu DNA polymerase, 4 µl of buffer for Pfu DNA polymerase (10 x) and 200 µ mol/l each of deoxyNTPs.
   A strain with which no amplified DNA fragment was detected in the above PCR was identified as a strain having pepD gene deletion and was designated as Escherichia coli JPD1.
(3) Preparation of a Strain in Which the pepD and pepN Genes on the Chromosomal DNA of Escherichia coli JM101 are Deleted
   Escherichia coli JPD1 obtained in the above (2) was transformed with pKD46,'spread on LB agar medium containing 100 mg/l ampicillin, and cultured at 30°C to select Escherichia coli JPD1 carrying pKD46 (hereinafter referred to as Escherichia coli JPD1/pKD46). The chloramphenicol resistance gene-containing DNA fragment for pepN gene deletion was introduced into Escherichia coli JPD1/pKD46 by electroporation to obtain a transformant in which the chloramphenicol resistance gene-containing DNA fragment for pepN gene deletion was integrated into the chromosomal DNA of Escherichia coli JPD1/pKD46 by homologous recombination.
   Subsequently, the same procedure as in the above (2) was carried out to obtain a strain in which the chloramphenicol resistance gene was deleted from the chromosomal DNA, which was designated as Escherichia coli JPDN2.
(4) Preparation of Strains in Which the pepN, pepA or pepB Gene or the dpp Operon on the Chromosomal DNA of Escherichia coli JM101 is Deleted and Strains Having Multiple Gene Deletion

The strains having pepN, pepA or pepB gene or dpp operon deletion were prepared according to the same procedure as in the above (2) using the respective chloramphenicol resistance gene-containing DNA fragments for gene or operon deletion prepared in the above (1).

Acquisition of the strains having gene deletions by the above method was confirmed by carrying out PCR in the same manner as in the above (2) using, as sets of primers, DNAs having the nucleotide sequences shown in SEQ ID NOS: 74 to' 81 which were designed and syntheseized based on inner nucleotide sequences of the respective genes to be deleted. That is, DNAs having the following nucleotide sequences were used as respective sets of primers for the confirmation of gene deletion: SEQ ID NOS: 74 and 75 for pepN deletion; SEQ ID NOS: 76 and 77 for pepA deletion; SEQ ID NOS: 78 and 79 for pepB deletion; and SEQ ID NOS: 80 and 81 for dpp operon deletion.

The thus obtained dpp operon-deleted strain, pepN gene-deleted strain, pepA gene-deleted strain and pepB gene-deleted strain were designated as Escherichia coli JDPP1, Escherichia coli JPN1, Escherichia coli JPA1 and Escherichia coli JPB7, respectively.

Further, strains having multiple gene deletions, i.e., deletions of two or more genes or operon selected from the group consisting of the pepD, pepN, pepA and pepB genes and the dpp operon were prepared according to the method of the above (3). Acquisition of the strains having multiple gene deletions was confirmed by PCR similar to that in the above (2). The thus obtained double gene-deleted strain having pepD gene and dpp operon deletions was designated as Escherichia coli JPDP49, triple gene-deleted strain having pepB, pepD and pepN gene deletions as Escherichia coli JPDNB43, triple gene-deleted strain having pepD and pepN gene and dpp operon deletions as Escherichia coli JPNDDP36, quadruple gene-deleted strain having pepA, pepD and pepN gene and dpp operon deletions as Escherichia coli JPNDAP5, and quadruple gene-deleted strain having pepB, pepD and pepN gene and dpp operon deletions as Escherichia coli JPNDBP7. The genes deleted in the gene-deleted strains are shown in Table 2.

**Table 2**

| Strain | Deleted gene |
|---|---|
| JM101 | none |
| JDPP1 | dpp operon |
| JPN1 | pepN |
| JPA1 | pepA |
| JPB7 | pepB |
| JPD1 | pepD |
| JPDN2 | pepD, pepN |
| JPNDB43 | pepB, pepD, pepN |
| JPDP49 | pepD, dpp operon |
| JPNDDP36 | pepD, pepN, dpp operon |
| JPNDAP5 | pepA, pepD, pepN, dpp operon |
| JPNDBP7 | pepB, pepD, pepN, dpp operon |

### Experimental Example 17

### Evaluation of Productivity of L-Ala-L-Gln and L-Ala-L-Ala by Escherichia coli Strains in Which Peptidase and Dipeptide-permeating/transporting Protein Activities are Lost

The strains having deletions of genes encoding various peptidases and dipeptide-permeating/transporting protein which were obtained in Experimental Example 16 were transformed using the plasmid pPE56 constructed in Experimental Example 15 to obtain ampicillin-resistant transformants.

Each of the obtained transformants was inoculated into 8 ml of LB medium containing 50 µg/ml ampicillin in a test tube and cultured at 28°C for 17 hours. The resulting culture was added to 8 ml of an aqueous medium [16 g/l dipotassium hydrogenphosphate, 14 g/l potassium dihydrogenphosphate, 5 g/l ammonium sulfate, 1 g/l citric acid (anhydrous), 0.5 g/l Casamino acid (Difco), 1 g/l L-Pro, 2.5 g/l L-Ala, 2.5 g/l L-Gln, 10 g/l glucose, 10 mg/l vitamin B₁, 25 mg/l magnesium sulfate heptahydrate and 50 mg/l ferrous sulfate heptahydrate; pH adjusted to 7.2 with 10 mol/l sodium hydroxide solution; L-Gln was added after sterilization by filtration of a 10-fold conc. solution; glucose, vitamin B₁, magnesium sulfate heptahydrate and ferrous sulfate heptahydrate were added after separate steam sterilization] containing 100 µg/ml ampicillin and amino acids in a test tube in an amount of 1% and subjected to reaction at 30°C for 24 hours. The resulting aqueous medium was centrifuged to obtain a supernatant.

The product in the supernatant was derivatized by the F-moc method and then analyzed by HPLC. The HPLC analysis was carried out using ODS-HG5 (Nomura Kagaku Co., Ltd.) as a separation column and solution A (6 ml/l acetic acid and 20% (v/v) acetonitrile, pH adjusted to 4.8 with triethylamine) and solution B (6 ml/l acetic acid and 70% (v/v) acetonitrile, pH adjusted to 4.8 with triethylamine) as eluents. The ratio of solution A to solution B was 8:2 during the first 5 minutes of elution and thereafter changed with a linear gradient so that the ratio became 1:1 at 20 minutes after the start of elution. The results of analysis are shown in Table 3.

**Table 3**

| Strain | Deleted gene | Ala-Gln (g/l) | Ala-Ala (g/l) |
|---|---|---|---|
| JM101 | none | 0 | 0 |
| JDPP1 | dpp operon | 0.02 | 0.01 |
| JPN1 | pepN | 0.01 | 0.01 |
| JPA1 | pepA | 0.01 | 0.01 |
| JPB7 | pepB | 0.01 | 0.01 |
| JPD1 | pepD | 0.01 | 0.01 |
| JPDN2 | pepD, pepN | 0.02 | 0.03 |
| JPNDB43 | pepB, pepD, pepN | 0.05 | 0.12 |
| JPDP49 | pepD, dpp operon | 0.11 | 0.08 |
| JPNDDP36 | pepD, pepN, dpp operon | 0.16 | 0.21 |
| JPNDAP5 | pepA, pepD, pepN, dpp operon | 0.28 | 0.26 |
| JPNDBP7 | pepB, pepD, pepN, dpp operon | 0.43 | 0.22 |

As can be seen from Table 3, small amounts of dipeptides were formed and accumulated by use of the microorganisms having deletions of two or less kinds of peptidase genes or one operon encoding a peptide-permeating/transporting protein, whereas the amounts of dipeptides formed and accumulated were greatly increased by use of the microorganisms having deletions of one or more kinds of peptidase genes and one operon encoding a peptide-permeating/transporting protein or microorganisms having deletions of three or more kinds of peptidase genes.

### Experimental Example 18

### Evaluation of Productivity of L-Alanyl-L-valine (hereinafter referred to as L-Ala-L-Val) by Escherichia coli Strains in Which Peptidase and Peptide-permeating/transporting Protein Activities are Lost

Similarly to Experimental Example 17, the Escherichia coli strains having deletions of genes encoding various peptidases and peptide-permeating/transporting protein were transformed using pPE56. Each of the obtained transformants was inoculated into 8 ml of LB medium containing 50 µg/ml ampicillin in a test tube and cultured at 28°C for 17 hours. The resulting culture was added to 8 ml of an aqueous medium [16 g/l dipotassium hydrogenphosphate, 14 g/l potassium dihydrogenphosphate, 5 g/l ammonium sulfate, 1 g/l citric acid (anhydrous), 0.5 g/l Casamino acid (Difco), 1 g/l L-Pro, 2.5 g/l L-Ala, 2.5 g/l L-Val, 10 g/l glucose, 10 mg/l vitamin B₁, 25 mg/l magnesium sulfate heptahydrate and 50 mg/l ferrous sulfate heptahydrate; pH adjusted to 7.2 with 10 mol/l sodium hydroxide solution; glucose, vitamin B₁, magnesium sulfate heptahydrate and ferrous sulfate heptahydrate were added after separate steam sterilization] containing 100 µg/ml ampicillin and amino acids in a test tube in an amount of 1% and subjected to reaction at 30°C for 24 hours. The resulting aqueous medium was centrifuged to obtain a supernatant.

The product in the culture supernatant was analyzed by the method described in Experimental Example 17. The results are shown in Table 4.

**Table 4**

| Strain | Deleted gene | Ala-Val (g/l) |
|---|---|---|
| JM101 | none | 0 |
| JDPP1 | dpp operon | 0 |
| JPN1 | pepN | 0 |
| JPA1 | pepA | 0 |
| JPB7 | pepB | 0 |
| JPD1 | pepD | 0 |
| JPDN2 | pepD, pepN | 0 |
| JPNDB43 | pepB, pepD, pepN | 0.04 |
| JPDP49 | pepD, dpp operon | 0.11 |
| JPNDDP36 | pepD, pepN, dpp operon | 0.22 |
| JPNDBP7 | pepB, pepD, pepN, dpp operon | 0.20 |

As can be seen from Table 4, the dipeptide was not produced by use of the microorganisms having deletions of two or less kinds of peptidase genes or one operon encoding a peptide-permeating/transporting protein, whereas the dipeptide was produced by use of the microorganisms having deletions of three or more kinds of peptidase genes or microorganisms having deletions of one or more kinds of peptidase genes and one operon encoding a peptide-permeating/transporting protein.

### Experimental Example 19

### Evaluation of Productivity of Glycyl-L-glutamine (hereinafter referred to as Gly-L-Gln) by Escherichia coli Strains in Which Peptidase and Dipeptide-permeating/transporting Protein Activities are Lost

Similarly to Experimental Example 17, the strains having deletions of various peptidase genes and an operon encoding a dipeptide-permeating/transporting protein were transformed using pPE56. Each of the obtained transformants was inoculated into 8 ml of LB medium containing 50 µg/ml ampicillin in a test tube and cultured at 28°C for 17 hours.

The resulting culture was added to 8 ml of an aqueous medium [16 g/l dipotassium hydrogenphosphate, 14 g/l potassium dihydrogenphosphate, 5 g/l ammonium sulfate, 1 g/l citric acid (anhydrous), 0.5 g/l Casamino acid (Difco), 1 g/l L-Pro, 2.5 g/l Gly, 2.5 g/l L-Gln, 10 g/l glucose, 10 mg/l vitamin B₁, 25 mg/l magnesium sulfate heptahydrate and 50 mg/l ferrous sulfate heptahydrate; pH adjusted to 7.2 with 10 mol/l sodium hydroxide solution; L-Gln was added after sterilization by filtration of a 10-fold conc. solution; glucose, vitamin B₁, magnesium sulfate heptahydrate and ferrous sulfate heptahydrate were added after separate steam sterilization] containing 100 µg/ml ampicillin and amino acids in a test tube in an amount of 1% and subjected to reaction at 30°C for 24 hours. The resulting aqueous medium was centrifuged to obtain a supernatant.

The product in the culture supernatant was analyzed by the method described in Experimental Example 17. The results are shown in Table 5.

**Table 5**

| Strain | Deleted gene | Gly-Gln (g/l) |
|---|---|---|
| JM101 | none | 0 |
| JDPP1 | dpp operon | 0 |
| JPDN2 | pepD, pepN | 0 |
| JPNDB43 | pepB, pepD, pepN | 0.01 |
| JPNDDP36 | pepD, pepN, dpp operon | 0.02 |
| JPNDBP7 | pepB, pepD, pepN, dpp operon | 0.03 |

As can be seen from Table 5, the dipeptide was not produced by use of the microorganisms having deletions of two or less kinds of peptidase genes or one operon encoding a peptide-permeating/transporting protein, whereas the dipeptide was produced by use of the microorganisms having deletions of three or more kinds of peptidase genes or microorganisms having deletions of two or more kinds of peptidase genes and one operon encoding a peptide-permeating/transporting protein.

Certain embodiments of the present invention are illustrated in the following examples. These examples are not to be construed as limiting the scope of the invention.

### Example 1

### Enzymatic Process for Production of Dipeptides or Dipeptide Derivatives Using Amino Acids or Amino Acid Derivatives as Substrates

A reaction mixture (0.1 ml) comprising 40 mg/l purified His-tagged recombinant enzyme obtained in Experimental Example 4, 100 mmol/l Tris-HCl (pH 9.0), 30 mmol/l magnesium chloride, 10 mmol/l ATP and 10 mmol/l each of amino acid and amino acid derivative shown in Table 6 was prepared, and reaction was carried out at 37°C for 2 hours.

After the completion of reaction, a termination buffer (4 mol/l urea, 100 mmol/l EDTA disodium salt) (100 times amount of the reaction mixture) was added to the reaction mixture to terminate the reaction, and the amount of ADP formed when ATP was consumed by enzymatic reaction was determined by HPLC, whereby it was confirmed that the reaction proceeded. The HPLC analysis was carried out by using Develosil C30-UG-5 (150 x 4.6 mm, Nomura Kagaku Co., Ltd.) as a column and a solution comprising 200 mmol/l acetic acid and 200 mmol/l triethylamine (pH 6.6) as a mobile phase at a flow rate of 1.0 ml/min at room temperature, and by measuring ultraviolet absorption at 254 nm. The results are shown in Table 6.

**Table 6-1**

| Amino acid added | Amount of ADP formed (mmol/1) |
|---|---|
| none | 0.05 |
| Ala | 0.13 |
| Ala + cyc(5)Ala | 5.67 |
| Ala + cyc(3)Ala | 3.64 |
| Ala + cyc(6)Ala | 3.78 |

**Table 6-2**

| Amino acid added | Amount of ADP formed (mmol/l) |
|---|---|
| none | 0.05 |
| Ala | 0.13 |
| Phe | 0.02 |
| Ala + Phe | 5.84 |
| Ala + Cl-Phe | 4.78 |
| Cl-Ala + Phe | 2.75 |
| CN-Ala + Phe | 1.11 |
| Ala + F-Phe | 4.44 |
| Ala + Ni-Phe | 4.42 |
| Ala + NH₂-Phe | 1.89 |
| Ala + Kynurenin | 2.11 |

**Table 6-3**

| Amino acid added | Amount of ADP formed (mmol/l) |
|---|---|
| none | 0.05 |
| Ala | 0.13 |
| Glu | 0.13 |
| Ala + Glu(OMe) | 2.60 |
| Ala + Glu(OEt) | 3.93 |
| Ala + Glu(OtBu) | 6.43 |
| Ala + Glu(OBzl) | 6.01 |

**Table 6-4**

| Amino acid added | Amount of ADP formed (mmol/l) |
|---|---|
| none | 0.05 |
| Ala | 0.13 |
| Asp | 0.16 |
| Ala + Asp(OMe) | 0.54 |
| Ala + Asp(OtBu) | 4.42 |
| Ala + Asp(OBzl) | 3.23 , |

**Table 6-5**

| Amino acid added | Amount of ADP formed (mmol/l) |
|---|---|
| none | 0.05 |
| Ala | 0.13 |
| Lys | 0.17 |
| Ala + Lys(Ac) | 0.75 |
| Ala + Lys(Boc) | 3.64 |

Tables 6-1 to 6-5 respectively show the results of experiments using the following substrates : 6-1, L-Ala and L-Ala derivatives; 6-2, L-Ala and L-Phe derivatives; 6-3, L-Ala and L-Glu derivatives; 6-4, L-Ala and L-Asp derivatives; and 6-5, L-Ala and L-Lys derivatives. The abbreviations for the amino acids and amino acid derivatives used as the substrates are as follows.
Ala: L-alanine
Phe: L-phenylalanine
Glu: L-glutamic acid
Asp: L-aspartic acid
Lys: L-lysine
Cl-Ala: β-chloro-L-alanine
CN-Ala: β-cyano-L-alanine
cyc(5)Ala: β-cyclopentyl-DL-alanine
cyc(3)Ala: β-cyclopropylalanine
cyc(6)Ala: β-cyclohexylalanine
Cl-Phe: 4-chlorophenylalanine
F-Phe: 4-fluorophenylalanine
Ni-Phe: p-nitrophenylalanine
NH₂-Phe: p-aminophenylalanine
Phe-NH₂ : phenylalanine amide
Kynurenin: L-kynurenin
Glu(OMe): glutamic acid-γ-methyl ester
Glu(OEt): glutamic acid-γ-ethyl ester
Glu(OtBu): glutamic acid-γ-butyl ester
Glu(OBzl): glutamic acid-γ-benzyl ester
Asp(OMe): aspartic acid-β-methyl ester
Asp(OtBu): aspartic acid-β-t-butyl ester
Asp(OBzl): aspartic acid-β-benzyl ester
Lys(Ac): acetyl-lysine
Lys(Boc): Boc-lysine

As shown in Table 6, the amounts of ADP formed in the control experiment using no substrate and the experiments respectively using L-Ala, L-Phe, L-Glu, L-Asp and L-Lys as the sole substrate were 0.02 to 0.16 mmol/l. On the other hand, as much as 0.54 to 6.43 mmol/l ADP was formed by using the combinations of amino acids and amino acid derivatives shown in Table 1.

The structural analysis of compounds that existed in the reaction mixtures obtained under reaction conditions similar to those as mentioned above was carried out by proton NMR analysis. The concentration of an amino acid or an amino acid derivative used as the substrate was 20mmol/L in reaction mixtures 1, 4, 10 and 11, and 10mmol/L in the remaining reaction mixtures, respectively.

The proton NMR analysis was carried out by using DMX500 manufactured by Bruker Co. under the conditions described below.
Temperature; 303K
Standard compound; 1 mmol/L 3-(Trimethylsilyl)-Propionic acid-D4 sodium salt (TSP)
Medium; light water (in the case of measuring reaction mixtures 4, 10 and 11) or heavy water (in the case of measuring the other reaction mixtures)

The structure of each of the compounds in the reaction mixtures was identified based on the Chemical shift of the proton of the α position. As the area of TSP was assumed to be an internal standard, the concentration of each of the compounds was calculated based on the area of the signal of the proton of the α position (Table 7). However, because the concentration of L-Ala-L-Ala was low, and because the signal of the proton of the α position overlapped with other signals, the concentration of L-Ala-L-Ala was calculated based on the area of the signal of the proton of the β position. The Chemical shift of the proton of the α position of each of the compounds are shown in parentheses (the unit is ppm).
The reaction mixture using Cl-Ala and Phe as substrates
(Reaction mixture 1)
   Cl-Ala(4.20), Phe(4.02), Cl-Ala-Phe(3.93, 4.50), Aziridine-2-carboxylic acid [Azc](2.73), Azc-Phe(2.59, 4.47)
   The reaction mixture using CN-Ala and Phe as substrates
(Reaction mixture 2)
   CN-Ala(3.87), Phe(4.00), CN-Ala-Phe(3.71, 4.48)
   The reaction mixture using Ala and C1-Phe as substrates
(Reaction mixture 3)
   Ala(3.79), Cl-Phe(3.97), Ala-Cl-Phe(3.90, 4.43)
   The reaction mixture using Ala and NH₂-Phe as substrates
(Reaction mixture 4)
   Ala(3.78), NH₂-Phe(3.93), Ala-NH₂-Phe(3.95, 4.39), Ala-Ala(B; 1.55, 1.36)
   The reaction mixture using Ala and Kinurenine as substrates (Reaction mixture 5)
   Ala(3.79), Kinurenine(4.16), Ala-Kinurenine(3.96, 4.64)
   The reaction mixture using Ala and Phe-NH₂ as substrates
(Reaction mixture 6)
   Ala(3.79), Phe-NH₂ (4.02), Ala-Phe-NH₂ (3.90, 4.60)
   The reaction mixture using Ala and Glu(OMe) as substrates
(Reaction mixture 7)
   Ala(3.79), Glu(OMe)(3.76), Ala-Glu(OMe)(4.05, 4.18), Ala-Ala(B; 1.55, 1.36)
   The reaction mixture using Ala and Glu(OtBu) as substrates
(Reaction mixture 8)
   Ala(3.79), Glu(OtBu)(3.76), Ala-Glu(OtBu)(4.04, 4.18)
   The reaction mixture using Ala and Asp(OtBu) as substrates
(Reaction mixture 9)
   Ala (3.81), Asp(OtBu)(3.98), Ala-Asp(OtBu)(4.04, 4.46)
   The reaction mixture using Ala and Lys(Boc) as substrates
(Reaction mixture 10)
   Ala(3.78), Lys (Boc) (3.73), Ala-Lys(Boc)(4.02, 4.14)
   The reaction mixture using Ala and cyc(3)Ala as substrates
(Reaction mixture 11)
   Ala(3.78), cyc(3)Ala (3.82), Ala-cyc(3)Ala(4.08, 4.24)
   The reaction mixture using Ala and cyc(6)Ala as substrates
(Reaction mixture 12)
   Ala(3.79), cyc(6)Ala (3.76), Ala-cyc(6)Ala(4.02, 4.22)

**Table 7**

| Reaction mixture No. | dipeptide | conc.(mmol/L) |
|---|---|---|
| 1 | Cl-Ala-Phe | 3.5 |
| | Azc-Phe | 5.3 |
| 2 | Cl-Ala | 8.4 |
| | (Cl-Ala)X2 | overlap |
| 3 | Ala-Cl-Phe | 4.3 |
| 4 | Ala-NH₂-Phe | 10.6 |
| | Ala-Ala | 0.3 |
| 5 | Ala-Kinurenine | 4.0 |
| 6 | Ala-Phe-NH₂ | 9.7 |
| 7 | Ala-Glu(OMe) | 6.5 |
| | Ala-Ala | 0.5 |
| 8 | Ala-Glu(OtBu) | 9.7 |
| 9 | Ala-Asp(OtBu) | 10.6 |
| 10 | Ala-Lys(Boc) | 6.6 |
| 11 | Ala-Cyc(3)Ala | 10.5 |
| 12 | Ala-Cyc(6)Ala | 6.6 |

The term "overlap" in the table means that an accurately fixed quantity could not be determined because the signals were overlapped.

It was thus revealed that a dipeptide derivative in which an amino acid and an amino acid derivative are directly linked by peptide bond can be produced using the amino acid and amino acid derivative as substrates according to the process of the present invention.

### Example 2

### Production of N-[2-(Acetylamino)propionyl]phenylalanine

L-Ala-L-Phe obtained in Experimental Example 6 (100 mg, 0.423 mmol) is suspended in methylene chloride (10 ml), and pyridine (10 ml) and acetic anhydride (1 ml, 11 mmol) are added to the suspension at room temperature. After stirring at room temperature for 24 hours, water is added and the resulting mixture is extracted three times with chloroform. The organic layer is washed with a saturated aqueous solution of sodium chloride and dried over anhydrous magnesium sulfate, and then the solvent is distilled away under reduced pressure to obtain N-[2-(acetylamino)propionyl]phenylalanine.

### Example 3

### Production of 1-{2-[N-((Acetylamino)acetyl)amino]-3-phenylpropionyl}piperidine

N-[2-(Acetylamino)propionyl]phenylalanine obtained in Example 2 (10 mg, 0.036 mmol) is suspended in N,N-dimethylformamide (5 ml), and 1-(3-dimethylaminopropyl)-3-ethylcarbodiimide hydrochloride (14 mg, 0.073 mmol), 1-hydroxybenzotriazole (15 mg, 0.11 mmol) and piperidine (40 µl, 0.40 mmol) are added to the suspension at room temperature, followed by stirring at 50°C for 24 hours. To the reaction mixture is added water, and the resulting mixture is extracted three times with chloroform. The organic layer is washed with 10% hydrochloric acid and a saturated aqueous solution of sodium chloride, and dried over anhydrous magnesium sulfate. After the solvent is distilled away under reduced pressure, the residue is purified by silica gel column chromatography to obtain 1-{2-[N-(2-(acetylamino)propionyl)amino]-3-phenylpropionyl}piperidine.

### SEQUENCE LISTING FREE TEXT

SEQ ID NO: 19 - Description of Artificial Sequence: Synthetic DNA
SEQ ID NO: 20 - Description of Artificial Sequence: Synthetic DNA
SEQ ID NO: 21 - Description of Artificial Sequence: Synthetic DNA
SEQ ID NO: 22 - Description of Artificial Sequence: Synthetic DNA
SEQ ID NO: 23 - Description of Artificial Sequence: Synthetic DNA
SEQ ID NO: 24 - Description of Artificial Sequence: Synthetic DNA
SEQ ID NO: 25 - Description of Artificial Sequence: Synthetic DNA
SEQ ID NO: 26 - Description of Artificial Sequence: Synthetic DNA
SEQ ID NO: 27 - Description of Artificial Sequence: Synthetic DNA
SEQ ID NO: 28 - Description of Artificial Sequence: Synthetic DNA
SEQ ID NO: 29 - Description of Artificial Sequence: Synthetic DNA
SEQ ID NO: 30 - Description of Artificial Sequence: Synthetic DNA
SEQ ID NO: 31 - Description of Artificial Sequence: Synthetic DNA
SEQ ID NO: 32 - Description of Artificial Sequence: Synthetic DNA
SEQ ID NO: 33 - Description of Artificial Sequence: Amino acid sequence used for database search
SEQ ID NO: 34 - Description of Artificial Sequence: Amino acid sequence used for database search
SEQ ID NO: 35 - Description of Artificial Sequence: Amino acid sequence used for database search
SEQ ID NO: 41 - Description of Artificial Sequence: Synthetic DNA
SEQ ID NO: 42 - Description of Artificial Sequence: Synthetic DNA
SEQ ID NO: 52 - Description of Artificial Sequence: Synthetic DNA
SEQ ID NO: 53 - Description of Artificial Sequence: Synthetic DNA
SEQ ID NO: 54 - Description of Artificial Sequence: Synthetic DNA
SEQ ID NO: 55 - Description of Artificial Sequence: Synthetic DNA
SEQ ID NO: 56 - Description of Artificial Sequence: Synthetic DNA
SEQ ID NO: 57- Description of Artificial Sequence: Synthetic DNA
SEQ ID NO: 58 - Description of Artificial Sequence: Synthetic DNA
SEQ ID NO: 59 - Description of Artificial Sequence: Synthetic DNA
SEQ ID NO: 60 - Description of Artificial Sequence: Synthetic DNA
SEQ ID NO: 61 - Description of Artificial Sequence: Synthetic DNA
SEQ ID NO: 62 - Description of Artificial Sequence: Synthetic DNA
SEQ ID NO: 63 - Description of Artificial Sequence: Synthetic DNA
SEQ ID NO: 64 - Description of Artificial Sequence: Synthetic DNA
SEQ ID NO: 65 - Description of Artificial Sequence: Synthetic DNA
SEQ ID NO: 66 - Description of Artificial Sequence: Synthetic DNA
SEQ ID NO: 67 - Description of Artificial Sequence: Synthetic DNA
SEQ ID NO: 68 - Description of Artificial Sequence: Synthetic DNA
SEQ ID NO: 69 - Description of Artificial Sequence: Synthetic DNA
SEQ ID NO: 70 - Description of Artificial Sequence: Synthetic DNA .
SEQ ID NO: 71 - Description of Artificial Sequence: Synthetic DNA
SEQ ID NO: 72 - Description of Artificial Sequence: Synthetic DNA
SEQ ID NO: 73 - Description of Artificial Sequence: Synthetic DNA
SEQ ID NO: 74 - Description of Artificial Sequence: Synthetic DNA
SEQ ID NO: 55 - Description of Artificial Sequence: Synthetic DNA
SEQ ID NO: 76 - Description of Artificial Sequence: Synthetic DNA
SEQ ID NO: 77 - Description of Artificial Sequence: Synthetic DNA
SEQ ID NO: 78 - Description of Artificial Sequence: Synthetic DNA
SEQ ID NO: 79 - Description of Artificial Sequence: Synthetic DNA
SEQ ID NO: 80 - Description of Artificial Sequence: Synthetic DNA
SEQ ID NO: 81 - Description of Artificial Sequence: Synthetic DNA
SEQ ID NO: 82 - Description of Artificial Sequence: Synthetic DNA
SEQ ID NO: 83 - Description of Artificial Sequence: Synthetic DNA
SEQ ID NO: 84 - Description of Artificial Sequence: Synthetic DNA
SEQ ID NO: 85 - Description of Artificial Sequence: Synthetic DNA

## Claims

1. A process for producing a dipeptide or a dipeptide derivative(PI) (hereinafter referred to as dipeptide or dipeptide derivative PI), which comprises: allowing the protein shown in any of the following [1] to [7], one or more kinds of amino acids or amino acid derivatives and ATP to be present in an aqueous medium; allowing the dipeptide or dipeptide derivative PI to form and accumulate in the aqueous medium; and recovering the dipeptide or dipeptide derivative PI from the aqueous medium;
[1] a protein having the amino acid sequence shown in any of SEQ ID NOS: 1 to 8;
[2] a protein consisting of an amino acid sequence wherein one or more amino acid residues are deleted, substituted or added in the amino acid sequence shown in any of SEQ ID NOS: 1 to 8 and having the activity to form the dipeptide or dipeptide derivative PI from one or more kinds of amino acids or amino acid derivatives;
[3] a protein consisting of an amino acid sequence which has 65% or more homology to the amino acid sequence shown in any of SEQ ID NOS: 1 to 8 and having the activity to form the dipeptide or dipeptide derivative PI from one or more kinds of amino acids or amino acid derivatives;
[4] a protein consisting of an amino acid sequence which has 80% or more homology to the amino acid sequence shown in SEQ ID NO: 17 and having the activity to form the dipeptide or dipeptide derivative PI from one or more kinds of amino acids or amino acid derivatives;
[5] a protein having the amino acid sequence shown in SEQ ID NO: 37 or 38;
[6] a protein consisting of an amino acid sequence wherein one or more amino acid residues are deleted, substituted or added in the amino acid sequence shown in SEQ ID NO: 37 or 38 and having the activity to form the dipeptide or dipeptide derivative PI from one or more kinds of amino acids or amino acid derivatives;
[7] a protein consisting of an amino acid sequence which has 65% or more homology to the amino acid sequence shown in SEQ ID NO: 37 or 38 and having the activity to form the dipeptide or dipeptide derivative PI from one or more kinds of amino acids or amino acid derivatives;
provided that a compound wherein the amino acids selected from the following Amino acid group A are the same or different, and are bound by peptide bond is excluded from the dipeptide or dipeptide derivative PI:
Amino acid group A; L-alanine, L-glutamine, L-glutamic acid, L-valine, L-leucine, L-isoleucine, L-proline, L-phenylalanine, L-tryptophan, L-methionine, L-serine, L-threonine, L-cysteine, L-asparagine, L-tyrosine, L-lysine, L-arginine, L-histidine, L-aspartic acid, L-α-aminobutanoic acid, L-azaserine, L-theanine, L-4-hydroxyproline, L-3-hydroxyproline, L-ornithine, L-citrulline, L-6-diazo-5-oxo-norleucine, glycine and β-alanine.

2. A process for producing a dipeptide or a dipeptide derivative (hereinafter referred to as dipeptide or dipeptide derivative PII), which comprises:
allowing the protein shown in any of following [1] to [7], one or more kinds of amino acids or amino acid derivatives and ATP to be present in an aqueous medium;
allowing dipeptide or dipeptide derivative PI to form and accumulate in the aqueous medium;
subjecting the dipeptide or dipeptide derivative PI, as such or after recovery, to modification to form the dipeptide or dipeptide derivative PII; and
recovering the dipeptide or dipeptide derivative PII;
[1] a protein having the amino acid sequence shown in any of SEQ ID NOS: 1 to 8;
[2] a protein consisting of an amino acid sequence wherein one or more amino acid residues are deleted, substituted or added in the amino acid sequence shown in any of SEQ ID NOS: 1 to 8 and having the activity to form the dipeptide or dipeptide derivative PI from one or more kinds of amino acids or amino acid derivatives;
[3] a protein consisting of an amino acid sequence which has 65% or more homology to the amino acid sequence shown in any of SEQ ID NOS: 1 to 8 and having the activity to form the dipeptide or dipeptide derivative PI from one or more kinds of amino acids or amino acid derivatives;
[4] a protein consisting of an amino acid sequence which has 80% or more homology to the amino acid sequence shown in SEQ ID NO: 17 and having the activity to form the dipeptide or dipeptide derivative PI from one or more kinds of amino acids or amino acid derivatives;
[5] a protein having the amino acid sequence shown in SEQ ID NO: 37 or 38;
[6] a protein consisting of an amino acid sequence wherein one or more amino acid residues are deleted, substituted or added in the amino acid sequence shown in SEQ ID NO: 37 or 38 and having the activity to form the dipeptide or dipeptide derivative PI from one or more kinds of amino acids or amino acid derivatives;
[7] a protein consisting of an amino acid sequence which has 65% or more homology to the amino acid sequence shown in SEQ ID NO: 37 or 38 and having the activity to form the dipeptide or dipeptide derivative PI from one or more kinds of amino acids or amino acid derivatives;
provided that a compound wherein the amino acids selected from the Amino acid group A described in the above are the same or different, and are bound by peptide bond is excluded from the dipeptide or dipeptide derivative PII.

3. A process for producing a dipeptide or a dipeptide derivative PI, which comprises:
allowing an enzyme source and one or more kinds of amino acids or amino acid derivatives to be present in an aqueous medium, said enzyme source being a culture or a treated matter of the culture of cells having the DNA selected from the following [1] to [5];
[1] DNA having the nucleotide sequence shown in any of SEQ ID NOS: 9 to 16 and 36;
[2] DNA which hybridizes with DNA having a nucleotide sequence complementary to the nucleotide sequence shown in' any of SEQ ID NOS: 9 to 16 and 36 under stringent conditions and which encodes a protein having the activity to form the dipeptide or dipeptide derivative PI from one or more kinds of amino acids or amino acid derivatives;
[3] DNA which hybridizes with DNA having a nucleotide sequence complementary to the nucleotide sequence shown in SEQ ID NO: 18 under stringent conditions and which encodes a protein having the activity to form the dipeptide or dipeptide derivative PI from one or more kinds of amino acids or amino acid derivatives;
[4] DNA having the nucleotide sequence shown in SEQ ID NO: 39 or 40;
[5] DNA which hybridizes with DNA having a nucleotide sequence complementary to the nucleotide sequence shown in SEQ ID NO: 39 or 40 under stringent conditions and which encodes a protein having the activity to form the dipeptide or dipeptide derivative PI from one or more kinds of amino acids or amino acid derivatives;
provided that a compound wherein the amino acids selected from the Amino acid group A described in the above are the same or different, and are bound by peptide bond is excluded from the dipeptide or dipeptide derivative PI.

4. A process for producing a dipeptide or a dipeptide derivative PII, which comprises:
allowing an enzyme source and one or more kinds of amino acids or amino acid derivatives to be present in an aqueous medium, said enzyme source being a culture or a treated matter of the culture of cells having the DNA selected from the following [1] to [5];
allowing dipeptide or dipeptide derivative PI to form and accumulate in the aqueous medium;
subjecting the dipeptide or dipeptide derivative PI, as such or after recovery, to modification to form the dipeptide or dipeptide derivative PII; and
recovering the dipeptide or dipeptide derivative PII;
[1] DNA having the nucleotide sequence shown in any of SEQ ID NOS: 9 to 16 and 36;
[2] DNA which hybridizes with DNA having a nucleotide sequence complementary to the nucleotide sequence shown in any of SEQ ID NOS: 9 to 16 and 36 under stringent conditions and which encodes a protein having the activity to form the dipeptide or dipeptide derivative PI from one or more kinds of amino acids or amino acid derivatives;
[3] DNA which hybridizes with DNA having a nucleotide sequence complementary to the nucleotide sequence shown in SEQ ID NO: 18 under stringent conditions and which encodes a protein having the activity to form the dipeptide or dipeptide derivative PI from one or more kinds of amino acids or amino acid derivatives;
[4] DNA having the nucleotide sequence shown in SEQ ID NO: 39 or 40;
[5] DNA which hybridizes with DNA having a nucleotide sequence complementary to the nucleotide sequence shown in SEQ ID NO: 39 or 40 under stringent conditions and which encodes a protein having the activity to form the dipeptide or dipeptide derivative PI from one or more kinds of amino acids or amino acid derivatives.

5. The process according to any of Claims 1 to 4, wherein the amino acids or amino acid derivatives are amino acids or amino acid derivatives represented by formula (I): 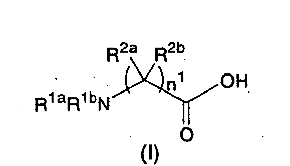 (wherein n¹ represents an integer of 1 to 3;
R^{1a} and R^{1b}, which may be the same or different, each represent a hydrogen atom, substituted or unsubstituted lower alkyl, substituted or unsubstituted lower alkenyl, substituted or unsubstituted lower alkynyl, substituted or unsubstituted aralkyl, substituted or unsubstituted lower alkanoyl, substituted or unsubstituted lower alkoxycarbonyl, substituted or unsubstituted aryl, or substituted or unsubstituted aroyl, or either R^{1a} or
R^{1b} may form a substituted or unsubstituted heterocyclic group together with the adjacent nitrogen atom, the carbon atom adjacent to the nitrogen atom and either R^{2a} or R^{2b} on the carbon atom; and
R^{2a} and R^{2b}, which may be the same or different, each represent a hydrogen atom, substituted or unsubstituted lower alkyl, substituted or unsubstituted aralkyl, substituted or unsubstituted aryl, or substituted or unsubstituted heterocyclic alkyl, or either R^{2a} or R^{2b} on the carbon atom adjacent to R^{1a}R^{1b}N may form a substituted or unsubstituted heterocyclic group together with the adjacent carbon atom, the nitrogen atom adjacent to the carbon atom and either R^{1a} or R^{1b}, and when n¹ is 2 or 3, two or three R^{2a}s and two or three R^{2b}s may be the same or different, respectively),
or formula (II): 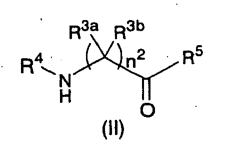 [wherein n² has the same significance as the above n¹;
R^{3a} and R^{3b}, which may be the same or different, each represent a hydrogen atom, substituted or unsubstituted lower alkyl, substituted or unsubstituted aralkyl, substituted or unsubstituted aryl, or substituted or unsubstituted heterocyclic alkyl, or either R^{3a} or R^{3b} on the carbon atom adjacent to R⁴HN may form a substituted or unsubstituted heterocyclic group together with the adjacent carbon atom, the nitrogen atom adjacent to the carbon atom and R⁴, and when n² is 2 or 3, two or three R^{3a}s and two or three R^{3b}s may be the same or different, respectively;
R⁴ represents a hydrogen atom, substituted or unsubstituted lower alkyl, substituted or unsubstituted lower alkenyl, substituted or unsubstituted lower alkynyl, substituted or unsubstituted aralkyl, substituted or unsubstituted lower alkanoyl, substituted or unsubstituted lower alkoxycarbonyl, substituted or unsubstituted aryl, or substituted or unsubstituted aroyl, or R⁴ may form a substituted or unsubstituted heterocyclic group together with the adjacent nitrogen atom, the carbon atom adjacent to the nitrogen atom and either R^{3a} or
R^{3b} on the carbon atom; and
R⁵ represents amino, hydroxy, substituted or unsubstituted lower alkoxy, mono(substituted or unsubstituted lower alkyl)amino, di(substituted or unsubstituted lower alkyl)amino, or an alicyclic heterocyclic group],
provided that when all the amino acids or amino acid derivatives are amino acids or amino acid derivatives represented by formula (I), at least one of R^{1a} and R^{1b} is a hydrogen atom, and when all the amino acids or amino acid derivatives are amino acids or amino acid derivatives represented by formula (II), R⁵ is hydroxy.

6. The process according to any of Claims 1 to 4, wherein the amino acids or amino acid derivatives are amino acids or amino acid derivatives represented by formula (III): 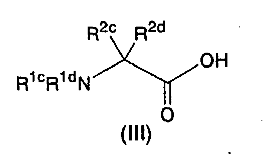 (wherein R^{1c} and R^{1d}, which may be the same or different, each represent a hydrogen atom, substituted or unsubstituted lower alkyl, substituted or unsubstituted lower alkenyl, substituted or unsubstituted lower alkynyl, substituted or unsubstituted aralkyl, substituted or unsubstituted lower alkanoyl, substituted or unsubstituted lower alkoxycarbonyl, substituted or unsubstituted aryl, or substituted or unsubstituted aroyl; and
R^{2c} and R^{2d}, which may be the same or different, each represent a hydrogen atom or substituted or unsubstituted lower alkyl),
or formula (IV): 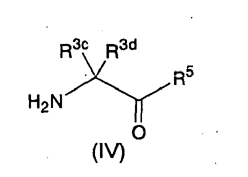 (wherein R^{3c} and R^{3d}, which may be the same or different, each represent a hydrogen atom, substituted or unsubstituted lower alkyl, substituted or unsubstituted aralkyl, or substituted or unsubstituted aryl; and
R⁵ has the same significance as defined above), provided that when all the amino acids or amino acid derivatives are amino acids or amino acid derivatives represented by formula (III), at least one of R^{1c} and
R^{1d} is a hydrogen atom, and when all the amino acids or amino acid derivatives are amino acids or amino acid derivatives represented by formula (IV), R⁵ is hydroxy.

7. The process according to any of Claims 1 to 4, wherein the amino acids or amino acid derivatives are amino acids or amino acid derivatives represented by formula (V): 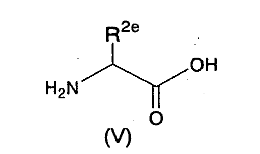 (wherein R^{2e} represents substituted or unsubstituted methyl),
or formula (VI): 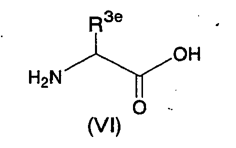 (wherein R^{3e} represents substituted or unsubstituted lower alkyl, substituted or unsubstituted aralkyl, or substituted or unsubstituted aryl).

8. The process according to any of Claims 1 to 4, wherein the amino acids or amino acid derivatives are amino acids selected from the group consisting of L-amino acids, glycine and β-alanine, or derivatives thereof.

9. The process according to Claim 8, wherein the L-amino acid is an L-amino acid selected from the group consisting of L-alanine, L-glutamine, L-glutamic acid, L-valine, L-leucine, L-isoleucine, L-proline, L-phenylalanine, L-tryptophan, L-methionine, L-serine, L-threonine, L-cysteine, L-asparagine, L-tyrosine, L-lysine, L-arginine, L-histidine, L-aspartic acid, L-α-aminobutyric acid, L-azaserine, L-theanine, L-4-hydroxyproline, L-3-hydroxyproline, L-ornithine, L-citrulline and L-6-diazo-5-oxo-norleucine.

10. The process according to any of Claims 1 to 5, wherein the dipeptide or dipeptide derivative PI is a dipeptide or a dipeptide derivative represented by formula (VIIa): 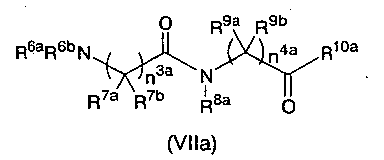 [wherein n^{3a} and n^{4a} each have the same significance as the above n¹;
R^{6a} and R^{6b}, which may be the same or different, each represent a hydrogen atom, substituted or unsubstituted lower alkyl, substituted or unsubstituted lower alkenyl, substituted or unsubstituted lower alkynyl, substituted or unsubstituted aralkyl, substituted or unsubstituted lower alkanoyl, substituted or unsubstituted lower alkoxycarbonyl, substituted or unsubstituted aryl, or substituted or unsubstituted aroyl, or either R^{6a} or R^{6b} may form a substituted or unsubstituted heterocyclic group together with the adjacent nitrogen atom, the carbon atom adjacent to the nitrogen atom and either R^{7a} or R^{7b} on the carbon atom;
R^{7a} and R^{7b}, which may be the same or different, each represent a hydrogen atom, substituted or unsubstituted lower alkyl, substituted or unsubstituted aralkyl, substituted or unsubstituted aryl, or substituted or unsubstituted heterocyclic alkyl, or either R^{7a} or R^{7b} on the carbon atom adjacent to R^{6a}R^{6b}N may form a substituted or unsubstituted heterocyclic group together with the adjacent carbon atom, the nitrogen atom adjacent to the carbon atom and either R^{6a} or R^{6b}, and when n^{3a} is 2 or 3, two or three R^{7a}s and two or three R^{7b}s may be the same or different, respectively;
R^{8a} represents a hydrogen atom, substituted or unsubstituted lower alkyl, substituted or unsubstituted lower alkenyl, substituted or unsubstituted lower alkynyl, substituted or unsubstituted aralkyl, substituted or unsubstituted lower alkanoyl, substituted or unsubstituted lower alkoxycarbonyl, substituted or unsubstituted aryl, or substituted or unsubstituted aroyl, or R^{8a} may form a substituted or unsubstituted heterocyclic group together with the adjacent nitrogen atom, the carbon atom adjacent to the nitrogen atom and bound to R^{9a} and R^{9b}, and either R^{9a} or R^{9b} on the carbon atom;
R^{9a} and R^{9b}, which may be the same or different, each represent a hydrogen atom, substituted or unsubstituted lower alkyl, substituted or unsubstituted aralkyl, substituted or unsubstituted aryl, or substituted or unsubstituted heterocyclic alkyl, or either R^{9a} or R^{9b} on the carbon atom adjacent to -R^{8a}N- may form a substituted or unsubstituted heterocyclic group together with the adjacent carbon atom, the nitrogen atom adjacent to the carbon atom and R^{8a}, and when n^{4a} is 2 or 3, two or three R^{9a}s and two or three R^{9b}s may be the same or different, respectively; and
R^{10a} represents amino, hydroxy, substituted or unsubstituted lower alkoxy, mono(substituted or unsubstituted lower alkyl)amino, di(substituted or unsubstituted lower alkyl)amino, or an alicyclic heterocyclic group].

11. The process according to any of Claims 1 to 5, wherein the dipeptide or dipeptide derivative PII is a dipeptide or a dipeptide derivative represented by formula (VIIb): 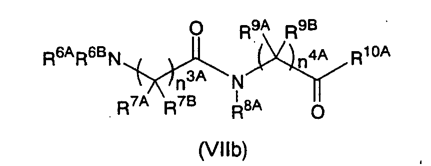 (wherein n^{3A} and n^{4A} each have the same significance as the above n¹;
R^{6A} and R^{6B}, which may be the same or different, each represent a hydrogen atom, substituted or unsubstituted lower alkyl, substituted or unsubstituted lower alkenyl, substituted or unsubstituted lower alkynyl, substituted or unsubstituted aralkyl, substituted or unsubstituted lower alkanoyl, substituted or unsubstituted lower alkoxycarbonyl, substituted or unsubstituted aryl, or substituted or unsubstituted aroyl, or either R^{6A} or
R^{6B} may form a substituted or unsubstituted heterocyclic group together with the adjacent nitrogen atom, the carbon atom adjacent to the nitrogen atom and either R^{7A} or R^{7B} on the carbon atom;
R^{7A} and R^{7B}, which may be the same or different, each represent a hydrogen atom, substituted or unsubstituted lower alkyl, substituted or unsubstituted aralkyl, substituted or unsubstituted aryl, or substituted or unsubstituted heterocyclic alkyl, or either R^{7A} or R^{7B} on the carbon atom adjacent to R^{6A}R^{6B}N may form a substituted or unsubstituted heterocyclic group together with the adjacent carbon atom, the nitrogen atom adjacent to the carbon atom and either R^{6A} or R^{6B}, and when n^{3A} is 2 or 3, two or three R^{7A}s and two or three R^{7B}s may be the same or different, respectively;
R^{8A} represents a hydrogen atom, substituted or unsubstituted lower alkyl, substituted or unsubstituted lower alkenyl, substituted or unsubstituted lower alkynyl, substituted or unsubstituted aralkyl, substituted or unsubstituted lower alkanoyl, substituted or unsubstituted lower alkoxycarbonyl, substituted or unsubstituted aryl, or substituted or unsubstituted'aroyl, or R^{8A} may form a substituted or unsubstituted heterocyclic group together with the adjacent nitrogen atom, the carbon atom adjacent to the nitrogen atom and bound to R^{9A} and R^{9B}, and either R^{9A} or R^{9B} on the carbon atom;
R^{9A} and R^{9B}, which may be the same or different, each represent a hydrogen atom, substituted or unsubstituted lower alkyl, substituted or unsubstituted aralkyl, substituted or unsubstituted aryl, or substituted or unsubstituted heterocyclic alkyl, or either R^{9A} or R^{9B} on the carbon atom adjacent to -R^{8A}N- may form a substituted or unsubstituted heterocyclic group together with the adjacent carbon atom, the nitrogen atom adjacent to the carbon atom and R^{8A}, and when n^{4A} is 2 or 3, two or three R^{9A}s and two or three R^{9B}s may be the same or different, respectively; and
R^{10A} has the same significance as the above R^{10a}).

12. The process according to any of Claims 1 to 6, wherein the dipeptide or dipeptide derivative PI is a dipeptide or a dipeptide derivative represented by formula (VIIIa): 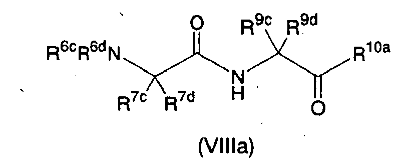 (wherein R^{6c} and R^{6d}, which may be the same or different, each represent a hydrogen atom, substituted or unsubstituted lower alkyl, substituted or unsubstituted lower alkenyl, substituted or unsubstituted lower alkynyl, substituted or unsubstituted aralkyl, substituted or unsubstituted lower alkanoyl, substituted or unsubstituted lower alkoxycarbonyl, substituted or unsubstituted aryl, or substituted or unsubstituted aroyl;
R^{7c} and R^{7d}, which may be the same or different, each represent a hydrogen atom or substituted or unsubstituted lower alkyl;
R^{9c} and R^{9d}, which may be the same or different, each represent a hydrogen atom, substituted or unsubstituted lower alkyl, substituted or unsubstituted aralkyl, or substituted or unsubstituted aryl; and
R^{10a} has the same significance as defined above) .

13. The process according to any of Claims 1 to 6, wherein the dipeptide or dipeptide derivative PII is a dipeptide or a dipeptide derivative represented by formula (VIIIb): 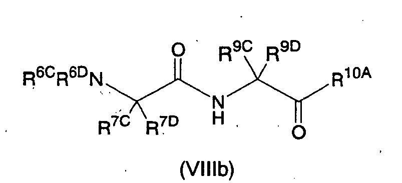 (wherein R^{6C}, R^{6D}, R^{7C}, R^{7D}, R^{9C} and R^{9D} have the same significances as the above R^{6c}, R^{6d}, R^{7c}, R^{7d}, R^{9c} and
R^{9d}, respectively; and
R^{10A} has the same significance as defined above).

14. The process according to any of Claims 1 to 7, wherein the dipeptide or dipeptide derivative PI is a dipeptide or a dipeptide derivative represented by formula (IXa): 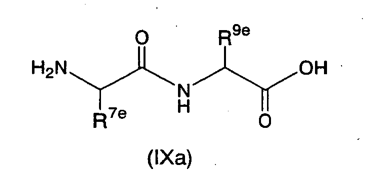 (wherein R^{7e} represents substituted or unsubstituted methyl ; and
R^{9e} represents substituted or unsubstituted lower alkyl, substituted or unsubstituted aralkyl, or substituted or unsubstituted aryl).

15. The process according to any of Claims 1 to 7, wherein the dipeptide or dipeptide derivative PII is a dipeptide or a dipeptide derivative represented by formula (IXb): 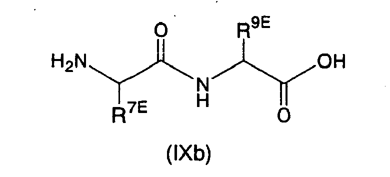 (wherein R^{7E} and R^{9E} have the same significances as the above R^{7e} and R^{9e}, respectively).

16. The process according to any of Claims 1 to 8, wherein the dipeptide or dipeptide derivative PI or dipeptide or dipeptide derivative PII is a dipeptide or a dipeptide derivative in which the same or different amino acids or amino acid derivatives selected from the group consisting of L-amino acids, glycine, β-alanine and their derivatives are linked with each other by peptide bond.

17. The process according to Claim 16, wherein the L-amino acid is an L-amino acid selected from the group consisting of L-alanine, L-glutamine, L-glutamic acid, L-valine, L-leucine, L-isoleucine, L-proline, L-phenylalanine, L-tryptophan, L-methionine, L-serine, L-threonine, L-cysteine, L-asparagine, L-tyrosine, L-lysine, L-arginine, L-histidine, L-aspartic acid, L-α-aminobutyric acid, L-azaserine, L-theanine, L-4-hydroxyproline, L-3-hydroxyproline, L-ornithine, L-citrulline and L-6-diazo-5-oxo-norleucine.

18. The process according to any of Claims 3 to 17, wherein the cells are cells of a microorganism.

19. The process according to Claim 18, wherein the microorganism is a procaryote.

20. The process according to Claim 19, wherein the procaryote is a microorganism in which the activities of one or more kinds of peptidases and one or more kinds of proteins having peptide-permeating/transporting activity (hereinafter referred to also as peptide-permeating/transporting proteins) are reduced or lost.

21. The process according to Claim 20, wherein the procaryote is a microorganism in which the activities of three or more kinds of peptidases are reduced or lost.

22. The process according to Claims 20 or 21, wherein the peptidase is a protein having the amino acid sequence shown in any of SEQ ID NOS: 43 to 46, or a protein having an amino acid sequence which has 80% or more homology to the amino acid sequence shown in any of SEQ ID NOS: 43 to 46 and having peptidase activity.

23. The process according to Claim 20 or 22, wherein the peptide-permeating/transporting protein is a protein having the amino acid sequence shown in any of SEQ ID NOS: 47 to 51, or a protein having an amino acid sequence which has 80% or more homology to the amino acid sequence shown in any of SEQ ID NOS: 47 to 51 and having peptide-permeating/transporting activity.

24. The process according to any of Claims 19 to 23, wherein the procaryote is a microorganism belonging to the genus Escherichia, Bacillus or Corynebacterium.

25. The process according to Claim 24, wherein the microorganism belonging to the genus Escherichia, Bacillus or Corynebacterium is Escherichia coli, Corynebacterium glutamicum, Corynebacterium ammoniagenes, Corynebacterium lactofermentum, Corynebacterium flavum, Corynebacterium efficiens, Bacillus subtilis or Bacillus megaterium.

26. The process according to any of Claims 3 to 25, wherein the treated matter of the culture is a treated matter which is selected from the group consisting of concentrated culture, dried culture, cells obtained by centrifuging the culture, dried cells, freeze-dried cells, surfactant-treated cells, ultrasonicated cells, mechanically-disrupted cells, solvent-treated cells, enzymatic-treated cells, protein fractionation of cells, immobilized cells, and an enzyme preparation obtained by extracting the cells, and which has the activity to form dipeptide or dipeptide derivative from one or more kinds of amino acids or amino acid derivatives.


<110> KYOWA HAKKO KOGYO CO., LTD.

<120> Process for producing dipeptides or dipeptide derivatives

<130> 1000P11696WO0

<150> JP2004-189008
<151> 2004-06-25

<160> 85

<170> PatentIn Ver. 2.1

<210> 1
<211> 472
<212> PRT
<213> Bacillus subtilis 168

<400> 1
Met Glu Arg Lys Thr Val Leu Val Ile Ala Asp Leu Gly Gly Cys Pro
  1               5                  10                  15

Pro His Met Phe Tyr Lys Ser Ala Ala Glu Lys Tyr Asn Leu Val Ser
             20                  25                  30

Phe Ile Pro Arg Pro Phe Ala Ile Thr Ala Ser His Ala Ala Leu Ile
         35                  40                  45

Glu Lys Tyr Ser Val Ala Val Ile Lys Asp Lys Asp Tyr Phe Lys Ser
     50                  55                  60

Leu Ala Asp Phe Glu His Pro Asp Ser Ile Tyr Trp Ala His Glu Asp
 65                  70                  75                  80

His Asn Lys Pro Glu Glu Glu Val Val Glu Gln Ile Val Lys Val Ala
                 85                  90                  95

Glu Met Phe Gly Ala Asp Ala Ile Thr Thr Asn Asn Glu Leu Phe Ile
            100                 105                 110

Ala Pro Met Ala Lys Ala Cys Glu Arg Leu Gly Leu Arg Gly Ala Gly
        115                 120                 125

Val Gln Ala Ala Glu Asn Ala Arg Asp Lys Asn Lys Met Arg Asp Ala

    130                 135                 140

Phe Asn Lys Ala Gly Val Lys Ser Ile Lys Asn Lys Arg Val Thr Thr
145                 150                 155                 160

Leu Glu Asp Phe Arg Ala Ala Leu Glu Glu Ile Gly Thr Pro Leu Ile
                165                 170                 175

Leu Lys Pro Thr Tyr Leu Ala Ser Ser Ile Gly Val Thr Leu Ile Thr
            180                 185                 190

Asp Thr Glu Thr Ala Glu Asp Glu Phe Asn Arg Val Asn Asp Tyr Leu
        195                 200                 205

Lys Ser Ile Asn Val Pro Lys Ala Val Thr Phe Glu Ala Pro Phe Ile

210                 215                 220

Ala Glu Glu Phe Leu Gln Gly Glu Tyr Gly Asp Trp Tyr Gln Thr Glu
225                 230                 235                 240

Gly Tyr Ser Asp Tyr Ile Ser Ile Glu Gly Ile Met Ala Asp Gly Glu
                245                 250                 255

Tyr Phe Pro Ile Ala Ile His Asp Lys Thr Pro Gln Ile Gly Phe Thr
            260                 265                 270

Glu Thr Ser His Ile Thr Pro Ser Ile Leu Asp Glu Glu Ala Lys Lys
        275                 280                 285

Lys Ile Val Glu Ala Ala Lys Lys Ala Asn Glu Gly Leu Gly Leu Gln
    290                 295                 300

Asn Cys Ala Thr His Thr Glu Ile Lys Leu Met Lys Asn Arg Glu Pro
305                 310                 315                 320

Gly Leu Ile Glu Ser Ala Ala Arg Phe Ala Gly Trp Asn Met Ile Pro
                325                 330                 335

Asn Ile Lys Lys Val Phe Gly Leu Asp Met Ala Gln Leu Leu Leu Asp
            340                 345                 350

Val Leu Cys Phe Gly Lys Asp Ala Asp Leu Pro Asp Gly Leu Leu Asp
        355                 360                 365

Gln Glu Pro Tyr Tyr Val Ala Asp Cys His Leu Tyr Pro Gln His Phe
    370                 375                 380

Lys Gln Asn Gly Gln Ile Pro Glu Thr Ala Glu Asp Leu Val Ile Glu
385                 390                 395                 400

Ala Ile Asp Ile Pro Asp Gly Leu Leu Lys Gly Asp Thr Glu Ile Val
                405                 410                 415

Ser Phe Ser Ala Ala Ala Pro Gly Thr Ser Val Asp Leu Thr Leu Phe
            420                 425                 430

Glu Ala Phe Asn Ser Ile Ala Ala Phe Glu Leu Lys Gly Ser Asn Ser
        435                 440                 445

Gln Asp Val Ala Glu Ser Ile Arg Gln Ile Gln Gln His Ala Lys Leu
    450                 455                 460

Thr Ala Lys Tyr Val Leu Pro Val
465                 470


<210> 2
<211> 472
<212> PRT
<213> Bacillus subtilis ATCC6633

<400> 2
Met Glu Arg Lys Thr Val Leu Val Ile Ala Asp Leu Gly Gly Cys Pro
  1               5                  10                  15

Pro His Met Phe Tyr Lys Ser Ala Ala Glu Lys Tyr Asn Leu Val Ser
            20                  25                  30

Phe Ile Pro Arg Pro Phe Ala Ile Thr Ala Ser His Ala Ala Leu Ile
        35                  40                  45

Glu Lys Tyr Ser Val Ala Val Ile Lys Asp Lys Asp Tyr Phe Gln Ser
    50                  55                  60

Leu Ala Asp Phe Glu His Pro Asp Ser Ile Tyr Trp Ala His Glu Asp
65                  70                  75                  80

His Asp Lys Pro Glu Glu Glu Val Val Glu Gln Ile Val Lys Val Ala
                85                  90                  95

Gln Met Phe Glu Ala Asp Ala Ile Thr Thr Asn Asn Glu Leu Phe Ile
            100                 105                 110

Ala Pro Met Ala Lys Ala Cys Glu Arg Leu Gly Leu Arg Gly Ala Gly
        115                 120                 125

Val Gln Ala Ala Glu Asn Ala Arg Asp Lys Asn Lys Met Arg Asp Ala
    130                 135                 140

Phe Asn Lys Ala Gly Val Lys Ser Ile Lys Asn Lys Arg Val Thr Thr
145                 150                 155                 160

Leu Glu Asp Phe Arg Ala Ala Leu Glu Glu Ile Gly Thr Pro Leu Ile
                165                 170                 175

Leu Lys Pro Thr Tyr Leu Ala Ser Ser Ile Gly Val Thr Leu Ile Thr
            180                 185                 190

Asp Thr Glu Thr Ala Glu Asp Glu Phe Asn Arg Val Asn Asp Tyr Leu
        195                 200                 205

Lys Ser Ile Asn Val Pro Lys Ala Val Thr Phe Glu Ala Pro Phe Ile
    210                 215                 220

Ala Glu Glu Phe Leu Gln Gly Glu Tyr Gly Asp Trp Tyr Gln Thr Glu
225                 230                 235                 240

Gly Tyr Ser Asp Tyr Ile Ser Ile Glu Gly Ile Met Ala Asp Gly Glu
                245                 250                 255

Tyr Phe Pro Ile Ala Ile His Asp Lys Thr Pro Gln Ile Gly Phe Thr
            260                 265                 270

Glu Thr Ser His Ile Thr Pro Ser Ile Leu Asp Glu Glu Ala Lys Lys
        275                 280                 285

Lys Ile Val Glu Ala Ala Lys Lys Ala Asn Glu Gly Leu Gly Leu Gln
    290                 295                 300

Asn Cys Ala Thr His Thr Glu Ile Lys Leu Met Lys Asn Arg Glu Pro
305                 310                 315                 320

Gly Leu Ile Glu Ser Ala Ala Arg Phe Ala Gly Trp Asn Met Ile Pro
                325                 330                 335

Asn Ile Lys Lys Val Phe Gly Leu Asp Met Ala Gln Leu Leu Leu Asp
            340                 345                 350

Val Leu Cys Phe Gly Lys Asp Ala Asp Leu Pro Asp Gly Leu Leu Asp
        355                 360                 365

Gln Glu Pro Tyr Tyr Val Ala Asp Cys His Leu Tyr Pro Gln His Phe
    370                 375                 380

Lys Gln Asn Gly Gln Ile Pro Glu Thr Ala Glu Asp Leu Val Ile Glu
385                 390                 395                 400

Ala Ile Asp Ile Pro Asp Gly Leu Leu Lys Gly Asp Thr Glu Ile Val
                405                 410                 415

Thr Phe Ser Ala Ala Ala Pro Gly Thr Ser Val Asp Leu Thr Leu Phe
            420                 425                 430

Glu Ala Phe Asn Ser Ile Ala Ala Phe Glu Leu Lys Gly Ser Asn Ser
        435                 440                 445

Gln Asp Val Ala Glu Ser Ile Arg Gln Ile Gln Gln His Ala Lys Leu
    450                 455                 460

Thr Ala Lys Tyr Val Leu Pro Val
465                 470


<210> 3
<211> 472
<212> PRT
<213> Bacillus subtilis IAM1213

<400> 3
Met Glu Arg Lys Thr Val Leu Val Ile Ala Asp Leu Gly Gly Cys Pro
  1               5                  10                  15

Pro His Met Phe Tyr Lys Ser Ala Ala Glu Lys Tyr Asn Leu Val Ser
             20                  25                  30

Phe Ile Pro Arg Pro Phe Ala Ile Thr Ala Ser His Ala Ala Leu Ile
         35                  40                  45

Glu Lys Tyr Ser Val Ala Val Ile Lys Asp Lys Asp Tyr Phe Lys Ser
     50                  55                  60

Leu Ala Asp Phe Glu His Pro Asp Ser Ile Tyr Trp Ala His Glu Asp
 65                  70                  75                  80

His Asn Lys Pro Glu Glu Glu Val Val Glu Gln Ile Val Lys Val Ala
                 85                  90                  95

Glu Met Phe Gly Ala Asp Ala Ile Thr Thr Asn Asn Glu Leu Phe Ile
            100                 105                 110

Ala Pro Met Ala Lys Ala Cys Glu Arg Leu Gly Leu Arg Gly Ala Gly
        115                 120                 125

Val Gln Ala Ala Glu Asn Ala Arg Asp Lys Asn Lys Met Arg Asp Ala
    130                 135                 140

Phe Asn Lys Ala Gly Val Lys Ser Ile Lys Asn Lys Arg Val Thr Thr
145                 150                 155                 160

Leu Glu Asp Phe Arg Ala Ala Leu Glu Glu Ile Gly Thr Pro Leu Ile
                165                 170                 175

Leu Lys Pro Thr Tyr Leu Ala Ser Ser Ile Gly Val Thr Leu Ile Thr
            180                 185                 190

Asp Thr Glu Thr Ala Glu Asp Glu Phe Asn Arg Val Asn Asp Tyr Leu
        195                 200                 205

Lys Ser Ile Asn Val Pro Lys Ala Val Thr Phe Glu Ala Pro Phe Ile
    210                 215                 220

Ala Glu Glu Phe Leu Gln Gly Glu Tyr Gly Asp Trp Tyr Gln Thr Glu
225                 230                 235                 240

Gly Tyr Ser Asp Tyr Ile Ser Ile Glu Gly Ile Met Ala Asp Gly Glu
                245                 250                 255

Tyr Phe Pro Ile Ala Ile His Asp Lys Thr Pro Gln Ile Gly Phe Thr
            260                 265                 270

Glu Thr Ser His Ile Thr Pro Ser Ile Leu Asp Glu Glu Ala Lys Lys
        275                 280                 285

Lys Ile Val Glu Ala Ala Lys Lys Ala Asn Glu Gly Leu Gly Leu Gln
    290                 295                 300

Asn Cys Ala Thr His Thr Glu Ile Lys Leu Met Lys Asn Arg Glu Pro
305                 310                 315                 320

Gly Leu Ile Glu Ser Ala Ala Arg Phe Ala Gly Trp Asn Met Ile Pro
                325                 330                 335

Asn Ile Lys Lys Val Phe Gly Leu Asp Met Ala Gln Leu Leu Leu Asp
            340                 345                 350

Val Leu Cys Phe Gly Lys Asp Ala Asp Leu Pro Asp Gly Leu Leu Asp
        355                 360                 365

Gln Glu Pro Tyr Tyr Val Ala Asp Cys His Leu Tyr Pro Gln His Phe
    370                 375                 380

Lys Gln Asn Gly Gln Ile Pro Glu Thr Ala Glu Asp Leu Val Ile Glu
385                 390                 395                 400

Ala Ile Asp Leu Pro Asp Gly Leu Leu Lys Gly Asp Thr Glu Ile Val
                405                 410                 415

Ser Phe Ser Ala Ala Ala Pro Gly Thr Ser Val Asp Leu Thr Leu Phe
            420                 425                 430

Glu Ala Phe Asn Ser Ile Ala Ala Phe Glu Leu Lys Gly Ser Asn Ser
        435                 440                 445

Gln Asp Val Ala Glu Ser Ile Arg Gln Ile Gln Gln His Ala Lys Leu
    450                 455                 460

Thr Ala Lys Tyr Val Leu Pro Val
465                 470

<210> 4
<211> 472
<212> PRT
<213> Bacillus subtilis IAM1107

<400> 4
Met Glu Arg Lys Thr Val Leu Val Ile Ala Asp Leu Gly Gly Cys Pro
1               5                   10                  15

Pro His Met Phe Tyr Lys Ser Ala Ala Glu Lys Tyr Asn Leu Val Ser
            20                  25                  30

Phe Ile Pro Arg Pro Phe Ala Ile Thr Ala Ser His Ala Ala Leu Ile
        35                  40                  45

Glu Lys Tyr Ser Val Ala Val Val Lys Asp Lys Asp Tyr Phe Lys Ser
    50                  55                  60

Leu Ala Asp Phe Glu His Pro Asp Ser Ile Tyr Trp Ala His Glu Asp
65                  70                  75                  80

His Asn Lys Pro Glu Glu Glu Val Val Glu Gln Ile Val Lys Val Ala
                85                  90                  95

Glu Met Phe Gly Ala Asp Ala Ile Thr Thr Asn Asn Glu Leu Phe Ile
            100                 105                 110

Ala Pro Met Ala Lys Ala Cys Glu Arg Leu Gly Leu Arg Gly Ala Gly
        115                 120                 125

Val Gln Ala Ala Glu Asn Ala Arg Asp Lys Asn Lys Met Arg Asp Ala
    130                 135                 140

Phe Asn Lys Ala Gly Val Lys Ser Ile Lys Asn Lys Arg Val Thr Thr
145                 150                 155                 160

Leu Glu Asp Phe Arg Ala Ala Leu Glu Glu Ile Gly Thr Pro Leu Ile
                165                 170                 175

Leu Lys Pro Thr Tyr Leu Ala Ser Ser Ile Gly Val Thr Leu Ile Thr
            180                 185                 190

Asp Thr Glu Thr Ala Glu Asp Glu Phe Asn Arg Val Asn Asp Tyr Leu
        195                 200                 205

Lys Ser Ile Asn Val Pro Lys Ala Val Thr Phe Glu Ala Pro Phe Ile
    210                 215                 220

Ala Glu Glu Phe Leu Gln Gly Glu Tyr Gly Asp Trp Tyr Gln Thr Glu
225                 230                 235                 240

Gly Tyr Ser Asp Tyr Ile Ser Ile Glu Gly Ile Met Ala Asp Gly Glu
                245                 250                 255

Tyr Phe Pro Ile Ala Ile His Asp Lys Thr Pro Gln Ile Gly Phe Thr

            260                 265                 270

Glu Thr Ser His Ile Thr Pro Ser Ile Leu Asp Glu Glu Ala Lys Lys
        275                 280                 285

Lys Ile Val Glu Ala Ala Lys Lys Ala Asn Glu Gly Leu Gly Leu Gln
    290                 295                 300

Asn Cys Ala Thr His Thr Glu Val Lys Leu Met Lys Asn Arg Glu Pro
305                 310                 315                 320

Gly Leu Ile Glu Ser Ala Ala Arg Phe Ala Gly Trp Asn Met Ile Pro
                325                 330                 335

Asn Ile Lys Lys Val Phe Gly Leu Asp Met Ala Gln Leu Leu Leu Asp
            340                 345                 350

Val Leu Cys Phe Gly Lys Asp Ala Asp Leu Pro Asp Gly Leu Leu Asp
        355                 360                 365

Gln Glu Pro Tyr Tyr Val Ala Asp Cys His Leu Tyr Pro Gln His Phe
    370                 375                 380

Lys Gln Asn Gly Gln Ile Pro Glu Thr Ala Glu Asp Leu Val Ile Glu
385                 390                 395                 400

Ala Ile Asp Ile Pro Asp Gly Leu Leu Lys Gly Asp Thr Glu Ile Phe
                405                 410                 415

Ser Phe Ser Ala Ala Ala Pro Gly Thr Ser Val Asp Leu Thr Leu Phe
            420                 425                 430

Glu Ala Phe Asn Ser Ile Ala Ala Phe Glu Leu Lys Gly Ser Asn Ser
        435                 440                 445

Gln Asp Val Ala Glu Ser Ile Arg Gln Ile Gln Gln His Ala Lys Leu
    450                 455                 460

Thr Ala Lys Tyr Val Leu Pro Val
465                 470

<210> 5
<211> 472
<212> PRT
<213> Bacillus subtilis IAM1214

<400> 5
Met Glu Arg Lys Thr Val Leu Val Ile Ala Asp Leu Gly Gly Cys Pro
1               5                   10                  15

Pro His Met Phe Tyr Lys Ser Ala Ala Glu Lys Tyr Asn Leu Val Ser
            20                  25                  30

Phe Ile Pro Arg Pro Phe Ala Ile Thr Ala Ser His Ala Ala Leu Ile
        35                  40                  45

Glu Lys Tyr Ser Val Ala Val Ile Lys Asp Lys Asp Tyr Phe Gln Ser
    50                  55                  60

Leu Ala Asp Phe Glu His Pro Asp Ser Ile Tyr Trp Ala His Glu Asp
65                  70                  75                  80

His Asp Lys Pro Glu Glu Glu Val Val Glu Gln Ile Val Lys Val Ala
                85                  90                  95

Gln Met Phe Glu Ala Asp Ala Ile Thr Thr Asn Asn Glu Leu Phe Ile
            100                 105                 110

Ala Pro Met Ala Lys Ala Cys Glu Arg Leu Gly Leu Arg Gly Ala Gly
        115                 120                 125

Val Gln Ala Ala Glu Asn Ala Arg Asp Lys Asn Lys Met Arg Asp Ala
    130                 135                 140

Phe Asn Lys Ala Gly Val Lys Ser Ile Lys Asn Lys Arg Val Thr Thr
145                 150                 155                 160

Leu Glu Asp Phe Arg Ala Ala Leu Glu Glu Ile Gly Thr Pro Leu Ile
                165                 170                 175

Leu Lys Pro Thr Tyr Leu Ala Ser Ser Ile Gly Val Thr Leu Ile Thr
            180                 185                 190

Asp Thr Glu Thr Ala Glu Asp Glu Phe Asn Arg Val Asn Asp Tyr Leu
        195                 200                 205

Lys Ser Ile Asn Val Pro Lys Ala Val Thr Phe Glu Ala Pro Phe Ile
    210                 215                 220

Ala Glu Glu Phe Leu Gln Gly Glu Tyr Gly Asp Trp Tyr Gln Thr Glu
225                 230                 235                 240

Gly Tyr Ser Asp Tyr Ile Ser Ile Glu Gly Ile Met Ala Asp Gly Glu
                245                 250                 255

Tyr Phe Pro Ile Ala Ile His Asp Lys Thr Pro Gln Ile Gly Phe Thr
            260                 265                 270

Glu Thr Ser His Ile Thr Pro Ser Ile Leu Asp Glu Glu Ala Lys Lys
        275                 280                 285

Lys Ile Val Glu Ala Ala Lys Lys Ala Asn Glu Gly Leu Gly Leu Gln
    290                 295                 300

Asn Cys Ala Thr His Thr Glu Ile Lys Leu Met Lys Asn Arg Glu Pro
305                 310                 315                 320

Gly Leu Ile Glu Ser Ala Ala Arg Phe Ala Gly Trp Asn Met Ile Pro
                325                 330                 335

Asn Ile Lys Lys Val Phe Gly Leu Asp Met Ala Gln Leu Leu Leu Asp
            340                 345                 350

Val Leu Cys Phe Gly Lys Asp Ala Asp Leu Pro Asp Gly Leu Leu Asp
        355                 360                 365

Gln Glu Pro Tyr Tyr Val Ala Asp Cys His Leu Tyr Pro Gln His Phe
    370                 375                 380

Lys Gln Asn Gly Gln Ile Pro Glu Thr Ala Glu Asp Leu Val Ile Glu
385                 390                 395                 400

Ala Ile Asp Ile Pro Asp Gly Leu Leu Lys Gly Asp Thr Glu Ile Val
                405                 410                 415

Thr Phe Ser Ala Ala Ala Pro Gly Thr Ser Val Asp Leu Thr Leu Phe
            420                 425                 430

Glu Ala Phe Asn Ser Ile Ala Ala Phe Glu Leu Lys Gly Ser Asn Ser
        435                 440                 445

Gln Asp Val Ala Glu Ser Ile Arg Gln Ile Gln Gln His Ala Lys Leu
    450                 455                 460

Thr Ala Lys Tyr Val Leu Pro Val
465                 470

<210> 6
<211> 472
<212> PRT
<213> Bacillus subtilis ATCC21555

<400> 6
Met Glu Arg Lys Thr Val Leu Val Ile Ala Asp Leu Gly Gly Cys Pro
  1               5                  10                  15

Pro His Met Phe Tyr Lys Ser Ala Ala Glu Lys Tyr Asn Leu Val Ser
             20                  25                  30

Phe Ile Pro Arg Pro Phe Ala Ile Thr Ala Ser His Ala Ala Leu Ile
         35                  40                  45

Glu Lys Tyr Ser Ile Ala Val Ile Lys Asp Lys Asp Tyr Phe Lys Ser
     50                  55                  60

Leu Ala Asp Phe Glu His Pro Asp Ser Ile Tyr Trp Ala His Glu Asp
 65                  70                  75                  80

His Asp Lys Pro Glu Glu Glu Val Val Glu Glu Ile Val Lys Val Ala
                 85                  90                  95

Asp Met Phe Gly Val Asp Ala Ile Thr Thr Asn Asn Glu Leu Phe Ile
            100                 105                 110

Ala Pro Met Ala Lys Ala Cys Lys Arg Leu Gly Leu Arg Gly Ala Gly
        115                 120                 125

Val Gln Ala Ala Glu Asn Ala Arg Asp Lys Asn Lys Met Arg Ala Ala
    130                 135                 140

Phe Asn Arg Ala Gly Val Lys Ser Ile Lys Asn Lys Arg Val Thr Thr
145                 150                 155                 160

Leu Glu Asp Phe Arg Ala Ala Leu Gln Glu Ile Gly Thr Pro Leu Ile
                165                 170                 175

Leu Lys Pro Thr Tyr Leu Ala Ser Ser Ile Gly Val Thr Leu Ile Lys

180                 185                 190

Glu Met Glu Thr Ala Glu Ala Glu Phe Asn Arg Val Asn Glu Tyr Leu
        195                 200                 205

Lys Ser Ile Asn Val Pro Lys Ala Val Thr Phe Glu Ala Pro Phe Ile
    210                 215                 220

Ala Glu Glu Phe Leu Gln Gly Glu Tyr Asp Asp Trp Tyr Glu Thr Ser
225                 230                 235                 240

Gly Tyr Ser Asp Tyr Ile Ser Ile Glu Gly Ile Met Ala Asp Gly Glu
                245                 250                 255

Tyr Phe Pro Val Ala Ile His Asp Lys Thr Pro Gln Ile Gly Phe Thr
            260                 265                 270

Glu Thr Ala His Ile Thr Pro Ser Ile Leu Asp Asp Asp Ala Lys Arg
        275                 280                 285

Lys Ile Val Glu Ala Ala Lys Lys Ala Asn Glu Gly Leu Gly Leu Glu
    290                 295                 300

Asn Cys Ala Thr His Thr Glu Ile Lys Leu Met Lys Asn Arg Glu Ala
305                 310                 315                 320

Gly Leu Ile Glu Ser Ala Ala Arg Phe Ala Gly Trp Asn Met Ile Pro
                325                 330                 335

Asn Ile Lys Lys Val Phe Gly Val Asp Met Ala Gln Leu Leu Leu Asp
            340                 345                 350

Val Leu Cys Tyr Gly Lys Glu Ala Asp Leu Pro Lys Gly Leu Leu Glu
        355                 360                 365

Gln Glu Pro Cys Tyr Val Ala Asp Cys His Leu Tyr Pro Gln His Phe
    370                 375                 380

Lys Glu Asn Gly Gln Leu Pro Glu Thr Val Val Asp Phe Val Ile Glu
385                 390                 395                 400

Ser Ile Glu Ile Pro Asp Gly Val Leu Lys Gly Asp Thr Glu Leu Val

                405                 410                 415

Ser Phe Ser Ala Ala Glu Ala Gly Thr Ser Val Asp Leu Arg Leu Phe
            420                 425                 430

Glu Ala Phe Asn Ser Ile Ala Ala Phe Glu Leu Lys Gly Ser Asn Ser
        435                 440                 445

Asn Asp Val Ala Glu Ser Ile Lys Gln Ile Gln Gln Ala Lys Leu
    450                 455                 460

Thr Ala Lys Tyr Ala Leu Ser Val
465                 470

<210> 7
<211> 472

<212> PRT
<213> Bacillus amyloliquefaciens IFO3022

<400> 7
   Met Glu Arg Lys Thr Val Leu Val Ile Ala Asp Leu Gly Gly Cys Pro
   1               5                   10                  15
   Pro His Met Phe Tyr Lys Ser Ala Ala Glu Lys Tyr Asn Leu Val Ser
               20                  25                  30
   Phe Ile Pro Arg Pro Phe Ala Ile Thr Ala Ser His Ala Ala Leu Ile
           35                  40                  45
   Glu Lys Tyr Ser Val Ala Val Ile Lys Asp Lys Asp Tyr Phe Lys Ser
       50                  55                  60
   Leu Ala Asp Phe Glu His Pro Asp Ser Ile Tyr Trp Ala His Glu Asp
   65                  70                  75                  80
   His Asp Lys Pro Glu Glu Glu Val Val Glu Glu Ile Val Lys Val Ala
                   85                  90                  95
   Gly Met Phe Ala Val Asp Ala Ile Thr Thr Asn Asn Glu Leu Phe Ile
               100                 105                 110
   Ala Pro Met Ala Lys Ala Cys Glu Arg Leu Gly Leu Arg Gly Ala Gly
           115                 120                 125
   Val Gln Ala Ala Glu Asn Ala Arg Asp Lys Asn Lys Met Arg Ala Ala
       130                 135                 140
   Phe Asn Arg Ala Gly Val Lys Ser Ile Lys Asn Arg Arg Val Thr Thr
   145                 150                 155                 160
   Leu Glu Asp Phe Arg Ala Ala Leu Gln Glu Ile Gly Thr Pro Leu Ile
                   165                 170                 175
   Leu Lys Pro Thr Tyr Leu Ala Ser Ser Ile Gly Val Thr Leu Ile Lys
               180                 185                 190
   Glu Arg Glu Thr Ala Glu Ala Glu Phe Asn Arg Val Asn Glu Tyr Leu
           195                 200                 205
   Lys Ser Ile Asn Val Pro Lys Ala Val Thr Phe Glu Ala Pro Phe Ile
       210                 215                 220
   Ala Glu Glu Phe Leu Gln Gly Glu Tyr Asp Asp Trp Tyr Glu Thr Ser
   225                 230                 235                 240
   Gly Tyr Ser Asp Tyr Ile Ser Ile Glu Gly Ile Met Ala Asp Gly Glu
                   245                 250                 255
   Tyr Phe Pro Val Ala Ile His Asp Lys Thr Pro Gln Ile Gly Phe Thr
               260                 265                 270
   Glu Thr Ser His Ile Thr Pro Ser Ile Leu Asp Asp Asp Ala Lys Arg
           275                 280                 285
   Lys Ile Val Glu Ala Ala Lys Lys Ala Asn Glu Gly Leu Gly Leu Glu
       290                 295                 300
   Asn Cys Ala Thr His Thr Glu Ile Lys Leu Met Lys Asn Arg Glu Ala
   305                 310                 315                 320
   Gly Leu Ile Glu Ser Ala Ala Arg Phe Ala Gly Trp Asn Met Ile Pro
                   325                 330                 335
   Asn Ile Lys Lys Val Phe Gly Val Asp Met Ala Gln Leu Leu Leu Asp
               340                 345                 350
   Val Leu Cys Phe Gly Lys Glu Ala Asp Leu Pro Lys Gly Leu Leu Glu
           355                 360                 365
   Gln Glu Pro Cys Tyr Val Ala Asp Cys His Leu Tyr Pro Gln His Phe
       370                 375                 380
   Lys Glu Asn Gly Gln Leu Pro Glu Thr Ala Val Asp Phe Val Ile Glu
   385                 390                 395                 400
   Ser Ile Asp Ile Pro Asp Gly Val Leu Lys Gly Asp Thr Glu Ile Val
                   405                 410                 415
   Ser Phe Ser Ala Ala Glu Ala Gly Thr Ser Val Asp Leu Arg Leu Phe
               420                 425                 430
   Glu Ala Phe Asn Ser Ile Ala Ala Phe Glu Leu Lys Gly Ser Asn Ser
           435                 440                 445
   Gly Asp Val Ala Glu Ser Ile Lys Gln Ile Gln Gln Gln Ala Lys Leu
       450                 455                 460
   Thr Ala Lys Tyr Ala Leu Pro Val
   465                 470


<210> 8
<211> 476
<212> PRT
<213> Bacillus pumilus NRRL B-12025

<400> 26
Val Leu Ser Leu Ser Lys Lys Thr Val Leu Val Ile Ala Asp Leu Gly
  1               5                  10                  15

Gly Cys Pro Pro His Met Phe Tyr Glu Ser Val Ala Ala Ser Tyr His
             20                  25                  30

Ile Val Ser Tyr Ile Pro Arg Pro Phe Ala Ile Thr Lys Gly His Ala
         35                  40                  45

Glu Leu Ile Glu Lys Tyr Ser Ile Ala Val Ile Lys Asp Arg Asp Tyr
     50                  55                  60

Phe Glu Thr His Pro Ser Phe Glu His Pro Asp Ser Ile Tyr Trp Ala
 65                  70                  75                  80

His Asp Asp Tyr Pro Lys Ser Glu Glu Glu Val Val Glu Asp Phe Ile
                 85                  90                  95

Arg Val Ala Ser Phe Phe Lys Ala Asp Ala Ile Thr Thr Asn Asn Glu

            100                 105                 110

Leu Phe Ile Ala Pro Met Ala Lys Ala Ala Glu Arg Leu Gly Leu Arg
        115                 120                 125

Gly Ala Gly Val Lys Ala Ala Glu Met Ala Arg Asp Lys Ser Gln Met
    130                 135                 140

Arg Ala Ala Phe Asn Ala Ser Gly Val Lys Ala Val Lys Thr Gln Pro
145                 150                 155                 160

Val Thr Thr Leu Ser Asp Phe Gln Gln Ala Ile Glu Ser Ile Gly Thr
                165                 170                 175

Pro Leu Ile Leu Lys Pro Thr Tyr Leu Ala Ser Ser Ile Gly Val Thr
            180                 185                 190

Leu Phe His Asp Lys Ala Gly Ser Asp Asp Leu Phe Leu Gln Val Gln
        195                 200                 205

Ser Tyr Leu Glu Thr Ile Pro Val Pro Asp Ala Val Thr Tyr Glu Ala
    210                 215                 220

Pro Phe Val Ala Glu Thr Tyr Leu Glu Gly Ala Tyr Glu Asp Trp Tyr
225                 230                 235                 240

Glu Asp Glu Gly Tyr Ala Asp Tyr Val Ser Val Glu Gly Leu Val Val
                245                 250                 255

Glu Gly Glu Tyr Leu Pro Phe Val Ile His Asp Lys Thr Pro Gln Ile
            260                 265                 270

Gly Phe Thr Glu Thr Ala His Ile Thr Pro Thr Ile Leu Asp Asn Glu
        275                 280                 285

Ala Lys Gln Ile Ile Ile Glu Ala Ala Arg Lys Ala Asn Glu Gly Leu
    290                 295                 300

Gly Leu Glu His Cys Ala Thr His Thr Glu Ile Lys Leu Met Lys Asn
305                 310                 315                 320

Arg Glu Thr Gly Leu Ile Glu Ala Ala Ala Arg Phe Ala Gly Trp Asn
                325                 330                 335

Met Ile Pro Asn Ile Lys Lys Val Phe Gly Val Asp Met Ala Lys Leu
            340                 345                 350

Leu Ile Asp Val Leu Val Asp Gly Lys Lys Ala Val Leu Pro Lys Gln
        355                 360                 365

Leu Leu Ser Gly His Thr Phe Tyr Val Ala Asp Cys His Leu Tyr Pro
    370                 375                 380

Gln His Phe Lys Glu Ser Gly Leu Ile Pro Pro Glu Ala Thr His Ile
385                 390                 395                 400

Thr Ile Asp His Val Ser Ile Pro Gln Glu Ala Phe Val Gly Asp Thr
                405                 410                 415

Ala Ile Val Ser Gln Ser Phe Pro Ala Lys Gly Thr Ile Val Asp Leu

            420                 425                 430

Glu Leu Phe Glu Ala Phe Asn Gly Ile Val Ser Leu Glu Leu Lys Gly
        435                 440                 445

Ser Ser Ser Gln Asp Val Ala Ala Ser Ile Arg Asn Ile Gln Lys Gln
    450                 455                 460

Ala Thr Ile Gln Leu Met Asp Glu Leu Val Lys Gly

465                 470                 475

<210> 9
<211> 1416
<212> DNA
<213> Bacillus subtilis 168

<400> 9
atg gag aga aaa aca gta ttg gtc atc gct gat ctt gga ggc tgc ccg 48
Met Glu Arg Lys Thr Val Leu Val Ile Ala Asp Leu Gly Gly Cys Pro

1               5                   10                  15

ccg cac atg ttt tat aaa agc gct gct gaa aaa tat aac ctg gtc agc 96
Pro His Met Phe Tyr Lys Ser Ala Ala Glu Lys Tyr Asn Leu Val Ser
            20                  25                  30

ttt att cca aga cct ttt gca att aca gcc tcc cat gca gca ttg att 144
Phe Ile Pro Arg Pro Phe Ala Ile Thr Ala Ser His Ala Ala Leu Ile
        35                  40                  45

gaa aaa tac tcg gtc gcg gtc ata aaa gat aaa gac tat ttt aag agt 192
Glu Lys Tyr Ser Val Ala Val Ile Lys Asp Lys Asp Tyr Phe Lys Ser
    50                  55                  60

tta gct gat ttt gaa cac cct gat tcc att tat tgg gcg cat gaa gat 240
Leu Ala Asp Phe Glu His Pro Asp Ser Ile Tyr Trp Ala His Glu Asp
65                  70                  75                  80

cat aac aag cct gag gaa gag gtc gtc gag caa atc gtc aag gtt gcc 288
His Asn Lys Pro Glu Glu Glu Val Val Glu Gln Ile Val Lys Val Ala
                85                  90                  95

gaa atg ttt ggg gcg gat gcc atc aca aca aac aat gaa tta ttc att 336
Glu Met Phe Gly Ala Asp Ala Ile Thr Thr Asn Asn Glu Leu Phe Ile
            100                 105                 110

gct ccg atg gcg aaa gcc tgt gaa cgt ctg ggc ttg aga ggt gcc ggc 384
Ala Pro Met Ala Lys Ala Cys Glu Arg Leu Gly Leu Arg Gly Ala Gly
        115                 120                 125

gtg cag gca gcc gaa aat gcc aga gat aaa aat aaa atg agg gac gct 432
Val Gln Ala Ala Glu Asn Ala Arg Asp Lys Asn Lys Met Arg Asp Ala
    130                 135                 140

ttt aat aag gcc gga gtc aaa tcg atc aaa aac aaa cga gtc aca act 480
Phe Asn Lys Ala Gly Val Lys Ser Ile Lys Asn Lys Arg Val Thr Thr
145                 150                 155                 160

ctt gaa gat ttc cgt gct gct ctt gaa gag atc ggc aca cct ctt atc     528
Leu Glu Asp Phe Arg Ala Ala Leu Glu Glu Ile Gly Thr Pro Leu Ile
                165                 170                 175

tta aag cct aca tac tta gcg agt tct atc ggt gta acg ctg att acg     576
Leu Lys Pro Thr Tyr Leu Ala Ser Ser Ile Gly Val Thr Leu Ile Thr
            180                 185                 190

gac act gag acg gca gaa gat gaa ttt aac aga gtc aat gac tat ctg     624
Asp Thr Glu Thr Ala Glu Asp Glu Phe Asn Arg Val Asn Asp Tyr Leu
        195                 200                 205

aaa tca att aac gtg cca aag gcg gtt acg ttt gaa gcg ccg ttt atc     672
Lys Ser Ile Asn Val Pro Lys Ala Val Thr Phe Glu Ala Pro Phe Ile
    210                 215                 220

gct gaa gaa ttt tta cag ggt gag tac gga gac tgg tat caa aca gaa     720
Ala Glu Glu Phe Leu Gln Gly Glu Tyr Gly Asp Trp Tyr Gln Thr Glu
225                 230                 235                 240

ggg tac tcc gac tat atc agt ata gaa ggc atc atg gct gac ggt gag     768
Gly Tyr Ser Asp Tyr Ile Ser Ile Glu Gly Ile Met Ala Asp Gly Glu
                245                 250                 255

tat ttc ccg atc gcc att cat gat aaa acg ccg caa atc ggg ttt aca     816
Tyr Phe Pro Ile Ala Ile His Asp Lys Thr Pro Gln Ile Gly Phe Thr
            260                 265                 270

gag aca tcc cac att acg ccg tcc att ctg gat gaa gag gca aaa aag     864
Glu Thr Ser His Ile Thr Pro Ser Ile Leu Asp Glu Glu Ala Lys Lys
        275                 280                 285

aaa att gtc gaa gct gcc aaa aag gca aat gaa ggg ctt gga ctg caa     912
Lys Ile Val Glu Ala Ala Lys Lys Ala Asn Glu Gly Leu Gly Leu Gln
    290                 295                 300

aat tgc gca aca cat aca gag atc aag cta atg aaa aac aga gaa ccg     960
Asn Cys Ala Thr His Thr Glu Ile Lys Leu Met Lys Asn Arg Glu Pro
305                 310                 315                 320

ggt tta ata gag tcg gca gcc aga ttt gcc ggc tgg aat atg atc ccc     1008
Gly Leu Ile Glu Ser Ala Ala Arg Phe Ala Gly Trp Asn Met Ile Pro
                325                 330                 335

aat att aaa aag gtc ttt ggc ctt gat atg gcg caa tta tta tta gat     1056
Asn Ile Lys Lys Val Phe Gly Leu Asp Met Ala Gln Leu Leu Leu Asp
            340                 345                 350

gtc ctc tgt ttc gga aaa gac gcc gat ctg ccg gac gga tta ttg gat     1104
Val Leu Cys Phe Gly Lys Asp Ala Asp Leu Pro Asp Gly Leu Leu Asp
        355                 360                 365

caa gag cct tat tat gtt gcc gac tgc cat ttg tac ccg cag cat ttc     1152
Gln Glu Pro Tyr Tyr Val Ala Asp Cys His Leu Tyr Pro Gln His Phe
    370                 375                 380

aaa caa aat ggc caa att cct gaa act gct gag gat ttg gtc att gaa     1200
Lys Gln Asn Gly Gln Ile Pro Glu Thr Ala Glu Asp Leu Val Ile Glu
385                 390                 395                 400

gcg atc gat att ccg gac ggg ctt tta aaa ggg gat act gaa atc gtt     1248
Ala Ile Asp Ile Pro Asp Gly Leu Leu Lys Gly Asp Thr Glu Ile Val
                405                 410                 415

tct ttt tcg gcc gca gca cca ggc act tca gtt gat ttg aca ttg ttt     1296
Ser Phe Ser Ala Ala Ala Pro Gly Thr Ser Val Asp Leu Thr Leu Phe
            420                 425                 430

gaa gct ttc aat tcc att gct gca ttt gaa ctg aaa ggc agt aat tca     1344
Glu Ala Phe Asn Ser Ile Ala Ala Phe Glu Leu Lys Gly Ser Asn Ser
        435                 440                 445

cag gat gtg gct gaa tca atc aga caa att cag cag cat gcg aag ctg     1392
Gln Asp Val Ala Glu Ser Ile Arg Gln Ile Gln Gln His Ala Lys Leu
    450                 455                 460

acg gca aag tat gtg ctg cca gta                                     1416
Thr Ala Lys Tyr Val Leu Pro Val
465                 470


<210> 10
<211> 1416
<212> DNA
<213> Bacillus subtilis ATCC6633

<400> 10
atg gag aga aaa aca gta ttg gtc atc gct gat ctt gga ggc tgc ccg     48
Met Glu Arg Lys Thr Val Leu Val Ile Ala Asp Leu Gly Gly Cys Pro
  1               5                  10                  15

ccg cac atg ttt tat aaa agc gct gct gaa aaa tat aac ctg gtt agc     96
Pro His Met Phe Tyr Lys Ser Ala Ala Glu Lys Tyr Asn Leu Val Ser
             20                  25                  30

ttt att ccg aga cct ttt gca ata aca gcc tcc cat gca gca ctg att     144
Phe Ile Pro Arg Pro Phe Ala Ile Thr Ala Ser His Ala Ala Leu Ile
         35                  40                  45

gaa aaa tac tcg gtc gcg gtc ata aaa gat aaa gac tat ttt cag agc     192
Glu Lys Tyr Ser Val Ala Val Ile Lys Asp Lys Asp Tyr Phe Gln Ser
     50                  55                  60

tta gct gat ttt gag cat ccc gat tca att tat tgg gcg cat gag gat     240
Leu Ala Asp Phe Glu His Pro Asp Ser Ile Tyr Trp Ala His Glu Asp
 65                  70                  75                  80

cat gac aag cct gaa gaa gag gtt gtc gag caa atc gtc aag gtt gcc     288
His Asp Lys Pro Glu Glu Glu Val Val Glu Gln Ile Val Lys Val Ala
                 85                  90                  95

caa atg ttt gag gcg gac gcc atc aca aca aac aat gaa tta ttc att     336
Gln Met Phe Glu Ala Asp Ala Ile Thr Thr Asn Asn Glu Leu Phe Ile
            100                 105                 110

gcc ccg atg gcg aaa gcc tgt gaa cgc ctt ggc ctg agg ggc gcc gga     384
Ala Pro Met Ala Lys Ala Cys Glu Arg Leu Gly Leu Arg Gly Ala Gly
        115                 120                 125

gtg cag gca gcg gaa aat gcc aga gat aaa aat aaa atg agg gac gct     432

Val Gln Ala Ala Glu Asn Ala Arg Asp Lys Asn Lys Met Arg Asp Ala
    130                 135                 140

ttt aat aag gcg gga gtc aaa tcg atc aaa aac aaa cga gtc aca act     480
Phe Asn Lys Ala Gly Val Lys Ser Ile Lys Asn Lys Arg Val Thr Thr
145                 150                 155                 160

ctt gag gat ttt cgt gct gca ctt gaa gag atc ggc aca cct cta atc     528
Leu Glu Asp Phe Arg Ala Ala Leu Glu Glu Ile Gly Thr Pro Leu Ile
                165                 170                 175

tta aag cct aca tac tta gcg agt tca atc ggc gta acg ctg att acc     576
Leu Lys Pro Thr Tyr Leu Ala Ser Ser Ile Gly Val Thr Leu Ile Thr
            180                 185                 190

gac acg gag acg gca gaa gat gaa ttt aac aga gtc aat gac tac ctg     624
Asp Thr Glu Thr Ala Glu Asp Glu Phe Asn Arg Val Asn Asp Tyr Leu
        195                 200                 205

aaa tcg att aac gtg ccg aag gcg gtc aca ttt gaa gca ccg ttt att     672
Lys Ser Ile Asn Val Pro Lys Ala Val Thr Phe Glu Ala Pro Phe Ile
    210                 215                 220

gct gag gaa ttt tta cag ggt gag tac gga gac tgg tat caa aca gaa     720
Ala Glu Glu Phe Leu Gln Gly Glu Tyr Gly Asp Trp Tyr Gln Thr Glu
225                 230                 235                 240

ggg tac tcc gac tat atc agc ata gaa ggc att atg gca gat ggt gag     768
Gly Tyr Ser Asp Tyr Ile Ser Ile Glu Gly Ile Met Ala Asp Gly Glu
                245                 250                 255

tat ttt ccg atc gcc att cat gac aaa acg ccg caa att gga ttt aca     816
Tyr Phe Pro Ile Ala Ile His Asp Lys Thr Pro Gln Ile Gly Phe Thr
            260                 265                 270

gag aca tca cat att acg cca tcc att ctg gat gaa gag gcg aaa aag     864
Glu Thr Ser His Ile Thr Pro Ser Ile Leu Asp Glu Glu Ala Lys Lys
        275                 280                 285

aaa att gtc gaa gcg gct aaa aag gca aat gaa ggg ctt gga ctg caa     912
Lys Ile Val Glu Ala Ala Lys Lys Ala Asn Glu Gly Leu Gly Leu Gln
    290                 295                 300

aat tgc gca aca cat aca gaa atc aag cta atg aaa aac aga gaa ccg     960
Asn Cys Ala Thr His Thr Glu Ile Lys Leu Met Lys Asn Arg Glu Pro
305                 310                 315                 320

ggt tta ata gag tcg gct gcc aga ttc gca ggc tgg aat atg att cct     1008
Gly Leu Ile Glu Ser Ala Ala Arg Phe Ala Gly Trp Asn Met Ile Pro
                325                 330                 335

aac att aaa aag gtt ttc ggc ctt gat atg gcg caa tta tta tta gat     1056
Asn Ile Lys Lys Val Phe Gly Leu Asp Met Ala Gln Leu Leu Leu Asp
            340                 345                 350

gtt ctc tgt ttc gga aaa gat gct gat ctg ccg gac ggg tta ttg gat     1104
Val Leu Cys Phe Gly Lys Asp Ala Asp Leu Pro Asp Gly Leu Leu Asp
        355                 360                 365

caa gag cct tac tat gtt gct gac tgc cat ctg tac cct cag cat ttc     1152
Gln Glu Pro Tyr Tyr Val Ala Asp Cys His Leu Tyr Pro Gln His Phe
    370                 375                 380

aaa caa aat ggc cag atc cct gaa act gcc gag gat ttg gta atc gaa     1200
Lys Gln Asn Gly Gln Ile Pro Glu Thr Ala Glu Asp Leu Val Ile Glu
385                 390                 395                 400

gcg atc gat att ccg gat ggg ctt ttg aag ggt gat aca gaa atc gtt     1248
Ala Ile Asp Ile Pro Asp Gly Leu Leu Lys Gly Asp Thr Glu Ile Val
                405                 410                 415

act ttt tcg gct gcg gca cca gga aca tca gtt gat ttg aca ctg ttt     1296
Thr Phe Ser Ala Ala Ala Pro Gly Thr Ser Val Asp Leu Thr Leu Phe
            420                 425                 430

gaa gcc ttc aac tcc att gct gca ttt gaa ctg aaa ggc agc aat tca     1344
Glu Ala Phe Asn Ser Ile Ala Ala Phe Glu Leu Lys Gly Ser Asn Ser
        435                 440                 445

cag gat gtg gct gaa tca atc aga caa att cag cag cat gcg aag ctg     1392
Gln Asp Val Ala Glu Ser Ile Arg Gln Ile Gln Gln His Ala Lys Leu
    450                 455                 460

acg gca aag tat gtg ctg cca gta                                     1416
Thr Ala Lys Tyr Val Leu Pro Val
465                 470

<210> 11
<211> 1416
<212> DNA
<213> Bacillus subtilis IAM1213

<400> 11
atg gag aga aaa aca gta ttg gtc atc gct gat ctt gga ggc tgc ccg     48
Met Glu Arg Lys Thr Val Leu Val Ile Ala Asp Leu Gly Gly Cys Pro
1               5                   10                  15

ccg cac atg ttt tat aaa agc gct gct gaa aaa tat aac ctg gtc agc     96
Pro His Met Phe Tyr Lys Ser Ala Ala Glu Lys Tyr Asn Leu Val Ser
            20                  25                  30

ttt att cca aga cct ttt gca att aca gcc tcc cat gca gca ttg att     144
Phe Ile Pro Arg Pro Phe Ala Ile Thr Ala Ser His Ala Ala Leu Ile
        35                  40                  45

gaa aaa tac tcg gtc gcg gtc ata aaa gat aaa gac tat ttt aag agt     192
Glu Lys Tyr Ser Val Ala Val Ile Lys Asp Lys Asp Tyr Phe Lys Ser
    50                  55                  60

tta gct gat ttt gag cat cct gac tcc att tat tgg gcg cat gag gat     240
Leu Ala Asp Phe Glu His Pro Asp Ser Ile Tyr Trp Ala His Glu Asp
65                  70                  75                  80

cat aac aag cct gag gaa gag gtc gtc gag caa atc gtc aag gtt gcc     288
His Asn Lys Pro Glu Glu Glu Val Val Glu Gln Ile Val Lys Val Ala
                85                  90                  95

gaa atg ttt ggg gcg gat gcc atc aca aca aac aat gaa tta ttc att 336
Glu Met Phe Gly Ala Asp Ala Ile Thr Thr Asn Asn Glu Leu Phe Ile
            100                 105                 110

gct ccg atg gcg aaa gcc tgt gaa cgt ctg ggc ctg aga ggt gcc ggc 384
Ala Pro Met Ala Lys Ala Cys Glu Arg Leu Gly Leu Arg Gly Ala Gly
        115                 120                 125

gtg cag gca gcc gaa aat gcc aga gat aaa aat aaa atg agg gac gct 432
Val Gln Ala Ala Glu Asn Ala Arg Asp Lys Asn Lys Met Arg Asp Ala
    130                 135                 140

ttt aat aag gcc gga gtc aaa tcg atc aaa aac aaa cga gtc aca act 480
Phe Asn Lys Ala Gly Val Lys Ser Ile Lys Asn Lys Arg Val Thr Thr
145                 150                 155                 160

ctc gaa gat ttc cgt gct gct ctt gaa gag atc ggc aca cct ctt atc 528
Leu Glu Asp Phe Arg Ala Ala Leu Glu Glu Ile Gly Thr Pro Leu Ile
                165                 170                 175

tta aag cct aca tac tta gcg agt tca atc ggt gta acg ctg att acg 576
Leu Lys Pro Thr Tyr Leu Ala Ser Ser Ile Gly Val Thr Leu Ile Thr
            180                 185                 190

gac act gag acg gca gaa gat gaa ttt aac aga gtc aat gac tat ctg 624
Asp Thr Glu Thr Ala Glu Asp Glu Phe Asn Arg Val Asn Asp Tyr Leu
        195                 200                 205

aaa tca att aac gtg cca aag gcg gtt acg ttt gaa gcg ccg ttt atc 672
Lys Ser Ile Asn Val Pro Lys Ala Val Thr Phe Glu Ala Pro Phe Ile
    210                 215                 220

gct gaa gaa ttt tta cag ggt gag tac gga gac tgg tat caa aca gaa 720
Ala Glu Glu Phe Leu Gln Gly Glu Tyr Gly Asp Trp Tyr Gln Thr Glu
225                 230                 235                 240

ggg tac tcc gac tat atc agt ata gaa ggc atc atg gct gac ggt gag 768

Gly Tyr Ser Asp Tyr Ile Ser Ile Glu Gly Ile Met Ala Asp Gly Glu
                245                 250                 255

tat ttc ccg atc gcc att cat gat aaa acg ccg caa atc ggg ttt aca 816
Tyr Phe Pro Ile Ala Ile His Asp Lys Thr Pro Gln Ile Gly Phe Thr
            260                 265                 270

gag aca tcc cac att acg ccg tcc att ctg gat gaa gag gca aaa aag 864
Glu Thr Ser His Ile Thr Pro Ser Ile Leu Asp Glu Glu Ala Lys Lys
        275                 280                 285

aaa att gtc gaa gct gcc aaa aag gca aat gaa ggt ctt ggc ctg caa 912
Lys Ile Val Glu Ala Ala Lys Lys Ala Asn Glu Gly Leu Gly Leu Gln
    290                 295                 300

aat tgc gca aca cat aca gag atc aag cta atg aaa aat aga gaa ccg 960
Asn Cys Ala Thr His Thr Glu Ile Lys Leu Met Lys Asn Arg Glu Pro
305                 310                 315                 320

ggt tta att gaa tcg gca gcc aga ttc gcc ggc tgg aat atg atc ccc 1008
Gly Leu Ile Glu Ser Ala Ala Arg Phe Ala Gly Trp Asn Met Ile Pro

                325                 330                 335

aat att aaa aag gtc ttt ggc ctt gat atg gcg caa tta tta tta gat 1056
Asn Ile Lys Lys Val Phe Gly Leu Asp Met Ala Gln Leu Leu Leu Asp
            340                 345                 350

gtc ctt tgt ttc gga aaa gac gcc gat ctg ccg gac gga tta ttg gat 1104
Val Leu Cys Phe Gly Lys Asp Ala Asp Leu Pro Asp Gly Leu Leu Asp
        355                 360                 365

caa gag cct tat tat gtt gcc gac tgc cat ttg tac ccg caa cat ttc 1152
Gln Glu Pro Tyr Tyr Val Ala Asp Cys His Leu Tyr Pro Gln His Phe
    370                 375                 380

aaa caa aat ggc cag att cca gaa act gct gag gat ttg gtc att gaa 1200
Lys Gln Asn Gly Gln Ile Pro Glu Thr Ala Glu Asp Leu Val Ile Glu
385                 390                 395                 400

gcg atc gat ctg cct gac ggg ctt tta aaa ggg gat act gag atc gtt 1248
Ala Ile Asp Leu Pro Asp Gly Leu Leu Lys Gly Asp Thr Glu Ile Val
                405                 410                 415

tct ttt tcg gcc gca gca cca gga act tca gtt gat ttg aca ttg ttt 1296
Ser Phe Ser Ala Ala Ala Pro Gly Thr Ser Val Asp Leu Thr Leu Phe
            420                 425                 430

gaa gct ttc aat tcc att gct gca ttt gaa ctg aaa ggc agt aat tca 1344
Glu Ala Phe Asn Ser Ile Ala Ala Phe Glu Leu Lys Gly Ser Asn Ser
        435                 440                 445

cag gat gtg gct gaa tca atc aga caa att cag cag cat gcg aag ctg 1392
Gln Asp Val Ala Glu Ser Ile Arg Gln Ile Gln Gln His Ala Lys Leu
    450                 455                 460

acg gca aag tat gtg ctg cca gta 1416
Thr Ala Lys Tyr Val Leu Pro Val
465                 470

<210> 12
<211> 1416

<212> DNA
<213> Bacillus subtilis IAM1107

<400> 12
atg gag aga aaa aca gta ttg gtc atc gct gat ctt gga ggc tgc ccg 48
Met Glu Arg Lys Thr Val Leu Val Ile Ala Asp Leu Gly Gly Cys Pro
1               5                   10                  15

ccg cac atg ttt tat aaa agc gct gct gaa aaa tat aac ctg gtc agc 96
Pro His Met Phe Tyr Lys Ser Ala Ala Glu Lys Tyr Asn Leu Val Ser
            20                  25                  30

ttt att cca aga cct ttt gca att aca gcc tcc cat gca gca ttg att 144
Phe Ile Pro Arg Pro Phe Ala Ile Thr Ala Ser His Ala Ala Leu Ile
        35                  40                  45

gaa aaa tac tcg gtc gcg gtc gta aaa gat aaa gac tat ttt aag agt 192
Glu Lys Tyr Ser Val Ala Val Val Lys Asp Lys Asp Tyr Phe Lys Ser

50                  55                  60

tta gct gat ttt gag cat cct gac tcc att tat tgg gcg cat gag gat     240
Leu Ala Asp Phe Glu His Pro Asp Ser Ile Tyr Trp Ala His Glu Asp
65                  70                  75                  80

cat aac aag cct gag gaa gag gtc gtc gag caa atc gtc aag gtt gcc     288
His Asn Lys Pro Glu Glu Glu Val Val Glu Gln Ile Val Lys Val Ala
                85                  90                  95

gaa atg ttc ggg gcg gat gcc atc aca aca aac aat gaa tta ttc att     336
Glu Met Phe Gly Ala Asp Ala Ile Thr Thr Asn Asn Glu Leu Phe Ile
            100                 105                 110

gct ccg atg gcg aaa gcc tgt gaa cgt ctg ggc ttg aga ggt gcc ggc     384
Ala Pro Met Ala Lys Ala Cys Glu Arg Leu Gly Leu Arg Gly Ala Gly
        115                 120                 125

gtg cag gca gcc gaa aat gcc aga gat aaa aat aaa atg agg gac gct     432
Val Gln Ala Ala Glu Asn Ala Arg Asp Lys Asn Lys Met Arg Asp Ala
    130                 135                 140

ttt aat aag gcc gga gtc aaa tcg atc aaa aac aaa cga gtc aca act     480
Phe Asn Lys Ala Gly Val Lys Ser Ile Lys Asn Lys Arg Val Thr Thr
145                 150                 155                 160

ctt gaa gat ttc cgt gct gct ctt gaa gag atc ggc aca cct ctt atc     528
Leu Glu Asp Phe Arg Ala Ala Leu Glu Glu Ile Gly Thr Pro Leu Ile
                165                 170                 175

tta aag cct aca tac tta gcg agt tct atc ggt gta acg ctg att acg     576
Leu Lys Pro Thr Tyr Leu Ala Ser Ser Ile Gly Val Thr Leu Ile Thr
            180                 185                 190

gac act gag acg gca gaa gat gaa ttt aac aga gtc aat gac tat ctg     624
Asp Thr Glu Thr Ala Glu Asp Glu Phe Asn Arg Val Asn Asp Tyr Leu
        195                 200                 205

aaa tca att aac gtg cca aag gcg gtt acg ttt gaa gcg ccg ttt atc     672
Lys Ser Ile Asn Val Pro Lys Ala Val Thr Phe Glu Ala Pro Phe Ile
    210                 215                 220

gct gaa gaa ttt tta cag ggt gag tac gga gac tgg tat caa aca gaa     720
Ala Glu Glu Phe Leu Gln Gly Glu Tyr Gly Asp Trp Tyr Gln Thr Glu
225                 230                 235                 240

ggg tac tcc gac tat atc agt ata gaa ggc atc atg gct gac ggt gag     768
Gly Tyr Ser Asp Tyr Ile Ser Ile Glu Gly Ile Met Ala Asp Gly Glu
                245                 250                 255

tat ttc ccg atc gcc att cat gat aaa acg ccg caa atc ggg ttt aca     816
Tyr Phe Pro Ile Ala Ile His Asp Lys Thr Pro Gln Ile Gly Phe Thr
            260                 265                 270

gag aca tcc cac att acg ccg tcc att ctg gat gaa gag gca aaa aag     864
Glu Thr Ser His Ile Thr Pro Ser Ile Leu Asp Glu Glu Ala Lys Lys
        275                 280                 285

aaa att gtc gaa gct gcc aaa aag gca aat gaa ggg ctt ggc ctg caa     912
Lys Ile Val Glu Ala Ala Lys Lys Ala Asn Glu Gly Leu Gly Leu Gln

290                 295                 300

aat tgc gca aca cat aca gag gtc aag cta atg aaa aac aga gaa ccg     960
Asn Cys Ala Thr His Thr Glu Val Lys Leu Met Lys Asn Arg Glu Pro
305                 310                 315                 320

ggt tta att gaa tcg gca gcc aga ttt gcc ggc tgg aat atg atc cct     1008
Gly Leu Ile Glu Ser Ala Ala Arg Phe Ala Gly Trp Asn Met Ile Pro
                325                 330                 335

aac att aaa aag gtt ttc ggc ctt gat atg gcg caa tta tta tta gat     1056
Asn Ile Lys Lys Val Phe Gly Leu Asp Met Ala Gln Leu Leu Leu Asp
            340                 345                 350

gtc ctc tgt ttc gga aaa gat gcc gat ctg ccg gac gga tta ttg gat     1104
Val Leu Cys Phe Gly Lys Asp Ala Asp Leu Pro Asp Gly Leu Leu Asp
        355                 360                 365

caa gag cct tac tat gtc gcc gac tgc cat ttg tac ccg cag cat ttc     1152
Gln Glu Pro Tyr Tyr Val Ala Asp Cys His Leu Tyr Pro Gln His Phe
    370                 375                 380

aaa caa aat ggc cag att cca gaa acc gct gag gat ttg gtc att gaa     1200
Lys Gln Asn Gly Gln Ile Pro Glu Thr Ala Glu Asp Leu Val Ile Glu
385                 390                 395                 400

gcg atc gat att ccg gac ggg ctt tta aaa ggg gat act gaa atc ttt     1248
Ala Ile Asp Ile Pro Asp Gly Leu Leu Lys Gly Asp Thr Glu Ile Phe
                405                 410                 415

tct ttt tcg gcc gca gca cca ggc act tca gtt gat ttg aca ttg ttt     1296
Ser Phe Ser Ala Ala Ala Pro Gly Thr Ser Val Asp Leu Thr Leu Phe
            420                 425                 430

gaa gct ttc aat tcc att gct gca ttt gaa ctg aaa ggc agt aat tca     1344
Glu Ala Phe Asn Ser Ile Ala Ala Phe Glu Leu Lys Gly Ser Asn Ser
        435                 440                 445

cag gat gtg gct gaa tca atc aga caa att cag cag cat gcg aag ctg     1392
Gln Asp Val Ala Glu Ser Ile Arg Gln Ile Gln Gln His Ala Lys Leu
    450                 455                 460

acg gca aag tat gtg ctg cca gta                                     1416
Thr Ala Lys Tyr Val Leu Pro Val
465                 470

<210> 13
<211> 1416
<212> DNA
<213> Bacillus subtilis IAM1214

<400> 13
   atg gag aga aaa aca gta ttg gtc atc gct gat ctt gga ggc tgc ccg     48
   Met Glu Arg Lys Thr Val Leu Val Ile Ala Asp Leu Gly Gly Cys Pro
     1               5                  10                  15
   ccg cac atg ttt tat aaa agc gct gct gaa aaa tat aac ctg gtt agc     96
   Pro His Met Phe Tyr Lys Ser Ala Ala Glu Lys Tyr Asn Leu Val Ser
                20                  25                  30
   ttt att ccg aga cct ttt gca ata aca gcc tcc cat gca gca ctg att 144
   Phe Ile Pro Arg Pro Phe Ala Ile Thr Ala Ser His Ala Ala Leu Ile
   35 40 45
   gaa aaa tac tcg gtc gcg gtc ata aaa gat aaa gac tat ttt cag agc 192
   Glu Lys Tyr Ser Val Ala Val Ile Lys Asp Lys Asp Tyr Phe Gln Ser
   50 55 60
   tta gct gat ttt gag cat ccc gat tca att tat tgg gcg cat gag gat 240
   Leu Ala Asp Phe Glu His Pro Asp Ser Ile Tyr Trp Ala His Glu Asp
   65 70 75 80
   cat gac aag cct gaa gaa gag gtt gtc gag caa atc gtc aag gtt gcc 288
   His Asp Lys Pro Glu Glu Glu Val Val Glu Gln Ile Val Lys Val Ala
   85 90 95
   caa atg ttt gag gcg gac gcc atc aca aca aac aat gaa tta ttc att 336
   Gln Met Phe Glu Ala Asp Ala Ile Thr Thr Asn Asn Glu Leu Phe Ile
   100 105 110
   gcc ccg atg gcg aaa gcc tgt gaa cgc ctt ggc ctg agg ggc gcc gga 384
   Ala Pro Met Ala Lys Ala Cys Glu Arg Leu Gly Leu Arg Gly Ala Gly
   115 120 125
   gtg cag gca gcg gaa aat gcc aga gat aaa aat aaa atg agg gac gct 432
   Val Gln Ala Ala Glu Asn Ala Arg Asp Lys Asn Lys Met Arg Asp Ala
   130 135 140
   ttt aat aag gcg gga gtc aaa tcg atc aaa aac aaa cga gtc aca act 480
   Phe Asn Lys Ala Gly Val Lys Ser Ile Lys Asn Lys Arg Val Thr Thr
   145 150 155 160
   ctt gag gat ttt cgt gct gca ctt gaa gag atc ggc aca cct cta atc 528
   Leu Glu Asp Phe Arg Ala Ala Leu Glu Glu Ile Gly Thr Pro Leu Ile
   165 170 175
   tta aag cct aca tac tta gcg agt tca atc ggc gta acg ctg att acc 576
   Leu Lys Pro Thr Tyr Leu Ala Ser Ser Ile Gly Val Thr Leu Ile Thr
   180 185 190
   gac acg gag acg gca gaa gat gaa ttt aac aga gtc aat gac tac ctg 624
   Asp Thr Glu Thr Ala Glu Asp Glu Phe Asn Arg Val Asn Asp Tyr Leu
   195 200 205
   aaa tcg att aac gtg ccg aag gcg gtc aca ttt gaa gca ccg ttt att 672
   Lys Ser Ile Asn Val Pro Lys Ala Val Thr Phe Glu Ala Pro Phe Ile
   210 215 220
   gct gag gaa ttt tta cag ggt gag tac gga gac tgg tat caa aca gaa 720
   Ala Glu Glu Phe Leu Gln Gly Glu Tyr Gly Asp Trp Tyr Gln Thr Glu
   225 230 235 240
   ggg tac tcc gac tat atc agc ata gaa ggc att atg gca gat ggt gag 768
   Gly Tyr Ser Asp Tyr Ile Ser Ile Glu Gly Ile Met Ala Asp Gly Glu
   245 250 255
   tat ttt ccg atc gcc att cat gac aaa acg ccg caa att gga ttt aca 816
   Tyr Phe Pro Ile Ala Ile His Asp Lys Thr Pro Gln Ile Gly Phe Thr
   260 265 270
   gag aca tca cat att acg cca tcc att ctg gat gaa gag gcg aaa aag     864
   Glu Thr Ser His Ile Thr Pro Ser Ile Leu Asp Glu Glu Ala Lys Lys
           275                 280                 285
   aaa att gtc gaa gcg gct aaa aag gca aat gaa ggg ctt gga ctg caa     912
   Lys Ile Val Glu Ala Ala Lys Lys Ala Asn Glu Gly Leu Gly Leu Gln
       290                 295                 300
   aat tgc gca aca cat aca gaa atc aag cta atg aaa aac aga gaa ccg     960
   Asn Cys Ala Thr His Thr Glu Ile Lys Leu Met Lys Asn Arg Glu Pro
   305                 310                 315                 320
   ggt tta ata gag tcg gct gcc aga ttc gca ggc tgg aat atg att cct    1008
   Gly Leu Ile Glu Ser Ala Ala Arg Phe Ala Gly Trp Asn Met Ile Pro
                   325                 330                 335
   aac att aaa aag gtt ttc ggc ctt gat atg gcg caa tta tta tta gat    1056
   Asn Ile Lys Lys Val Phe Gly Leu Asp Met Ala Gln Leu Leu Leu Asp
               340                 345                 350
   gtt ctc tgt ttc gga aaa gat gct gat ctg ccg gac ggg tta ttg gat    1104
   Val Leu Cys Phe Gly Lys Asp Ala Asp Leu Pro Asp Gly Leu Leu Asp
           355                 360                 365
   caa gag cct tac tat gtt gct gac tgc cat ctg tac cct cag cat ttc    1152
   Gln Glu Pro Tyr Tyr Val Ala Asp Cys His Leu Tyr Pro Gln His Phe
       370                 375                 380
   aaa caa aat ggc cag atc cct gaa act gcc gag gat ttg gta atc gaa    1200
   Lys Gln Asn Gly Gln Ile Pro Glu Thr Ala Glu Asp Leu Val Ile Glu
   385                 390                 395                 400
   gcg atc gat att ccg gat ggg ctt ttg aag ggt gat aca gaa atc gtt    1248
   Ala Ile Asp Ile Pro Asp Gly Leu Leu Lys Gly Asp Thr Glu Ile Val
                   405                 410                 415
   act ttt tcg gct gcg gca cca gga aca tca gtt gat ttg aca ctg ttt    1296
   Thr Phe Ser Ala Ala Ala Pro Gly Thr Ser Val Asp Leu Thr Leu Phe
               420                 425                 430
   gaa gcc ttc aac tcc att gct gca ttt gaa ctg aaa ggc agc aat tca    1344
   Glu Ala Phe Asn Ser Ile Ala Ala Phe Glu Leu Lys Gly Ser Asn Ser
           435                 440                 445
   cag gat gtg gct gaa tca atc aga caa att cag cag cat gcg aag ctg    1392
   Gln Asp Val Ala Glu Ser Ile Arg Gln Ile Gln Gln His Ala Lys Leu
       450                 455                 460
   acg gca aag tat gtg ctg cca gta                                    1416
   Thr Ala Lys Tyr Val Leu Pro Val
   465                 470

<210> 14
<211> 1416
<212> DNA
<213> Bacillus subtilis ATCC21555

<400> 14

atg gag aga aaa aca gta ttg gtt atc gct gat ctt ggg ggc tgc ccg 48
Met Glu Arg Lys Thr Val Leu Val Ile Ala Asp Leu Gly Gly Cys Pro
1               5                   10                  15

ccg cat atg ttt tac aaa agc gca gcc gaa aaa tac aac ctc gtc agc 96
Pro His Met Phe Tyr Lys Ser Ala Ala Glu Lys Tyr Asn Leu Val Ser
            20                  25                  30

ttt att ccg aga ccc ttt gca att aca gcc tct cat gcg gcc tta att 144
Phe Ile Pro Arg Pro Phe Ala Ile Thr Ala Ser His Ala Ala Leu Ile
        35                  40                  45

gaa aaa tac tcg att gcg gtc att aaa gat aaa gac tat ttt aag agt 192
Glu Lys Tyr Ser Ile Ala Val Ile Lys Asp Lys Asp Tyr Phe Lys Ser
    50                  55                  60

ctg gct gat ttt gaa cat ccc gat tcg att tat tgg gct cat gaa gat 240
Leu Ala Asp Phe Glu His Pro Asp Ser Ile Tyr Trp Ala His Glu Asp
65                  70                  75                  80

cat gac aaa cct gag gaa gaa gtc gtc gaa gaa atc gtg aaa gtg gcc 288
His Asp Lys Pro Glu Glu Glu Val Val Glu Glu Ile Val Lys Val Ala
                85                  90                  95

gac atg ttt ggg gtt gac gcc att acg acc aac aat gaa ctg ttt atc 336
Asp Met Phe Gly Val Asp Ala Ile Thr Thr Asn Asn Glu Leu Phe Ile
            100                 105                 110

gct ccg atg gca aaa gcg tgt aaa cgt ctc ggc ctg cgg gga gcg ggc 384
Ala Pro Met Ala Lys Ala Cys Lys Arg Leu Gly Leu Arg Gly Ala Gly
        115                 120                 125

gta cag gcc gct gaa aac gcc aga gat aaa aat aaa atg aga gcc gcc 432
Val Gln Ala Ala Glu Asn Ala Arg Asp Lys Asn Lys Met Arg Ala Ala
    130                 135                 140

ttc aac cgg gcc ggc gtc aaa tcc atc aaa aac aaa cgg gtg acg acc 480
Phe Asn Arg Ala Gly Val Lys Ser Ile Lys Asn Lys Arg Val Thr Thr
145                 150                 155                 160

ctg gaa gat ttc cgc gcc gcg ctt cag gaa atc gga acg ccg ctt att 528
Leu Glu Asp Phe Arg Ala Ala Leu Gln Glu Ile Gly Thr Pro Leu Ile
                165                 170                 175

ctg aag cct aca tat ctg gca agc tcg atc ggc gtg acg ctt att aaa 576
Leu Lys Pro Thr Tyr Leu Ala Ser Ser Ile Gly Val Thr Leu Ile Lys
            180                 185                 190

gag atg gaa acg gcc gaa gct gaa ttc aac aga gtc aat gag tac ttg 624
Glu Met Glu Thr Ala Glu Ala Glu Phe Asn Arg Val Asn Glu Tyr Leu
        195                 200                 205

aaa tcg att aat gta ccg aaa gcg gtg acg ttt gaa gcg ccg ttt atc 672
Lys Ser Ile Asn Val Pro Lys Ala Val Thr Phe Glu Ala Pro Phe Ile
    210                 215                 220

gcg gaa gaa ttc ttg cag ggc gag tat gat gac tgg tac gaa aca agc 720
Ala Glu Glu Phe Leu Gln Gly Glu Tyr Asp Asp Trp Tyr Glu Thr Ser
225                 230                 235                 240

ggt tat tcc gac tat atc agc atc gaa ggc atc atg gcc gac gga gaa 768
Gly Tyr Ser Asp Tyr Ile Ser Ile Glu Gly Ile Met Ala Asp Gly Glu
                245                 250                 255

tac ttc ccc gtt gcg atc cat gat aaa aca ccg caa atc gga ttc acg 816
Tyr Phe Pro Val Ala Ile His Asp Lys Thr Pro Gln Ile Gly Phe Thr
            260                 265                 270

gag aca gcg cat att acg ccg tcc atc ctg gat gat gac gcc aag cgg 864
Glu Thr Ala His Ile Thr Pro Ser Ile Leu Asp Asp Asp Ala Lys Arg
        275                 280                 285

aaa atc gtc gaa gct gcc aag aag gcg aat gaa gga ctc ggc ctc gaa 912
Lys Ile Val Glu Ala Ala Lys Lys Ala Asn Glu Gly Leu Gly Leu Glu
    290                 295                 300

aac tgt gca acg cat aca gaa ata aaa tta atg aaa aac cgg gaa gcc 960
Asn Cys Ala Thr His Thr Glu Ile Lys Leu Met Lys Asn Arg Glu Ala
305                 310                 315                 320

gga ctg att gag tca gcg gcc aga ttc gcg gga tgg aat atg att ccg 1008
Gly Leu Ile Glu Ser Ala Ala Arg Phe Ala Gly Trp Asn Met Ile Pro
                325                 330                 335

aat att aaa aag gtc ttc ggc gtt gat atg gcg cag cta tta ttg gat 1056
Asn Ile Lys Lys Val Phe Gly Val Asp Met Ala Gln Leu Leu Leu Asp
            340                 345                 350

gtt ctc tgt tac gga aaa gaa gct gat ctg ccg aaa gga tta ttg gag 1104
Val Leu Cys Tyr Gly Lys Glu Ala Asp Leu Pro Lys Gly Leu Leu Glu
        355                 360                 365

cag gag cca tgc tat gtc gca gac tgc cac ttg tat cct cag cat ttc 1152
Gln Glu Pro Cys Tyr Val Ala Asp Cys His Leu Tyr Pro Gln His Phe
    370                 375                 380

aaa gag aac ggc cag ctg cct gag acg gtt gtc gat ttc gtc att gaa 1200
Lys Glu Asn Gly Gln Leu Pro Glu Thr Val Val Asp Phe Val Ile Glu
385                 390                 395                 400

agc att gaa att cct gac ggc gtc tta aag gga gac act gaa ctc gtt 1248
Ser Ile Glu Ile Pro Asp Gly Val Leu Lys Gly Asp Thr Glu Leu Val
                405                 410                 415

tct ttc tca gcg gct gag gcg ggt acg tca gtg gat ctg cgg ctg ttc 1296
Ser Phe Ser Ala Ala Glu Ala Gly Thr Ser Val Asp Leu Arg Leu Phe
            420                 425                 430

gaa gcg ttc aac agc att gcg gcg ttt gag ctg aaa gga agc aat tcg 1344
Glu Ala Phe Asn Ser Ile Ala Ala Phe Glu Leu Lys Gly Ser Asn Ser
        435                 440                 445

aac gac gtg gcc gaa tca atc aaa caa att cag cag cag gcg aag ctg 1392
Asn Asp Val Ala Glu Ser Ile Lys Gln Ile Gln Gln Gln Ala Lys Leu
    450                 455                 460

act gca aag tat gcg tta tcg gta 1416
Thr Ala Lys Tyr Ala Leu Ser Val

<210> 15
<211> 1416
<212> DNA
<213> Bacillus amyloliquefaciens IFO3022

<400> 15
atg gag aga aaa aca gta ttg gtt atc gct gac ctt ggg gga tgc ccg    48
Met Glu Arg Lys Thr Val Leu Val Ile Ala Asp Leu Gly Gly Cys Pro
1               5                   10                  15

ccg cat atg ttt tac aaa agc gca gcc gaa aaa tac aac ctc gtc agc    96
Pro His Met Phe Tyr Lys Ser Ala Ala Glu Lys Tyr Asn Leu Val Ser
            20                  25                  30

ttt att ccg aga cct ttt gca att aca gcc tct cat gcg gca tta att    144
Phe Ile Pro Arg Pro Phe Ala Ile Thr Ala Ser His Ala Ala Leu Ile
        35                  40                  45

gaa aaa tac tcg gtc gcg gtc ata aaa gat aaa gac tat ttt aag agt    192
Glu Lys Tyr Ser Val Ala Val Ile Lys Asp Lys Asp Tyr Phe Lys Ser
    50                  55                  60

ctg gct gat ttt gag cat ccc gat tcg att tac tgg gct cat gaa gat    240
Leu Ala Asp Phe Glu His Pro Asp Ser Ile Tyr Trp Ala His Glu Asp
65                  70                  75                  80

cat gac aaa cct gag gaa gaa gta gtc gaa gaa atc gtc aag gtg gcc    288
His Asp Lys Pro Glu Glu Glu Val Val Glu Glu Ile Val Lys Val Ala
                85                  90                  95

ggc atg ttc gcg gtt gac gcc att acg acc aac aat gaa ctg ttt atc    336
Gly Met Phe Ala Val Asp Ala Ile Thr Thr Asn Asn Glu Leu Phe Ile
            100                 105                 110

gct ccg atg gca aaa gcg tgt gaa cgt ctc ggc ctg cgg gga gcg ggc    384
Ala Pro Met Ala Lys Ala Cys Glu Arg Leu Gly Leu Arg Gly Ala Gly
        115                 120                 125

gta cag gcc gct gaa aat gcc aga gat aaa aac aaa atg aga gcc gct    432
Val Gln Ala Ala Glu Asn Ala Arg Asp Lys Asn Lys Met Arg Ala Ala
    130                 135                 140

ttc aac cgg gcc ggc gtc aag tct atc aaa aac aga cgg gtg acg acg    480

Phe Asn Arg Ala Gly Val Lys Ser Ile Lys Asn Arg Arg Val Thr Thr
145                 150                 155                 160

ctg gaa gat ttc cgc gcc gcg ctt cag gaa atc gga acg ccg ctc att    528
Leu Glu Asp Phe Arg Ala Ala Leu Gln Glu Ile Gly Thr Pro Leu Ile
                165                 170                 175

ctg aag cct aca tat ctg gcg agc tcc atc ggc gtg acg ctc atc aaa    576
Leu Lys Pro Thr Tyr Leu Ala Ser Ser Ile Gly Val Thr Leu Ile Lys
            180                 185                 190

gag agg gaa acg gcc gaa gcc gaa ttt aac aga gtc aat gaa tac ctg    624
Glu Arg Glu Thr Ala Glu Ala Glu Phe Asn Arg Val Asn Glu Tyr Leu
        195                 200                 205

aag tcg atc aac gta ccg aaa gcg gtc acg ttt gaa gcg ccg ttt atc    672

Lys Ser Ile Asn Val Pro Lys Ala Val Thr Phe Glu Ala Pro Phe Ile
    210                 215                 220

gcg gaa gaa ttt ttg cag ggc gag tat gac gac tgg tac gaa aca agc     720
Ala Glu Glu Phe Leu Gln Gly Glu Tyr Asp Asp Trp Tyr Glu Thr Ser
225                 230                 235                 240

ggt tat tcc gac tat atc agc ata gaa ggc atc atg gcc gac gga gaa     768
Gly Tyr Ser Asp Tyr Ile Ser Ile Glu Gly Ile Met Ala Asp Gly Glu
                245                 250                 255

tac ttc cct gtc gca att cat gat aaa aca ccg caa atc gga ttc acg     816
Tyr Phe Pro Val Ala Ile His Asp Lys Thr Pro Gln Ile Gly Phe Thr
            260                 265                 270

gag aca tcg cat att acg ccg tcc atc ctg gat gat gac gcg aag cgg     864
Glu Thr Ser His Ile Thr Pro Ser Ile Leu Asp Asp Asp Ala Lys Arg
        275                 280                 285

aaa atc gtc gaa gca gcc aaa aag gcg aat gaa gga ctc ggc ctc gaa     912
Lys Ile Val Glu Ala Ala Lys Lys Ala Asn Glu Gly Leu Gly Leu Glu
    290                 295                 300

aac tgc gca acc cat aca gag att aaa tta atg aaa aac cgg gaa gcc     960
Asn Cys Ala Thr His Thr Glu Ile Lys Leu Met Lys Asn Arg Glu Ala
305                 310                 315                 320

gga ctg att gaa tca gcg gca cga ttt gcg ggc tgg aac atg att ccg     1008
Gly Leu Ile Glu Ser Ala Ala Arg Phe Ala Gly Trp Asn Met Ile Pro
                325                 330                 335

aat att aaa aag gtc ttc ggc gtc gat atg gcg cag ctg tta ttg gat     1056
Asn Ile Lys Lys Val Phe Gly Val Asp Met Ala Gln Leu Leu Leu Asp
            340                 345                 350

gtt ctc tgt ttc gga aaa gaa gcc gat ctg ccg aaa gga tta ttg gag     1104
Val Leu Cys Phe Gly Lys Glu Ala Asp Leu Pro Lys Gly Leu Leu Glu
        355                 360                 365

cag gag ccg tgc tat gtc gcc gac tgc cac ttg tat cct cag cat ttc     1152
Gln Glu Pro Cys Tyr Val Ala Asp Cys His Leu Tyr Pro Gln His Phe
    370                 375                 380

aaa gag aac ggc cag ctg cct gag acg gct gtc gat ttc gtc att gaa     1200
Lys Glu Asn Gly Gln Leu Pro Glu Thr Ala Val Asp Phe Val Ile Glu
385                 390                 395                 400

agc att gac att ccc gac ggc gtc tta aag gga gac acc gaa atc gtt     1248
Ser Ile Asp Ile Pro Asp Gly Val Leu Lys Gly Asp Thr Glu Ile Val
                405                 410                 415

tct ttc tcg gcg gcc gag gcg ggt aca tcc gtg gat ctg cgg ctg ttc     1296
Ser Phe Ser Ala Ala Glu Ala Gly Thr Ser Val Asp Leu Arg Leu Phe
            420                 425                 430

gaa gcg ttc aac agc att gcg gcg ttc gag ctg aaa gga agc aat tcg     1344
Glu Ala Phe Asn Ser Ile Ala Ala Phe Glu Leu Lys Gly Ser Asn Ser
        435                 440                 445

ggt gac gtg gcc gaa tca atc aaa caa att cag cag cag gcg aag ctg     1392

Gly Asp Val Ala Glu Ser Ile Lys Gln Ile Gln Gln Gln Ala Lys Leu
    450                 455                 460

act gca aag tat gcg tta ccg gta                                    1416
Thr Ala Lys Tyr Ala Leu Pro Val

<210> 16
<211> 1428
<212> DNA
<213> Bacillus pumilus NRRL B-12025

<400> 16
gtg ctt tca ttg agt aaa aaa act gta ctt gtc att gct gac tta gga    48
Val Leu Ser Leu Ser Lys Lys Thr Val Leu Val Ile Ala Asp Leu Gly
  1               5                  10                  15

ggg tgc ccg ccc cat atg ttt tat gaa agc gtg gcg gca tca tac cat    96
Gly Cys Pro Pro His Met Phe Tyr Glu Ser Val Ala Ala Ser Tyr His
             20                  25                  30

atc gtt tct tat atc cca aga ccc ttt gcg att aca aag gga cat gcc    144
Ile Val Ser Tyr Ile Pro Arg Pro Phe Ala Ile Thr Lys Gly His Ala
         35                  40                  45

gag cta atc gaa aaa tac tcc att gcc gtc atc aaa gac cgt gat tat    192
Glu Leu Ile Glu Lys Tyr Ser Ile Ala Val Ile Lys Asp Arg Asp Tyr
     50                  55                  60

ttt gag aca cac cct tct ttt gaa cac cct gat tct att tac tgg gca    240
Phe Glu Thr His Pro Ser Phe Glu His Pro Asp Ser Ile Tyr Trp Ala
 65                  70                  75                  80

cat gat gat tat cca aaa tca gaa gaa gaa gtt gtg gaa gac ttc att    288
His Asp Asp Tyr Pro Lys Ser Glu Glu Glu Val Val Glu Asp Phe Ile
                 85                  90                  95

cga gta gct tcc ttt ttc aaa gca gat gca atc acg acc aat aat gaa    336
Arg Val Ala Ser Phe Phe Lys Ala Asp Ala Ile Thr Thr Asn Asn Glu
            100                 105                 110

tta ttc att gca ccg atg gca aag gcc gct gaa cgt ctt ggg cta cga    384
Leu Phe Ile Ala Pro Met Ala Lys Ala Ala Glu Arg Leu Gly Leu Arg
        115                 120                 125

ggt gcc ggt gtc aag gca gcc gaa atg gcg cgt gat aaa agc caa atg    432
Gly Ala Gly Val Lys Ala Ala Glu Met Ala Arg Asp Lys Ser Gln Met
    130                 135                 140

agg gct gca ttc aat gcc tct ggc gtc aaa gcg gtg aaa act cag cct    480
Arg Ala Ala Phe Asn Ala Ser Gly Val Lys Ala Val Lys Thr Gln Pro
145                 150                 155                 160

gtc acg act tta tct gat ttc caa caa gcc att gag tct atc gga aca    528
Val Thr Thr Leu Ser Asp Phe Gln Gln Ala Ile Glu Ser Ile Gly Thr
                165                 170                 175

ccg ctc att tta aag cct aca tat tta gcc agt tct att ggc gtc acc    576
Pro Leu Ile Leu Lys Pro Thr Tyr Leu Ala Ser Ser Ile Gly Val Thr
            180                 185                 190

ttg ttt cat gac aaa gcc gga agt gat gac ttg ttt tta caa gta caa 624
Leu Phe His Asp Lys Ala Gly Ser Asp Asp Leu Phe Leu Gln Val Gln
        195                 200                 205

tcg tat ttg gaa acc ata cca gtc cca gac gct gtc acg tat gaa gca 672
Ser Tyr Leu Glu Thr Ile Pro Val Pro Asp Ala Val Thr Tyr Glu Ala
    210                 215                 220

ccg ttt gtc gct gaa aca tat tta gag ggt gct tac gaa gat tgg tat 720
Pro Phe Val Ala Glu Thr Tyr Leu Glu Gly Ala Tyr Glu Asp Trp Tyr
225                 230                 235                 240

gaa gac gaa gga tat gct gat tat gtc agt gta gaa ggg ctg gtc gta 768
Glu Asp Glu Gly Tyr Ala Asp Tyr Val Ser Val Glu Gly Leu Val Val
                245                 250                 255

gag ggc gaa tat ctc cct ttt gtc ata cat gat aaa acc cct caa atc 816
Glu Gly Glu Tyr Leu Pro Phe Val Ile His Asp Lys Thr Pro Gln Ile
            260                 265                 270

ggc ttt aca gaa acg gct cat atc act ccg acg atc tta gac aat gaa 864
Gly Phe Thr Glu Thr Ala His Ile Thr Pro Thr Ile Leu Asp Asn Glu
        275                 280                 285

gcc aag caa atc atc att gaa gca gca agg aag gca aat gaa ggg cta 912
Ala Lys Gln Ile Ile Ile Glu Ala Ala Arg Lys Ala Asn Glu Gly Leu
    290                 295                 300

ggt ctt gaa cat tgt gca acc cat aca gaa atc aaa ctc atg aaa aat 960
Gly Leu Glu His Cys Ala Thr His Thr Glu Ile Lys Leu Met Lys Asn
305                 310                 315                 320

cga gaa act gga ctg atc gag gca gcg gct cga ttc gct ggc tgg aat 1008
Arg Glu Thr Gly Leu Ile Glu Ala Ala Ala Arg Phe Ala Gly Trp Asn
                325                 330                 335

atg atc ccg aat att aaa aaa gtc ttt ggc gtc gat atg gcg aag cta 1056
Met Ile Pro Asn Ile Lys Lys Val Phe Gly Val Asp Met Ala Lys Leu
            340                 345                 350

ttg att gat gta tta gtt gat ggt aaa aag gct gta ctg cca aaa cag 1104
Leu Ile Asp Val Leu Val Asp Gly Lys Lys Ala Val Leu Pro Lys Gln
        355                 360                 365

ctg ctt tct gga cat aca ttt tat gta gcg gac tgc cac ctg tac cct 1152
Leu Leu Ser Gly His Thr Phe Tyr Val Ala Asp Cys His Leu Tyr Pro
    370                 375                 380

cag cat ttt aaa gag agt ggg ctt atc ccg cct gaa gcc aca cat att 1200
Gln His Phe Lys Glu Ser Gly Leu Ile Pro Pro Glu Ala Thr His Ile
385                 390                 395                 400

acc att gat cat gtg tct att ccg cag gaa gca ttc gtt gga gat act 1248
Thr Ile Asp His Val Ser Ile Pro Gln Glu Ala Phe Val Gly Asp Thr
                405                 410                 415

gcg att gtc agt caa tca ttc cct gcc aaa ggg act att gtg gat ctt 1296
Ala Ile Val Ser Gln Ser Phe Pro Ala Lys Gly Thr Ile Val Asp Leu
            420                 425                 430

gaa tta ttt gaa gct ttt aat gga atc gta tct ctt gaa tta aaa gga    1344
Glu Leu Phe Glu Ala Phe Asn Gly Ile Val Ser Leu Glu Leu Lys Gly
        435                 440                 445

tca tcc tca caa gat gtt gcc gcg tcc atc cgc aac att cag aaa cag    1392
Ser Ser Ser Gln Asp Val Ala Ala Ser Ile Arg Asn Ile Gln Lys Gln
    450                 455                 460

gca acg att cag tta atg gat gaa tta gtg aag gga                    1428
Ala Thr Ile Gln Leu Met Asp Glu Leu Val Lys Gly
465                 470                 475

<210> 17
<211> 93
<212> PRT
<213> Bacillus subtilis 168

<400> 17
Gly Ala Gly Val Gln Ala Ala Glu Asn Ala Arg Asp Lys Asn Lys Met
  1               5                  10                  15

Arg Asp Ala Phe Asn Lys Ala Gly Val Lys Ser Ile Lys Asn Lys Arg
             20                  25                  30

Val Thr Thr Leu Glu Asp Phe Arg Ala Ala Leu Glu Glu Ile Gly Thr
         35                  40                  45

Pro Leu Ile Leu Lys Pro Thr Tyr Leu Ala Ser Ser Ile Gly Val Thr
     50                  55                  60

Leu Ile Thr Asp Thr Glu Thr Ala Glu Asp Glu Phe Asn Arg Val Asn
 65                  70                  75                  80

Asp Tyr Leu Lys Ser Ile Asn Val Pro Lys Ala Val Thr
                 85                  90

<210> 18
<211> 279
<212> DNA
<213> Bacillus subtilis 168

<400> 18
ggt gcc ggc gtg cag gca gcc gaa aat gcc aga gat aaa aat aaa atg     48
Gly Ala Gly Val Gln Ala Ala Glu Asn Ala Arg Asp Lys Asn Lys Met
  1               5                  10                  15

agg gac gct ttt aat aag gcc gga gtc aaa tcg atc aaa aac aaa cga     96
Arg Asp Ala Phe Asn Lys Ala Gly Val Lys Ser Ile Lys Asn Lys Arg
             20                  25                  30

gtc aca act ctt gaa gat ttc cgt gct gct ctt gaa gag atc ggc aca    144
Val Thr Thr Leu Glu Asp Phe Arg Ala Ala Leu Glu Glu Ile Gly Thr
         35                  40                  45

cct ctt atc tta aag cct aca tac tta gcg agt tct atc ggt gta acg    192
Pro Leu Ile Leu Lys Pro Thr Tyr Leu Ala Ser Ser Ile Gly Val Thr
     50                  55                  60

ctg att acg gac act gag acg gca gaa gat gaa ttt aac aga gtc aat     240
Leu Ile Thr Asp Thr Glu Thr Ala Glu Asp Glu Phe Asn Arg Val Asn
 65                  70                  75                  80

gac tat ctg aaa tca att aac gtg cca aag gcg gtt acg                 279
Asp Tyr Leu Lys Ser Ile Asn Val Pro Lys Ala Val Thr
                85                  90

<210> 19
<211> 30
<212> DNA
<213> Artificial Sequence

<220>
<223> Description of Artificial Sequence: Synthetic DNA

<400> 19
attctcgagt agagaaggag tgttttacat                                    30

<210> 20
<211> 30
<212> DNA
<213> Artificial Sequence

<220>
<223> Description of Artificial Sequence: Synthetic DNA

<400> 20
ttaggatcct catactggca gcacatactt                                    30

<210> 21
<211> 24
<212> DNA
<213> Artificial Sequence

<220>
<223> Description of Artificial Sequence: Synthetic DNA

<400> 21
caagaattct catgtttgac agct                                          24

<210> 22
<211> 28
<212> DNA
<213> Artificial Sequence

<220>
<223> Description of Artificial Sequence: Synthetic DNA

<400> 22
taactcgaga ttcccttttt acgtgaac                                      28

<210> 23
<211> 27
<212> DNA
<213> Artificial Sequence

<220>
<223> Description of Artificial Sequence: Synthetic DNA

<400> 23
ttaaccatgg agagaaaaac agtattg                                          27

<210> 24
<211> 30
<212> DNA
<213> Artificial Sequence

<220>
<223> Description of Artificial Sequence: Synthetic DNA

<400> 24
atatggatcc tactggcagc acatactttg                                       30

<210> 25
<211> 21
<212> DNA
<213> Artificial Sequence

<220>
<223> Description of Artificial Sequence: Synthetic DNA

<400> 25
caccgcagac ggaggataca c                                                21


<210> 26
<211> 22
<212> DNA
<213> Artificial Sequence

<220>
<223> Description of Artificial Sequence: Synthetic DNA

<400> 26
cggacgtcac ccaataatcg tg                                               22


<210> 27
<211> 23
<212> DNA
<213> Artificial Sequence

<220>
<223> Description of Artificial Sequence: Synthetic DNA

<400> 27
ccgatggcra aagcstgtra acg                                              23


<210> 28
<211> 21
<212> DNA
<213> Artificial Sequence

<220>

<223> Description of Artificial Sequence: Synthetic DNA

<400> 28
cggcagatcr gcdtcttttc c                                                21


<210> 29
<211> 26
<212> DNA
<213> Artificial Sequence

<220>
<223> Description of Artificial Sequence: Synthetic DNA

<400> 29
gctaggtctt gaacattgtg caaccc                                           26


<210> 30
<211> 23
<212> DNA
<213> Artificial Sequence

<220>
<223> Description of Artificial Sequence: Synthetic DNA

<400> 30
ggtgttccga tagactcaat ggc                                              23


<210> 31
<211> 44
<212> DNA
<213> Artificial Sequence

<220>
<223> Description of Artificial Sequence: Synthetic DNA

<400> 31
catgccatgg agaaaaaaac tgtacttgtc attgctgact tagg                 □@□@□@ 44



<210> 32
<211> 38
<212> DNA
<213> Artificial Sequence

<220>
<223> Description of Artificial Sequence: Synthetic DNA

<400> 32
cgcggatccc ttcactaatt catccattaa ctgaatcg                        □@□@□@38


<210> 33
<211> 12
<212> PRT
<213> Artificial Sequence

<220>
<221> UNSURE
<222> (3)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (4)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (9)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (10)
<223> Xaa represents any amino acid selected from Leu, Ile, Val, Met and Ala

<220>
<221> UNSURE
<222> (11)
<223> Xaa represents Glu, Ser or Ala

<220>
<221> UNSURE
<222> (12)
<223> Xaa represents Gly, Ser or Ala

<220>
<223> Description of Artificial Sequence: Amino acid sequence used for data base
search

<400> 33
His Gly Xaa Xaa Gly Gln Asp Gly Xaa Xaa Xaa Xaa
                5                   10


<210> 34
<211> 28
<212> PRT
<213> Artificial Sequence

<220>
<221> UNSURE
<222> (1)
<223> Xaa represents Leu Ile or Val

<220>
<221> UNSURE
<222> (2)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE

<222> (3)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (4)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (5)
<223> Xaa represents Gly or Ala

<220>
<221> UNSURE
<222> (6)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (7)
<223> Xaa represents any amino acid selected from Gly, Ser, Ala, Ile and Val

<220>
<221> UNSURE
<222> (9)
<223> Xaa represents any amino acid selected from Leu, Ile, Val, Met, Cys and
Ala

<220>
<221> UNSURE
<222> (11)
<223> Xaa represents any amino acid selected from Leu, Ile, Val, Met, Phe and
Ala

<220>
<221> UNSURE
<222> (12)
<223> Xaa represents any amino acid selected from Leu, Ile, Val, Met, Phe and
Ala

<220>
<221> UNSURE
<222> (13)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (14)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val


<220>
<221> UNSURE
<222> (15)

<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (16)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (17)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (18)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (19)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (20)
<223> Xaa represents Leu, Ile or Val

<220>
<221> UNSURE
<222> (21)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (23)
<223> Xaa represents any amino acid selected from Leu, Ile, Val, Ala and Pro

<220>
<221> UNSURE
<222> (25)
<223> Xaa represents Ser, Thr or Pro

<220>
<221> UNSURE
<222> (26)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (28)
<223> Xaa represents Gly or Ala

<220>

<223> Description of Artificial Sequence: Amino acid sequence used for data base
search

<400> 34
Xaa Xaa Xaa Xaa Xaa Xaa Xaa Arg Xaa Asp Xaa Xaa Xaa Xaa Xaa Xaa
                5                   10                  15

Xaa Xaa Xaa Xaa Xaa Gln Xaa Asn Xaa Xaa Pro Xaa
            20                  25


<210> 35
<211> 30
<212> PRT
<213> Artificial Sequence

<220>
<221> UNSURE
<222> (1)
<223> Xaa represents Leu Ile or Val

<220>
<221> UNSURE
<222> (2)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (3)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (4)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (5)
<223> Xaa represents Gly or Ala

<220>
<221> UNSURE
<222> (6)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (7)
<223> Xaa represents any amino acid selected from Gly, Ser, Ala, Ile and Val

<220>
<221> UNSURE
<222> (9)
<223> Xaa represents any amino acid selected from Leu, Ile, Val, Met, Cys and
Ala

<220>
<221> UNSURE
<222> (11)
<223> Xaa represents any amino acid selected from Leu, Ile, Val, Met, Phe and
Ala

<220>
<221> UNSURE
<222> (12)
<223> Xaa represents any amino acid selected from Leu, Ile, Val, Met, Phe and
Ala

<220>
<221> UNSURE
<222> (13)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (14)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (15)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (16)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (17)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (18)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (19)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (20)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (21)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (22)
<223> Xaa represents Leu, Ile or Val

<220>
<221> UNSURE
<222> (23)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (25)
<223> Xaa represents any amino acid selected from Leu, Ile, Val, Ala and Pro

<220>
<221> UNSURE
<222> (27)
<223> Xaa represents Ser, Thr or Pro

<220>
<221> UNSURE
<222> (28)
<223> Xaa represents any amino acid selected from Ala, Arg, Asn, Asp, Cys, Glu,
Gln, Gly, His, Ile, Leu, Lys, Met, Phe, Pro, Ser, Thr, Trp, Tyr and Val

<220>
<221> UNSURE
<222> (30)
<223> Xaa represents Gly or Ala

<220>
<223> Description of Artificial Sequence: Amino acid sequence used for data base
search

<400> 35
Xaa Xaa Xaa Xaa Xaa Xaa Xaa Arg Xaa Asp Xaa Xaa Xaa Xaa Xaa Xaa
                5                   10                  15

Xaa Xaa Xaa Xaa Xaa Xaa Xaa Gln Xaa Asn Xaa Xaa Pro Xaa
            20                  25                  30


<210> 36
<211> 1416
<212> DNA
<213> Bacillus subtilis ATCC 15245 and Bacillus subtilis IAM 1033

<400> 36
atg gag aga aaa aca gta ttg gtc atc gct gat ctt gga ggc tgc ccg    48
Met Glu Arg Lys Thr Val Leu Val Ile Ala Asp Leu Gly Gly Cys Pro
  1               5                  10                  15

ccg cac atg ttt tat aaa agc gct gct gaa aaa tat aac ctg gtc agc     96
Pro His Met Phe Tyr Lys Ser Ala Ala Glu Lys Tyr Asn Leu Val Ser
            20                  25                  30

ttt att cca aga cct ttt gca att aca gcc tcc cat gca gca ttg att     144
Phe Ile Pro Arg Pro Phe Ala Ile Thr Ala Ser His Ala Ala Leu Ile
        35                  40                  45

gaa aaa tac tcg gtc gcg gtc ata aaa gat aaa gac tat ttt aag agt     192
Glu Lys Tyr Ser Val Ala Val Ile Lys Asp Lys Asp Tyr Phe Lys Ser
    50                  55                  60

tta gct gat ttt gag cat cct gat tcc att tat tgg gcg cat gag gat     240
Leu Ala Asp Phe Glu His Pro Asp Ser Ile Tyr Trp Ala His Glu Asp
65                  70                  75                  80

cat aac aag cct gag gaa gag gtc gtc gag caa atc gtc aag gtt gcc     288
His Asn Lys Pro Glu Glu Glu Val Val Glu Gln Ile Val Lys Val Ala
                85                  90                  95

gaa atg ttt ggg gcg gat gcc atc aca aca aac aat gaa tta ttc att     336
Glu Met Phe Gly Ala Asp Ala Ile Thr Thr Asn Asn Glu Leu Phe Ile
            100                 105                 110

gct ccg atg gcg aaa gcc tgt gaa cgt ctg ggc ctg aga ggt gcc ggc     384
Ala Pro Met Ala Lys Ala Cys Glu Arg Leu Gly Leu Arg Gly Ala Gly
        115                 120                 125

gtg cag gca gcc gaa aat gcc aga gat aaa aat aaa atg agg gac gct     432
Val Gln Ala Ala Glu Asn Ala Arg Asp Lys Asn Lys Met Arg Asp Ala
    130                 135                 140

ttt aat aag gcc gga gtc aaa tcg atc aaa aac aaa cga gtc aca act     480
Phe Asn Lys Ala Gly Val Lys Ser Ile Lys Asn Lys Arg Val Thr Thr
145                 150                 155                 160

ctt gaa gat ttc cgt gct gct ctt gaa gag atc ggc aca cct ctt atc     528
Leu Glu Asp Phe Arg Ala Ala Leu Glu Glu Ile Gly Thr Pro Leu Ile
                165                 170                 175

tta aag cct aca tac tta gcg agt tca atc ggt gta acg ctg att acg     576
Leu Lys Pro Thr Tyr Leu Ala Ser Ser Ile Gly Val Thr Leu Ile Thr
            180                 185                 190

gac act gag acg gca gaa gat gaa ttt aac aga gtc aat gac tat ctg     624
Asp Thr Glu Thr Ala Glu Asp Glu Phe Asn Arg Val Asn Asp Tyr Leu
        195                 200                 205

aaa tca att aac gtg cca aag gcg gtt acg ttt gaa gcg ccg ttt atc     672
Lys Ser Ile Asn Val Pro Lys Ala Val Thr Phe Glu Ala Pro Phe Ile
    210                 215                 220

gct gaa gaa ttt tta cag ggt gag tac gga gac tgg tat caa aca gaa     720
Ala Glu Glu Phe Leu Gln Gly Glu Tyr Gly Asp Trp Tyr Gln Thr Glu
225                 230                 235                 240

ggg tac tcc gac tat atc agt ata gaa ggc atc atg gct gac ggt gag     768
Gly Tyr Ser Asp Tyr Ile Ser Ile Glu Gly Ile Met Ala Asp Gly Glu
                245                 250                 255

tat ttc ccg atc gcc att cat gat aaa acg ccg caa atc ggg ttt aca 816

Tyr Phe Pro Ile Ala Ile His Asp Lys Thr Pro Gln Ile Gly Phe Thr
            260                 265                 270

gag aca tcc cac att acg ccg tcc att ctg gat gaa gag gca aaa aag 864
Glu Thr Ser His Ile Thr Pro Ser Ile Leu Asp Glu Glu Ala Lys Lys
        275                 280                 285

aaa att gtc gaa gct gcc aaa aag gca aat gaa ggg ctt ggc ctg caa 912
Lys Ile Val Glu Ala Ala Lys Lys Ala Asn Glu Gly Leu Gly Leu Gln
    290                 295                 300

aat tgc gca aca cat aca gag atc aag cta atg aaa aac aga gaa ccg 960
Asn Cys Ala Thr His Thr Glu Ile Lys Leu Met Lys Asn Arg Glu Pro
305                 310                 315                 320

ggt tta ata gag tcg gca gcc aga ttc gca ggc tgg aat atg att cct 1008
Gly Leu Ile Glu Ser Ala Ala Arg Phe Ala Gly Trp Asn Met Ile Pro
                325                 330                 335

aat att aaa aag gtc ttt ggc ctt gat atg gcg caa tta tta tta gat 1056
Asn Ile Lys Lys Val Phe Gly Leu Asp Met Ala Gln Leu Leu Leu Asp
            340                 345                 350

gtc ctc tgt ttc gga aaa gac gcc gat ctg ccg gac gga tta ttg gat 1104
Val Leu Cys Phe Gly Lys Asp Ala Asp Leu Pro Asp Gly Leu Leu Asp
        355                 360                 365

caa gag cct tat tat gtt gcc gac tgc cat ttg tac ccg cag cat ttc 1152
Gln Glu Pro Tyr Tyr Val Ala Asp Cys His Leu Tyr Pro Gln His Phe
    370                 375                 380

aaa caa aat ggc cag att cca gaa acc gct gag gat ttg gtc att gaa 1200
Lys Gln Asn Gly Gln Ile Pro Glu Thr Ala Glu Asp Leu Val Ile Glu
385                 390                 395                 400

gcg atc gat att ccg gac ggg ctt tta aaa ggg gat act gaa atc gtt 1248
Ala Ile Asp Ile Pro Asp Gly Leu Leu Lys Gly Asp Thr Glu Ile Val
                405                 410                 415

tca ttt tca gcc gca gca cca ggc act tca gtt gat ttg aca ttg ttt 1296
Ser Phe Ser Ala Ala Ala Pro Gly Thr Ser Val Asp Leu Thr Leu Phe
            420                 425                 430

gaa gct ttc aat tcc att gct gca ttt gaa ctg aaa ggc agt aat tca 1344
Glu Ala Phe Asn Ser Ile Ala Ala Phe Glu Leu Lys Gly Ser Asn Ser
        435                 440                 445

cag gat gtg gct gaa tca atc aga caa att cag cag cat gca aag ctg 1392
Gln Asp Val Ala Glu Ser Ile Arg Gln Ile Gln Gln His Ala Lys Leu
    450                 455                 460
acg gca aag tat gtg ctg cca gta 1416
Thr Ala Lys Tyr Val Leu Pro Val
465                 470

<210> 37
<211> 239

<212> PRT
<213> Streptomyces noursei IFO15452

<400> 37
Met Leu Ala Gly Leu Val Pro Ala Pro Asp His Gly Met Arg Glu Glu
1               5                   10                  15

Ile Leu Gly Asp Arg Ser Arg Leu Ile Arg Gln Arg Gly Glu His Ala
            20                  25                  30

Leu Ile Gly Ile Ser Ala Gly Asn Ser Tyr Phe Ser Gln Lys Asn Thr
        35                  40                  45

Val Met Leu Leu Gln Trp Ala Gly Gln Arg Phe Glu Arg Thr Asp Val
    50                  55                  60

Val Tyr Val Asp Thr His Ile Asp Glu Met Leu Ile Ala Asp Gly Arg
65                  70                  75                  80

Ser Ala Gln Glu Ala Glu Arg Ser Val Lys Arg Thr Leu Lys Asp Leu
                85                  90                  95

Arg Arg Arg Leu Arg Arg Ser Leu Glu Ser Val Gly Asp His Ala Glu
            100                 105                 110

Arg Phe Arg Val Arg Ser Leu Ser Glu Leu Gln Glu Thr Pro Glu Tyr
        115                 120                 125

Arg Ala Val Arg Glu Arg Thr Asp Arg Ala Phe Glu Glu Asp Ala Glu
    130                 135                 140

Phe Ala Thr Ala Cys Glu Asp Met Val Arg Ala Val Val Met Asn Arg
145                 150                 155                 160

Pro Gly Asp Gly Val Gly Ile Ser Ala Glu His Leu Arg Ala Gly Leu
                165                 170                 175

Asn Tyr Val Leu Ala Glu Ala Pro Leu Phe Ala Asp Ser Pro Gly Val
            180                 185                 190

Phe Ser Val Pro Ser Ser Val Leu Cys Tyr His Ile Asp Thr Pro Ile
        195                 200                 205

Thr Ala Phe Leu Ser Arg Arg Glu Thr Gly Phe Arg Ala Ala Glu Gly

    210                 215                 220

Gln Ala Tyr Val Val Val Arg Pro Gln Glu Leu Ala Asp Ala Ala
225                 230                 235


<210> 38
<211> 239
<212> PRT
<213> Streptomyces alborus IFO15452

<400> 38
Met Leu Ala Gly Leu Val Pro Ala Leu Asp His Ser Met Arg Glu Glu
1               5                   10                  15

Ile Leu Gly Asn Arg Gly Arg Lys Ile Arg Gln Arg Gly Glu His Ala
            20                  25                  30

Leu Ile Gly Ile Ser Ala Gly Asn Ser Tyr Phe Ser Gln Lys Asn Val
        35                  40                  45

Thr Met Leu Leu Gln Trp Ala Gly Gln His Phe Glu Arg Thr Asp Val
    50                  55                  60

Val Tyr Val Asp Thr His Ile Asp Asp Met Leu Met Ala Asp Gly Arg
65                  70                  75                  80

Ser Ala Gln Glu Ala Glu Lys Ser Val Lys Arg Thr Leu Lys Asp Leu
                85                  90                  95

Arg Arg Arg Leu Arg Arg Ser Leu Glu Ser Val Gly Asp His Ser Glu
            100                 105                 110

Arg Phe Arg Val Arg Ser Leu Ser Glu Ile Gln Glu Thr Pro Glu Tyr
        115                 120                 125

Arg Ala Ala Arg Glu Ser Thr Asp Arg Ala Phe Arg Glu Asp Gly Glu
    130                 135                 140

Phe Ala Thr Val Cys Glu Glu Met Val Arg Ala Val Val Met Asn Arg
145                 150                 155                 160

Pro Gly Asp Gly Val Asp Ile Ser Glu Glu His Leu Arg Ala Gly Leu
                165                 170                 175

Asn Tyr Val Leu Ala Glu Ala Pro Leu Phe Ala Asp Ser Pro Gly Val
            180                 185                 190

Phe Ser Val Pro Ser Ser Val Leu Cys Tyr His Ile Pro Thr Pro Val
        195                 200                 205

Ser Thr Phe Leu Ala His Arg Glu Thr Gly Phe Gln Ala Ala Gln Gly
    210                 215                 220

Gln Ala Tyr Val Val Val Arg Pro Gln Glu Leu Ala Asp Ala Ala
225                 230                 235

<210> 39
<211> 717
<212> DNA
<213> Streptomyces noursei IFO15452

<400> 39
atg ctt gca ggc tta gtt ccc gcg ccg gac cac gga atg cgg gaa gaa      48
Met Leu Ala Gly Leu Val Pro Ala Pro Asp His Gly Met Arg Glu Glu
1               5                   10                  15

ata ctt ggc gac cgc agc cga ttg atc cgg caa cgc ggt gag cac gcc      96
Ile Leu Gly Asp Arg Ser Arg Leu Ile Arg Gln Arg Gly Glu His Ala
            20                  25                  30

ctc atc gga atc agt gcg ggc aac agt tat ttc agc cag aag aac acc      144
Leu Ile Gly Ile Ser Ala Gly Asn Ser Tyr Phe Ser Gln Lys Asn Thr
        35                  40                  45

gtc atg ctg ctg caa tgg gcc ggg cag cgt ttc gag cgc acc gat gtc    192

Val Met Leu Leu Gln Trp Ala Gly Gln Arg Phe Glu Arg Thr Asp Val
    50                  55                  60

gtc tat gtc gac acc cac atc gac gag atg ctg atc gcc gac ggc cgc    240
Val Tyr Val Asp Thr His Ile Asp Glu Met Leu Ile Ala Asp Gly Arg
65                  70                  75                  80

agc gcg cag gag gcc gag cgg tcg gtc aaa cgc acg ctc aag gat ctg    288
Ser Ala Gln Glu Ala Glu Arg Ser Val Lys Arg Thr Leu Lys Asp Leu
                85                  90                  95

cgg cgc aga ctc cgg cgc tcg ctg gag agc gtg ggc gac cac gcc gag    336
Arg Arg Arg Leu Arg Arg Ser Leu Glu Ser Val Gly Asp His Ala Glu
            100                 105                 110

cgg ttc cgt gtc cgg tcc ctg tcc gag ctc cag gag acc cct gag tac    384
Arg Phe Arg Val Arg Ser Leu Ser Glu Leu Gln Glu Thr Pro Glu Tyr
        115                 120                 125

cgg gcc gta cgc gag cgc acc gac cgg gcc ttc gag gag gac gcc gaa    432
Arg Ala Val Arg Glu Arg Thr Asp Arg Ala Phe Glu Glu Asp Ala Glu
    130                 135                 140

ttc gcc acc gcc tgc gag gac atg gtg cgg gcc gtg gtg atg aac cgg    480
Phe Ala Thr Ala Cys Glu Asp Met Val Arg Ala Val Val Met Asn Arg
145                 150                 155                 160

ccc ggt gac ggc gtc ggc atc tcc gcg gaa cac ctg cgg gcc ggt ctg    528
Pro Gly Asp Gly Val Gly Ile Ser Ala Glu His Leu Arg Ala Gly Leu
                165                 170                 175

aac tac gtg ctg gcc gag gcc ccg ctc ttc gcg gac tcg ccc gga gtc    576
Asn Tyr Val Leu Ala Glu Ala Pro Leu Phe Ala Asp Ser Pro Gly Val
            180                 185                 190

ttc tcc gtc ccc tcc tcg gtg ctc tgc tac cac atc gac acc ccg atc    624
Phe Ser Val Pro Ser Ser Val Leu Cys Tyr His Ile Asp Thr Pro Ile
        195                 200                 205

acg gcg ttc ctg tcc cgg cgc gag acc ggt ttc cgg gcg gcc gag gga    672
Thr Ala Phe Leu Ser Arg Arg Glu Thr Gly Phe Arg Ala Ala Glu Gly
    210                 215                 220

cag gcg tac gtc gtc gtc agg ccc cag gag ctg gcc gac gcg gcc        717
Gln Ala Tyr Val Val Val Arg Pro Gln Glu Leu Ala Asp Ala Ala
225                 230                 235


<210> 40
<211> 717
<212> DNA
<213> Streptomyces alborus IFO15452

<400> 40
atg ctt gca ggc tta gtt ccc gcg ctg gac cac agc atg cgg gaa gaa    48
Met Leu Ala Gly Leu Val Pro Ala Leu Asp His Ser Met Arg Glu Glu
  1               5                  10                  15

ata ctt ggc aat cgc ggc cga aag atc cgg caa cgc ggt gag cac gct     96
Ile Leu Gly Asn Arg Gly Arg Lys Ile Arg Gln Arg Gly Glu His Ala
            20                  25                  30

ctc att gga atc agt gcg ggc aac agt tat ttc agc cag aag aac gtc     144
Leu Ile Gly Ile Ser Ala Gly Asn Ser Tyr Phe Ser Gln Lys Asn Val
        35                  40                  45

acc atg ctg ctg caa tgg gcc ggg cag cat ttc gag cgc acg gat gtc     192
Thr Met Leu Leu Gln Trp Ala Gly Gln His Phe Glu Arg Thr Asp Val
    50                  55                  60

gtc tac gtg gac acg cac atc gac gac atg ctg atg gcg gac ggc cgc     240
Val Tyr Val Asp Thr His Ile Asp Asp Met Leu Met Ala Asp Gly Arg
65                  70                  75                  80

agc gcg cag gaa gcc gag aag tcg gtc aag cgc acg ctc aag gat ctg     288
Ser Ala Gln Glu Ala Glu Lys Ser Val Lys Arg Thr Leu Lys Asp Leu
                85                  90                  95

cgg cgc agg ctg cgg cgc tcg ttg gaa agc gtg ggc gac cac agc gag     336
Arg Arg Arg Leu Arg Arg Ser Leu Glu Ser Val Gly Asp His Ser Glu
            100                 105                 110

cgg ttc cgc gtc cgg tcc ctg tcc gag atc cag gag acc cct gag tac     384
Arg Phe Arg Val Arg Ser Leu Ser Glu Ile Gln Glu Thr Pro Glu Tyr
        115                 120                 125

cgg gcc gca cgc gag tcc acc gac cgg gcc ttc cgc gag gac ggc gag     432
Arg Ala Ala Arg Glu Ser Thr Asp Arg Ala Phe Arg Glu Asp Gly Glu
    130                 135                 140

ttc gcc acc gtc tgc gag gag atg gtg cgc gcc gtg gtg atg aac cgg     480
Phe Ala Thr Val Cys Glu Glu Met Val Arg Ala Val Val Met Asn Arg
145                 150                 155                 160

ccc ggt gac ggc gtc gac atc tcg gag gaa cac ctg cgg gcc ggt ctg     528
Pro Gly Asp Gly Val Asp Ile Ser Glu Glu His Leu Arg Ala Gly Leu
                165                 170                 175

aac tac gtg ctc gcc gag gcc ccg ctc ttc gcg gac tcg ccc ggc gtg     576
Asn Tyr Val Leu Ala Glu Ala Pro Leu Phe Ala Asp Ser Pro Gly Val
            180                 185                 190

ttc tcc gtc ccc tcg tcg gtg ctc tgc tac cac atc ccc acc ccg gta     624
Phe Ser Val Pro Ser Ser Val Leu Cys Tyr His Ile Pro Thr Pro Val
        195                 200                 205

tcg acg ttc ctg gcc cat cgc gag acc ggt ttc cag gcg gct cag ggt     672
Ser Thr Phe Leu Ala His Arg Glu Thr Gly Phe Gln Ala Ala Gln Gly
    210                 215                 220

cag gca tac gtc gtc gtc agg ccg cag gag ctg gcc gac gcg gcc         717
Gln Ala Tyr Val Val Val Arg Pro Gln Glu Leu Ala Asp Ala Ala
225                 230                 235

<210> 41
<211> 32

<212> DNA
<213> Artificial Sequence

<220>
<223> Description of Artificial Sequence: Synthetic DNA

<400> 41
agagccatgg gacttgcagg cttagttccc gc                                32


<210> 42
<211> 29
<212> DNA
<213> Artificial Sequence

<220>
<223> Description of Artificial Sequence: Synthetic DNA

<400> 42
agagagatct ggccgcgtcg gccagctcc                                    29


<210> 43
<211> 503
<212> PRT
<213> Escherichia coli

<400> 43
Met Glu Phe Ser Val Lys Ser Gly Ser Pro Glu Lys Gln Arg Ser Ala
 1               5                  10                  15

Cys Ile Val Val Gly Val Phe Glu Pro Arg Arg Leu Ser Pro Ile Ala
            20                  25                  30

Glu Gln Leu Asp Lys Ile Ser Asp Gly Tyr Ile Ser Ala Leu Leu Arg
        35                  40                  45

Arg Gly Glu Leu Glu Gly Lys Pro Gly Gln Thr Leu Leu Leu His His
    50                  55                  60

Val Pro Asn Val Leu Ser Glu Arg Ile Leu Leu Ile Gly Cys Gly Lys
 65                  70                  75                  80

Glu Arg Glu Leu Asp Glu Arg Gln Tyr Lys Gln Val Ile Gln Lys Thr
                85                  90                  95

Ile Asn Thr Leu Asn Asp Thr Gly Ser Met Glu Ala Val Cys Phe Leu
            100                 105                 110

Thr Glu Leu His Val Lys Gly Arg Asn Asn Tyr Trp Lys Val Arg Gln

        115                 120                 125

Ala Val Glu Thr Ala Lys Glu Thr Leu Tyr Ser Phe Asp Gln Leu Lys
    130                 135                 140

Thr Asn Lys Ser Glu Pro Arg Arg Pro Leu Arg Lys Met Val Phe Asn
145                 150                 155                 160

Val Pro Thr Arg Arg Glu Leu Thr Ser Gly Glu Arg Ala Ile Gln His

                165                 170                 175

Gly Leu Ala Ile Ala Ala Gly Ile Lys Ala Ala Lys Asp Leu Gly Asn
            180                 185                 190

Met Pro Pro Asn Ile Cys Asn Ala Ala Tyr Leu Ala Ser Gln Ala Arg
        195                 200                 205

Gln Leu Ala Asp Ser Tyr Ser Lys Asn Val Ile Thr Arg Val Ile Gly
    210                 215                 220

Glu Gln Gln Met Lys Glu Leu Gly Met His Ser Tyr Leu Ala Val Gly
225                 230                 235                 240

Gln Gly Ser Gln Asn Glu Ser Leu Met Ser Val Ile Glu Tyr Lys Gly
                245                 250                 255

Asn Ala Ser Glu Asp Ala Arg Pro Ile Val Leu Val Gly Lys Gly Leu
            260                 265                 270

Thr Phe Asp Ser Gly Gly Ile Ser Ile Lys Pro Ser Glu Gly Met Asp
        275                 280                 285

Glu Met Lys Tyr Asp Met Cys Gly Ala Ala Ala Val Tyr Gly Val Met
    290                 295                 300

Arg Met Val Ala Glu Leu Gln Leu Pro Ile Asn Val Ile Gly Val Leu
305                 310                 315                 320

Ala Gly Cys Glu Asn Met Pro Gly Gly Arg Ala Tyr Arg Pro Gly Asp
                325                 330                 335

Val Leu Thr Thr Met Ser Gly Gln Thr Val Glu Val Leu Asn Thr Asp
            340                 345                 350

Ala Glu Gly Arg Leu Val Leu Cys Asp Val Leu Thr Tyr Val Glu Arg
        355                 360                 365

Phe Glu Pro Glu Ala Val Ile Asp Val Ala Thr Leu Thr Gly Ala Cys
    370                 375                 380

Val Ile Ala Leu Gly His His Ile Thr Gly Leu Met Ala Asn His Asn
385                 390                 395                 400

Pro Leu Ala His Glu Leu Ile Ala Ala Ser Glu Gln Ser Gly Asp Arg
                405                 410                 415

Ala Trp Arg Leu Pro Leu Gly Asp Glu Tyr Gln Glu Gln Leu Glu Ser
            420                 425                 430

Asn Phe Ala Asp Met Ala Asn Ile Gly Gly Arg Pro Gly Gly Ala Ile
        435                 440                 445

Thr Ala Gly Cys Phe Leu Ser Arg Phe Thr Arg Lys Tyr Asn Trp Ala
    450                 455                 460

His Leu Asp Ile Ala Gly Thr Ala Trp Arg Ser Gly Lys Ala Lys Gly
465                 470                 475                 480

Ala Thr Gly Arg Pro Val Ala Leu Leu Ala Gln Phe Leu Leu Asn Arg

485                 490                 495

Ala Gly Phe Asn Gly Glu Glu
            500

<210> 44
<211> 427
<212> PRT
<213> Escherichia coli

<400> 44
Met Thr Glu Ala Met Lys Ile Thr Leu Ser Thr Gln Pro Ala Asp Ala
1               5                   10                  15

Arg Trp Gly Glu Lys Ala Thr Tyr Ser Ile Asn Asn Asp Gly Ile Thr
            20                  25                  30

Leu His Leu Asn Gly Ala Asp Asp Leu Gly Leu Ile Gln Arg Ala Ala
        35                  40                  45

Arg Lys Ile Asp Gly Leu Gly Ile Lys His Val Gln Leu Ser Gly Glu
    50                  55                  60

Gly Trp Asp Ala Asp Arg Cys Trp Ala Phe Trp Gln Gly Tyr Lys Ala
65                  70                  75                  80

Pro Lys Gly Thr Arg Lys Val Val Trp Pro Asp Leu Asp Asp Ala Gln
                85                  90                  95

Arg Gln Glu Leu Asp Asn Arg Leu Met Ile Ile Asp Trp Val Arg Asp
            100                 105                 110

Thr Ile Asn Ala Pro Ala Glu Glu Leu Gly Pro Ser Gln Leu Ala Gln
        115                 120                 125

Arg Ala Val Asp Leu Ile Ser Asn Val Ala Gly Asp Arg Val Thr Tyr
    130                 135                 140

Arg Ile Thr Lys Gly Glu Asp Leu Arg Glu Gln Gly Tyr Met Gly Leu
145                 150                 155                 160

His Thr Val Gly Arg Gly Ser Glu Arg Ser Pro Val Leu Leu Ala Leu
                165                 170                 175

Asp Tyr Asn Pro Thr Gly Asp Lys Glu Ala Pro Val Tyr Ala Cys Leu
            180                 185                 190

Val Gly Lys Gly Ile Thr Phe Asp Ser Gly Gly Tyr Ser Ile Lys Gln
        195                 200                 205

Thr Ala Phe Met Asp Ser Met Lys Ser Asp Met Gly Gly Ala Ala Thr
    210                 215                 220

Val Thr Gly Ala Leu Ala Phe Ala Ile Thr Arg Gly Leu Asn Lys Arg
225                 230                 235                 240

Val Lys Leu Phe Leu Cys Cys Ala Asp Asn Leu Ile Ser Gly Asn Ala
                245                 250                 255

Phe Lys Leu Gly Asp Ile Ile Thr Tyr Arg Asn Gly Lys Lys Val Glu
            260                 265                 270

Val Met Asn Thr Asp Ala Glu Gly Arg Leu Val Leu Ala Asp Gly Leu
        275                 280                 285

Ile Asp Ala Ser Ala Gln Lys Pro Glu Met Ile Ile Asp Ala Ala Thr
    290                 295                 300

Leu Thr Gly Ala Ala Lys Thr Ala Leu Gly Asn Asp Tyr His Ala Leu
305                 310                 315                 320

Phe Ser Phe Asp Asp Ala Leu Ala Gly Arg Leu Leu Ala Ser Ala Ala
                325                 330                 335

Gln Glu Asn Glu Pro Phe Trp Arg Leu Pro Leu Ala Glu Phe His Arg
            340                 345                 350

Ser Gln Leu Pro Ser Asn Phe Ala Glu Leu Asn Asn Thr Gly Ser Ala
        355                 360                 365

Ala Tyr Pro Ala Gly Ala Ser Thr Ala Ala Gly Phe Leu Ser His Phe
    370                 375                 380

Val Glu Asn Tyr Gln Gln Gly Trp Leu His Ile Asp Cys Ser Ala Thr
385                 390                 395                 400

Tyr Arg Lys Ala Pro Val Glu Gln Trp Ser Ala Gly Ala Thr Gly Leu
                405                 410                 415

Gly Val Arg Thr Ile Ala Asn Leu Leu Thr Ala
            420                 425


<210> 45
<211> 485
<212> PRT
<213> Escherichia coli

<400> 45
Met Ser Glu Leu Ser Gln Leu Ser Pro Gln Pro Leu Trp Asp Ile Phe
  1               5                  10                  15

Ala Lys Ile Cys Ser Ile Pro His Pro Ser Tyr His Glu Glu Gln Leu
             20                  25                  30

Ala Glu Tyr Ile Val Gly Trp Ala Lys Glu Lys Gly Phe His Val Glu
         35                  40                  45

Arg Asp Gln Val Gly Asn Ile Leu Ile Arg Lys Pro Ala Thr Ala Gly
     50                  55                  60

Met Glu Asn Arg Lys Pro Val Val Leu Gln Ala His Leu Asp Met Val
 65                  70                  75                  80

Pro Gln Lys Asn Asn Asp Thr Val His Asp Phe Thr Lys Asp Pro Ile
                 85                  90                  95

Gln Pro Tyr Ile Asp Gly Glu Trp Val Lys Ala Arg Gly Thr Thr Leu
            100                 105                 110

Gly Ala Asp Asn Gly Ile Gly Met Ala Ser Ala Leu Ala Val Leu Ala
        115                 120                 125

Asp Glu Asn Val Val His Gly Pro Leu Glu Val Leu Leu Thr Met Thr
    130                 135                 140

Glu Glu Ala Gly Met Asp Gly Ala Phe Gly Leu Gln Gly Asn Trp Leu
145                 150                 155                 160

Gln Ala Asp Ile Leu Ile Asn Thr Asp Ser Glu Glu Glu Gly Glu Ile
                165                 170                 175

Tyr Met Gly Cys Ala Gly Gly Ile Asp Phe Thr Ser Asn Leu His Leu
            180                 185                 190

Asp Arg Glu Ala Val Pro Ala Gly Phe Glu Thr Phe Lys Leu Thr Leu
        195                 200                 205

Lys Gly Leu Lys Gly Gly His Ser Gly Gly Glu Ile His Val Gly Leu
    210                 215                 220

Gly Asn Ala Asn Lys Leu Leu Val Arg Phe Leu Ala Gly His Ala Glu
225                 230                 235                 240

Glu Leu Asp Leu Arg Leu Ile Asp Phe Asn Gly Gly Thr Leu Arg Asn
                245                 250                 255

Ala Ile Pro Arg Glu Ala Phe Ala Thr Ile Ala Val Ala Ala Asp Lys
            260                 265                 270

Val Asp Val Leu Lys Ser Leu Val Asn Thr Tyr Gln Glu Ile Leu Lys
        275                 280                 285

Asn Glu Leu Ala Glu Lys Glu Lys Asn Leu Ala Leu Leu Leu Asp Ser
    290                 295                 300

Val Ala Asn Asp Lys Ala Ala Leu Ile Ala Lys Ser Arg Asp Thr Phe
305                 310                 315                 320

Ile Arg Leu Leu Asn Ala Thr Pro Asn Gly Val Ile Arg Asn Ser Asp
                325                 330                 335

Val Ala Lys Gly Val Val Glu Thr Ser Leu Asn Val Gly Val Val Thr
            340                 345                 350

Met Thr Asp Asn Asn Val Glu Ile His Cys Leu Ile Arg Ser Leu Ile
        355                 360                 365

Asp Ser Gly Lys Asp Tyr Val Val Ser Met Leu Asp Ser Leu Gly Lys
    370                 375                 380

Leu Ala Gly Ala Lys Thr Glu Ala Lys Gly Ala Tyr Pro Gly Trp Gln
385                 390                 395                 400

Pro Asp Ala Asn Ser Pro Val Met His Leu Val Arg Glu Thr Tyr Gln
                405                 410                 415

Arg Leu Phe Asn Lys Thr Pro Asn Ile Gln Ile Ile His Ala Gly Leu
            420                 425                 430

Glu Cys Gly Leu Phe Lys Lys Pro Tyr Pro Glu Met Asp Met Val Ser
        435                 440                 445

Ile Gly Pro Thr Ile Thr Gly Pro His Ser Pro Asp Glu Gln Val His
    450                 455                 460

Ile Glu Ser Val Gly His Tyr Trp Thr Leu Leu Thr Glu Leu Leu Lys
465                 470                 475                 480

Glu Ile Pro Ala Lys
                485

<210> 46
<211> 870
<212> PRT
<213> Escherichia coli

<400> 46
Met Thr Gln Gln Pro Gln Ala Lys Tyr Arg His Asp Tyr Arg Ala Pro
  1               5                  10                  15

Asp Tyr Gln Ile Thr Asp Ile Asp Leu Thr Phe Asp Leu Asp Ala Gln
             20                  25                  30

Lys Thr Val Val Thr Ala Val Ser Gln Ala Val Arg His Gly Ala Ser
         35                  40                  45

Asp Ala Pro Leu Arg Leu Asn Gly Glu Asp Leu Lys Leu Val Ser Val
     50                  55                  60

His Ile Asn Asp Glu Pro Trp Thr Ala Trp Lys Glu Glu Glu Gly Ala
 65                  70                  75                  80

Leu Val Ile Ser Asn Leu Pro Glu Arg Phe Thr Leu Lys Ile Ile Asn
                 85                  90                  95

Glu Ile Ser Pro Ala Ala Asn Thr Ala Leu Glu Gly Leu Tyr Gln Ser
            100                 105                 110

Gly Asp Ala Leu Cys Thr Gln Cys Glu Ala Glu Gly Phe Arg His Ile
        115                 120                 125

Thr Tyr Tyr Leu Asp Arg Pro Asp Val Leu Ala Arg Phe Thr Thr Lys
    130                 135                 140

Ile Ile Ala Asp Lys Ile Lys Tyr Pro Phe Leu Leu Ser Asn Gly Asn
145                 150                 155                 160

Arg Val Ala Gln Gly Glu Leu Glu Asn Gly Arg His Trp Val Gln Trp
                165                 170                 175

Gln Asp Pro Phe Pro Lys Pro Cys Tyr Leu Phe Ala Leu Val Ala Gly
            180                 185                 190

Asp Phe Asp Val Leu Arg Asp Thr Phe Thr Thr Arg Ser Gly Arg Glu
        195                 200                 205

Val Ala Leu Glu Leu Tyr Val Asp Arg Gly Asn Leu Asp Arg Ala Pro
    210                 215                 220

Trp Ala Met Thr Ser Leu Lys Asn Ser Met Lys Trp Asp Glu Glu Arg
225                 230                 235                 240

Phe Gly Leu Glu Tyr Asp Leu Asp Ile Tyr Met Ile Val Ala Val Asp
                245                 250                 255

Phe Phe Asn Met Gly Ala Met Glu Asn Lys Gly Leu Asn Ile Phe Asn
            260                 265                 270

Ser Lys Tyr Val Leu Ala Arg Thr Asp Thr Ala Thr Asp Lys Asp Tyr
        275                 280                 285

Leu Asp Ile Glu Arg Val Ile Gly His Glu Tyr Phe His Asn Trp Thr
    290                 295                 300

Gly Asn Arg Val Thr Cys Arg Asp Trp Phe Gln Leu Ser Leu Lys Glu
305                 310                 315                 320

Gly Leu Thr Val Phe Arg Asp Gln Glu Phe Ser Ser Asp Leu Gly Ser
                325                 330                 335

Arg Ala Val Asn Arg Ile Asn Asn Val Arg Thr Met Arg Gly Leu Gln
            340                 345                 350

Phe Ala Glu Asp Ala Ser Pro Met Ala His Pro Ile Arg Pro Asp Met
        355                 360                 365

Val Ile Glu Met Asn Asn Phe Tyr Thr Leu Thr Val Tyr Glu Lys Gly
    370                 375                 380

Ala Glu Val Ile Arg Met Ile His Thr Leu Leu Gly Glu Glu Asn Phe
385                 390                 395                 400

Gln Lys Gly Met Gln Leu Tyr Phe Glu Arg His Asp Gly Ser Ala Ala
                405                 410                 415

Thr Cys Asp Asp Phe Val Gln Ala Met Glu Asp Ala Ser Asn Val Asp
            420                 425                 430

Leu Ser His Phe Arg Arg Trp Tyr Ser Gln Ser Gly Thr Pro Ile Val
        435                 440                 445

Thr Val Lys Asp Asp Tyr Asn Pro Glu Thr Glu Gln Tyr Thr Leu Thr
    450                 455                 460

Ile Ser Gln Arg Thr Pro Ala Thr Pro Asp Gln Ala Glu Lys Gln Pro
465                 470                 475                 480

Leu His Ile Pro Phe Ala Ile Glu Leu Tyr Asp Asn Glu Gly Lys Val
                485                 490                 495

Ile Pro Leu Gln Lys Gly Gly His Pro Val Asn Ser Val Leu Asn Val
            500                 505                 510

Thr Gln Ala Glu Gln Thr Phe Val Phe Asp Asn Val Tyr Phe Gln Pro
        515                 520                 525

Val Pro Ala Leu Leu Cys Glu Phe Ser Ala Pro Val Lys Leu Glu Tyr
    530                 535                 540

Lys Trp Ser Asp Gln Gln Leu Thr Phe Leu Met Arg His Ala Arg Asn
545                 550                 555                 560

Asp Phe Ser Arg Trp Asp Ala Ala Gln Ser Leu Leu Ala Thr Tyr Ile
                565                 570                 575

Lys Leu Asn Val Ala Arg His Gln Gln Gly Gln Pro Leu Ser Leu Pro
            580                 585                 590

Val His Val Ala Asp Ala Phe Arg Ala Val Leu Leu Asp Glu Lys Ile
        595                 600                 605

Asp Pro Ala Leu Ala Ala Glu Ile Leu Thr Leu Pro Ser Val Asn Glu
    610                 615                 620

Met Ala Glu Leu Phe Asp Ile Ile Asp Pro Ile Ala Ile Ala Glu Val
625                 630                 635                 640

Arg Glu Ala Leu Thr Arg Thr Leu Ala Thr Glu Leu Ala Asp Glu Leu
                645                 650                 655

Leu Ala Ile Tyr Asn Ala Asn Tyr Gln Ser Glu Tyr Arg Val Glu His
            660                 665                 670

Glu Asp Ile Ala Lys Arg Thr Leu Arg Asn Ala Cys Leu Arg Phe Leu
        675                 680                 685

Ala Phe Gly Glu Thr His Leu Ala Asp Val Leu Val Ser Lys Gln Phe
    690                 695                 700

His Glu Ala Asn Asn Met Thr Asp Ala Leu Ala Ala Leu Ser Ala Ala
705                 710                 715                 720

Val Ala Ala Gln Leu Pro Cys Arg Asp Ala Leu Met Gln Glu Tyr Asp
                725                 730                 735

Asp Lys Trp His Gln Asn Gly Leu Val Met Asp Lys Trp Phe Ile Leu
            740                 745                 750

Gln Ala Thr Ser Pro Ala Ala Asn Val Leu Glu Thr Val Arg Gly Leu
        755                 760                 765

Leu Gln His Arg Ser Phe Thr Met Ser Asn Pro Asn Arg Ile Arg Ser
    770                 775                 780

Leu Ile Gly Ala Phe Ala Gly Ser Asn Pro Ala Ala Phe His Ala Glu
785                 790                 795                 800

Asp Gly Ser Gly Tyr Leu Phe Leu Val Glu Met Leu Thr Asp Leu Asn
                805                 810                 815

Ser Arg Asn Pro Gln Val Ala Ser Arg Leu Ile Glu Pro Leu Ile Arg
            820                 825                 830

Leu Lys Arg Tyr Asp Ala Lys Arg Gln Glu Lys Met Arg Ala Ala Leu
        835                 840                 845

Glu Gln Leu Lys Gly Leu Glu Asn Leu Ser Gly Asp Leu Tyr Glu Lys
    850                 855                 860

Ile Thr Lys Ala Leu Ala
865                 870

<210> 47
<211> 535
<212> PRT
<213> Escherichia coli

<400> 47
Met Arg Ile Ser Leu Lys Lys Ser Gly Met Leu Lys Leu Gly Leu Ser
1               5                   10                  15

Leu Val Ala Met Thr Val Ala Ala Ser Val Gln Ala Lys Thr Leu Val
            20                  25                  30

Tyr Cys Ser Glu Gly Ser Pro Glu Gly Phe Asn Pro Gln Leu Phe Thr
        35                  40                  45

Ser Gly Thr Thr Tyr Asp Ala Ser Ser Val Pro Leu Tyr Asn Arg Leu
    50                  55                  60

Val Glu Phe Lys Ile Gly Thr Thr Glu Val Ile Pro Gly Leu Ala Glu
65                  70                  75                  80

Lys Trp Glu Val Ser Glu Asp Gly Lys Thr Tyr Thr Phe His Leu Arg
                85                  90                  95

Lys Gly Val Lys Trp His Asp Asn Lys Glu Phe Lys Pro Thr Arg Glu
            100                 105                 110

Leu Asn Ala Asp Asp Val Val Phe Ser Phe Asp Arg Gln Lys Asn Ala
        115                 120                 125

Gln Asn Pro Tyr His Lys Val Ser Gly Gly Ser Tyr Glu Tyr Phe Glu
    130                 135                 140

Gly Met Gly Leu Pro Glu Leu Ile Ser Glu Val Lys Lys Val Asp Asp
145                 150                 155                 160

Asn Thr Val Gln Phe Val Leu Thr Arg Pro Glu Ala Pro Phe Leu Ala
                165                 170                 175

Asp Leu Ala Met Asp Phe Ala Ser Ile Leu Ser Lys Glu Tyr Ala Asp
            180                 185                 190

Ala Met Met Lys Ala Gly Thr Pro Glu Lys Leu Asp Leu Asn Pro Ile
        195                 200                 205

Gly Thr Gly Pro Phe Gln Leu Gln Gln Tyr Gln Lys Asp Ser Arg Ile
    210                 215                 220

Arg Tyr Lys Ala Phe Asp Gly Tyr Trp Gly Thr Lys Pro Gln Ile Asp
225                 230                 235                 240

Thr Leu Val Phe Ser Ile Thr Pro Asp Ala Ser Val Arg Tyr Ala Lys
                245                 250                 255

Leu Gln Lys Asn Glu Cys Gln Val Met Pro Tyr Pro Asn Pro Ala Asp

260                 265                 270

Ile Ala Arg Met Lys Gln Asp Lys Ser Ile Asn Leu Met Glu Met Pro
        275                 280                 285

Gly Leu Asn Val Gly Tyr Leu Ser Tyr Asn Val Gln Lys Lys Pro Leu
    290                 295                 300

Asp Asp Val Lys Val Arg Gln Ala Leu Thr Tyr Ala Val Asn Lys Asp
305                 310                 315                 320

Ala Ile Ile Lys Ala Val Tyr Gln Gly Ala Gly Val Ser Ala Lys Asn
                325                 330                 335

Leu Ile Pro Pro Thr Met Trp Gly Tyr Asn Asp Asp Val Gln Asp Tyr
            340                 345                 350

Thr Tyr Asp Pro Glu Lys Ala Lys Ala Leu Leu Lys Glu Ala Gly Leu
        355                 360                 365

Glu Lys Gly Phe Ser Ile Asp Leu Trp Ala Met Pro Val Gln Arg Pro
    370                 375                 380

Tyr Asn Pro Asn Ala Arg Arg Met Ala Glu Met Ile Gln Ala Asp Trp
385                 390                 395                 400

Ala Lys Val Gly Val Gln Ala Lys Ile Val Thr Tyr Glu Trp Gly Glu
                405                 410                 415

Tyr Leu Lys Arg Ala Lys Asp Gly Glu His Gln Thr Val Met Met Gly
            420                 425                 430

Trp Thr Gly Asp Asn Gly Asp Pro Asp Asn Phe Phe Ala Thr Leu Phe
        435                 440                 445

Ser Cys Ala Ala Ser Glu Gln Gly Ser Asn Tyr Ser Lys Trp Cys Tyr
    450                 455                 460

Lys Pro Phe Glu Asp Leu Ile Gln Pro Ala Arg Ala Thr Asp Asp His
465                 470                 475                 480

Asn Lys Arg Val Glu Leu Tyr Lys Gln Ala Gln Val Val Met His Asp
                485                 490                 495

Gln Ala Pro Ala Leu Ile Ile Ala His Ser Thr Val Phe Glu Pro Val
            500                 505                 510

Arg Lys Glu Val Lys Gly Tyr Val Val Asp Pro Leu Gly Lys His His
        515                 520                 525

Phe Glu Asn Val Ser Ile Glu
    530                 535

<210> 48
<211> 339
<212> PRT
<213> Escherichia coli

<400> 48

Met Leu Gln Phe Ile Leu Arg Arg Leu Gly Leu Val Ile Pro Thr Phe
1               5                   10                  15

Ile Gly Ile Thr Leu Leu Thr Phe Ala Phe Val His Met Ile Pro Gly
            20                  25                  30

Asp Pro Val Met Ile Met Ala Gly Glu Arg Gly Ile Ser Pro Glu Arg
        35                  40                  45

His Ala Gln Leu Leu Ala Glu Leu Gly Leu Asp Lys Pro Met Trp Gln
    50                  55                  60

Gln Tyr Leu His Tyr Ile Trp Gly Val Met His Gly Asp Leu Gly Ile
65                  70                  75                  80

Ser Met Lys Ser Arg Ile Pro Val Trp Glu Glu Phe Val Pro Arg Phe
                85                  90                  95

Gln Ala Thr Leu Glu Leu Gly Val Cys Ala Met Ile Phe Ala Thr Ala
            100                 105                 110

Val Gly Ile Pro Val Gly Val Leu Ala Ala Val Lys Arg Gly Ser Ile
        115                 120                 125

Phe Asp His Thr Ala Val Gly Leu Ala Leu Thr Gly Tyr Ser Met Pro
    130                 135                 140

Ile Phe Trp Trp Gly Met Met Leu Ile Met Leu Val Ser Val His Trp
145                 150                 155                 160

Asn Leu Thr Pro Val Ser Gly Arg Val Ser Asp Met Val Phe Leu Asp
                165                 170                 175

Asp Ser Asn Pro Leu Thr Gly Phe Met Leu Ile Asp Thr Ala Ile Trp
            180                 185                 190

Gly Glu Asp Gly Asn Phe Ile Asp Ala Val Ala His Met Ile Leu Pro
        195                 200                 205

Ala Ile Val Leu Gly Thr Ile Pro Leu Ala Val Ile Val Arg Met Thr
    210                 215                 220

Arg Ser Ser Met Leu Glu Val Leu Gly Glu Asp Tyr Ile Arg Thr Ala
225                 230                 235                 240

Arg Ala Lys Gly Leu Thr Arg Met Arg Val Ile Ile Val His Ala Leu
                245                 250                 255

Arg Asn Ala Met Leu Pro Val Val Thr Val Ile Gly Leu Gln Val Gly
            260                 265                 270

Thr Leu Leu Ala Gly Ala Ile Leu Thr Glu Thr Ile Phe Ser Trp Pro
        275                 280                 285

Gly Leu Gly Arg Trp Leu Ile Asp Ala Leu Gln Arg Arg Asp Tyr Pro
    290                 295                 300

Val Val Gln Gly Gly Val Leu Leu Val Ala Thr Met Ile Ile Leu Val
305                 310                 315                 320

Asn Leu Leu Val Asp Leu Leu Tyr Gly Val Val Asn Pro Arg Ile Arg
                325                 330                 335

His Lys Lys


<210> 49
<211> 300
<212> PRT
<213> Escherichia coli

<400> 49
Met Ser Gln Val Thr Glu Asn Lys Val Ile Ser Ala Pro Val Pro Met
  1               5                  10                  15

Thr Pro Leu Gln Glu Phe Trp His Tyr Phe Lys Arg Asn Lys Gly Ala
             20                  25                  30

Val Val Gly Leu Val Tyr Val Val Ile Val Leu Phe Ile Ala Ile Phe
         35                  40                  45

Ala Asn Trp Ile Ala Pro Tyr Asn Pro Ala Glu Gln Phe Arg Asp Ala
     50                  55                  60

Leu Leu Ala Pro Pro Ala Trp Gln Glu Gly Gly Ser Met Ala His Leu
 65                  70                  75                  80

Leu Gly Thr Asp Asp Val Gly Arg Asp Val Leu Ser Arg Leu Met Tyr
                 85                  90                  95

Gly Ala Arg Leu Ser Leu Leu Val Gly Cys Leu Val Val Val Leu Ser
            100                 105                 110

Leu Ile Met Gly Val Ile Leu Gly Leu Ile Ala Gly Tyr Phe Gly Gly
        115                 120                 125

Leu Val Asp Asn Ile Ile Met Arg Val Val Asp Ile Met Leu Ala Leu
    130                 135                 140

Pro Ser Leu Leu Leu Ala Leu Val Leu Val Ala Ile Phe Gly Pro Ser
145                 150                 155                 160

Ile Gly Asn Ala Ala Leu Ala Leu Thr Phe Val Ala Leu Pro His Tyr
                165                 170                 175

Val Arg Leu Thr Arg Ala Ala Val Leu Val Glu Val Asn Arg Asp Tyr
            180                 185                 190

Val Thr Ala Ser Arg Val Ala Gly Ala Gly Ala Met Arg Gln Met Phe
        195                 200                 205

Ile Asn Ile Phe Pro Asn Cys Leu Ala Pro Leu Ile Val Gln Ala Ser
    210                 215                 220

Leu Gly Phe Ser Asn Ala Ile Leu Asp Met Ala Ala Leu Gly Phe Leu
225                 230                 235                 240

Gly Met Gly Ala Gln Pro Pro Thr Pro Glu Trp Gly Thr Met Leu Ser
                245                 250                 255

Asp Val Leu Gln Phe Ala Gln Ser Ala Trp Trp Val Val Thr Phe Pro
            260                 265                 270

Gly Leu Ala Ile Leu Leu Thr Val Leu Ala Phe Asn Leu Met Gly Asp
        275                 280                 285

Gly Leu Arg Asp Ala Leu Asp Pro Lys Leu Lys Gln
    290                 295                 300

<210> 50
<211> 327
<212> PRT
<213> Escherichia coli

<400> 50
Met Ala Leu Leu Asn Val Asp Lys Leu Ser Val His Phe Gly Asp Glu
1               5                   10                  15

Ser Ala Pro Phe Arg Ala Val Asp Arg Ile Ser Tyr Ser Val Lys Gln
            20                  25                  30

Gly Glu Val Val Gly Ile Val Gly Glu Ser Gly Ser Gly Lys Ser Val
        35                  40                  45

Ser Ser Leu Ala Ile Met Gly Leu Ile Asp Tyr Pro Gly Arg Val Met
    50                  55                  60

Ala Glu Lys Leu Glu Phe Asn Gly Gln Asp Leu Gln Arg Ile Ser Glu
65                  70                  75                  80

Lys Glu Arg Arg Asn Leu Val Gly Ala Glu Val Ala Met Ile Phe Gln
                85                  90                  95

Asp Pro Met Thr Ser Leu Asn Pro Cys Tyr Thr Val Gly Phe Gln Ile
            100                 105                 110

Met Glu Ala Ile Lys Val His Gln Gly Gly Asn Lys Ser Thr Arg Arg
        115                 120                 125

Gln Arg Ala Ile Asp Leu Leu Asn Gln Val Gly Ile Pro Asp Pro Ala
    130                 135                 140

Ser Arg Leu Asp Val Tyr Pro His Gln Leu Ser Gly Gly Met Ser Gln
145                 150                 155                 160

Arg Val Met Ile Ala Met Ala Ile Ala Cys Arg Pro Lys Leu Leu Ile
                165                 170                 175

Ala Asp Glu Pro Thr Thr Ala Leu Asp Val Thr Ile Gln Ala Gln Ile
            180                 185                 190

Ile Glu Leu Leu Leu Glu Leu Gln Gln Lys Glu Asn Met Ala Leu Val
        195                 200                 205

Leu Ile Thr His Asp Leu Ala Leu Val Ala Glu Ala Ala His Lys Ile
    210                 215                 220

Ile Val Met Tyr Ala Gly Gln Val Val Glu Thr Gly Asp Ala His Ala
225                 230                 235                 240

Ile Phe His Ala Pro Arg His Pro Tyr Thr Gln Ala Leu Leu Arg Ala
                245                 250                 255

Leu Pro Glu Phe Ala Gln Asp Lys Glu Arg Leu Ala Ser Leu Pro Gly
            260                 265                 270

Val Val Pro Gly Lys Tyr Asp Arg Pro Asn Gly Cys Leu Leu Asn Pro
        275                 280                 285

Arg Cys Pro Tyr Ala Thr Asp Arg Cys Arg Ala Glu Glu Pro Ala Leu
    290                 295                 300

Asn Met Leu Ala Asp Gly Arg Gln Ser Lys Cys His Tyr Pro Leu Asp
305                 310                 315                 320

Asp Ala Gly Arg Pro Thr Leu
                325

<210> 51
<211> 334
<212> PRT
<213> Escherichia coli

<400> 51
Met Ser Thr Gln Glu Ala Thr Leu Gln Gln Pro Leu Leu Gln Ala Ile
1               5                   10                  15

Asp Leu Lys Lys His Tyr Pro Val Lys Lys Gly Met Phe Ala Pro Glu
            20                  25                  30

Arg Leu Val Lys Ala Leu Asp Gly Val Ser Phe Asn Leu Glu Arg Gly
        35                  40                  45

Lys Thr Leu Ala Val Val Gly Glu Ser Gly Cys Gly Lys Ser Thr Leu
    50                  55                  60

Gly Arg Leu Leu Thr Met Ile Glu Met Pro Thr Gly Gly Glu Leu Tyr
65                  70                  75                  80

Tyr Gln Gly Gln Asp Leu Leu Lys His Asp Pro Gln Ala Gln Lys Leu
                85                  90                  95

Arg Arg Gln Lys Ile Gln Ile Val Phe Gln Asn Pro Tyr Gly Ser Leu
            100                 105                 110

Asn Pro Arg Lys Lys Val Gly Gln Ile Leu Glu Glu Pro Leu Leu Ile
        115                 120                 125

Asn Thr Ser Leu Ser Lys Glu Gln Arg Arg Glu Lys Ala Leu Ser Met
    130                 135                 140

Met Ala Lys Val Gly Leu Lys Thr Glu His Tyr Asp Arg Tyr Pro His
145                 150                 155                 160

Met Phe Ser Gly Gly Gln Arg Gln Arg Ile Ala Ile Ala Arg Gly Leu
                165                 170                 175

Met Leu Asp Pro Asp Val Val Ile Ala Asp Glu Pro Val Ser Ala Leu

180                 185                 190

Asp Val Ser Val Arg Ala Gln Val Leu Asn Leu Met Met Asp Leu Gln
        195                 200                 205

Gln Glu Leu Gly Leu Ser Tyr Val Phe Ile Ser His Asp Leu Ser Val
    210                 215                 220

Val Glu His Ile Ala Asp Glu Val Met Val Met Tyr Leu Gly Arg Cys
225                 230                 235                 240

Val Glu Lys Gly Thr Lys Asp Gln Ile Phe Asn Asn Pro Arg His Pro
                245                 250                 255

Tyr Thr Gln Ala Leu Leu Ser Ala Thr Pro Arg Leu Asn Pro Asp Asp
            260                 265                 270

Arg Arg Glu Arg Ile Lys Leu Ser Gly Glu Leu Pro Ser Pro Leu Asn
        275                 280                 285

Pro Pro Pro Gly Cys Ala Phe Asn Ala Arg Cys Arg Arg Arg Phe Gly
    290                 295                 300

Pro Cys Thr Gln Leu Gln Pro Gln Leu Lys Asp Tyr Gly Gly Gln Leu
305                 310                 315                 320

Val Ala Cys Phe Ala Val Asp Gln Asp Glu Asn Pro Gln Arg
                325                 330

<210> 52
<211> 34
<212> DNA
<213> Artificial Sequence

<220>
<223> Description of Artificial Sequence: Synthetic DNA

<400> 52
gaagttccta tactttctag agaataggaa cttc                                34

<210> 53
<211> 1509
<212> DNA
<213> Escherichia coli

<400> 53
atg gag ttt agt gta aaa agc ggt agc ccg gag aaa cag cgg agt gcc      48
Met Glu Phe Ser Val Lys Ser Gly Ser Pro Glu Lys Gln Arg Ser Ala
  1               5                  10                  15

tgc atc gtc gtg ggc gtc ttc gaa cca cgt cgc ctt tct ccg att gca      96
Cys Ile Val Val Gly Val Phe Glu Pro Arg Arg Leu Ser Pro Ile Ala
             20                  25                  30

gaa cag ctc gat aaa atc agc gat ggg tac atc agc gcc ctg cta cgt     144
Glu Gln Leu Asp Lys Ile Ser Asp Gly Tyr Ile Ser Ala Leu Leu Arg
         35                  40                  45

cgg ggc gaa ctg gaa gga aaa ccg ggg cag aca ttg ttg ctg cac cat 192
Arg Gly Glu Leu Glu Gly Lys Pro Gly Gln Thr Leu Leu Leu His His
    50                  55                  60

gtt ccg aat gta ctt tcc gag cga att ctc ctt att ggt tgc ggc aaa 240
Val Pro Asn Val Leu Ser Glu Arg Ile Leu Leu Ile Gly Cys Gly Lys
65                  70                  75                  80

gaa cgt gag ctg gat gag cgt cag tac aag cag gtt att cag aaa acc 288
Glu Arg Glu Leu Asp Glu Arg Gln Tyr Lys Gln Val Ile Gln Lys Thr
                85                  90                  95

att aat acg ctg aat gat act ggc tca atg gaa gcg gtc tgc ttt ctg 336
Ile Asn Thr Leu Asn Asp Thr Gly Ser Met Glu Ala Val Cys Phe Leu
            100                 105                 110

act gag ctg cac gtt aaa ggc cgt aac aac tac tgg aaa gtg cgt cag 384
Thr Glu Leu His Val Lys Gly Arg Asn Asn Tyr Trp Lys Val Arg Gln
        115                 120                 125

gct gtc gag acg gca aaa gag acg ctc tac agt ttc gat cag ctg aaa 432
Ala Val Glu Thr Ala Lys Glu Thr Leu Tyr Ser Phe Asp Gln Leu Lys
    130                 135                 140

acg aac aag agc gaa ccg cgt cgt ccg ctg cgt aag atg gtg ttc aac 480
Thr Asn Lys Ser Glu Pro Arg Arg Pro Leu Arg Lys Met Val Phe Asn
145                 150                 155                 160

gtg ccg acc cgc cgt gaa ctg acc agc ggt gag cgc gcg atc cag cac 528
Val Pro Thr Arg Arg Glu Leu Thr Ser Gly Glu Arg Ala Ile Gln His
                165                 170                 175

ggt ctg gcg att gcc gcc ggg att aaa gca gca aaa gat ctc ggc aat 576
Gly Leu Ala Ile Ala Ala Gly Ile Lys Ala Ala Lys Asp Leu Gly Asn
            180                 185                 190

atg ccg ccg aat atc tgt aac gcc gct tac ctc gct tca caa gcg cgc 624
Met Pro Pro Asn Ile Cys Asn Ala Ala Tyr Leu Ala Ser Gln Ala Arg
        195                 200                 205

cag ctg gct gac agc tac agc aag aat gtc atc acc cgc gtt atc ggc 672
Gln Leu Ala Asp Ser Tyr Ser Lys Asn Val Ile Thr Arg Val Ile Gly
    210                 215                 220

gaa cag cag atg aaa gag ctg ggg atg cat tcc tat ctg gcg gtc ggt 720
Glu Gln Gln Met Lys Glu Leu Gly Met His Ser Tyr Leu Ala Val Gly
225                 230                 235                 240

cag ggt tcg caa aac gaa tcg ctg atg tcg gtg att gag tac aaa ggc 768
Gln Gly Ser Gln Asn Glu Ser Leu Met Ser Val Ile Glu Tyr Lys Gly
                245                 250                 255

aac gcg tcg gaa gat gca cgc cca atc gtg ctg gtg ggt aaa ggt tta 816
Asn Ala Ser Glu Asp Ala Arg Pro Ile Val Leu Val Gly Lys Gly Leu
            260                 265                 270

acc ttc gac tcc ggc ggt atc tcg atc aag cct tca gaa ggc atg gat 864
Thr Phe Asp Ser Gly Gly Ile Ser Ile Lys Pro Ser Glu Gly Met Asp
        275                 280                 285

gag atg aag tac gat atg tgc ggt gcg gca gcg gtt tac ggc gtg atg     912
Glu Met Lys Tyr Asp Met Cys Gly Ala Ala Ala Val Tyr Gly Val Met
    290                 295                 300

cgg atg gtc gcg gag cta caa ctg ccg att aac gtt atc ggc gtg ttg     960
Arg Met Val Ala Glu Leu Gln Leu Pro Ile Asn Val Ile Gly Val Leu
305                 310                 315                 320

gca ggc tgc gaa aac atg cct ggc gga cga gcc tat cgt ccg ggc gat     1008
Ala Gly Cys Glu Asn Met Pro Gly Gly Arg Ala Tyr Arg Pro Gly Asp
                325                 330                 335

gtg tta acc acc atg tcc ggt caa acc gtt gaa gtg ctg aac acc gac     1056
Val Leu Thr Thr Met Ser Gly Gln Thr Val Glu Val Leu Asn Thr Asp
            340                 345                 350

gct gaa ggc cgc ctg gta ctg tgc gac gtg tta act tac gtt gag cgt     1104
Ala Glu Gly Arg Leu Val Leu Cys Asp Val Leu Thr Tyr Val Glu Arg
        355                 360                 365

ttt gag ccg gaa gcg gtg att gac gtg gcg acg ctg acc ggt gcc tgc     1152
Phe Glu Pro Glu Ala Val Ile Asp Val Ala Thr Leu Thr Gly Ala Cys
    370                 375                 380

gtg atc gcg ctg ggt cat cat att act ggt ctg atg gcg aac cat aat     1200
Val Ile Ala Leu Gly His His Ile Thr Gly Leu Met Ala Asn His Asn
385                 390                 395                 400

ccg ctg gcc cat gaa ctg att gcc gcg tct gaa caa tcc ggt gac cgc     1248
Pro Leu Ala His Glu Leu Ile Ala Ala Ser Glu Gln Ser Gly Asp Arg
                405                 410                 415

gca tgg cgc tta ccg ctg ggt gac gag tat cag gaa caa ctg gag tcc     1296
Ala Trp Arg Leu Pro Leu Gly Asp Glu Tyr Gln Glu Gln Leu Glu Ser
            420                 425                 430

aat ttt gcc gat atg gcg aac att ggc ggt cgt cct ggt ggg gcg att     1344
Asn Phe Ala Asp Met Ala Asn Ile Gly Gly Arg Pro Gly Gly Ala Ile
        435                 440                 445

acc gca ggt tgc ttc ctg tca cgc ttt acc cgt aag tac aac tgg gcg     1392
Thr Ala Gly Cys Phe Leu Ser Arg Phe Thr Arg Lys Tyr Asn Trp Ala
    450                 455                 460

cac ctg gat atc gcc ggt acc gcc tgg cgt tct ggt aaa gca aaa ggc     1440
His Leu Asp Ile Ala Gly Thr Ala Trp Arg Ser Gly Lys Ala Lys Gly
465                 470                 475                 480

gcc acc ggt cgt ccg gta gcg ttg ctg gca cag ttc ctg tta aac cgc     1488
Ala Thr Gly Arg Pro Val Ala Leu Leu Ala Gln Phe Leu Leu Asn Arg
                485                 490                 495

gct ggg ttt aac ggc gaa gag                                         1509
Ala Gly Phe Asn Gly Glu Glu
            500

<210> 54
<211> 1281

<212> DNA
<213> Escherichia coli

<400> 54
atg aca gaa gcg atg aag att acc ctc tct acc caa cct gcc gac gcg     48
Met Thr Glu Ala Met Lys Ile Thr Leu Ser Thr Gln Pro Ala Asp Ala
1               5                   10                  15

cgc tgg gga gaa aaa gca act tac agc att aat aat gac ggc att acc     96
Arg Trp Gly Glu Lys Ala Thr Tyr Ser Ile Asn Asn Asp Gly Ile Thr
            20                  25                  30

ctg cat ttg aac ggg gca gac gat ctg ggg ctg atc cag cgt gcg gcg     144
Leu His Leu Asn Gly Ala Asp Asp Leu Gly Leu Ile Gln Arg Ala Ala
        35                  40                  45

cgc aag att gac ggt ctg ggc atc aag cat gtt cag tta agc ggt gaa     192
Arg Lys Ile Asp Gly Leu Gly Ile Lys His Val Gln Leu Ser Gly Glu
    50                  55                  60

ggc tgg gat gcg gat cgc tgc tgg gca ttc tgg caa ggt tac aaa gcc     240
Gly Trp Asp Ala Asp Arg Cys Trp Ala Phe Trp Gln Gly Tyr Lys Ala
65                  70                  75                  80

ccg aaa ggc acg cgt aaa gtg gtg tgg ccg gat ctg gac gat gcc cag     288
Pro Lys Gly Thr Arg Lys Val Val Trp Pro Asp Leu Asp Asp Ala Gln
                85                  90                  95

cgc cag gaa ctg gat aac cgc ctg atg atc atc gac tgg gtg cgt gac     336
Arg Gln Glu Leu Asp Asn Arg Leu Met Ile Ile Asp Trp Val Arg Asp
            100                 105                 110

acc atc aac gca ccg gca gaa gaa ttg gga cca tcg caa ctg gca cag     384
Thr Ile Asn Ala Pro Ala Glu Glu Leu Gly Pro Ser Gln Leu Ala Gln
        115                 120                 125

cgt gct gtt gat ctg atc agc aac gtc gcg ggc gat cgt gtg act tat     432
Arg Ala Val Asp Leu Ile Ser Asn Val Ala Gly Asp Arg Val Thr Tyr
    130                 135                 140

cgg atc acc aaa ggc gaa gat ctg cgt gag caa ggt tat atg ggg ctg     480
Arg Ile Thr Lys Gly Glu Asp Leu Arg Glu Gln Gly Tyr Met Gly Leu
145                 150                 155                 160

cac aca gtc gga cgc ggt tca gaa cgt tct ccg gta ttg ctg gcg ctg     528
His Thr Val Gly Arg Gly Ser Glu Arg Ser Pro Val Leu Leu Ala Leu
                165                 170                 175

gat tac aac cca act ggc gat aaa gaa gcg cca gtg tac gcg tgc ctg     576
Asp Tyr Asn Pro Thr Gly Asp Lys Glu Ala Pro Val Tyr Ala Cys Leu
            180                 185                 190

gta ggt aaa ggt atc act ttt gac tcc ggc ggc tac agc atc aaa cag     624
Val Gly Lys Gly Ile Thr Phe Asp Ser Gly Gly Tyr Ser Ile Lys Gln
        195                 200                 205

act gcg ttt atg gac tcg atg aag tcg gac atg ggc ggc gcg gca acg     672
Thr Ala Phe Met Asp Ser Met Lys Ser Asp Met Gly Gly Ala Ala Thr
    210                 215                 220

gtt acc ggg gcg ctg gca ttt gcc att acg cgc gga ctg aac aag cgc     720
Val Thr Gly Ala Leu Ala Phe Ala Ile Thr Arg Gly Leu Asn Lys Arg
225                 230                 235                 240

gtg aag ctg ttc ctc tgc tgt gcg gat aac ctg att agc ggc aat gcg     768
Val Lys Leu Phe Leu Cys Cys Ala Asp Asn Leu Ile Ser Gly Asn Ala
                245                 250                 255

ttc aag ctg ggc gat atc atc acc tat cgc aac ggt aaa aaa gtt gaa     816
Phe Lys Leu Gly Asp Ile Ile Thr Tyr Arg Asn Gly Lys Lys Val Glu
            260                 265                 270

gtg atg aac act gat gcc gaa ggg cgt ctg gtg ctt gcc gat ggt ctg     864
Val Met Asn Thr Asp Ala Glu Gly Arg Leu Val Leu Ala Asp Gly Leu
        275                 280                 285

att gat gcc agt gcg cag aaa ccg gaa atg atc att gat gcg gcg acc     912
Ile Asp Ala Ser Ala Gln Lys Pro Glu Met Ile Ile Asp Ala Ala Thr
    290                 295                 300

ctc acc ggg gcg gcg aaa act gcg ctg ggt aat gat tat cac gcg ctg     960
Leu Thr Gly Ala Ala Lys Thr Ala Leu Gly Asn Asp Tyr His Ala Leu
305                 310                 315                 320

ttc agt ttt gac gat gcg ctg gcc ggt cgc ttg ctg gcg agt gcc gcg     1008
Phe Ser Phe Asp Asp Ala Leu Ala Gly Arg Leu Leu Ala Ser Ala Ala
                325                 330                 335

cag gag aac gaa ccg ttc tgg cgt ctg ccg ctg gcg gag ttc cac cgc     1056
Gln Glu Asn Glu Pro Phe Trp Arg Leu Pro Leu Ala Glu Phe His Arg
            340                 345                 350

agc cag ctg ccg tct aac ttt gcc gaa ctg aac aat acc gga agc gcg     1104
Ser Gln Leu Pro Ser Asn Phe Ala Glu Leu Asn Asn Thr Gly Ser Ala
        355                 360                 365

gcg tat ccg gca ggc gcg agc acg gcg gcg ggc ttc ctg tcg cac ttt     1152
Ala Tyr Pro Ala Gly Ala Ser Thr Ala Ala Gly Phe Leu Ser His Phe
    370                 375                 380

gtt gag aac tat cag caa ggc tgg ctg cat atc gac tgc tcg gcg act     1200
Val Glu Asn Tyr Gln Gln Gly Trp Leu His Ile Asp Cys Ser Ala Thr
385                 390                 395                 400

tac cgt aaa gcg ccg gtt gaa cag tgg tct gcg ggc gct acg gga ctt     1248
Tyr Arg Lys Ala Pro Val Glu Gln Trp Ser Ala Gly Ala Thr Gly Leu
                405                 410                 415

ggt gtg cgc acg ata gct aat ctg tta acg gcg                         1281
Gly Val Arg Thr Ile Ala Asn Leu Leu Thr Ala
            420                 425

<210> 55
<211> 1455
<212> DNA
<213> Escherichia coli

<400> 55
gtg tct gaa ctg tct caa tta tct cca cag ccg ctg tgg gat att ttt     48

Val Ser Glu Leu Ser Gln Leu Ser Pro Gln Pro Leu Trp Asp Ile Phe
1               5                   10                  15

gcc aaa atc tgt tct att cct cac ccg tcc tat cat gaa gag caa ctc     96
Ala Lys Ile Cys Ser Ile Pro His Pro Ser Tyr His Glu Glu Gln Leu
            20                  25                  30

gct gaa tac att gtt ggt tgg gca aaa gag aaa ggt ttc cat gtc gaa     144
Ala Glu Tyr Ile Val Gly Trp Ala Lys Glu Lys Gly Phe His Val Glu
        35                  40                  45

cgc gat cag gta ggt aat atc ctg att cgt aaa cct gct acc gca ggt     192
Arg Asp Gln Val Gly Asn Ile Leu Ile Arg Lys Pro Ala Thr Ala Gly
    50                  55                  60

atg gaa aat cgt aaa ccg gtc gtc tta cag gcc cac ctc gat atg gtg     240
Met Glu Asn Arg Lys Pro Val Val Leu Gln Ala His Leu Asp Met Val
65                  70                  75                  80

ccg cag aaa aat aac gac acc gtg cat gac ttc acg aaa gat cct atc     288
Pro Gln Lys Asn Asn Asp Thr Val His Asp Phe Thr Lys Asp Pro Ile
                85                  90                  95

cag cct tat att gat ggc gaa tgg gtt aaa gcg cgc ggc acc acg ctg     336
Gln Pro Tyr Ile Asp Gly Glu Trp Val Lys Ala Arg Gly Thr Thr Leu
            100                 105                 110

ggt gcg gat aac ggc att ggt atg gcc tct gcg ctg gcg gtt ctg gct     384
Gly Ala Asp Asn Gly Ile Gly Met Ala Ser Ala Leu Ala Val Leu Ala
        115                 120                 125

gac gaa aac gtg gtt cac ggc ccg ctg gaa gtg ctg ctg acc atg acc     432
Asp Glu Asn Val Val His Gly Pro Leu Glu Val Leu Leu Thr Met Thr
    130                 135                 140

gaa gaa gcc ggt atg gac ggt gcg ttc ggc tta cag ggc aac tgg ttg     480
Glu Glu Ala Gly Met Asp Gly Ala Phe Gly Leu Gln Gly Asn Trp Leu
145                 150                 155                 160

cag gct gat att ctg att aac acc gac tcc gaa gaa gaa ggt gaa atc     528
Gln Ala Asp Ile Leu Ile Asn Thr Asp Ser Glu Glu Glu Gly Glu Ile
                165                 170                 175

tac atg ggt tgt gcg ggg ggt atc gac ttc acc tcc aac ctg cat tta     576
Tyr Met Gly Cys Ala Gly Gly Ile Asp Phe Thr Ser Asn Leu His Leu
            180                 185                 190

gat cgt gaa gcg gtt cca gct ggt ttt gaa acc ttc aag tta acc tta     624
Asp Arg Glu Ala Val Pro Ala Gly Phe Glu Thr Phe Lys Leu Thr Leu
        195                 200                 205

aaa ggt ctg aaa ggc ggt cac tcc ggc ggg gaa atc cac gtt ggg ctg     672
Lys Gly Leu Lys Gly Gly His Ser Gly Gly Glu Ile His Val Gly Leu
    210                 215                 220

ggt aat gcc aac aaa ctg ctg gtg cgc ttc ctg gcg ggt cat gcg gaa     720
Gly Asn Ala Asn Lys Leu Leu Val Arg Phe Leu Ala Gly His Ala Glu
225                 230                 235                 240

gaa ctg gat ctg cgc ctt atc gat ttc aac ggc ggc aca ctg cgt aac     768

Glu Leu Asp Leu Arg Leu Ile Asp Phe Asn Gly Gly Thr Leu Arg Asn
                245                 250                 255

gcc atc ccg cgt gaa gcc ttt gcg acc att gct gtc gca gct gat aaa      816
Ala Ile Pro Arg Glu Ala Phe Ala Thr Ile Ala Val Ala Ala Asp Lys
            260                 265                 270

gtc gac gtc ctg aaa tct ctg gtg aat acc tat cag gag atc ctg aaa      864
Val Asp Val Leu Lys Ser Leu Val Asn Thr Tyr Gln Glu Ile Leu Lys
        275                 280                 285

aac gag ctg gca gaa aaa gag aaa aat ctg gcc ttg ttg ctg gac tct      912
Asn Glu Leu Ala Glu Lys Glu Lys Asn Leu Ala Leu Leu Leu Asp Ser
    290                 295                 300

gta gcg aac gat aaa gct gcc ctg att gcg aaa tct cgc gat acc ttt      960
Val Ala Asn Asp Lys Ala Ala Leu Ile Ala Lys Ser Arg Asp Thr Phe
305                 310                 315                 320

att cgt ctg ctg aac gcc acc ccg aac ggt gtg att cgt aac tcc gat      1008
Ile Arg Leu Leu Asn Ala Thr Pro Asn Gly Val Ile Arg Asn Ser Asp
                325                 330                 335

gta gcc aaa ggt gtg gtt gaa acc tcc ctg aac gtc ggt gtg gtg acc      1056
Val Ala Lys Gly Val Val Glu Thr Ser Leu Asn Val Gly Val Val Thr
            340                 345                 350

atg act gac aat aac gta gaa att cac tgc ctg atc cgt tca ctg atc      1104
Met Thr Asp Asn Asn Val Glu Ile His Cys Leu Ile Arg Ser Leu Ile
        355                 360                 365

gac agc ggt aaa gac tac gtg gtg agc atg ctg gat tcg ctg ggt aaa      1152
Asp Ser Gly Lys Asp Tyr Val Val Ser Met Leu Asp Ser Leu Gly Lys
    370                 375                 380

ctg gct ggc gcg aaa acc gaa gcg aaa ggc gca tat cct ggc tgg cag      1200
Leu Ala Gly Ala Lys Thr Glu Ala Lys Gly Ala Tyr Pro Gly Trp Gln
385                 390                 395                 400

ccg gac gct aat tct ccg gtg atg cat ctg gta cgt gaa acc tat cag      1248
Pro Asp Ala Asn Ser Pro Val Met His Leu Val Arg Glu Thr Tyr Gln
                405                 410                 415

cgc ctg ttc aac aag acg ccg aac atc cag att atc cac gcg ggc ctg      1296
Arg Leu Phe Asn Lys Thr Pro Asn Ile Gln Ile Ile His Ala Gly Leu
            420                 425                 430

gaa tgt ggt ctg ttc aaa aaa ccg tat ccg gaa atg gac atg gtt tct      1344
Glu Cys Gly Leu Phe Lys Lys Pro Tyr Pro Glu Met Asp Met Val Ser
        435                 440                 445

atc ggg cca act atc acc ggt cca cac tct ccg gat gag caa gtt cac      1392
Ile Gly Pro Thr Ile Thr Gly Pro His Ser Pro Asp Glu Gln Val His
    450                 455                 460

atc gaa agc gta ggt cat tac tgg aca ctg ctg act gaa ctg ctg aaa      1440
Ile Glu Ser Val Gly His Tyr Trp Thr Leu Leu Thr Glu Leu Leu Lys
465                 470                 475                 480

gaa att ccg gcg aag                                                1455
Glu Ile Pro Ala Lys
                485

<210> 56
<211> 2610
<212> DNA
<213> Escherichia coli

<400> 56
atg act caa cag cca caa gcc aaa tac cgt cac gat tat cgt gcg ccg    48
Met Thr Gln Gln Pro Gln Ala Lys Tyr Arg His Asp Tyr Arg Ala Pro
 1               5                  10                  15

gat tac cag att act gat att gac ttg acc ttt gac ctc gac gcg caa    96
Asp Tyr Gln Ile Thr Asp Ile Asp Leu Thr Phe Asp Leu Asp Ala Gln
            20                  25                  30

aag acg gtc gtt acc gcg gtc agc cag gct gtc cgt cat ggt gca tca    144
Lys Thr Val Val Thr Ala Val Ser Gln Ala Val Arg His Gly Ala Ser
        35                  40                  45

gat gct ccc ctt cgt ctc aac ggc gaa gac ctc aaa ctg gtt tct gtt    192
Asp Ala Pro Leu Arg Leu Asn Gly Glu Asp Leu Lys Leu Val Ser Val
    50                  55                  60

cat att aat gat gag ccg tgg acc gcc tgg aaa gaa gaa gag ggc gca    240
His Ile Asn Asp Glu Pro Trp Thr Ala Trp Lys Glu Glu Glu Gly Ala
65                  70                  75                  80

ctg gtt atc agt aat ttg ccg gag cgt ttt acg ctt aag atc att aat    288
Leu Val Ile Ser Asn Leu Pro Glu Arg Phe Thr Leu Lys Ile Ile Asn
                85                  90                  95

gaa ata agc ccg gcg gcg aat acc gcg ctg gaa ggg ctt tat cag tca    336
Glu Ile Ser Pro Ala Ala Asn Thr Ala Leu Glu Gly Leu Tyr Gln Ser
            100                 105                 110

ggc gat gcg ctt tgc acc cag tgt gaa gcc gaa ggt ttc cgc cat att    384
Gly Asp Ala Leu Cys Thr Gln Cys Glu Ala Glu Gly Phe Arg His Ile
        115                 120                 125

acg tat tat ctc gac cgc ccg gac gtg ctg gcg cgt ttt acc acc aaa    432
Thr Tyr Tyr Leu Asp Arg Pro Asp Val Leu Ala Arg Phe Thr Thr Lys
    130                 135                 140

att att gcc gat aaa atc aaa tat ccc ttc ctg ctt tcc aat ggt aac    480
Ile Ile Ala Asp Lys Ile Lys Tyr Pro Phe Leu Leu Ser Asn Gly Asn
145                 150                 155                 160

cgc gtt gcg caa ggc gaa ctg gaa aac gga cgc cat tgg gta cag tgg    528
Arg Val Ala Gln Gly Glu Leu Glu Asn Gly Arg His Trp Val Gln Trp
                165                 170                 175

cag gac ccg ttc ccg aaa ccg tgc tac ctg ttt gcg ctg gtg gca ggc    576
Gln Asp Pro Phe Pro Lys Pro Cys Tyr Leu Phe Ala Leu Val Ala Gly
            180                 185                 190

gac ttt gat gta ctg cgc gat acc ttt acc acg cgt tct ggt cgc gaa    624

Asp Phe Asp Val Leu Arg Asp Thr Phe Thr Thr Arg Ser Gly Arg Glu
        195                 200                 205

gta gca ctg gag ctg tac gtc gat cgc ggc aac ctt gat cgc gcg ccg 672
Val Ala Leu Glu Leu Tyr Val Asp Arg Gly Asn Leu Asp Arg Ala Pro
    210                 215                 220

tgg gcg atg acc tcg ctg aaa aac tcc atg aaa tgg gat gaa gaa cgc 720

Trp Ala Met Thr Ser Leu Lys Asn Ser Met Lys Trp Asp Glu Glu Arg
225                 230                 235                 240

ttt ggc ctg gag tat gac ctc gac atc tat atg atc gtc gcg gtg gat 768
Phe Gly Leu Glu Tyr Asp Leu Asp Ile Tyr Met Ile Val Ala Val Asp
                245                 250                 255

ttc ttc aat atg ggc gca atg gag aat aag ggg ctg aat atc ttt aac 816
Phe Phe Asn Met Gly Ala Met Glu Asn Lys Gly Leu Asn Ile Phe Asn
            260                 265                 270

tcc aaa tat gtg ctg gcc cgc acc gac acc gcc acc gac aaa gat tac 864
Ser Lys Tyr Val Leu Ala Arg Thr Asp Thr Ala Thr Asp Lys Asp Tyr
        275                 280                 285

ctc gat att gaa cgc gtt atc ggc cat gaa tat ttc cat aac tgg acc 912
Leu Asp Ile Glu Arg Val Ile Gly His Glu Tyr Phe His Asn Trp Thr
    290                 295                 300

ggt aac cga gtg acc tgt cgc gac tgg ttc cag ctc agc ctg aaa gaa 960
Gly Asn Arg Val Thr Cys Arg Asp Trp Phe Gln Leu Ser Leu Lys Glu
305                 310                 315                 320

ggt tta acc gtc ttc cgc gat cag gag ttc agc tct gac ctt ggt tcc 1008
Gly Leu Thr Val Phe Arg Asp Gln Glu Phe Ser Ser Asp Leu Gly Ser
                325                 330                 335

cgc gca gtt aac cgc atc aat aat gta cgc acc atg cgc gga ttg cag 1056
Arg Ala Val Asn Arg Ile Asn Asn Val Arg Thr Met Arg Gly Leu Gln
            340                 345                 350

ttt gca gaa gac gcc agc ccg atg gcg cac ccg atc cgc ccg gat atg 1104
Phe Ala Glu Asp Ala Ser Pro Met Ala His Pro Ile Arg Pro Asp Met
        355                 360                 365

gtc att gag atg aac aac ttc tac acc ctg acc gtt tac gag aag ggc 1152
Val Ile Glu Met Asn Asn Phe Tyr Thr Leu Thr Val Tyr Glu Lys Gly
    370                 375                 380

gcg gaa gtg att cgc atg atc cac acc ctg ctt ggc gaa gaa aac ttc 1200
Ala Glu Val Ile Arg Met Ile His Thr Leu Leu Gly Glu Glu Asn Phe
385                 390                 395                 400

cag aaa ggg atg cag ctt tat ttc gag cgt cat gat ggt agt gca gcg 1248
Gln Lys Gly Met Gln Leu Tyr Phe Glu Arg His Asp Gly Ser Ala Ala
                405                 410                 415

acc tgt gac gac ttt gtg cag gcg atg gaa gat gcg tcg aat gtc gat 1296
Thr Cys Asp Asp Phe Val Gln Ala Met Glu Asp Ala Ser Asn Val Asp
            420                 425                 430

ctc tcc cat ttc cgc cgt tgg tac agc cag tcc ggt aca ccg att gtg     1344
Leu Ser His Phe Arg Arg Trp Tyr Ser Gln Ser Gly Thr Pro Ile Val
        435                 440                 445

acc gtc aaa gac gac tac aat ccg gaa acc gag cag tac acc ctg acc     1392
Thr Val Lys Asp Asp Tyr Asn Pro Glu Thr Glu Gln Tyr Thr Leu Thr
    450                 455                 460

atc agc cag cgc acg cca gcc acg ccg gat cag gca gaa aaa cag ccg     1440
Ile Ser Gln Arg Thr Pro Ala Thr Pro Asp Gln Ala Glu Lys Gln Pro
465                 470                 475                 480

ctg cat att ccg ttt gcc atc gaa ctg tat gat aac gaa ggc aaa gtg     1488
Leu His Ile Pro Phe Ala Ile Glu Leu Tyr Asp Asn Glu Gly Lys Val
                485                 490                 495

atc ccg ttg cag aaa ggc ggt cat ccg gtg aat tcc gtg ctg aac gtc     1536
Ile Pro Leu Gln Lys Gly Gly His Pro Val Asn Ser Val Leu Asn Val
            500                 505                 510

act cag gcg gaa cag acc ttt gtc ttt gat aat gtc tac ttc cag ccg     1584
Thr Gln Ala Glu Gln Thr Phe Val Phe Asp Asn Val Tyr Phe Gln Pro
        515                 520                 525

gtg cct gcg ctg ctg tgc gaa ttc tct gcg cca gtg aaa ctg gaa tat     1632
Val Pro Ala Leu Leu Cys Glu Phe Ser Ala Pro Val Lys Leu Glu Tyr
    530                 535                 540

aag tgg agc gat cag caa ctg acc ttc ctg atg cgt cat gcg cgt aat     1680
Lys Trp Ser Asp Gln Gln Leu Thr Phe Leu Met Arg His Ala Arg Asn
545                 550                 555                 560

gat ttc tcc cgc tgg gat gcg gcg caa agt ttg ctg gca acc tac atc     1728
Asp Phe Ser Arg Trp Asp Ala Ala Gln Ser Leu Leu Ala Thr Tyr Ile
                565                 570                 575

aag ctg aac gtc gcg cgt cat cag caa ggt cag ccg ctg tct ctg ccg     1776
Lys Leu Asn Val Ala Arg His Gln Gln Gly Gln Pro Leu Ser Leu Pro
            580                 585                 590

gtg cat gtg gct gat gct ttc cgc gcg gta ctg ctt gat gag aag att     1824
Val His Val Ala Asp Ala Phe Arg Ala Val Leu Leu Asp Glu Lys Ile
        595                 600                 605

gat cca gcg ctg gcg gca gaa atc ctg acg ctg cct tct gtc aat gaa     1872
Asp Pro Ala Leu Ala Ala Glu Ile Leu Thr Leu Pro Ser Val Asn Glu
    610                 615                 620

atg gct gaa ttg ttc gat atc atc gac ccg att gct att gcc gaa gta     1920
Met Ala Glu Leu Phe Asp Ile Ile Asp Pro Ile Ala Ile Ala Glu Val
625                 630                 635                 640

cgc gaa gca ctc act cgt act ctg gcg act gaa ctg gcg gat gag cta     1968
Arg Glu Ala Leu Thr Arg Thr Leu Ala Thr Glu Leu Ala Asp Glu Leu
                645                 650                 655

ctg gct att tac aac gcg aat tac cag agc gag tac cgt gtt gag cat     2016
Leu Ala Ile Tyr Asn Ala Asn Tyr Gln Ser Glu Tyr Arg Val Glu His
            660                 665                 670

gaa gat att gca aaa cgc act ctg cgt aat gcc tgc ctg cgt ttc ctc    2064
Glu Asp Ile Ala Lys Arg Thr Leu Arg Asn Ala Cys Leu Arg Phe Leu
        675                 680                 685

gct ttt ggt gaa acg cat ctg gct gat gtg ctg gtg agc aag cag ttc    2112
Ala Phe Gly Glu Thr His Leu Ala Asp Val Leu Val Ser Lys Gln Phe
    690                 695                 700

cac gaa gca aac aat atg act gat gcg ctg gcg gcg ctt tct gcg gcg    2160
His Glu Ala Asn Asn Met Thr Asp Ala Leu Ala Ala Leu Ser Ala Ala
705                 710                 715                 720

gtt gcc gca cag ctg cct tgc cgt gac gcg ctg atg cag gag tac gac    2208
Val Ala Ala Gln Leu Pro Cys Arg Asp Ala Leu Met Gln Glu Tyr Asp

                    725                 730                 735

gac aag tgg cat cag aac ggt ctg gtg atg gat aaa tgg ttt atc ctg    2256
Asp Lys Trp His Gln Asn Gly Leu Val Met Asp Lys Trp Phe Ile Leu
            740                 745                 750

caa gcc acc agc ccg gcg gcg aat gtg ctg gag acg gtg cgc ggc ctg    2304
Gln Ala Thr Ser Pro Ala Ala Asn Val Leu Glu Thr Val Arg Gly Leu
        755                 760                 765

ttg cag cat cgc tca ttt acc atg agc aac ccg aac cgt att cgt tcg    2352
Leu Gln His Arg Ser Phe Thr Met Ser Asn Pro Asn Arg Ile Arg Ser
    770                 775                 780

ttg att ggc gcg ttt gcg ggc agc aat ccg gca gcg ttc cat gcc gaa    2400
Leu Ile Gly Ala Phe Ala Gly Ser Asn Pro Ala Ala Phe His Ala Glu
785                 790                 795                 800

gat ggc agc ggt tac ctg ttc ctg gtg gaa atg ctt acc gac ctc aac    2448
Asp Gly Ser Gly Tyr Leu Phe Leu Val Glu Met Leu Thr Asp Leu Asn
                805                 810                 815

agc cgt aac ccg cag gtg gct tca cgt ctg att gaa ccg ctg att cgc    2496
Ser Arg Asn Pro Gln Val Ala Ser Arg Leu Ile Glu Pro Leu Ile Arg
            820                 825                 830

ctg aaa cgt tac gat gcc aaa cgt cag gag aaa atg cgc gcg gcg ctg    2544
Leu Lys Arg Tyr Asp Ala Lys Arg Gln Glu Lys Met Arg Ala Ala Leu
        835                 840                 845

gaa cag ttg aaa ggg ctg gaa aat ctc tct ggc gat ctg tac gag aag    2592
Glu Gln Leu Lys Gly Leu Glu Asn Leu Ser Gly Asp Leu Tyr Glu Lys
    850                 855                 860

ata act aaa gca ctg gct                                            2610
Ile Thr Lys Ala Leu Ala
865                 870

<210> 57
<211> 1605
<212> DNA
<213> Escherichia coli

<400> 57

atg cgt att tcc ttg aaa aag tca ggg atg ctg aag ctt ggt ctc agc    48
Met Arg Ile Ser Leu Lys Lys Ser Gly Met Leu Lys Leu Gly Leu Ser
1               5                   10                  15

ctg gtg gct atg acc gtc gca gca agt gtt cag gct aaa act ctg gtt    96
Leu Val Ala Met Thr Val Ala Ala Ser Val Gln Ala Lys Thr Leu Val
            20                  25                  30

tat tgc tca gaa gga tct ccg gaa ggg ttt aac ccg cag ctg ttt acc    144
Tyr Cys Ser Glu Gly Ser Pro Glu Gly Phe Asn Pro Gln Leu Phe Thr
        35                  40                  45

tcc ggc acc acc tat gac gcc tct tcc gtc ccg ctt tat aac cgt ctg    192
Ser Gly Thr Thr Tyr Asp Ala Ser Ser Val Pro Leu Tyr Asn Arg Leu
    50                  55                  60

gtt gaa ttt aaa atc ggc acc acc gaa gtg atc ccg ggc ctc gct gaa    240
Val Glu Phe Lys Ile Gly Thr Thr Glu Val Ile Pro Gly Leu Ala Glu
65                  70                  75                  80

aag tgg gaa gtc agc gaa gac ggt aaa acc tat acc ttc cat ctg cgt    288
Lys Trp Glu Val Ser Glu Asp Gly Lys Thr Tyr Thr Phe His Leu Arg
                85                  90                  95

aaa ggt gtg aag tgg cac gac aat aaa gaa ttc aaa ccg acg cgt gaa    336
Lys Gly Val Lys Trp His Asp Asn Lys Glu Phe Lys Pro Thr Arg Glu
            100                 105                 110

ctg aac gcc gat gat gtg gtg ttc tcg ttc gat cgt cag aaa aac gcg    384
Leu Asn Ala Asp Asp Val Val Phe Ser Phe Asp Arg Gln Lys Asn Ala
        115                 120                 125

caa aac ccg tac cat aaa gtt tct ggc ggc agc tac gaa tac ttc gaa    432
Gln Asn Pro Tyr His Lys Val Ser Gly Gly Ser Tyr Glu Tyr Phe Glu
    130                 135                 140

ggc atg ggc ttg cca gag ctg atc agt gaa gtg aaa aag gtg gac gac    480
Gly Met Gly Leu Pro Glu Leu Ile Ser Glu Val Lys Lys Val Asp Asp
145                 150                 155                 160

aac acc gtt cag ttt gtg ctg act cgc ccg gaa gcg ccg ttc ctc gct    528
Asn Thr Val Gln Phe Val Leu Thr Arg Pro Glu Ala Pro Phe Leu Ala
                165                 170                 175

gac ctg gca atg gac ttc gcc tct att ctg tca aaa gaa tat gct gat    576
Asp Leu Ala Met Asp Phe Ala Ser Ile Leu Ser Lys Glu Tyr Ala Asp
            180                 185                 190

gcg atg atg aaa gcc ggt aca ccg gaa aaa ctg gac ctc aac cca atc    624
Ala Met Met Lys Ala Gly Thr Pro Glu Lys Leu Asp Leu Asn Pro Ile
        195                 200                 205

gga acc ggt ccg ttc cag tta cag cag tat caa aaa gat tcc cgt atc    672
Gly Thr Gly Pro Phe Gln Leu Gln Gln Tyr Gln Lys Asp Ser Arg Ile
    210                 215                 220

cgc tac aaa gcg ttt gat ggc tac tgg ggc acc aaa ccg cag atc gat    720
Arg Tyr Lys Ala Phe Asp Gly Tyr Trp Gly Thr Lys Pro Gln Ile Asp
225                 230                 235                 240

acg ctg gtt ttc tct att acc cct gac gct tcc gtg cgt tac gcg aaa 768
Thr Leu Val Phe Ser Ile Thr Pro Asp Ala Ser Val Arg Tyr Ala Lys
                245                 250                 255

ttg cag aag aat gaa tgc cag gtg atg ccg tac ccg aac ccg gca gat 816
Leu Gln Lys Asn Glu Cys Gln Val Met Pro Tyr Pro Asn Pro Ala Asp
            260                 265                 270

atc gct cgc atg aag cag gat aaa tcc atc aat ctg atg gaa atg ccg 864
Ile Ala Arg Met Lys Gln Asp Lys Ser Ile Asn Leu Met Glu Met Pro
        275                 280                 285

ggg ctg aac gtc ggt tat ctc tcg tat aac gtg cag aaa aaa cca ctc 912
Gly Leu Asn Val Gly Tyr Leu Ser Tyr Asn Val Gln Lys Lys Pro Leu
    290                 295                 300

gat gac gtg aaa gtt cgc cag gct ctg acc tac gcg gtg aac aaa gac 960
Asp Asp Val Lys Val Arg Gln Ala Leu Thr Tyr Ala Val Asn Lys Asp
305                 310                 315                 320

gcg atc atc aaa gcg gtt tat cag ggc gcg ggc gta tca gcg aaa aac 1008
Ala Ile Ile Lys Ala Val Tyr Gln Gly Ala Gly Val Ser Ala Lys Asn
                325                 330                 335

ctg atc ccg cca acc atg tgg ggc tat aac gac gac gtt cag gac tac 1056
Leu Ile Pro Pro Thr Met Trp Gly Tyr Asn Asp Asp Val Gln Asp Tyr
            340                 345                 350

acc tac gat cct gaa aaa gcg aaa gcc ttg ctg aaa gaa gcg ggt ctg 1104
Thr Tyr Asp Pro Glu Lys Ala Lys Ala Leu Leu Lys Glu Ala Gly Leu
        355                 360                 365

gaa aaa ggt ttc tcc atc gac ctg tgg gcg atg ccg gta caa cgt ccg 1152
Glu Lys Gly Phe Ser Ile Asp Leu Trp Ala Met Pro Val Gln Arg Pro
    370                 375                 380

tat aac ccg aac gct cgc cgc atg gcg gag atg att cag gca gac tgg 1200
Tyr Asn Pro Asn Ala Arg Arg Met Ala Glu Met Ile Gln Ala Asp Trp
385                 390                 395                 400

gcg aaa gtc ggc gtg cag gcc aaa att gtc acc tac gaa tgg ggt gag 1248
Ala Lys Val Gly Val Gln Ala Lys Ile Val Thr Tyr Glu Trp Gly Glu
                405                 410                 415

tac ctc aag cgt gcg aaa gat ggc gag cac cag acg gta atg atg ggc 1296
Tyr Leu Lys Arg Ala Lys Asp Gly Glu His Gln Thr Val Met Met Gly
            420                 425                 430

tgg act ggc gat aac ggg gat ccg gat aac ttc ttc gcc acc ctg ttc 1344
Trp Thr Gly Asp Asn Gly Asp Pro Asp Asn Phe Phe Ala Thr Leu Phe
        435                 440                 445

agc tgc gcc gcc tct gaa caa ggc tcc aac tac tca aaa tgg tgc tac 1392
Ser Cys Ala Ala Ser Glu Gln Gly Ser Asn Tyr Ser Lys Trp Cys Tyr
    450                 455                 460

aaa ccg ttt gaa gat ctg att caa ccg gcg cgt gct acc gac gac cac 1440
Lys Pro Phe Glu Asp Leu Ile Gln Pro Ala Arg Ala Thr Asp Asp His
465                 470                 475                 480

aat aaa cgc gtt gaa ctg tac aaa caa gcg cag gtg gtg atg cac gat     1488
Asn Lys Arg Val Glu Leu Tyr Lys Gln Ala Gln Val Val Met His Asp
                485                 490                 495

cag gct ccg gca ctg atc atc gct cac tcc acc gtg ttt gaa ccg gta     1536
Gln Ala Pro Ala Leu Ile Ile Ala His Ser Thr Val Phe Glu Pro Val
            500                 505                 510

cgt aaa gaa gtt aaa ggc tat gtg gtt gat cca tta ggc aaa cat cac     1584
Arg Lys Glu Val Lys Gly Tyr Val Val Asp Pro Leu Gly Lys His His
        515                 520                 525

ttc gaa aac gtc tct atc gaa                                         1605
Phe Glu Asn Val Ser Ile Glu
    530                 535


<210> 58
<211> 1017
<212> DNA
<213> Escherichia coli

<400> 58
atg ttg cag ttt att ctc cga cgt ttg gga ctc gtc atc ccc acg ttt     48
Met Leu Gln Phe Ile Leu Arg Arg Leu Gly Leu Val Ile Pro Thr Phe
  1               5                  10                  15

atc ggt att acc ctt ctc aca ttt gcc ttt gtc cac atg atc ccg ggc     96
Ile Gly Ile Thr Leu Leu Thr Phe Ala Phe Val His Met Ile Pro Gly
             20                  25                  30

gat ccg gtg atg atc atg gcg ggc gaa cgt ggg atc tcc cca gag cgt     144
Asp Pro Val Met Ile Met Ala Gly Glu Arg Gly Ile Ser Pro Glu Arg
         35                  40                  45

cac gcg cag ctg ctg gct gaa ctc ggc tta gat aaa ccg atg tgg cag     192
His Ala Gln Leu Leu Ala Glu Leu Gly Leu Asp Lys Pro Met Trp Gln
     50                  55                  60

cag tat ctc cat tac att tgg ggc gtt atg cat ggc gat cta ggc att     240
Gln Tyr Leu His Tyr Ile Trp Gly Val Met His Gly Asp Leu Gly Ile
 65                  70                  75                  80

tca atg aaa agc cgc atc ccg gtt tgg gaa gag ttc gtg ccg cgc ttc     288
Ser Met Lys Ser Arg Ile Pro Val Trp Glu Glu Phe Val Pro Arg Phe
                 85                  90                  95

cag gcc acg ctg gaa ctt ggc gtc tgc gcg atg att ttt gct acg gca     336
Gln Ala Thr Leu Glu Leu Gly Val Cys Ala Met Ile Phe Ala Thr Ala
            100                 105                 110

gtc ggt att ccg gtc ggc gtg ctg gct gcg gtt aaa cgc ggt tcc att     384
Val Gly Ile Pro Val Gly Val Leu Ala Ala Val Lys Arg Gly Ser Ile
        115                 120                 125

ttc gat cac aca gcg gtt ggc ctg gcg ctg aca ggt tat tca atg cct     432
Phe Asp His Thr Ala Val Gly Leu Ala Leu Thr Gly Tyr Ser Met Pro
    130                 135                 140

atc ttc tgg tgg ggc atg atg ctg atc atg ctg gtt tcg gtg cac tgg     480

Ile Phe Trp Trp Gly Met Met Leu Ile Met Leu Val Ser Val His Trp
145                 150                 155                 160

aac ctg acg ccc gtc tcc ggt cgc gtg agc gat atg gtg ttc ctc gat     528
Asn Leu Thr Pro Val Ser Gly Arg Val Ser Asp Met Val Phe Leu Asp
                165                 170                 175

gac tcc aat ccg tta acc ggt ttt atg cta atc gac acc gcc atc tgg     576
Asp Ser Asn Pro Leu Thr Gly Phe Met Leu Ile Asp Thr Ala Ile Trp
            180                 185                 190

ggt gaa gac ggc aac ttt atc gat gcc gtc gcc cat atg atc ttg cct     624
Gly Glu Asp Gly Asn Phe Ile Asp Ala Val Ala His Met Ile Leu Pro
        195                 200                 205

gcc att gtg ctg ggt act att ccg ctg gcg gtc att gtg cgt atg aca     672
Ala Ile Val Leu Gly Thr Ile Pro Leu Ala Val Ile Val Arg Met Thr
    210                 215                 220

cgc tcc tcg atg ctg gaa gtg ctg ggc gag gat tac atc cgc acc gcg     720
Arg Ser Ser Met Leu Glu Val Leu Gly Glu Asp Tyr Ile Arg Thr Ala
225                 230                 235                 240

cgc gcc aaa ggg cta acc cgc atg cgg gtg att atc gtc cat gcg ctg     768
Arg Ala Lys Gly Leu Thr Arg Met Arg Val Ile Ile Val His Ala Leu
                245                 250                 255

cgt aac gcg atg ctg ccg gtg gtg acc gtt atc ggc ctg cag gtg gga     816
Arg Asn Ala Met Leu Pro Val Val Thr Val Ile Gly Leu Gln Val Gly
            260                 265                 270

aca ttg ctg gcg ggg gcg att ctg acc gaa acc atc ttc tcg tgg ccc     864
Thr Leu Leu Ala Gly Ala Ile Leu Thr Glu Thr Ile Phe Ser Trp Pro
        275                 280                 285

ggt ctg gga cgc tgg ttg att gac gca ctg caa cgc cgc gac tat ccg     912
Gly Leu Gly Arg Trp Leu Ile Asp Ala Leu Gln Arg Arg Asp Tyr Pro
    290                 295                 300

gta gtg cag ggc ggc gta ttg ctg gtg gcg acg atg att atc ctc gtc     960
Val Val Gln Gly Gly Val Leu Leu Val Ala Thr Met Ile Ile Leu Val
305                 310                 315                 320

aac ttg ctg gtc gat ctg ctg tac ggc gtg gtg aac ccg cgt att cgt     1008
Asn Leu Leu Val Asp Leu Leu Tyr Gly Val Val Asn Pro Arg Ile Arg
                325                 330                 335

cat aag aag                                                         1017
His Lys Lys

<210> 59
<211> 900
<212> DNA
<213> Escherichia coli

<400> 59
atg tca cag gtt act gaa aat aaa gtg att agc gca ccg gtg ccg atg     48
Met Ser Gln Val Thr Glu Asn Lys Val Ile Ser Ala Pro Val Pro Met
  1               5                  10                  15

acc ccg tta cag gag ttc tgg cac tat ttt aaa cgc aac aaa ggc gcg 96
Thr Pro Leu Gln Glu Phe Trp His Tyr Phe Lys Arg Asn Lys Gly Ala
            20                  25                  30

gtc gtc ggg ctg gtt tac gtc gtc atc gtg ctg ttc atc gcg atc ttt 144
Val Val Gly Leu Val Tyr Val Val Ile Val Leu Phe Ile Ala Ile Phe
        35                  40                  45

gcc aac tgg att gca ccc tat aac ccg gcg gaa cag ttc cgc gat gca 192
Ala Asn Trp Ile Ala Pro Tyr Asn Pro Ala Glu Gln Phe Arg Asp Ala
    50                  55                  60

ctg ctc gcc ccg cca gcc tgg cag gaa ggc ggc agc atg gcg cac ttg 240
Leu Leu Ala Pro Pro Ala Trp Gln Glu Gly Gly Ser Met Ala His Leu
65                  70                  75                  80

ctg ggc acc gat gac gta ggc cgt gat gtg ctg tcg cgc ctg atg tac 288
Leu Gly Thr Asp Asp Val Gly Arg Asp Val Leu Ser Arg Leu Met Tyr
                85                  90                  95

ggt gcg cgc ctg tcg ctg ctg gtt ggc tgt ctg gta gtt gtg tta tcg 336
Gly Ala Arg Leu Ser Leu Leu Val Gly Cys Leu Val Val Val Leu Ser
            100                 105                 110

ctg att atg ggc gtt att ctc ggc ctg atc gcc ggt tac ttt ggc ggc 384
Leu Ile Met Gly Val Ile Leu Gly Leu Ile Ala Gly Tyr Phe Gly Gly
        115                 120                 125

ctg gtc gat aac atc att atg cgc gtg gtc gat atc atg ctg gcg ctg 432
Leu Val Asp Asn Ile Ile Met Arg Val Val Asp Ile Met Leu Ala Leu
    130                 135                 140

cca agt ctg ctg ctg gcg ctg gtg ctg gtg gca att ttc ggc ccg tcg 480
Pro Ser Leu Leu Leu Ala Leu Val Leu Val Ala Ile Phe Gly Pro Ser
145                 150                 155                 160

att ggt aac gcc gcg ctg gca ctg acc ttc gtt gcc ttg ccg cac tat 528
Ile Gly Asn Ala Ala Leu Ala Leu Thr Phe Val Ala Leu Pro His Tyr
                165                 170                 175

gtg cgc tta acc cgc gcc gcc gtg ctg gtg gaa gtt aac cgc gat tac 576
Val Arg Leu Thr Arg Ala Ala Val Leu Val Glu Val Asn Arg Asp Tyr
            180                 185                 190

gtc acc gcg tct cgc gtg gcg ggt gcc ggg gcg atg cgt cag atg ttt 624
Val Thr Ala Ser Arg Val Ala Gly Ala Gly Ala Met Arg Gln Met Phe
        195                 200                 205

att aac atc ttc ccg aac tgc ctt gcg ccg ctg att gtt cag gcg tcg 672
Ile Asn Ile Phe Pro Asn Cys Leu Ala Pro Leu Ile Val Gln Ala Ser
    210                 215                 220

ctc ggt ttc tct aac gcc att ctc gat atg gct gct ctt ggt ttc ctc 720
Leu Gly Phe Ser Asn Ala Ile Leu Asp Met Ala Ala Leu Gly Phe Leu
225                 230                 235                 240

ggc atg ggg gca cag ccg cca acg cct gag tgg ggc acc atg ctc tcc 768
Gly Met Gly Ala Gln Pro Pro Thr Pro Glu Trp Gly Thr Met Leu Ser
                245                 250                 255

gac gtg ttg cag ttc gcg caa agc gcc tgg tgg gtc gtg acc ttc ccg 816
Asp Val Leu Gln Phe Ala Gln Ser Ala Trp Trp Val Val Thr Phe Pro
            260                 265                 270

ggt ctg gcg atc ctg ctg acg gtg ctg gca ttt aac ctg atg ggt gac 864
Gly Leu Ala Ile Leu Leu Thr Val Leu Ala Phe Asn Leu Met Gly Asp
        275                 280                 285

ggt ctg cgt gac gcg ctc gat ccc aaa ctg aag cag 900
Gly Leu Arg Asp Ala Leu Asp Pro Lys Leu Lys Gln
    290                 295                 300

<210> 60
<211> 981
<212> DNA
<213> Escherichia coli

<400> 60
atg gcg tta tta aat gta gat aaa tta tcg gtg cat ttc ggc gac gaa 48
Met Ala Leu Leu Asn Val Asp Lys Leu Ser Val His Phe Gly Asp Glu
1               5                   10                  15

agc gcg ccg ttc cgc gcc gta gac cgc atc agc tac agc gta aaa cag 96
Ser Ala Pro Phe Arg Ala Val Asp Arg Ile Ser Tyr Ser Val Lys Gln
            20                  25                  30

ggc gaa gtg gtc ggg att gtg ggt gag tcc ggc tcc ggt aag tcg gtc 144
Gly Glu Val Val Gly Ile Val Gly Glu Ser Gly Ser Gly Lys Ser Val
        35                  40                  45

agt tca ctg gcg att atg ggg ctg att gat tat ccg ggc cgc gta atg 192
Ser Ser Leu Ala Ile Met Gly Leu Ile Asp Tyr Pro Gly Arg Val Met
    50                  55                  60

gca gaa aaa ctg gag ttt aac ggc cag gat ttg cag cgt atc tca gaa 240
Ala Glu Lys Leu Glu Phe Asn Gly Gln Asp Leu Gln Arg Ile Ser Glu
65                  70                  75                  80

aaa gag cgc cgc aac ctg gtg ggt gcc gaa gtg gcg atg atc ttc cag 288
Lys Glu Arg Arg Asn Leu Val Gly Ala Glu Val Ala Met Ile Phe Gln
                85                  90                  95

gac ccg atg acc agc ctt aac ccg tgc tac acc gtg ggt ttc cag att 336
Asp Pro Met Thr Ser Leu Asn Pro Cys Tyr Thr Val Gly Phe Gln Ile
            100                 105                 110

atg gaa gcg att aag gtg cat cag ggc ggc aac aaa agt acc cgc cgt 384
Met Glu Ala Ile Lys Val His Gln Gly Gly Asn Lys Ser Thr Arg Arg
        115                 120                 125

cag cga gcg att gac ctg ctg aat cag gtc ggt att ccc gat ccg gca 432
Gln Arg Ala Ile Asp Leu Leu Asn Gln Val Gly Ile Pro Asp Pro Ala
    130                 135                 140

tcg cgt ctg gat gtt tac ccg cat cag ctt tcc ggc ggc atg agc cag 480
Ser Arg Leu Asp Val Tyr Pro His Gln Leu Ser Gly Gly Met Ser Gln
145                 150                 155                 160

cgc gtg atg atc gcc atg gcg att gcc tgt cgg cca aaa ctg ctg att 528
Arg Val Met Ile Ala Met Ala Ile Ala Cys Arg Pro Lys Leu Leu Ile
                165                 170                 175

gcc gat gaa ccg acc acc gcg ctg gac gtg acc att cag gcg caa atc 576
Ala Asp Glu Pro Thr Thr Ala Leu Asp Val Thr Ile Gln Ala Gln Ile
            180                 185                 190

atc gaa cta ctg ctg gag cta cag cag aaa gag aac atg gcg ctg gtg 624
Ile Glu Leu Leu Leu Glu Leu Gln Gln Lys Glu Asn Met Ala Leu Val
        195                 200                 205

tta att acc cat gac ctg gcg ctg gtg gcg gaa gcg gca cat aaa atc 672
Leu Ile Thr His Asp Leu Ala Leu Val Ala Glu Ala Ala His Lys Ile
    210                 215                 220

atc gtg atg tat gca ggc cag gtg gtg gaa acc ggt gat gcg cac gcc 720
Ile Val Met Tyr Ala Gly Gln Val Val Glu Thr Gly Asp Ala His Ala
225                 230                 235                 240

atc ttc cat gcg ccg cgt cac ccg tat act cag gca ttg ctg cgt gcg 768
Ile Phe His Ala Pro Arg His Pro Tyr Thr Gln Ala Leu Leu Arg Ala
                245                 250                 255

ctg cca gaa ttt gct cag gac aaa gaa cgt ctg gcg tcg ttg cca ggt 816
Leu Pro Glu Phe Ala Gln Asp Lys Glu Arg Leu Ala Ser Leu Pro Gly
            260                 265                 270

gtc gtt ccc ggc aag tac gac cgc ccg aac ggc tgc ctg ctt aac ccg 864
Val Val Pro Gly Lys Tyr Asp Arg Pro Asn Gly Cys Leu Leu Asn Pro
        275                 280                 285

cgc tgc ccc tat gcc act gac aga tgt cgc gct gaa gaa ccg gcg ctg 912
Arg Cys Pro Tyr Ala Thr Asp Arg Cys Arg Ala Glu Glu Pro Ala Leu
    290                 295                 300

aat atg ctc gct gac ggg cgt cag tcc aaa tgc cat tac cca ctt gat 960
Asn Met Leu Ala Asp Gly Arg Gln Ser Lys Cys His Tyr Pro Leu Asp
305                 310                 315                 320

gat gcc ggg agg ccg aca cta 981
Asp Ala Gly Arg Pro Thr Leu
                325

<210> 61
<211> 1002
<212> DNA
<213> Escherichia coli

<400> 61
atg agt acg caa gag gcc acc ctg caa caa ccg ctg ttg cag gct atc 48
Met Ser Thr Gln Glu Ala Thr Leu Gln Gln Pro Leu Leu Gln Ala Ile
1               5                   10                  15

gac ctg aaa aaa cat tat ccg gtg aag aaa ggc atg ttc gcg ccg gaa 96
Asp Leu Lys Lys His Tyr Pro Val Lys Lys Gly Met Phe Ala Pro Glu
            20                  25                  30

cgt ctg gtt aaa gcg ctg gat ggc gtt tcg ttt aac ctt gaa cgt ggc 144

Arg Leu Val Lys Ala Leu Asp Gly Val Ser Phe Asn Leu Glu Arg Gly
        35                  40                  45

aaa acg ctg gca gta gtg ggc gaa tct ggc tgc ggt aaa tcg acc ctc 192
Lys Thr Leu Ala Val Val Gly Glu Ser Gly Cys Gly Lys Ser Thr Leu
    50                  55                  60

ggt cgg ttg ctg acg atg att gaa atg ccc acc ggt ggc gag ctg tat 240
Gly Arg Leu Leu Thr Met Ile Glu Met Pro Thr Gly Gly Glu Leu Tyr
65                  70                  75                  80

tac cag ggg cag gat ctg ctt aag cac gat ccg cag gcg cag aag ctg 288
Tyr Gln Gly Gln Asp Leu Leu Lys His Asp Pro Gln Ala Gln Lys Leu
                85                  90                  95

cgt cgg cag aaa atc cag atc gtc ttc cag aac cct tac ggt tcg ctg 336
Arg Arg Gln Lys Ile Gln Ile Val Phe Gln Asn Pro Tyr Gly Ser Leu
            100                 105                 110

aat ccg cgt aaa aaa gtc ggg caa att ctt gaa gag ccg ctg ctg atc 384
Asn Pro Arg Lys Lys Val Gly Gln Ile Leu Glu Glu Pro Leu Leu Ile
        115                 120                 125

aac acc agc tta agc aaa gaa cag cgt cgg gaa aaa gcc ctg tcg atg 432
Asn Thr Ser Leu Ser Lys Glu Gln Arg Arg Glu Lys Ala Leu Ser Met
    130                 135                 140

atg gcg aaa gtc ggc ctg aaa acc gag cac tat gac cgc tat ccg cat 480
Met Ala Lys Val Gly Leu Lys Thr Glu His Tyr Asp Arg Tyr Pro His
145                 150                 155                 160

atg ttc tcc ggc ggt cag cgt cag cgt atc gcc atc gcc cgt ggt ctg 528
Met Phe Ser Gly Gly Gln Arg Gln Arg Ile Ala Ile Ala Arg Gly Leu
                165                 170                 175

atg ctc gac ccg gat gtg gtg att gcc gat gaa ccg gtt tcc gcg ctg 576
Met Leu Asp Pro Asp Val Val Ile Ala Asp Glu Pro Val Ser Ala Leu
            180                 185                 190

gat gtt tca gtg cgc gcg cag gtg ctg aat ctg atg atg gat ttg cag 624
Asp Val Ser Val Arg Ala Gln Val Leu Asn Leu Met Met Asp Leu Gln
        195                 200                 205

cag gag ttg ggg ctg tct tat gtc ttt atc tcc cac gac ctg tcg gtg 672
Gln Glu Leu Gly Leu Ser Tyr Val Phe Ile Ser His Asp Leu Ser Val
    210                 215                 220

gtg gag cac att gct gat gaa gtg atg gtg atg tac ctg ggc cgc tgc 720
Val Glu His Ile Ala Asp Glu Val Met Val Met Tyr Leu Gly Arg Cys
225                 230                 235                 240

gtg gag aag gga acg aaa gac caa atc ttc aat aac ccg cgc cat ccg 768
Val Glu Lys Gly Thr Lys Asp Gln Ile Phe Asn Asn Pro Arg His Pro
                245                 250                 255

tac act cag gcg cta ctt tcc gcg acg ccg cgc ctg aac ccg gac gat 816
Tyr Thr Gln Ala Leu Leu Ser Ala Thr Pro Arg Leu Asn Pro Asp Asp
            260                 265                 270

cgc cgc gag cgc atc aag ctc agc ggt gaa cta cca agc cca ctg aat 864

Arg Arg Glu Arg Ile Lys Leu Ser Gly Glu Leu Pro Ser Pro Leu Asn
        275                 280                 285

cca ccg ccg ggt tgc gcc ttc aac gcc cgc tgt cgt cgg cgc ttc ggc     912
Pro Pro Pro Gly Cys Ala Phe Asn Ala Arg Cys Arg Arg Arg Phe Gly
    290                 295                 300

ccc tgc acc cag ttg cag ccg cag cta aaa gac tac ggc ggt caa ctg     960
Pro Cys Thr Gln Leu Gln Pro Gln Leu Lys Asp Tyr Gly Gly Gln Leu
305                 310                 315                 320

gta gct tgt ttt gct gtt gat cag gat gaa aat ccg cag cgt             1002
Val Ala Cys Phe Ala Val Asp Gln Asp Glu Asn Pro Gln Arg
                325                 330

<210> 62
<211> 56
<212> DNA
<213> Artificial Sequence

<220>
<223> Description of Artificial Sequence: Synthetic DNA

<400> 62
ctaaccctgt gacctgcaat actgttttgc gggtgagtgt aggctggagc tgcttc     56

<210> 63
<211> 56
<212> DNA
<213> Artificial Sequence

<220>
<223> Description of Artificial Sequence: Synthetic DNA

<400> 63
gaaactgccg gaaggcgatt aaacgccatc cggcagcata tgaatatcct ccttag     56

<210> 64
<211> 56
<212> DNA
<213> Artificial Sequence

<220>
<223> Description of Artificial Sequence: Synthetic DNA

<400> 64
ttacgcaaca ggaatagact gaacaccaga ctctatgtgt aggctggagc tgcttc     56

<210> 65
<211> 56
<212> DNA
<213> Artificial Sequence

<220>
<223> Description of Artificial Sequence: Synthetic DNA

<400> 65
agaaaacagg ggtaaattcc ccgaatggcg gcgctacata tgaatatcct ccttag     56


<210> 66
<211> 56
<212> DNA
<213> Artificial Sequence

<220>
<223> Description of Artificial Sequence: Synthetic DNA


<400> 66
atggagttta gtgtaaaaag cggtagcccg gagaaagtgt aggctggagc tgcttc     56


<210> 67
<211> 56
<212> DNA
<213> Artificial Sequence

<220>
<223> Description of Artificial Sequence: Synthetic DNA

<400> 67
ttactcttcg ccgttaaacc cagcgcggtt taacagcata tgaatatcct ccttag     56


<210> 68
<211> 56
<212> DNA
<213> Artificial Sequence

<220>
<223> Description of Artificial Sequence: Synthetic DNA

<400> 68
atgacagaag cgatgaagat taccctctct acccaagtgt aggctggagc tgcttc     56


<210> 69
<211> 56
<212> DNA
<213> Artificial Sequence

<220>
<223> Description of Artificial Sequence: Synthetic DNA

<400> 69
ttacgccgtt aacagattag ctatcgtgcg cacacccata tgaatatcct ccttag     56


<210> 70
<211> 56
<212> DNA
<213> Artificial Sequence

<220>
<223> Description of Artificial Sequence: Synthetic DNA

<400> 70
gcatccccac ctcataacgt tgacccgacc gggcaagtgt aggctggagc tgcttc     56

<210> 71
<211> 56
<212> DNA
<213> Artificial Sequence

<220>
<223> Description of Artificial Sequence: Synthetic DNA

<400> 71
ctgtacggca ttttgctatg cttgtcgcca ctgttgcata tgaatatcct ccttag     56

<210> 72
<211> 21
<212> DNA
<213> Artificial Sequence

<220>
<223> Description of Artificial Sequence: Synthetic DNA

<400> 72
gtgtctgaac tgtctcaatt a                                           21

<210> 73
<211> 21
<212> DNA
<213> Artificial Sequence

<220>
<223> Description of Artificial Sequence: Synthetic DNA

<400> 73
cggaatttct ttcagcagtt c                                           21

<210> 74
<211> 21
<212> DNA
<213> Artificial Sequence

<220>
<223> Description of Artificial Sequence: Synthetic DNA

<400> 74
atgactcaac agccacaagc c                                           21

<210> 75
<211> 21
<212> DNA
<213> Artificial Sequence

<220>
<223> Description of Artificial Sequence: Synthetic DNA

<400> 75
tgctttagtt atcttctcgt a                                                21

<210> 76
<211> 21
<212> DNA
<213> Artificial Sequence

<220>
<223> Description of Artificial Sequence: Synthetic DNA

<400> 76
agtgcctgca tcgtcgtggg c                                                21

<210> 77
<211> 21
<212> DNA
<213> Artificial Sequence

<220>
<223> Description of Artificial Sequence: Synthetic DNA

<400> 77
ggcgcctttt gctttaccag a                                                21

<210> 78
<211> 21
<212> DNA
<213> Artificial Sequence

<220>
<223> Description of Artificial Sequence: Synthetic DNA

<400> 78
gacgcgcgct ggggagaaaa a                                                21

<210> 79
<211> 21
<212> DNA
<213> Artificial Sequence

<220>
<223> Description of Artificial Sequence: Synthetic DNA

<400> 79
cgtagcgccc gcagaccact g                                                21

<210> 80
<211> 21
<212> DNA
<213> Artificial Sequence

<220>
<223> Description of Artificial Sequence: Synthetic DNA

<400> 80
atgcgtattt ccttgaaaaa g                                                21

<210> 81
<211> 21
<212> DNA
<213> Artificial Sequence

<220>
<223> Description of Artificial Sequence: Synthetic DNA

<400> 81
ttattcgata gagacgtttt c                                                21

<210> 82
<211> 29
<212> DNA
<213> Artificial Sequence

<220>
<223> Description of Artificial Sequence: Synthetic DNA

<400> 82
tacactcgag attaaagagg agaaattaa                                        29

<210> 83
<211> 30
<212> DNA
<213> Artificial Sequence

<220>
<223> Description of Artificial Sequence: Synthetic DNA

<400> 83
ttaggatcct catactggca gcacatactt                                       30

<210> 84
<211> 24
<212> DNA
<213> Artificial Sequence

<220>
<223> Description of Artificial Sequence: Synthetic DNA

<400> 84
caagaattct catgtttgac agct                                             24

<210> 85
<211> 28
<212> DNA
<213> Artificial Sequence

<220>
<223> Description of Artificial Sequence: Synthetic DNA

<400> 85
   taactcgaga ttcccttttt acgtgaac 28